# EUROPEAN PATENT APPLICATION

(11) **EP 1 273 660 A2**
(43) Date of publication of application: **08.01.2003**
(21) Application number: 02001160.7
(22) Date of filing: 25.01.2002
(51) Int. Cl.: C12N 15/12, C07K 14/705, C07K 16/28, G01N 33/53, G01N 33/68, C12Q 1/68, A61K 38/17, A61K 39/00, A61K 48/00

(54) **Human sodium-hydrogen exchanger like protein 1**

(30) Priority: 30.01.2001 WO PCT/US01/00666; 23.05.2001 US 864761; 21.12.2001 US 343331 P
(71) Applicant: Aeomica, Inc., Sunnyvale, CA 94086-4520 (US)
(72) Inventor: Gu, Yizhong, Cupertino, California 95014 (US)
(74) Representative: Rollins, Anthony John

(57) **Abstract**

The invention provides isolated nucleic acids that encode human sodium-hydrogen exchanger like protein 1 (NHELP1), and fragments thereof, vectors for propagating and expressing NHELP1 nucleic acids, host cells comprising the nucleic acids and vectors of the present invention, proteins, protein fragments, and protein fusions of the novel NHELP1 isoforms, and antibodies thereto. The invention further provides transgenic cells and non-human organisms comprising human NHELP1 nucleic acids, and transgenic cells and non-human organisms with targeted disruption of the endogenous orthologue of the human NHELP1 gene. The invention further provides pharmaceutical formulations of the nucleic acids, proteins, and antibodies of the present invention, and diagnostic, investigational, and therapeutic methods based on the NHELP1 nucleic acids, proteins, and antibodies of the present invention.

## Description

### FIELD OF THE INVENTION

The present invention relates to novel human sodium-hydrogen exchanger like protein 1 (NHELP1). More specifically, the invention provides isolated nucleic acid molecules encoding NHELP1, fragments thereof, vectors and host cells comprising isolated nucleic acid molecules encoding NHELP1, NHELP1 polypeptides, antibodies, transgenic cells and non-human organisms, and diagnostic, therapeutic, and investigational methods of using the same.

### BACKGROUND OF THE INVENTION

The luminal ionic composition of many, if not all, intracellular compartments differs from the surrounding cytoplasm and is an important determinant of their function. The establishment of this differential composition is achieved through the concerted actions of distinct integral membrane ion carriers, including pumps, channels, and transporters. For example, alkalinization of the mitochondrial matrix, driven by the respiratory chain, contributes to the inner membrane H⁺ gradient used to drive ATP synthesis (Saraste, *Science* 283:1488-1493 (1999)) and, indirectly, to extrude matrix Ca²⁺ through the functional coupling of Na⁺ /H⁺ and Na⁺ /Ca²⁺ antiport pathways (Garlid *et al., Methods Enzymol.* 260:331-348 (1995); Brierley *et al., J. Bioenerg. Biomembr.* 26:519-526 (1994); Babcock *et al., J. Cell Biol.* 136:833-844 (1997)).

Organelles of the secretory and endocytic pathways are distinguished by their luminal acidity, which is generated by the activity of a vacuolar-type H⁺-ATPase (V-ATPase). Mellman *et al., Annu. Rev. Biochem.* 55:663-700 (1986); Forgac, *J. Biol. Chem.* 274:12951-12954 (1999). At present, little is known about the mechanisms controlling the steady-state [H⁺] within the lumen of different endomembrane compartments. Although distinct isoforms of some of the V-ATPase subunits have been reported in different tissues (Puopolo et *al., J. Biol. Chem.* 267:3696-3706 (1992); Nishi and Forgac, *J. Biol. Chem.* 275:6824-6830 (2000)) or specialized cell types (Gluck, *J. Exp. Biol.* 172:29-37 (1992)), there is no clear evidence that the V-ATPase functions differently in particular organelles within a single mammalian cell. Rather, the luminal [H⁺] is thought to be regulated by a complex interplay between the V-ATPase and unidentified leak pathways for protons, based on the rapid dissipation of the transmembrane proton chemical gradient (DpH) observed after inhibiting the V-ATPase with macrolide antibiotics. Demaurex et al., *J. Biol. Chem.* 273:2044-2051 (1998); Farinas and verkman, *J*. *Biol. Chem.* 274:7603-7606 (1999); Kim et al., J. *Cell Biol.* 134:1387-1399 (1996).

A few components of this leak pathway have been identified recently. For example, one such component in the Golgi complex was recently identified as a Zn²⁺ -inhibitable H⁺ conductance. Schapiro and Grinstein, *J. Biol. Chem.* 275:21025-21032 (2000). Several Na+/H+ exchangers (NHEs) have also been identified which are involved in the controlling of luminal cation composition of various cellular organelles. Orlowski and Grinstein, *J. Biol. Chem.* 272:22373-22376 (1997); Yun *et al., Am. J. Physiol.* 269:G1-G11 (1995); Numata and Orlowski, *J. Biol. Chem.* 276:17387-17394 (2001). These findings highlights the H⁺ leak as a key determinant of organellar pH and emphasizes the need to identify additional molecular components of this pathway.

To date, seven NHE isoforms have been identified. These isoforms exhibit considerable differences in their primary structures, tissue distribution, membrane localization, biochemical and pharmacological properties, and responsiveness to various stimuli. Numata *et al., J. Biol. Chem.* 273:6951-6959 (1998); Numata and Orlowski, *J. Biol. Chem.* 276:17387-17394 (2001). Mammalian NHEs participate in a wide array of other essential cellular processes, including control of intracellular pH, maintenance of cellular volume, and reabsorption of Na⁺ across renal, intestinal, and other epithelia. Activation of NHE activity also facilitates growth factor-induced proliferation of certain cell types (Grinstein *et al., Biochim. Biophys. Acta* 988:73-97 (1989)) and is associated with events leading to apoptosis. Rajotte *et al., J. Biol. Chem.* 267:9980-9987 (1992); Li and Eastman, *J. Biol. Chem.* 270:3203-3211 (1995); Zhu and Loh, *Biochim. Biophys. Acta* 1269:122-128 (1995).

Imbalance of the luminal ionic composition could lead to severe consequences. Studies have shown that V-ATPases are involved in the development of drug resistance to breast cancer treatments. Martinez-Zaguilan *et al., Biochem. Pharm.* 57:1037-1046 (1999). The overexpression of some V-ATPases may also play a crucial role in tumor progression as it is characteristic of invasive pancreatic tumors. Ohta *et al, Br. J. Cancer* 73:1511-1517 (1996). Another series of recent studies have implicated the endocytotic role of V-ATPase domains in the development of acquired immunodeficiency syndrome (AIDS). Lu *et al*., *Immunity* 8:647-656 (1998); Mandic *et al., Mol. Biol. Cell* 12:463-473 (2001).

Given the roles of NHE in maintaining cation ion homeostasis and normal cellular function, there is a need to identify and to characterize additional human proteins with structural and functional similarities to NHE.

### SUMMARY OF THE INVENTION

The present invention solves these and other needs in the art by providing isolated nucleic acids that encode NHELP1, and fragments thereof.

In other aspects, the invention provides vectors for propagating and expressing the nucleic acids of the present invention, host cells comprising the nucleic acids and vectors of the present invention, proteins, protein fragments, and protein fusions of the NHELP1, and antibodies thereto.

The invention further provides pharmaceutical formulations of the nucleic acids, proteins, and antibodies of the present invention.

In other aspects, the invention provides transgenic cells and non-human organisms comprising NHELP1 nucleic acids, and transgenic cells and non-human organisms with targeted disruption of the endogenous orthologue of the NHELP1.

The invention additionally provides diagnostic, investigational, and therapeutic methods based on the NHELP1 nucleic acids, proteins, and antibodies of the present invention.

The invention further provides medical and diagnostic uses of the NHELP1 nucleic acid, proteins and antibodies.

### BRIEF DESCRIPTION OF THE DRAWINGS

The above and other objects and advantages of the present invention will be apparent upon consideration of the following detailed description taken in conjunction with the accompanying drawings, in which like characters refer to like parts throughout, and in which:
FIG. 1 (a) schematizes the protein domain structure of NHELP1 and FIG. 1 (b) shows the alignment of the Na_H_Exchanger dommain with that of other proteins;
FIG. 2 is a map showing the genomic structure of NHELP1 encoded at chromosome 3q23; and
FIG. 3 presents the nucleotide and predicted amino acid sequences of NHELP1.

### DETAILED DESCRIPTION OF THE INVENTION

Mining the sequence of the human genome for novel human genes, the present inventors have identified a human sodium-hydrogen exchanger like protein (NHELP1), which functions in maintaining cation ion homeostasis of a cellular organelle.

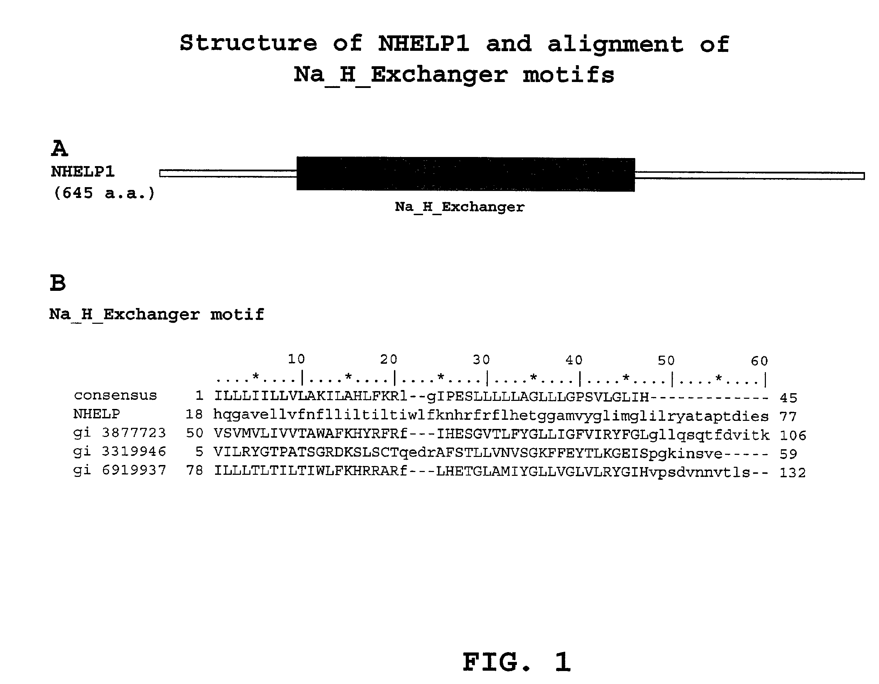

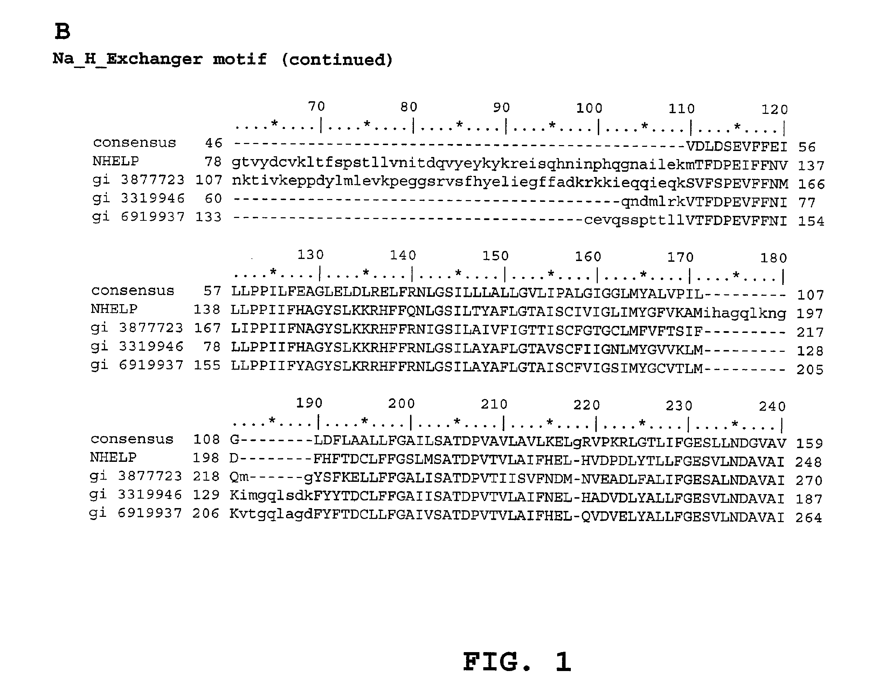

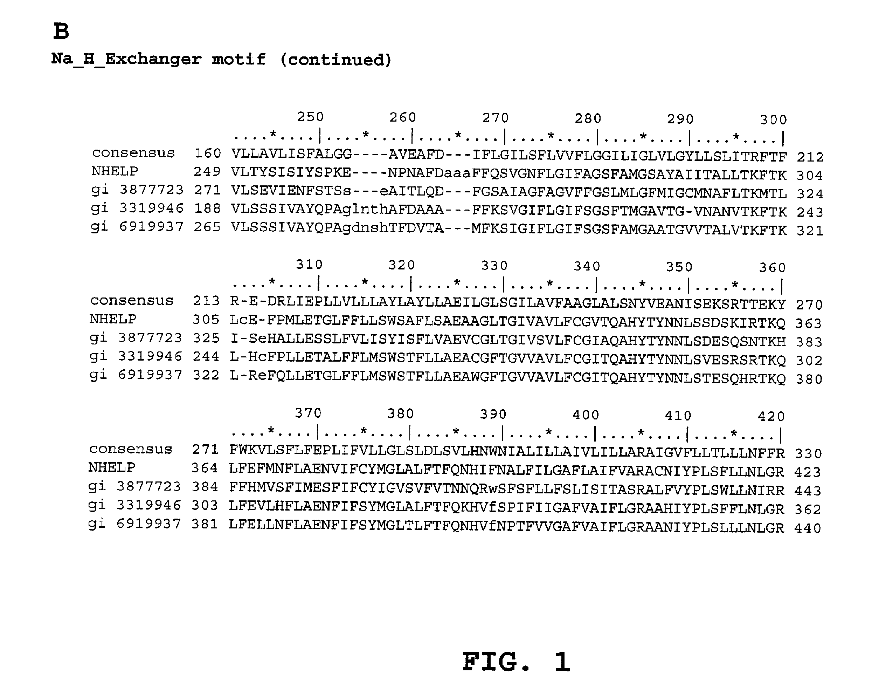

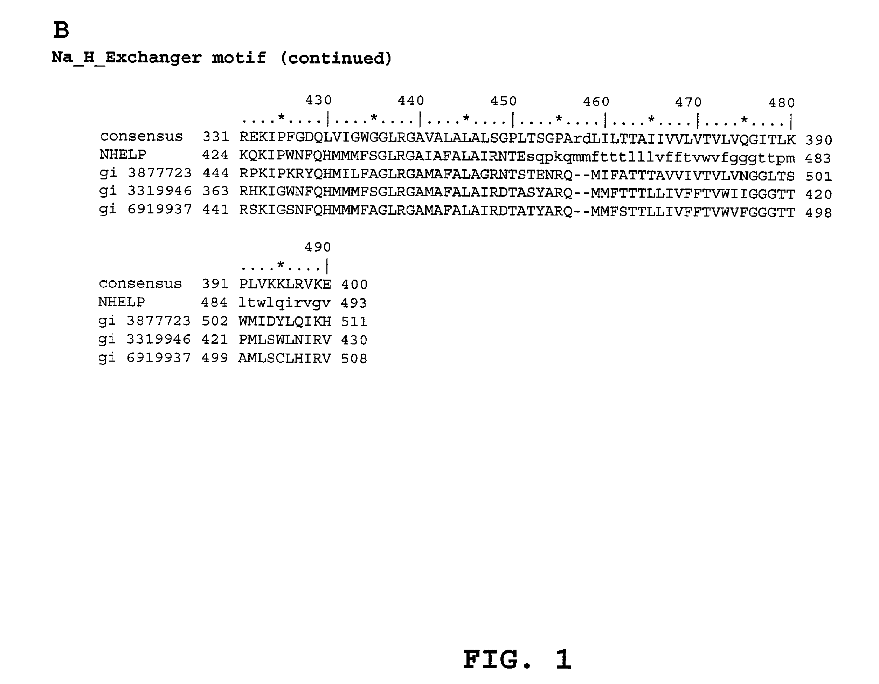

As schematized in FIG. 1, the newly isolated gene product shares certain protein domains and an overall structural organization with other human sodium/hydrogen exchangers. The shared structural features strongly imply that NHELP1 plays a role similar to that of other human sodium/hydrogen exchangers in maintaining cation ion homeostasis.

Like the other human sodium/hydrogen exchanger isoforms, NHELP1 contains a Na_H_Exchanger domain. In MHELP1, the Na_H_Exchanger motif ocurrs at amino acids 128-454 (http://www.ncbi.nlm.nih.gov/Structure/cdd/wrpsb.cgi/). Other signatures of the newly isolated NHELP1 proteins were identified by searching the PROSITE database (http://www.expasy.ch/tools/scnpsitl.html). These include three *N*-glycosylation sites (96 - 99, 352 - 355, 516 - 519), four protein kinase C phosphorylation sites (9 - 11, 149 - 151, 258 - 260 and 522 - 524), one tyrosine kinase phosphorylation site (6 - 14), a leucine zipper (311 - 332), a amidation site, seven Casein kinase II phosphorylation sites, and eleven *N*-myristoylation sites.

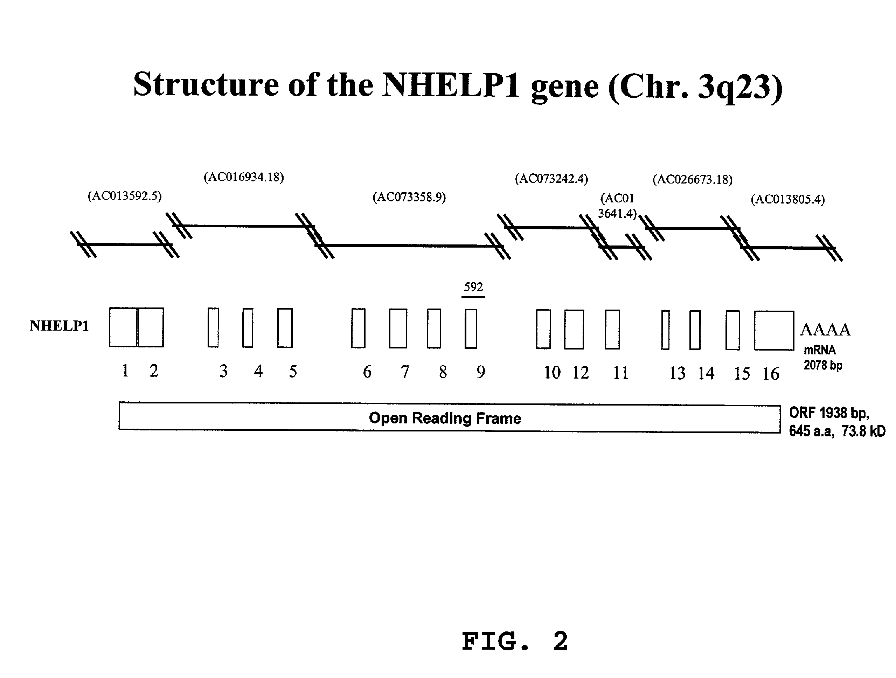

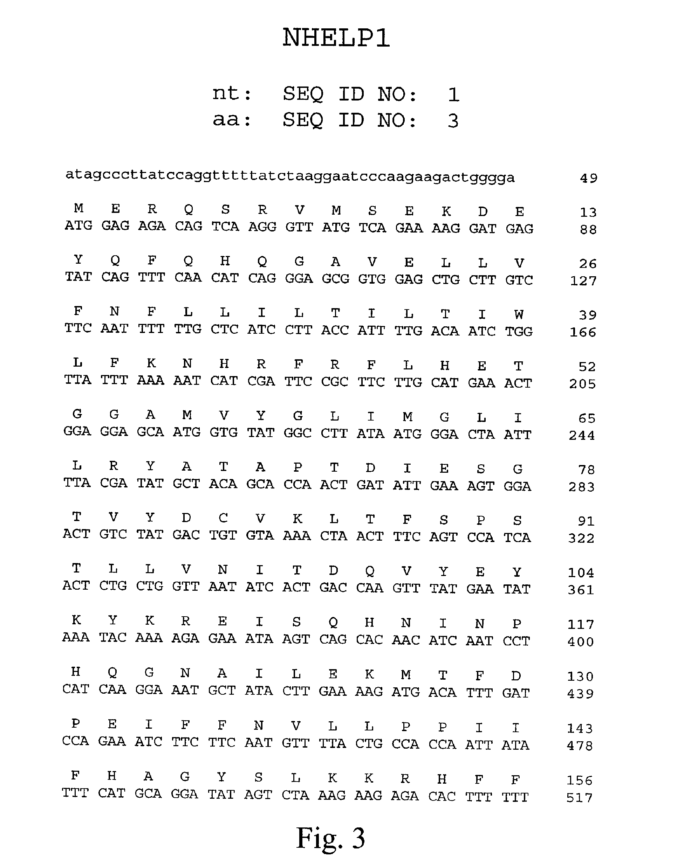

FIG. 2 shows the genomic organization of NHELP1.

At the top is shown the seven bacterial artificial chromosomes (BACs), with GenBank accession numbers, that span the NHELP1 locus. The genome-derived single-exon probe first used to demonstrate expression from this locus is shown below the BACs and labeled "592". The 592 bp probe includes sequence drawn from exon 9 and surrounding introns.

As shown in FIG. 2, NHELP1, encoding a protein of 645 amino acids, comprises exons 1 - 16. The predicted molecular weights, prior to any post_translational modification, is 72.6 kD. The cDNA clone appears full length, with the open reading frame starting with a methionine and terminating with a stop codon.

As further discussed in the examples herein, expression of NHELP1 was assessed using hybridization to genome-derived single exon microarrays. Microarray analysis of exon nine showed expression in all tissues tested, including brain, adult liver, fetal liver, adrenal, bone marrow, prostate, testis as well as a cell line, hela.

As more fully described below, the present invention provides isolated nucleic acids that encode NHELP1 and fragments thereof. The invention further provides vectors for propagation and expression of the nucleic acids of the present invention, host cells comprising the nucleic acids and vectors of the present invention, proteins, protein fragments, and protein fusions of the present invention, and antibodies specific for all or any one of the isoforms. The invention provides pharmaceutical formulations of the nucleic acids, proteins, and antibodies of the present invention. The invention further provides transgenic cells and non-human organisms comprising human NHELP1 nucleic acids, and transgenic cells and non-human organisms with targeted disruption of the endogenous orthologue of the human NHELP1. The invention additionally provides diagnostic, investigational, and therapeutic methods based on the NHELP1 nucleic acids, proteins, and antibodies of the present invention.

### DEFINITIONS

Unless defined otherwise, all technical and scientific terms used herein have the meaning commonly understood by one of ordinary skill in the art to which this invention belongs.

As used herein, "nucleic acid" (synonymously, "polynucleotide") includes polynucleotides having natural nucleotides in native 5'-3' phosphodiester linkage - *e.g.,* DNA or RNA - as well as polynucleotides that have nonnatural nucleotide analogues, nonnative internucleoside bonds, or both, so long as the nonnatural polynucleotide is capable of sequence-discriminating basepairing under experimentally desired conditions. Unless otherwise specified, the term "nucleic acid" includes any topological conformation; the term thus explicitly comprehends single-stranded, double-stranded, partially duplexed, triplexed, hairpinned, circular, and padlocked conformations.

As used herein, an "isolated nucleic acid" is a nucleic acid molecule that exists in a physical form that is nonidentical to any nucleic acid molecule of identical sequence as found in nature; "isolated" does not require, although it does not prohibit, that the nucleic acid so described has itself been physically removed from its native environment.

For example, a nucleic acid can be said to be "isolated" when it includes nucleotides and/or internucleoside bonds not found in nature. When instead composed of natural nucleosides in phosphodiester linkage, a nucleic acid can be said to be "isolated" when it exists at a purity not found in nature, where purity can be adjudged with respect to the presence of nucleic acids of other sequence, with respect to the presence of proteins, with respect to the presence of lipids, or with respect the presence of any other component of a biological cell, or when the nucleic acid lacks sequence that flanks an otherwise identical sequence in an organism's genome, or when the nucleic acid possesses sequence not identically present in nature.

As so defined, "isolated nucleic acid" includes nucleic acids integrated into a host cell chromosome at a heterologous site, recombinant fusions of a native fragment to a heterologous sequence, recombinant vectors present as episomes or as integrated into a host cell chromosome.

As used herein, an isolated nucleic acid "encodes" a reference polypeptide when at least a portion of the nucleic acid, or its complement, can be directly translated to provide the amino acid sequence of the reference polypeptide, or when the isolated nucleic acid can be used, alone or as part of an expression vector, to express the reference polypeptide *in vitro,* in a prokaryotic host cell, or in a eukaryotic host cell.

As used herein, the term "exon" refers to a nucleic acid sequence found in genomic DNA that is bioinformatically predicted and/or experimentally confirmed to contribute contiguous sequence to a mature mRNA transcript.

As used herein, the phrase "open reading frame" and the equivalent acronym "ORF" refer to that portion of a transcript-derived nucleic acid that can be translated in its entirety into a sequence of contiguous amino acids. As so defined, an ORF has length, measured in nucleotides, exactly divisible by 3. As so defined, an ORF need not encode the entirety of a natural protein.

As used herein, the phrase "ORF-encoded peptide" refers to the predicted or actual translation of an ORF.

As used herein, the phrase "degenerate variant" of a reference nucleic acid sequence intends all nucleic acid sequences that can be directly translated, using the standard genetic code, to provide an amino acid sequence identical to that translated from the reference nucleic acid sequence.

As used herein, the term "microarray" and the equivalent phrase "nucleic acid microarray" refer to a substrate-bound collection of plural nucleic acids, hybridization to each of the plurality of bound nucleic acids being separately detectable. The substrate can be solid or porous, planar or non-planar, unitary or distributed.

As so defined, the term "microarray" and phrase "nucleic acid microarray" include all the devices so called in Schena (ed.), DNA Microarrays: A Practical Approach (Practical Approach Series), Oxford University Press (1999) (ISBN: 0199637768); *Nature Genet.* 21(1)(suppl):1 - 60 (1999); and Schena (ed.), Microarray Biochip: Tools and Technology, Eaton Publishing Company/BioTechniques Books Division (2000) (ISBN: 1881299376), the disclosures of which are incorporated herein by reference in their entireties.

As so defined, the term "microarray" and phrase "nucleic acid microarray" also include substrate-bound collections of plural nucleic acids in which the plurality of nucleic acids are distributably disposed on a plurality of beads, rather than on a unitary planar substrate, as is described, *inter alia,* in Brenner *et al., Proc. Natl. Acad. Sci. USA* 97(4):166501670 (2000), the disclosure of which is incorporated herein by reference in its entirety; in such case, the term "microarray" and phrase "nucleic acid microarray" refer to the plurality of beads in aggregate.

As used herein with respect to solution phase hybridization, the term "probe", or equivalently, "nucleic acid probe" or "hybridization probe", refers to an isolated nucleic acid of known sequence that is, or is intended to be, detectably labeled. As used herein with respect to a nucleic acid microarray, the term "probe" (or equivalently "nucleic acid probe" or "hybridization probe") refers to the isolated nucleic acid that is, or is intended to be, bound to the substrate. In either such context, the term "target" refers to nucleic acid intended to be bound to probe by sequence complementarity.

As used herein, the expression "probe comprising SEQ ID NO:X", and variants thereof, intends a nucleic acid probe, at least a portion of which probe has either (i) the sequence directly as given in the referenced SEQ ID NO:X, or (ii) a sequence complementary to the sequence as given in the referenced SEQ ID NO:X, the choice as between sequence directly as given and complement thereof dictated by the requirement that the probe be complementary to the desired target.

As used herein, the phrases "expression of a probe" and "expression of an isolated nucleic acid" and their linguistic equivalents intend that the probe or, (respectively, the isolated nucleic acid), or a probe (or, respectively, isolated nucleic acid) complementary in sequence thereto,can hybridize detectably under high stringency conditions to a sample of nucleic acids that derive from mRNA transcripts from a given source. For example, and by way of illustration only, expression of a probe in "liver" means that the probe can hybridize detectably under high stringency conditions to a sample of nucleic acids that derive from mRNA obtained from liver.

As used herein, "a single exon probe" comprises at least part of an exon ("reference exon") and can hybridize detectably under high stringency conditions to transcript-derived nucleic acids that include the reference exon. The single exon probe will not, however, hybridize detectably under high stringency conditions to nucleic acids that lack the reference exon and that consist of one or more exons that are found adjacent to the reference exon in the genome.

For purposes herein, "high stringency conditions" are defined for solution phase hybridization as aqueous hybridization *(i.e.,* free of formamide) in 6X SSC (where 20X SSC contains 3.0 M NaCl and 0.3 M sodium citrate), 1% SDS at 65°C for at least 8 hours, followed by one or more washes in 0.2X SSC, 0.1% SDS at 65°C. "Moderate stringency conditions" are defined for solution phase hybridization as aqueous hybridization (i.e., free of formamide) in 6X SSC, 1% SDS at 65°C for at least 8 hours, followed by one or more washes in 2x SSC, 0.1% SDS at room temperature.

For microarray-based hybridization, standard "high stringency conditions" are defined as hybridization in 50% formamide, 5X SSC, 0.2 µg/µl poly(dA), 0.2 µg/µl human cot1 DNA, and 0.5% SDS, in a humid oven at 42°C overnight, followed by successive washes of the microarray in 1X SSC, 0.2% SDS at 55°C for 5 minutes, and then 0.1X SSC, 0.2% SDS, at 55°C for 20 minutes. For microarray-based hybridization, "moderate stringency conditions", suitable for cross-hybridization to mRNA encoding structurally- and functionally-related proteins, are defined to be the same as those for high stringency conditions but with reduction in temperature for hybridization and washing to room temperature (approximately 25°C).

As used herein, the terms "protein", "polypeptide", and "peptide" are used interchangeably to refer to a naturally-occurring or synthetic polymer of amino acid monomers (residues), irrespective of length, where amino acid monomer here includes naturally-occurring amino acids, naturally-occurring amino acid structural variants, and synthetic non-naturally occurring analogs that are capable of participating in peptide bonds. The terms "protein", "polypeptide", and "peptide" explicitly permits of post-translational and post-synthetic modifications, such as glycosylation.

The term "oligopeptide" herein denotes a protein, polypeptide, or peptide having 25 or fewer monomeric subunits.

The phrases "isolated protein", "isolated polypeptide", "isolated peptide" and "isolated oligopeptide" refer to a protein (or respectively to a polypeptide, peptide, or oligopeptide) that is nonidentical to any protein molecule of identical amino acid sequence as found in nature; "isolated" does not require, although it does not prohibit, that the protein so described has itself been physically removed from its native environment.

For example, a protein can be said to be "isolated" when it includes amino acid analogues or derivatives not found in nature, or includes linkages other than standard peptide bonds.

When instead composed entirely of natural amino acids linked by peptide bonds, a protein can be said to be "isolated" when it exists at a purity not found in nature - where purity can be adjudged with respect to the presence of proteins of other sequence, with respect to the presence of non-protein compounds, such as nucleic acids, lipids, or other components of a biological cell, or when it exists in a composition not found in nature, such as in a host cell that does not naturally express that protein.

A "purified protein" (equally, a purified polypeptide, peptide, or oligopeptide) is an isolated protein, as above described, present at a concentration of at least 95%, as measured on a weight basis with respect to total protein in a composition. A "substantially purified protein" (equally, a substantially purified polypeptide, peptide, or oligopeptide) is an isolated protein, as above described, present at a concentration of at least 70%, as measured on a weight basis with respect to total protein in a composition.

As used herein, the phrase "protein isoforms" refers to a plurality of proteins having nonidentical primary amino acid sequence but that share amino acid sequence encoded by at least one common exon.

As used herein, the phrase "alternative splicing" and its linguistic equivalents includes all types of RNA processing that lead to expression of plural protein isoforms from a single gene; accordingly, the phrase "splice variant(s)" and its linguistic equivalents embraces mRNAs transcribed from a given gene that, however processed, collectively encode plural protein isoforms. For example, and by way of illustration only, splice variants can include exon insertions, exon extensions, exon truncations, exon deletions, alternatives in the 5' untranslated region ("5' UT") and alternatives in the 3' untranslated region ("3' UT"). Such 3' alternatives include, for example, differences in the site of RNA transcript cleavage and site of poly(A) addition. See, *e.g.,* Gautheret *et al*., *Genome Res.* 8:524-530 (1998).

As used herein, "orthologues" are separate occurrences of the same gene in multiple species. The separate occurrences have similar, albeit nonidentical, amino acid sequences, the degree of sequence similarity depending, in part, upon the evolutionary distance of the species from a common ancestor having the same gene.

As used herein, the term "paralogues" indicates separate occurrences of a gene in one species. The separate occurrences have similar, albeit nonidentical, amino acid sequences, the degree of sequence similarity depending, in part, upon the evolutionary distance from the gene duplication event giving rise to the separate occurrences.

As used herein, the term "homologues" is generic to "orthologues" and "paralogues".

As used herein, the term "antibody" refers to a polypeptide, at least a portion of which is encoded by at least one immunoglobulin gene, or fragment thereof, and that can bind specifically to a desired target molecule. The term includes naturally-occurring forms, as well as fragments and derivatives.

Fragments within the scope of the term "antibody" include those produced by digestion with various proteases, those produced by chemical cleavage and/or chemical dissociation, and those produced recombinantly, so long as the fragment remains capable of specific binding to a target molecule. Among such fragments are Fab, Fab', Fv, F(ab)'₂, and single chain Fv (scFv) fragments.

Derivatives within the scope of the term include antibodies (or fragments thereof) that have been modified in sequence, but remain capable of specific binding to a target molecule, including: interspecies chimeric and humanized antibodies; antibody fusions; heteromeric antibody complexes and antibody fusions, such as diabodies (bispecific antibodies), single-chain diabodies, and intrabodies (see, *e.g.*, Marasco (ed.), Intracellular Antibodies: Research and Disease Applications, Springer-Verlag New York, Inc. (1998) (ISBN: 3540641513), the disclosure of which is incorporated herein by reference in its entirety).

As used herein, antibodies can be produced by any known technique, including harvest from cell culture of native B lymphocytes, harvest from culture of hybridomas, recombinant expression systems, and phage display.

As used herein, "antigen" refers to a ligand that can be bound by an antibody; an antigen need not itself be immunogenic. The portions of the antigen that make contact with the antibody are denominated "epitopes".

"Specific binding" refers to the ability of two molecular species concurrently present in a heterogeneous (inhomogeneous) sample to bind to one another in preference to binding to other molecular species in the sample. Typically, a specific binding interaction will discriminate over adventitious binding interactions in the reaction by at least two-fold, more typically by at least 10-fold, often at least 100-fold; when used to detect analyte, specific binding is sufficiently discriminatory when determinative of the presence of the analyte in a heterogeneous (inhomogeneous) sample. Typically, the affinity or avidity of a specific binding reaction is least about 10⁻⁷ M, with specific binding reactions of greater specificity typically having affinity or avidity of at least 10⁻⁸ M to at least about 10⁻⁹ M.

As used herein, "molecular binding partners" - and equivalently, "specific binding partners" - refer to pairs of molecules, typically pairs of biomolecules, that exhibit specific binding. Nonlimiting examples are receptor and ligand, antibody and antigen, and biotin to any of avidin, streptavidin, neutrAvidin and captAvidin.

The term "antisense", as used herein, refers to a nucleic acid molecule sufficiently complementary in sequence, and sufficiently long in that complementary sequence, as to hybridize under intracellular conditions to (i) a target mRNA transcript or (ii) the genomic DNA strand complementary to that transcribed to produce the target mRNA transcript.

The term "portion", as used with respect to nucleic acids, proteins, and antibodies, is synonymous with "fragment".

### NUCLEIC ACID MOLECULES

In a first aspect, the invention provides isolated nucleic acids that encode NHELP1, variants having at least 65% sequence identity thereto, degenerate variants thereof, variants that encode NHELP1 proteins having conservative or moderately conservative substitutions, cross-hybridizing nucleic acids, and fragments thereof.

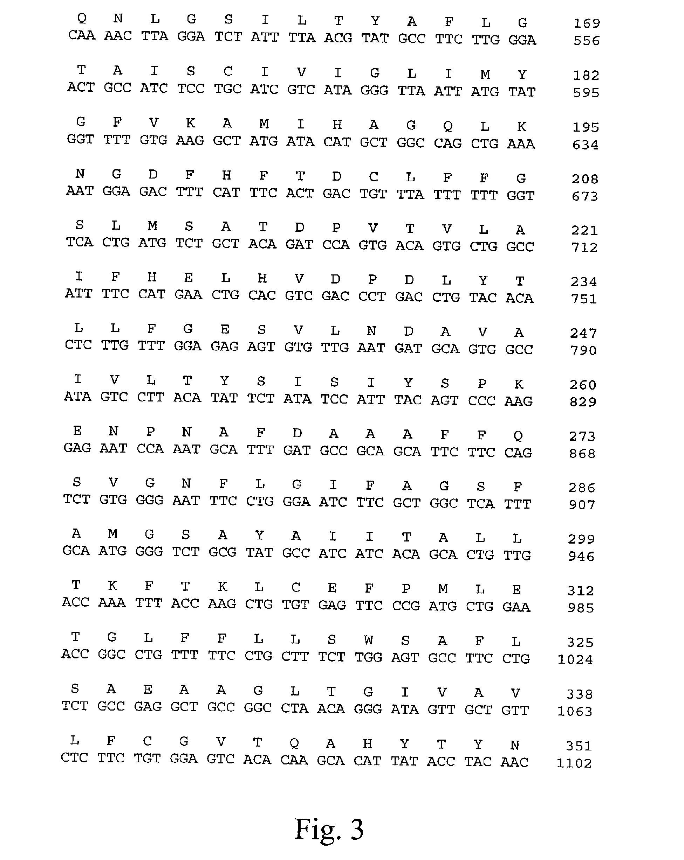

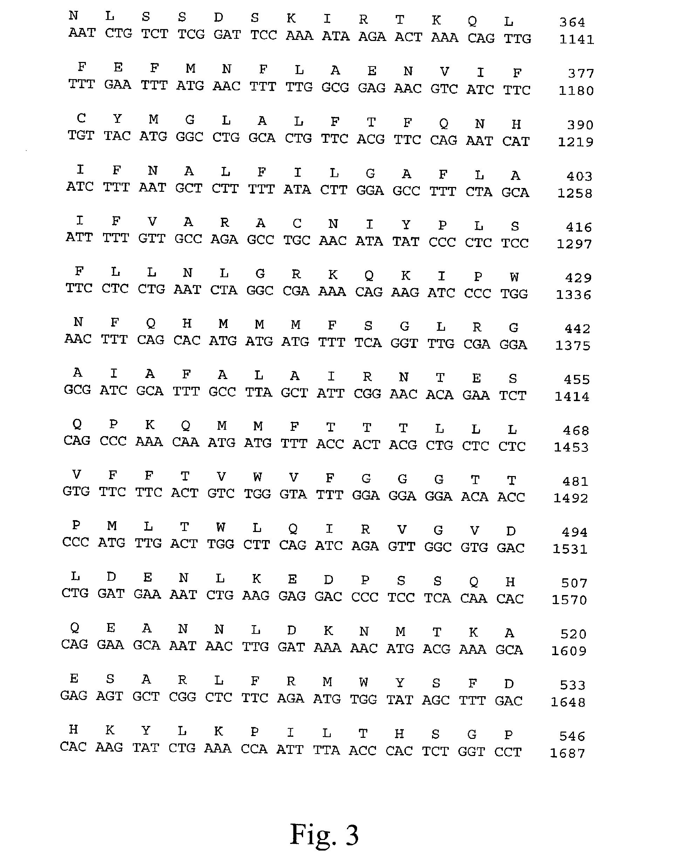

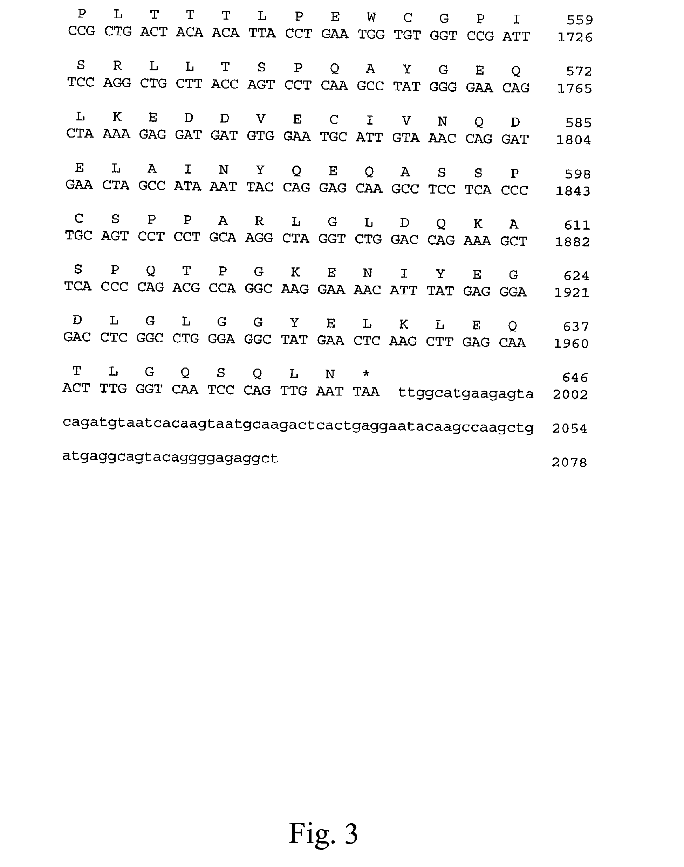

FIG. 3 presents the nucleotide sequence of the NHELP1 cDNA clone, with predicted amino acid translation; the sequences are further presented in the Sequence Listing, incorporated herein by reference in its entirety, in SEQ ID NOs: 1 (full length nucleotide sequence of human NHELP1 cDNA) and 3 (full length amino acid coding sequence of human NHELP1).

Unless otherwise indicated, each nucleotide sequence is set forth herein as a sequence of deoxyribonucleotides. It is intended, however, that the given sequence be interpreted as would be appropriate to the polynucleotide composition: for example, if the isolated nucleic acid is composed of RNA, the given sequence intends ribonucleotides, with uridine substituted for thymidine.

Unless otherwise indicated, nucleotide sequences of the isolated nucleic acids of the present invention were determined by sequencing a DNA molecule that had resulted, directly or indirectly, from at least one enzymatic polymerization reaction (*e.g*., reverse transcription and/or polymerase chain reaction) using an automated sequencer (such as the MegaBACE™ 1000, Amersham Biosciences, Sunnyvale, CA, USA), or by reliance upon such sequence or upon genomic sequence prior-accessioned into a public database. Unless otherwise indicated, all amino acid sequences of the polypeptides of the present invention were predicted by translation from the nucleic acid sequences so determined.

As a consequence, any nucleic acid sequence presented herein may contain errors introduced by erroneous incorporation of nucleotides during polymerization, by erroneous base calling by the automated sequencer (although such sequencing errors have been minimized for the nucleic acids directly determined herein, unless otherwise indicated, by the sequencing of each of the complementary strands of a duplex DNA), or by similar errors accessioned into the public database. Such errors can readily be identified and corrected by resequencing of the genomic locus using standard techniques.

Single nucleotide polymorphisms (SNPs) occur frequently in eukaryotic genomes - more than 1.4 million SNPs have already identified in the human genome, International Human Genome Sequencing Consortium, *Nature* 409:860 - 921 (2001) - and the sequence determined from one individual of a species may differ from other allelic forms present within the population. Additionally, small deletions and insertions, rather than single nucleotide polymorphisms, are not uncommon in the general population, and often do not alter the function of the protein.

Accordingly, it is an aspect of the present invention to provide nucleic acids not only identical in sequence to those described with particularity herein, but also to provide isolated nucleic acids at least about 65% identical in sequence to those described with particularity herein, typically at least about 70%, 75%, 80%, 85%, or 90% identical in sequence to those described with particularity herein, usefully at least about 91%, 92%, 93%, 94%, or 95% identical in sequence to those described with particularity herein, usefully at least about 96%, 97%, 98%, or 99% identical in sequence to those described with particularity herein, and, most conservatively, at least about 99.5%, 99.6%, 99.7%, 99.8% and 99.9% identical in sequence to those described with particularity herein. These sequence variants can be naturally occurring or can result from human intervention, as by random or directed mutagenesis.

For purposes herein, percent identity of two nucleic acid sequences is determined using the procedure of Tatiana *et al.,* "Blast 2 sequences - a new tool for comparing protein and nucleotide sequences", *FEMS Microbiol Lett.* 174:247-250 (1999), which procedure is effectuated by the computer program BLAST 2 SEQUENCES, available online at
http://www.ncbi.nlm.nih.gov/blast/bl2seq/bl2.html.
To assess percent identity of nucleic acids, the BLASTN module of BLAST 2 SEQUENCES is used with default values of (i) reward for a match: 1; (ii) penalty for a mismatch: -2; (iii) open gap 5 and extension gap 2 penalties; (iv) gap X_dropoff 50 expect 10 word size 11 filter, and both sequences are entered in their entireties.

As is well known, the genetic code is degenerate, with each amino acid except methionine translated from a plurality of codons, thus permitting a plurality of nucleic acids of disparate sequence to encode the identical protein. As is also well known, codon choice for optimal expression varies from species to species. The isolated nucleic acids of the present invention being useful for expression of NHELP1 proteins and protein fragments, it is, therefore, another aspect of the present invention to provide isolated nucleic acids that encode NHELP1 proteins and portions thereof not only identical in sequence to those described with particularity herein, but degenerate variants thereof as well.

As is also well known, amino acid substitutions occur frequently among natural allelic variants, with conservative substitutions often occasioning only *de minimis* change in protein function.

Accordingly, it is an aspect of the present invention to provide nucleic acids not only identical in sequence to those described with particularity herein, but also to provide isolated nucleic acids that encode NHELP1, and portions thereof, having conservative amino acid substitutions, and also to provide isolated nucleic acids that encode NHELP1, and portions thereof, having moderately conservative amino acid substitutions.

Although there are a variety of metrics for calling conservative amino acid substitutions, based primarily on either observed changes among evolutionarily related proteins or on predicted chemical similarity, for purposes herein a conservative replacement is any change having a positive value in the PAM250 log-likelihood matrix reproduced herein below *(see* Gonnet *et al., Science* 256(5062):1443-5 (1992)):

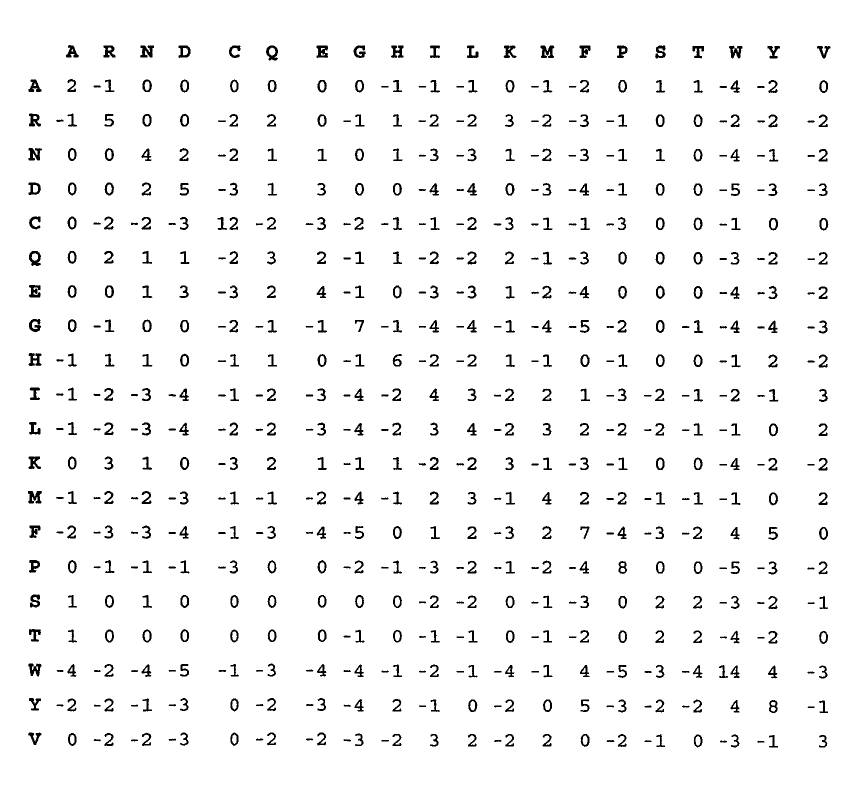

For purposes herein, a "moderately conservative" replacement is any change having a nonnegative value in the PAM250 log-likelihood matrix reproduced herein above.

As is also well known in the art, relatedness of nucleic acids can also be characterized using a functional test, the ability of the two nucleic acids to base-pair to one another at defined hybridization stringencies.

It is, therefore, another aspect of the invention to provide isolated nucleic acids not only identical in sequence to those described with particularity herein, but also to provide isolated nucleic acids ("cross-hybridizing nucleic acids") that hybridize under high stringency conditions (as defined herein below) to all or to a portion of various of the isolated NHELP1 nucleic acids of the present invention ("reference nucleic acids"), as well as cross-hybridizing nucleic acids that hybridize under moderate stringency conditions to all or to a portion of various of the isolated NHELP1 nucleic acids of the present invention.

Such cross-hybridizing nucleic acids are useful, *inter alia,* as probes for, and to drive expression of, proteins related to the proteins of the present invention as alternative isoforms, homologues, paralogues, and orthologues. Particularly useful orthologues are those from other primate species, such as chimpanzee, rhesus macaque, monkey, baboon, orangutan, and gorilla; from rodents, such as rats, mice, guinea pigs; from lagomorphs, such as rabbits; and from domestic livestock, such as cow, pig, sheep, horse, goat and chicken.

For purposes herein, high stringency conditions are defined as aqueous hybridization *(i.e.,* free of formamide) in 6X SSC (where 20X SSC contains 3.0 M NaCl and 0.3 M sodium citrate), 1% SDS at 65°C for at least 8 hours, followed by one or more washes in 0.2X SSC, 0.1% SDS at 65°C. For purposes herein, moderate stringency conditions are defined as aqueous hybridization (*i.e*., free of formamide) in 6X SSC, 1% SDS at 65°C for at least 8 hours, followed by one or more washes in 2x SSC, 0.1% SDS at room temperature.

The hybridizing portion of the reference nucleic acid is typically at least 15 nucleotides in length, often at least 17 nucleotides in length. Often, however, the hybridizing portion of the reference nucleic acid is at least 20 nucleotides in length, 25 nucleotides in length, and even 30 nucleotides, 35 nucleotides, 40 nucleotides, and 50 nucleotides in length. Of course, cross-hybridizing nucleic acids that hybridize to a larger portion of the reference nucleic acid - for example, to a portion of at least 50 nt, at least 100 nt, at least 150 nt, 200 nt, 250 nt, 300 nt, 350 nt, 400 nt, 450 nt, or 500 nt or more - or even to the entire length of the reference nucleic acid, are also useful.

The hybridizing portion of the cross-hybridizing nucleic acid is at least 75% identical in sequence to at least a portion of the reference nucleic acid. Typically, the hybridizing portion of the cross-hybridizing nucleic acid is at least 80%, often at least 85%, 86%, 87%, 88%, 89% or even at least 90% identical in sequence to at least a portion of the reference nucleic acid. Often, the hybridizing portion of the cross-hybridizing nucleic acid will be at least 91%, 92%, 93%, 94%, 95%, 96%, 97%, 98%, or 99% identical in sequence to at least a portion of the reference nucleic acid sequence. At times, the hybridizing portion of the cross-hybridizing nucleic acid will be at least 99.5% identical in sequence to at least a portion of the reference nucleic acid.

The invention also provides fragments of various of the isolated nucleic acids of the present invention.

By "fragments" of a reference nucleic acid is here intended isolated nucleic acids, however obtained, that have a nucleotide sequence identical to a portion of the reference nucleic acid sequence, which portion is at least 17 nucleotides and less than the entirety of the reference nucleic acid. As so defined, "fragments" need not be obtained by physical fragmentation of the reference nucleic acid, although such provenance is not thereby precluded.

In theory, an oligonucleotide of 17 nucleotides is of sufficient length as to occur at random less frequently than once in the three gigabase human genome, and thus to provide a nucleic acid probe that can uniquely identify the reference sequence in a nucleic acid mixture of genomic complexity. As is well known, further specificity can be obtained by probing nucleic acid samples of subgenomic complexity, and/or by using plural fragments as short as 17 nucleotides in length collectively to prime amplification of nucleic acids, as, *e.g.,* by polymerase chain reaction (PCR).

As further described herein below, nucleic acid fragments that encode at least 6 contiguous amino acids (*i.e.,* fragments of 18 nucleotides or more) are useful in directing the expression or the synthesis of peptides that have utility in mapping the epitopes of the protein encoded by the reference nucleic acid. *See, e.g.,* Geysen *et al*., "Use of peptide synthesis to probe viral antigens for epitopes to a resolution of a single amino acid," *Proc. Natl. Acad. Sci. USA* 81:3998-4002 (1984); and U.S. Pat. Nos. 4,708,871 and 5,595,915, the disclosures of which are incorporated herein by reference in their entireties.

As further described herein below, fragments that encode at least 8 contiguous amino acids (*i.e.,* fragments of 24 nucleotides or more) are useful in directing the expression or the synthesis of peptides that have utility as immunogens. *See, e.g*., Lerner, "Tapping the immunological repertoire to produce antibodies of predetermined specificity," Nature 299:592-596 (1982); Shinnick *et al*., "Synthetic peptide immunogens as vaccines," *Annu. Rev. Microbiol.* 37:425-46 (1983); Sutcliffe *et* al., "Antibodies that react with predetermined sites on proteins," *Science* 219:660-6 (1983), the disclosures of which are incorporated herein by reference in their entireties.

The nucleic acid fragment of the present invention is thus at least 17 nucleotides in length, typically at least 18 nucleotides in length, and often at least 24 nucleotides in length. Often, the nucleic acid of the present invention is at least 25 nucleotides in length, and even 30 nucleotides, 35 nucleotides, 40 nucleotides, or 45 nucleotides in length. Of course, larger fragments having at least 50 nt, at least 100 nt, at least 150 nt, 200 nt, 250 nt, 300 nt, 350 nt, 400 nt, 450 nt, or 500 nt or more are also useful, and at times preferred.

Having been based upon the mining of genomic sequence, rather than upon surveillance of expressed message, the present invention further provides isolated genome-derived nucleic acids that include portions of the NHELP1 gene.

The invention particularly provides genome-derived single exon probes.

As further described in commonly owned and copending U.S. patent application serial nos. 09/864,761, filed May 23, 2001; 09/774,203, filed January 29, 2001; and 09/632,366, filed August 3, 2000, the disclosures of which are incorporated herein by reference in their entireties, "a single exon probe" comprises at least part of an exon ("reference exon") and can hybridize detectably under high stringency conditions to transcript-derived nucleic acids that include the reference exon. The single exon probe will not, however, hybridize detectably under high stringency conditions to nucleic acids that lack the reference exon and instead consist of one or more exons that are found adjacent to the reference exon in the genome.

Genome-derived single exon probes typically further comprise, contiguous to a first end of the exon portion, a first intronic and/or intergenic sequence that is identically contiguous to the exon in the genome. Often, the genome-derived single exon probe further comprises, contiguous to a second end of the exonic portion, a second intronic and/or intergenic sequence that is identically contiguous to the exon in the genome.

The minimum length of genome-derived single exon probes is defined by the requirement that the exonic portion be of sufficient length to hybridize under high stringency conditions to transcript-derived nucleic acids. Accordingly, the exon portion is at least 17 nucleotides, typically at least 18 nucleotides, 20 nucleotides, 24 nucleotides, 25 nucleotides or even 30, 35, 40, 45, or 50 nucleotides in length, and can usefully include the entirety of the exon, up to 100 nt, 150 nt, 200 nt, 250 nt, 300 nt, 350 nt, 400 nt or even 500 nt or more in length.

The maximum length of genome-derived single exon probes is defined by the requirement that the probes contain portions of no more than one exon, that is, be unable to hybridize detectably under high stringency conditions to nucleic acids that lack the reference exon but include one or more exons that are found adjacent to the reference exon the genome.

Given variable spacing of exons through eukaryotic genomes, the maximum length of single exon probes of the present invention is typically no more than 25 kb, often no more than 20 kb, 15 kb, 10 kb or 7.5 kb, or even no more than 5 kb, 4 kb, 3 kb, or even no more than about 2.5 kb in length.

The genome-derived single exon probes of the present invention can usefully include at least a first terminal priming sequence not found in contiguity with the rest of the probe sequence in the genome, and often will contain a second terminal priming sequence not found in contiguity with the rest of the probe sequence in the genome.

The present invention also provides isolated genome-derived nucleic acids that include nucleic acid sequence elements that control transcription of the NHELP1 gene.

With a complete draft of the human genome now available, genomic sequences that are within the vicinity of the NHELP1 coding region (and that are additional to those described with particularity herein) can readily be obtained by PCR amplification.

The isolated nucleic acids of the present invention can be composed of natural nucleotides in native 5'-3' phosphodiester internucleoside linkage - *e.g*., DNA or RNA - or can contain any or all of nonnatural nucleotide analogues, nonnative internucleoside bonds, or post-synthesis modifications, either throughout the length of the nucleic acid or localized to one or more portions thereof.

As is well known in the art, when the isolated nucleic acid is used as a hybridization probe, the range of such nonnatural analogues, nonnative internucleoside bonds, or post-synthesis modifications will be limited to those that permit sequence-discriminating basepairing of the resulting nucleic acid. When used to direct expression or RNA or protein *in vitro* or *in vivo,* the range of such nonnatural analogues, nonnative internucleoside bonds, or post-synthesis modifications will be limited to those that permit the nucleic acid to function properly as a polymerization substrate. When the isolated nucleic acid is used as a therapeutic agent, the range of such changes will be limited to those that do not confer toxicity upon the isolated nucleic acid.

For example, when desired to be used as probes, the isolated nucleic acids of the present invention can usefully include nucleotide analogues that incorporate labels that are directly detectable, such as radiolabels or fluorophores, or nucleotide analogues that incorporate labels that can be visualized in a subsequent reaction, such as biotin or various haptens.

Common radiolabeled analogues include those labeled with ³³P, ³²P, and ³⁵S, such as α-³²P-dATP, α-³²P-dCTP, α-³²P-dGTP, α-³²P-dTTP, α-³²P-3'dATP, α-³²P-ATP, α-³²P-CTP, α-³²P-GTP, α-³²P-UTP, α-³⁵S-dATP, γ-³⁵S-GTP, γ-³³P-dATP, and the like.

Commercially available fluorescent nucleotide analogues readily incorporated into the nucleic acids of the present invention include Cy3-dCTP, Cy3-dUTP, Cy5-dCTP, Cy3-dUTP (Amersham Biosciences, Piscataway, New Jersey, USA), fluorescein-12-dUTP, tetramethylrhodamine-6-dUTP, Texas Red®-5-dUTP, Cascade Blue®-7-dUTP, BODIPY® FL-14-dUTP, BODIPY® TMR-14-dUTP, BODIPY® TR-14-dUTP, Rhodamine Green™-5-dUTP, Oregon Green® 488-5-dUTP, Texas Red®-12-dUTP, BODIPY® 630/650-14-dUTP, BODIPY® 650/665-14-dUTP, Alexa Fluor® 488-5-dUTP, Alexa Fluor® 532-5-dUTP, Alexa Fluor® 568-5-dUTP, Alexa Fluor® 594-5-dUTP, Alexa Fluor® 546-14-dUTP, fluorescein-12-UTP, tetramethylrhodamine-6-UTP, Texas Red®-5-UTP, Cascade Blue®-7-UTP, BODIPY® FL-14-UTP, BODIPY® TMR-14-UTP, BODIPY® TR-14-UTP, Rhodamine Green™-5-UTP, Alexa Fluor® 488-5-UTP, Alexa Fluor® 546-14-UTP (Molecular Probes, Inc. Eugene, OR, USA).

Protocols are available for custom synthesis of nucleotides having other fluorophores. Henegariu *et al.,* "Custom Fluorescent-Nucleotide Synthesis as an Alternative Method for Nucleic Acid Labeling," *Nature Biotechnol.* 18:345 - 348 (2000), the disclosure of which is incorporated herein by reference in its entirety.

Haptens that are commonly conjugated to nucleotides for subsequent labeling include biotin (biotin-11-dUTP, Molecular Probes, Inc., Eugene, OR, USA; biotin-21-UTP, biotin-21-dUTP, Clontech Laboratories, Inc., Palo Alto, CA, USA), digoxigenin (DIG-11-dUTP, alkali labile, DIG-11-UTP, Roche Diagnostics Corp., Indianapolis, IN, USA), and dinitrophenyl (dinitrophenyl-11-dUTP, Molecular Probes, Inc., Eugene, OR, USA).

As another example, when desired to be used for antisense inhibition of transcription or translation, the isolated nucleic acids of the present invention can usefully include altered, often nuclease-resistant, internucleoside bonds. *See* Hartmann *et al.* (eds.), Manual of Antisense Methodology (Perspectives in Antisense Science), Kluwer Law International (1999) (ISBN:079238539X); Stein *et al.* (eds.), Applied Antisense Oligonucleotide Technology, Wiley-Liss (cover (1998) (ISBN: 0471172790); Chadwick *et al.* (eds.), Oligonucleotides as Therapeutic Agents - Symposium No. 209, John Wiley & Son Ltd (1997) (ISBN: 0471972797), the disclosures of which are incorporated herein by reference in their entireties. Such altered internucloside bonds are often desired also when the isolated nucleic acid of the present invention is to be used for targeted gene correction, Gamper *et al., Nucl. Acids Res.* 28(21):4332-4339 (2000), the disclosures of which are incorporated herein by reference in its entirety.

Modified oligonucleotide backbones often preferred when the nucleic acid is to be used for antisense purposes are, for example, phosphorothioates, chiral phosphorothioates, phosphorodithioates, phosphotriesters, aminoalkylphosphotriesters, methyl and other alkyl phosphonates including 3'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates having normal 3'-5' linkages, 2'-5' linked analogs of these, and those having inverted polarity wherein the adjacent pairs of nucleoside units are linked 3'-5' to 5'-3' or 2'-5' to 5'-2'. Representative U.S. patents that teach the preparation of the above phosphorus-containing linkages include, but are not limited to, U.S. Pat. Nos. 3,687,808; 4,469,863; 4,476,301; 5,023,243; 5,177,196; 5,188,897; 5,264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,306; 5,550,111; 5,563,253; 5,571,799; 5,587,361; and 5,625,050, the disclosures of which are incorporated herein by reference in their entireties.

Preferred modified oligonucleotide backbones for antisense use that do not include a phosphorus atom have backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and CH₂ component parts. Representative U.S. patents that teach the preparation of the above backbones include, but are not limited to, U.S. Pat. Nos. 5,034,506; 5,166,315; 5,185,444; 5,214,134; 5,216,141; 5,235,033; 5,264,562; 5,264,564; 5,405,938; 5,434,257; 5,466,677; 5,470,967; 5,489,677; 5,541,307; 5,561,225; 5,596,086; 5,602,240; 5,610,289; 5,602,240; 5,608,046; 5,610,289; 5,618,704; 5,623,070; 5,663,312; 5,633,360; 5,677,437; and 5,677,439, the disclosures of which are incorporated herein by reference in their entireties.

In other preferred oligonucleotide mimetics, both the sugar and the internucleoside linkage are replaced with novel groups, such as peptide nucleic acids (PNA).

In PNA compounds, the phosphodiester backbone of the nucleic acid is replaced with an amide-containing backbone, in particular by repeating N-(2-aminoethyl) glycine units linked by amide bonds. Nucleobases are bound directly or indirectly to aza nitrogen atoms of the amide portion of the backbone, typically by methylene carbonyl linkages.

The uncharged nature of the PNA backbone provides PNA/DNA and PNA/RNA duplexes with a higher thermal stability than is found in DNA/DNA and DNA/RNA duplexes, resulting from the lack of charge repulsion between the PNA and DNA or RNA strand. In general, the Tm of a PNA/DNA or PNA/RNA duplex is 1°C higher per base pair than the Tm of the corresponding DNA/DNA or DNA/RNA duplex (in 100 mM NaCl).

The neutral backbone also allows PNA to form stable DNA duplexes largely independent of salt concentration. At low ionic strength, PNA can be hybridized to a target sequence at temperatures that make DNA hybridization problematic or impossible. And unlike DNA/DNA duplex formation, PNA hybridization is possible in the absence of magnesium. Adjusting the ionic strength, therefore, is useful if competing DNA or RNA is present in the sample, or if the nucleic acid being probed contains a high level of secondary structure.

PNA also demonstrates greater specificity in binding to complementary DNA. A PNA/DNA mismatch is more destabilizing than DNA/DNA mismatch. A single mismatch in mixed a PNA/DNA 15-mer lowers the Tm by 8-20°C (15°C on average). In the corresponding DNA/DNA duplexes, a single mismatch lowers the Tm by 4-16°C (11°C on average). Because PNA probes can be significantly shorter than DNA probes, their specificity is greater.

Additionally, nucleases and proteases do not recognize the PNA polyamide backbone with nucleobase sidechains. As a result, PNA oligomers are resistant to degradation by enzymes, and the lifetime of these compounds is extended both *in vivo* and *in vitro.* In addition, PNA is stable over a wide pH range.

Because its backbone is formed from amide bonds, PNA can be synthesized using a modified peptide synthesis protocol. PNA oligomers can be synthesized by both Fmoc and tBoc methods. Representative U.S. patents that teach the preparation of PNA compounds include, but are not limited to, U.S. Pat. Nos. 5,539,082; 5,714,331; and 5,719,262, each of which is herein incorporated by reference; automated PNA synthesis is readily achievable on commercial synthesizers (*see, e.g*., "PNA User's Guide," Rev. 2, February 1998, Perseptive Biosystems Part No. 60138, Applied Biosystems, Inc., Foster City, CA).

PNA chemistry and applications are reviewed, *inter alia,* in Ray *et al., FASEB J.* 14(9):1041-60 (2000); Nielsen *et al., Pharmacol Toxicol.* 86(1):3-7 (2000); Larsen *et al., Biochim Biophys Acta.* 1489(1):159-66 (1999); Nielsen, *Curr. Opin. Struct. Biol.* 9(3):353-7 (1999), and Nielsen, *Curr. Opin. Biotechnol.* 10(1):71-5 (1999), the disclosures of which are incorporated herein by reference in their entireties.

Differences from nucleic acid compositions found in nature - *e.g.,* nonnative bases, altered internucleoside linkages, post-synthesis modification - can be present throughout the length of the nucleic acid or can, instead, usefully be localized to discrete portions thereof. As an example of the latter, chimeric nucleic acids can be synthesized that have discrete DNA and RNA domains and demonstrated utility for targeted gene repair, as further described in U.S. Pat. Nos. 5,760,012 and 5,731,181, the disclosures of which are incorporated herein by reference in their entireties. As another example, chimeric nucleic acids comprising both DNA and PNA have been demonstrated to have utility in modified PCR reactions. *See* Misra *et al., Biochem.* 37: 1917-1925 (1998); *see also* Finn *et al., Nucl. Acids Res.* 24: 3357-3363 (1996), incorporated herein by reference.

Unless otherwise specified, nucleic acids of the present invention can include any topological conformation appropriate to the desired use; the term thus explicitly comprehends, among others, single-stranded, double-stranded, triplexed, quadruplexed, partially double-stranded, partially-triplexed, partially-quadruplexed, branched, hairpinned, circular, and padlocked conformations. Padlock conformations and their utilities are further described in Banér *et al*., *Curr. Opin. Biotechnol.* 12:11-15 (2001); Escude *et al.,* Proc. Natl. Acad. Sci. USA 14;96(19):10603-7 (1999); Nilsson *et al*., *Science* 265(5181):2085-8 (1994), the disclosures of which are incorporated herein by reference in their entireties. Triplex and quadruplex conformations, and their utilities, are reviewed in Praseuth *et al., Biochim. Biophys. Acta.* 1489(1):181-206 (1999); Fox, *Curr. Med. Chem.* 7(1):17-37 (2000); Kochetkova *et al., Methods Mol. Biol.* 130:189-201 (2000); Chan *et al., J. Mol. Med.* 75(4):267-82 (1997), the disclosures of which are incorporated herein by reference in their entireties.

The nucleic acids of the present invention can be detectably labeled.

Commonly-used labels include radionuclides, such as ³²P, ³³P, ³⁵S, ³H (and for NMR detection, ¹³C and ¹⁵N), haptens that can be detected by specific antibody or high affinity binding partner (such as avidin), and fluorophores.

As noted above, detectable labels can be incorporated by inclusion of labeled nucleotide analogues in the nucleic acid.

Such analogues can be incorporated by enzymatic polymerization, such as by nick translation, random priming, polymerase chain reaction (PCR), terminal transferase tailing, and end-filling of overhangs, for DNA molecules, and *in vitro* transcription driven, *e.g*., from phage promoters, such as T7, T3, and SP6, for RNA molecules. Commercial kits are readily available for each such labeling approach.

Analogues can also be incorporated during automated solid phase chemical synthesis.

As is well known, labels can also be incorporated after nucleic acid synthesis, with the 5' phosphate and 3' hydroxyl providing convenient sites for post-synthetic covalent attachment of detectable labels.

Various other post-synthetic approaches permit internal labeling of nucleic acids.

For example, fluorophores can be attached using a cisplatin reagent that reacts with the N7 of guanine residues (and, to a lesser extent, adenine bases) in DNA, RNA, and PNA to provide a stable coordination complex between the nucleic acid and fluorophore label (Universal Linkage System) (available from Molecular Probes, Inc., Eugene, OR, USA and Amersham Biosciences, Piscataway, NJ, USA); *see* Alers *et al., Genes, Chromosomes & Cancer*, Vol. 25, pp. 301 - 305 (1999); Jelsma *et al., J. NIH Res.* 5:82 (1994); Van Belkum *et al., BioTechniques* 16:148-153 (1994), incorporated herein by reference. As another example, nucleic acids can be labeled using a disulfide-containing linker (FastTag™ Reagent, Vector Laboratories, Inc., Burlingame, CA, USA) that is photo- or thermally coupled to the target nucleic acid using aryl azide chemistry; after reduction, a free thiol is available for coupling to a hapten, fluorophore, sugar, affinity ligand, or other marker.

Multiple independent or interacting labels can be incorporated into the nucleic acids of the present invention.

For example, both a fluorophore and a moiety that in proximity thereto acts to quench fluorescence can be included to report specific hybridization through release of fluorescence quenching, Tyagi *et al*., *Nature Biotechnol.* 14: 303-308 (1996); Tyagi *et al*., *Nature Biotechnol.* 16, 49-53 (1998); Sokol et *al., Proc. Natl. Acad. Sci. USA* 95: 11538-11543 (1998); Kostrikis *et al*., *Science* 279:1228-1229 (1998); Marras *et al*., *Genet. Anal.* 14: 151-156 (1999); U.S. Pat. Nos. 5,846,726, 5,925,517, 5925517, or to report exonucleotidic excision, U.S. Pat. No. 5,538,848; Holland *et al., Proc. Natl. Acad. Sci. USA* 88:7276-7280 (1991); Heid *et al., Genome Res.* 6(10):986-94 (1996); Kuimelis *et al., Nucleic Acids Symp Ser.* (37):255-6 (1997); U.S. Patent No. 5,723,591, the disclosures of which are incorporated herein by reference in their entireties.

So labeled, the isolated nucleic acids of the present invention can be used as probes, as further described below.

Nucleic acids of the present invention can also usefully be bound to a substrate. The substrate can porous or solid, planar or non-planar, unitary or distributed; the bond can be covalent or noncovalent. Bound to a substrate, nucleic acids of the present invention can be used as probes in their unlabeled state.

For example, the nucleic acids of the present invention can usefully be bound to a porous substrate, commonly a membrane, typically comprising nitrocellulose, nylon, or positively-charged derivatized nylon; so attached, the nucleic acids of the present invention can be used to detect NHELP1 nucleic acids present within a labeled nucleic acid sample, either a sample of genomic nucleic acids or a sample of transcript-derived nucleic acids, e.g. by reverse dot blot.

The nucleic acids of the present invention can also usefully be bound to a solid substrate, such as glass, although other solid materials, such as amorphous silicon, crystalline silicon, or plastics, can also be used. Such plastics include polymethylacrylic, polyethylene, polypropylene, polyacrylate, polymethylmethacrylate, polyvinylchloride, polytetrafluoroethylene, polystyrene, polycarbonate, polyacetal, polysulfone, celluloseacetate, cellulosenitrate, nitrocellulose, or mixtures thereof.

Typically, the solid substrate will be rectangular, although other shapes, particularly disks and even spheres, present certain advantages. Particularly advantageous alternatives to glass slides as support substrates for array of nucleic acids are optical discs, as described in Demers, "Spatially Addressable Combinatorial Chemical Arrays in CD-ROM Format," international patent publication WO 98/12559, incorporated herein by reference in its entirety.

The nucleic acids of the present invention can be attached covalently to a surface of the support substrate or applied to a derivatized surface in a chaotropic agent that facilitates denaturation and adherence by presumed noncovalent interactions, or some combination thereof.

The nucleic acids of the present invention can be bound to a substrate to which a plurality of other nucleic acids are concurrently bound, hybridization to each of the plurality of bound nucleic acids being separately detectable. At low density, *e.g.* on a porous membrane, these substrate-bound collections are typically denominated macroarrays; at higher density, typically on a solid support, such as glass, these substrate bound collections of plural nucleic acids are colloquially termed microarrays. As used herein, the term microarray includes arrays of all densities. It is, therefore, another aspect of the invention to provide microarrays that include the nucleic acids of the present invention.

The isolated nucleic acids of the present invention can be used as hybridization probes to detect, characterize, and quantify NHELP1 nucleic acids in, and isolate NHELP1 nucleic acids from, both genomic and transcript-derived nucleic acid samples. When free in solution, such probes are typically, but not invariably, detectably labeled; bound to a substrate, as in a microarray, such probes are typically, but not invariably unlabeled.

For example, the isolated nucleic acids of the present invention can be used as probes to detect and characterize gross alterations in the NHELP1 genomic locus, such as deletions, insertions, translocations, and duplications of the NHELP1 genomic locus through fluorescence *in situ* hybridization (FISH) to chromosome spreads. *See, e.g.,* Andreeff *et al.* (eds.), Introduction to Fluorescence *In Situ* Hybridization: Principles and Clinical Applications, John Wiley & Sons (1999) (ISBN: 0471013455), the disclosure of which is incorporated herein by reference in its entirety. The isolated nucleic acids of the present invention can be used as probes to assess smaller genomic alterations using, *e.g.,* Southern blot detection of restriction fragment length polymorphisms. The isolated nucleic acids of the present invention can be used as probes to isolate genomic clones that include the nucleic acids of the present invention, which thereafter can be restriction mapped and sequenced to identify deletions, insertions, translocations, and substitutions (single nucleotide polymorphisms, SNPs) at the sequence level.

The isolated nucleic acids of the present invention can also be used as probes to detect, characterize, and quantify NHELP1 nucleic acids in, and isolate NHELP1 nucleic acids from, transcript-derived nucleic acid samples.

For example, the isolated nucleic acids of the present invention can be used as hybridization probes to detect, characterize by length, and quantify NHELP1 mRNA by northern blot of total or poly-A⁺- selected RNA samples. For example, the isolated nucleic acids of the present invention can be used as hybridization probes to detect, characterize by location, and quantify NHELP1 message by *in situ* hybridization to tissue sections (see, *e.g*., Schwarchzacher *et al*., In Situ Hybridization, Springer-Verlag New York (2000) (ISBN: 0387915966), the disclosure of which is incorporated herein by reference in its entirety). For example, the isolated nucleic acids of the present invention can be used as hybridization probes to measure the representation of NHELP1 clones in a cDNA library. For example, the isolated nucleic acids of the present invention can be used as hybridization probes to isolate NHELP1 nucleic acids from cDNA libraries, permitting sequence level characterization of NHELP1 messages, including identification of deletions, insertions, truncations - including deletions, insertions, and truncations of exons in alternatively spliced forms - and single nucleotide polymorphisms.

All of the aforementioned probe techniques are well within the skill in the art, and are described at greater length in standard texts such as Sambrook *et al.,* Molecular Cloning: A Laboratory Manual (3^{rd} ed.), Cold Spring Harbor Laboratory Press (2001) (ISBN: 0879695773); Ausubel *et al.* (eds.), Short Protocols in Molecular Biology: A Compendium of Methods from Current Protocols in Molecular Biology (4^{th} ed.), John Wiley & Sons, 1999 (ISBN: 047132938X); and Walker et *al.* (eds.), The Nucleic Acids Protocols Handbook, Humana Press (2000) (ISBN: 0896034593), the disclosures of which are incorporated herein by reference in their entirety.

As described in the Examples herein below, the nucleic acids of the present invention can also be used to detect and quantify NHELP1 nucleic acids in transcript-derived samples - that is, to measure expression of the NHELP1 gene - when included in a microarray. Measurement of NHELP1 expression has particular utility in the diagnosis and treatmnet of cancer and AIDS, as further described in the Examples herein below.

As would be readily apparent to one of skill in the art, each NHELP1 nucleic acid probe - whether labeled, substrate-bound, or both - is thus currently available for use as a tool for measuring the level of NHELP1 expression in each of the tissues in which expression has already been confirmed, notably brain, adult liver, adrenal, bone marrow, fetal liver, testis and prostate, as well as a cell line, hela. The utility is specific to the probe: under high stringency conditions, the probe reports the level of expression of message specifically containing that portion of the NHELP1 gene included within the probe.

Measuring tools are well known in many arts, not just in molecular biology, and are known to possess credible, specific, and substantial utility. For example, U.S. Patent No. 6,016,191 describes and claims a tool for measuring characteristics of fluid flow in a hydrocarbon well; U.S. Patent No. 6,042,549 describes and claims a device for measuring exercise intensity; U.S. Patent No. 5,889,351 describes and claims a device for measuring viscosity and for measuring characteristics of a fluid; U.S. Patent No. 5,570,694 describes and claims a device for measuring blood pressure; U.S. Patent No. 5,930,143 describes and claims a device for measuring the dimensions of machine tools; U.S. Patent No. 5,279,044 describes and claims a measuring device for determining an absolute position of a movable element; U.S. Patent No. 5,186,042 describes and claims a device for measuring action force of a wheel; and U.S. Patent No. 4,246,774 describes and claims a device for measuring the draft of smoking articles such as cigarettes.

As for tissues not yet demonstrated to express NHELP1, the NHELP1 nucleic acid probes of the present invention are currently available as tools for surveying such tissues to detect the presence of NHELP1 nucleic acids.

Survey tools - *i.e.*, tools for determining the presence and/or location of a desired object by search of an area - are well known in many arts, not just in molecular biology, and are known to possess credible, specific, and substantial utility. For example, U.S. Patent No. 6,046,800 describes and claims a device for surveying an area for objects that move; U.S. Patent No. 6,025,201 describes and claims an apparatus for locating and discriminating platelets from non-platelet particles or cells on a cell-by-cell basis in a whole blood sample; U.S. Patent No. 5,990,689 describes and claims a device for detecting and locating anomalies in the electromagnetic protection of a system; U.S. Patent No. 5,984,175 describes and claims a device for detecting and identifying wearable user identification units; U.S. Patent No. 3,980,986 ("Oil well survey tool"), describes and claims a tool for finding the position of a drill bit working at the bottom of a borehole.

As noted above, the nucleic acid probes of the present invention are useful in constructing microarrays; the microarrays, in turn, are products of manufacture that are useful for measuring and for surveying gene expression.

When included on a microarray, each NHELP1 nucleic acid probe makes the microarray specifically useful for detecting that portion of the NHELP1 gene included within the probe, thus imparting upon the microarray device the ability to detect a signal where, absent such probe, it would have reported no signal. This utility makes each individual probe on such microarray akin to an antenna, circuit, firmware or software element included in an electronic apparatus, where the antenna, circuit, firmware or software element imparts upon the apparatus the ability newly and additionally to detect signal in a portion of the radio-frequency spectrum where previously it could not; such devices are known to have specific, substantial, and credible utility.

Changes in the level of expression need not be observed for the measurement of expression to have utility.

For example, where gene expression analysis is used to assess toxicity of chemical agents on cells, the failure of the agent to change a gene's expression level is evidence that the drug likely does not affect the pathway of which the gene's expressed protein is a part. Analogously, where gene expression analysis is used to assess side effects of pharmacologic agents - whether in lead compound discovery or in subsequent screening of lead compound derivatives - the inability of the agent to alter a gene's expression level is evidence that the drug does not affect the pathway of which the gene's expressed protein is a part.

WO 99/58720, incorporated herein by reference in its entirety, provides methods for quantifying the relatedness of a first and second gene expression profile and for ordering the relatedness of a plurality of gene expression profiles, without regard to the identity or function of the genes whose expression is used in the calculation.

Gene expression analysis, including gene expression analysis by microarray hybridization, is, of course, principally a laboratory-based art. Devices and apparatus used principally in laboratories to facilitate laboratory research are well-established to possess specific, substantial, and credible utility. For example, U.S. Patent No. 6,001,233 describes and claims a gel electrophoresis apparatus having a cam-activated clamp; for example, U.S. Patent No. 6,051,831 describes and claims a high mass detector for use in time-of-flight mass spectrometers; for example, U.S. Patent NO. 5,824,269 describes and claims a flow cytometer-as is well known, few gel electrophoresis apparatuses, TOF-MS devices, or flow cytometers are sold for consumer use.

Indeed, and in particular, nucleic acid microarrays, as devices intended for laboratory use in measuring gene expression, are well-established to have specific, substantial and credible utility. Thus, the microarrays of the present invention have at least the specific, substantial and credible utilities of the microarrays claimed as devices and articles of manufacture in the following U.S. patents, the disclosures of each of which is incorporated herein by reference: U.S. Patent Nos. 5,445,934 ("Array of oligonucleotides on a solid substrate"); 5,744,305 ("Arrays of materials attached to a substrate"); and 6,004,752 ("Solid support with attached molecules").

Genome-derived single exon probes and genome-derived single exon probe microarrays have the additional utility, *inter alia*, of permitting high-throughput detection of splice variants of the nucleic acids of the present invention, as further described in copending and commonly owned U.S. Patent application no. 09/632,366, filed August 3, 2000, the disclosure of which is incorporated herein by reference in its entirety.

The isolated nucleic acids of the present invention can also be used to prime synthesis of nucleic acid, for purpose of either analysis or isolation, using mRNA, cDNA, or genomic DNA as template.

For use as primers, at least 17 contiguous nucleotides of the isolated nucleic acids of the present invention will be used. Often, at least 18, 19, or 20 contiguous nucleotides of the nucleic acids of the present invention will be used, and on occasion at least 20, 22, 24, or 25 contiguous nucleotides of the nucleic acids of the present invention will be used, and even 30 nucleotides or more of the nucleic acids of the present invention can be used to prime specific synthesis.

The nucleic acid primers of the present invention can be used, for example, to prime first strand cDNA synthesis on an mRNA template.

Such primer extension can be done directly to analyze the message. Alternatively, synthesis on an mRNA template can be done to produce first strand cDNA. The first strand cDNA can thereafter be used, *inter alia,* directly as a single-stranded probe, as above-described, as a template for sequencing - permitting identification of alterations, including deletions, insertions, and substitutions, both normal allelic variants and mutations associated with abnormal phenotypes- or as a template, either for second strand cDNA synthesis *(e.g.,* as an antecedent to insertion into a cloning or expression vector), or for amplification.

The nucleic acid primers of the present invention can also be used, for example, to prime single base extension (SBE) for SNP detection *(see, e.g.,* U.S. Pat. No. 6,004,744, the disclosure of which is incorporated herein by reference in its entirety).

As another example, the nucleic acid primers of the present invention can be used to prime amplification of NHELP1 nucleic acids, using transcript-derived or genomic DNA as template.

Primer-directed amplification methods are now well-established in the art. Methods for performing the polymerase chain reaction (PCR) are compiled, *inter alia,* in McPherson, PCR (Basics: From Background to Bench), Springer Verlag (2000) (ISBN: 0387916008); Innis *et al.* (eds.), PCR Applications: Protocols for Functional Genomics, Academic Press (1999) (ISBN: 0123721857); Gelfand *et al.* (eds.), PCR Strategies, Academic Press (1998) (ISBN: 0123721822); Newton *et al.,* PCR, Springer-Verlag New York (1997) (ISBN: 0387915060); Burke (ed.), PCR: Essential Techniques, John Wiley & Son Ltd (1996) (ISBN: 047195697X); White (ed.), PCR Cloning Protocols: From Molecular Cloning to Genetic Engineering, Vol. 67, Humana Press (1996) (ISBN: 0896033430); McPherson et al. (eds.), PCR 2: A Practical Approach, Oxford University Press, Inc. (1995) (ISBN: 0199634254), the disclosures of which are incorporated herein by reference in their entireties. Methods for performing RT-PCR are collected, *e.g.,* in Siebert *et al.* (eds.), Gene Cloning and Analysis by RT-PCR, Eaton Publishing Company/Bio Techniques Books Division, 1998 (ISBN: 1881299147); Siebert (ed.), PCR Technique:RT-PCR, Eaton Publishing Company/BioTechniques Books (1995) (ISBN:1881299139), the disclosure of which is incorporated herein by reference in its entirety.

Isothermal amplification approaches, such as rolling circle amplification, are also now well-described. *See, e.g.,* Schweitzer *et al*., *Curr. Opin. Biotechnol.* 12(1):21-7 (2001); U.S. Patent Nos. 6,235,502, 6,221,603, 6,210,884, 6,183,960, 5,854,033, 5,714,320, 5,648,245, and international patent publications WO 97/19193 and WO 00/15779, the disclosures of which are incorporated herein by reference in their entireties. Rolling circle amplification can be combined with other techniques to facilitate SNP detection. See, *e.g*., Lizardi *et al*., *Nature Genet.* 19(3):225-32 (1998).

As further described below, nucleic acids of the present invention, inserted into vectors that flank the nucleic acid insert with a phage promoter, such as T7, T3, or SP6 promoter, can be used to drive *in vitro* expression of RNA complementary to either strand of the nucleic acid of the present invention. The RNA can be used, *inter alia,* as a single-stranded probe, in cDNA-mRNA subtraction, or for *in vitro* translation.

As will be further discussed herein below, nucleic acids of the present invention that encode NHELP1 protein or portions thereof can be used, *inter alia,* to express the NHELP1 proteins or protein fragments, either alone, or as part of fusion proteins.

Expression can be from genomic nucleic acids of the present invention, or from transcript-derived nucleic acids of the present invention.

Where protein expression is effected from genomic DNA, expression will typically be effected in eukaryotic, typically mammalian, cells capable of splicing introns from the initial RNA transcript. Expression can be driven from episomal vectors, such as EBV-based vectors, or can be effected from genomic DNA integrated into a host cell chromosome. As will be more fully described below, where expression is from transcript-derived (or otherwise intron-less) nucleic acids of the present invention, expression can be effected in wide variety of prokaryotic or eukaryotic cells.

Expressed *in vitro,* the protein, protein fragment, or protein fusion can thereafter be isolated, to be used, *inter alia,* as a standard in immunoassays specific for the proteins, or protein isoforms, of the present invention; to be used as a therapeutic agent, *e.g.,* to be administered as passive replacement therapy in individuals deficient in the proteins of the present invention, or to be administered as a vaccine; to be used for *in vitro* production of specific antibody, the antibody thereafter to be used, *e.g.,* as an analytical reagent for detection and quantitation of the proteins of the present invention or to be used as an immunotherapeutic agent.

The isolated nucleic acids of the present invention can also be used to drive *in vivo* expression of the proteins of the present invention. *In vivo* expression can be driven from a vector - typically a viral vector, often a vector based upon a replication incompetent retrovirus, an adenovirus, or an adeno-associated virus (AAV) - for purpose of gene therapy. *In vivo* expression can also be driven from signals endogenous to the nucleic acid or from a vector, often a plasmid vector, such as pVAX1 (Invitrogen, Carlsbad CA, USA), for purpose of "naked" nucleic acid vaccination, as further described in U.S. Pat. Nos. 5,589,466; 5,679,647; 5,804,566; 5,830,877; 5,843,913; 5,880,104; 5,958,891; 5,985,847; 6,017,897; 6,110,898; 6,204,250, the disclosures of which are incorporated herein by reference in their entireties.

The nucleic acids of the present invention can also be used for antisense inhibition of transcription or translation. *See* Phillips (ed.), Antisense Technology, Part B, Methods in Enzymology Vol. 314, Academic Press, Inc. (1999) (ISBN: 012182215X); Phillips (ed.), Antisense Technology, Part A, Methods in Enzymology Vol. 313, Academic Press, Inc. (1999) (ISBN: 0121822141); Hartmann *et al.* (eds.), Manual of Antisense Methodology (Perspectives in Antisense Science), Kluwer Law International (1999) (ISBN:079238539X); Stein *et al.* (eds.), Applied Antisense Oligonucleotide Technology, Wiley-Liss (cover (1998) (ISBN: 0471172790); Agrawal *et al.* (eds.), Antisense Research and Application, Springer-Verlag New York, Inc. (1998) (ISBN: 3540638334); Lichtenstein *et al.* (eds.), Antisense Technology: A Practical Approach, Vol. 185, Oxford University Press, INC. (1998) (ISBN: 0199635838); Gibson (ed.), Antisense and Ribozyme Methodology: Laboratory Companion, Chapman & Hall (1997) (ISBN: 3826100794); Chadwick *et al.* (eds.), Oligonucleotides as Therapeutic Agents - Symposium No. 209, John Wiley & Son Ltd (1997) (ISBN: 0471972797), the disclosures of which are incorporated herein by reference in their entireties.

Nucleic acids of the present invention, particularly cDNAs of the present invention, that encode full-length human NHELP1 protein isoforms, have additional, well-recognized, immediate, real world utility as commercial products of manufacture suitable for sale.

For example, Invitrogen Corp. (Carlsbad, CA, USA), through its Research Genetics subsidiary, sells full length human cDNAs cloned into one of a selection of expression vectors as GeneStorm® expression-ready clones; utility is specific for the gene, since each gene is capable of being ordered separately and has a distinct catalogue number, and utility is substantial, each clone selling for $650.00 US. Similarly, Incyte Genomics (Palo Alto, CA, USA) sells clones from public and proprietary sources in multi-well plates or individual tubes.

Nucleic acids of the present invention that include genomic regions encoding the human NHELP1 protein, or portions thereof, have yet further utilities.

For example, genomic nucleic acids of the present invention can be used as amplification substrates, *e.g.* for preparation of genome-derived single exon probes of the present invention, as described above and in copending and commonly-owned U.S. patent application nos. 09/864,761, filed May 23, 2001, 09/774,203, filed January 29, 2001, and 09/632,366, filed August 3, 2000, the disclosures of which are incorporated herein by reference in their entireties.

As another example, genomic nucleic acids of the present invention can be integrated non-homologously into the genome of somatic cells, *e.g.* CHO cells, COS cells, or 293 cells, with or without amplification of the insertional locus, in order, *e.g*., to create stable cell lines capable of producing the proteins of the present invention.

As another example, more fully described herein below, genomic nucleic acids of the present invention can be integrated nonhomologously into embryonic stem (ES) cells to create transgenic non-human animals capable of producing the proteins of the present invention.

Genomic nucleic acids of the present invention can also be used to target homologous recombination to the human NHELP1 locus. *See, e.g.,* U.S. Patent Nos. 6,187,305; 6,204,061; 5,631,153; 5,627,059; 5,487,992; 5,464,764; 5,614,396; 5,527,695 and 6,063,630; and Kmiec *et al.* (eds.), Gene Targeting Protocols, Vol. 133, Humana Press (2000) (ISBN: 0896033600); Joyner (ed.), Gene Targeting: A Practical Approach, Oxford University Press, Inc. (2000) (ISBN: 0199637938); Sedivy *et al.,* Gene Targeting, Oxford University Press (1998) (ISBN: 071677013X); Tymms *et al.* (eds.), Gene Knockout Protocols, Humana Press (2000) (ISBN: 0896035727); Mak *et al.* (eds.), The Gene Knockout FactsBook, Vol. 2, Academic Press, Inc. (1998) (ISBN: 0124660444); Torres *et al.,* Laboratory Protocols for Conditional Gene Targeting, Oxford University Press (1997) (ISBN: 019963677X); Vega (ed.), Gene Targeting, CRC Press, LLC (1994) (ISBN: 084938950X), the disclosures of which are incorporated herein by reference in their entireties.

Where the genomic region includes transcription regulatory elements, homologous recombination can be used to alter the expression of NHELP1, both for purpose of *in vitro* production of NHELP1 protein from human cells, and for purpose of gene therapy. *See, e.g*., U.S. Pat. Nos. 5,981,214, 6,048,524; 5,272,071.

Fragments of the nucleic acids of the present invention smaller than those typically used for homologous recombination can also be used for targeted gene correction or alteration, possibly by cellular mechanisms different from those engaged during homologous recombination.

For example, partially duplexed RNA/DNA chimeras have been shown to have utility in targeted gene correction, U.S. Pat. Nos. 5,945,339, 5,888,983, 5,871,984, 5,795,972, 5,780,296, 5,760,012, 5,756,325, 5,731,181, the disclosures of which are incorporated herein by reference in their entireties. So too have small oligonucleotides fused to triplexing domains have been shown to have utility in targeted gene correction, Culver *et al.,* "Correction of chromosomal point mutations in human cells with bifunctional oligonucleotides," *Nature Biotechnol.* 17(10):989-93 (1999), as have oligonucleotides having modified terminal bases or modified terminal internucleoside bonds, Gamper *et al*., *Nucl. Acids Res.* 28(21):4332-9 (2000), the disclosures of which are incorporated herein by reference.

The isolated nucleic acids of the present invention can also be used to provide the initial substrate for recombinant engineering of NHELP1 protein variants having desired phenotypic improvements. Such engineering includes, for example, site-directed mutagenesis, random mutagenesis with subsequent functional screening, and more elegant schemes for recombinant evolution of proteins, as are described, *inter alia,* in U.S. Pat. Nos. 6,180,406; 6,165,793; 6,117,679; and 6,096,548, the disclosures of which are incorporated herein by reference in their entireties.

Nucleic acids of the present invention can be obtained by using the labeled probes of the present invention to probe nucleic acid samples, such as genomic libraries, cDNA libraries, and mRNA samples, by standard techniques. Nucleic acids of the present invention can also be obtained by amplification, using the nucleic acid primers of the present invention, as further demonstrated in Example 1, herein below. Nucleic acids of the present invention of fewer than about 100 nt can also be synthesized chemically, typically by solid phase synthesis using commercially available automated synthesizers.

### "Full Length" NHELP1 Nucleic Acids

In a first series of nucleic acid embodiments, the invention provides isolated nucleic acids that encode the entirety of the NHELP1 protein. As discussed above, the "full-length" nucleic acids of the present invention can be used, *inter alia,* to express full length NHELP1 protein. The full-length nucleic acids can also be used as nucleic acid probes; used as probes, the isolated nucleic acids of these embodiments will hybridize to NHELP1.

In a first such embodiment, the invention provides an isolated nucleic acid comprising (i) the nucleotide sequence of SEQ ID NO: 1, or (ii) the complement of (i). The SEQ ID NO: 1 presents the entire cDNA of NHELP1, including the 5' untranslated (UT) region and 3' UT.

In a second embodiment, the invention provides an isolated nucleic acid comprising (i) the nucleotide sequence of SEQ ID NO: 2, (ii) a degenerate variant of the nucleotide sequence of SEQ ID NO: 2, or (iii) the complement of (i) or (ii). SEQ ID NO: 2 presents the open reading frame (ORF) from SEQ ID NO: 1.

In a third embodiment, the invention provides an isolated nucleic acid comprising (i) a nucleotide sequence that encodes a polypeptide with the amino acid sequence of SEQ ID NO: 3 or (ii) the complement of a nucleotide sequence that encodes a polypeptide with the amino acid sequence of SEQ ID NO: 3. SEQ ID NO: 3 provides the amino acid sequence of NHELP1.

In a fourth embodiment, the invention provides an isolated nucleic acid having a nucleotide sequence that (i) encodes a polypeptide having the sequence of SEQ ID NO: 3, (ii) encodes a polypeptide having the sequence of SEQ ID NO: 3 with conservative amino acid substitutions, or (iii) that is the complement of (i) or (ii), where SEQ ID NO: 3 provides the amino acid sequence of NHELP1.

### Selected Partial Nucleic Acids

In a second series of nucleic acid embodiments, the invention provides isolated nucleic acids that encode select portions of NHELP1. As will be further discussed herein below, these "partial" nucleic acids can be used, *inter alia,* to express specific portions of the NHELP1. These "partial" nucleic acids can also be used, *inter alia,* as nucleic probes.

In a first such embodiment, the invention provides an isolated nucleic acid comprising (i) the nucleotide sequence of SEQ ID NO: 4, (ii) a degenerate variant of SEQ ID NO: 6, or (iii) the complement of (i) or (ii), wherein the isolated nucleic acid is no more than about 100 kb in length, typically no more than about 75 kb in length, more typically no more than about 50 kb length. SEQ ID NO: 6 encodes a novel portion of NHELP1. Often, the isolated nucleic acids of this embodiment are no more than about 25 kb in length, often no more than about 15 kb in length, and frequently no more than about 10 kb in length.

In another embodiment, the invention provides an isolated nucleic acid comprising (i) a nucleotide sequence that encodes SEQ ID NO: 7 or (ii) the complement of a nucleotide sequence that encodes SEQ ID NO: 7, wherein the isolated nucleic acid is no more than about 100 kb in length, typically no more than about 75 kb in length, frequently no more than about 50 kb in length. SEQ ID NO: 7 is the amino acid sequence encoded by the portion of NHELP1 not found in any EST fragments. Often, the isolated nucleic acids of this embodiment are no more than about 25 kb in length, often no more than about 15 kb in length, and frequently no more than about 10 kb in length.

In another embodiment, the invention provides an isolated nucleic acid comprising (i) a nucleotide sequence that encodes SEQ ID NO: 7, (ii) a nucleotide sequence that encodes SEQ ID NO: 7 with conservative substitutions, or (iii) the complement of (i) or (ii), wherein the isolated nucleic acid is no more than about 100 kb in length, typically no more than about 75 kb in length, and often no more than about 50 kb in length. Often, the isolated nucleic acids of this embodiment are no more than about 25 kb in length, often no more than about 15 kb in length, and frequently no more than about 10 kb in length.

### Cross-Hybridizing Nucleic Acids

In another series of nucleic acid embodiments, the invention provides isolated nucleic acids that hybridize to various of the NHELP1 nucleic acids of the present invention. These cross-hybridizing nucleic acids can be used, *inter alia,* as probes for, and to drive expression of, proteins that are related to NHELP1 of the present invention as further isoforms, homologues, paralogues, or orthologues.

In a first such embodiment, the invention provides an isolated nucleic acid comprising a sequence that hybridizes under high stringency conditions to a probe the nucleotide sequence of which consists of at least 17 nt, 18, 19, 20, 21, 22, 23, 24, 25, 30, 40, or 50 nt of SEQ ID NO: 4 or the complement of SEQ ID NO: 4, wherein the isolated nucleic acid is no more than about 100 kb in length, typically no more than about 75 kb in length, and often no more than about 50 kb in length. Often, the isolated nucleic acids of this embodiment are no more than about 25 kb in length, often no more than about 15 kb in length, and frequently no more than about 10 kb in length.

In a further embodiment, the invention provides an isolated nucleic acid comprising a sequence that hybridizes under moderate stringency conditions to a probe the nucleotide sequence of which consists of at least 17 nt, 18, 19, 20, 21, 22, 23, 24, 25, 30, 40, or 50 nt of SEQ ID NO: 4 or the complement of SEQ ID NO: 4, wherein the isolated nucleic acid is no more than about 100 kb in length, typically no more than about 75 kb in length, and often no more than about 50 kb in length. Often, the isolated nucleic acids of this embodiment are no more than about 25 kb in length, often no more than about 15 kb in length, and frequently no more than about 10 kb in length.

In a further embodiment, the invention provides an isolated nucleic acid comprising a sequence that hybridizes under high stringency conditions to a hybridization probe the nucleotide sequence of which (i) encodes a polypeptide having the sequence of SEQ ID NO: 7, (ii) encodes a polypeptide having the sequence of SEQ ID NO: 7 with conservative amino acid substitutions, or (iii) is the complement of (i) or (ii), wherein the isolated nucleic acid is no more than about 100 kb in length, typically no more than about 75 kb in length, and often no more than about 50 kb in length. Often, the isolated nucleic acids of this embodiment are no more than about 25 kb in length, often no more than about 15 kb in length, and frequently no more than about 10 kb in length.

### Particularly Useful Nucleic Acids

Particularly useful among the above-described nucleic acids are those that are expressed, or the complement of which are expressed, in brain, adult liver, adrenal, bone marrow, fetal liver, testis and prostate, as well as hela cell line.

Also particularly useful among the above-described nucleic acids are those that encode, or the complement of which encode, a polypeptide having Na⁺/H⁺ exchange activity.

Other particularly useful embodiments of the nucleic acids above-described are those that encode, or the complement of which encode, a polypeptide having a Na_H_Exchanger domain.

### Nucleic Acid Fragments

In another series of nucleic acid embodiments, the invention provides fragments of various of the isolated nucleic acids of the present invention which prove useful, *inter alia,* as nucleic acid probes, as amplification primers, and to direct expression or synthesis of epitopic or immunogenic protein fragments.

In a first such embodiment, the invention provides an isolated nucleic acid comprising at least 17 nucleotides, 18 nucleotides, 20 nucleotides, 24 nucleotides, or 25 nucleotides of (i) SEQ ID NO: 4, (ii) a degenerate variant of SEQ ID NO: 6, or (iii) the complement of (i) or (ii), wherein the isolated nucleic acid is no more than about 100 kb in length, typically no more than about 75 kb in length, more typically no more than about 50 kb in length. Often, the isolated nucleic acids of this embodiment are no more than about 25 kb in length, often no more than about 15 kb in length, and frequently no more than about 10 kb in length.

The invention also provides an isolated nucleic acid comprising (i) a nucleotide sequence that encodes a peptide of at least 8 contiguous amino acids of SEQ ID NO: 7, (ii) a nucleotide sequence that encodes a peptide of at least 15 contiguous amino acids of SEQ ID NO: 7, or (iii) the complement of (i) or (ii), wherein the isolated nucleic acid is no more than about 100 kb in length, typically no more than about 75 kb in length, more typically no more than about 50 kb in length. Often, the isolated nucleic acids of this embodiment are no more than about 25 kb in length, often no more than about 15 kb in length, and frequently no more than about 10 kb in length.

The invention also provides an isolated nucleic acid comprising a nucleotide sequence that encodes (i) a polypeptide having the sequence of at least 8 contiguous amino acids of SEQ ID NO: 7 with conservative amino acid substitutions, (ii) a polypeptide having the sequence of at least 15 contiguous amino acids of SEQ ID NO: 7 with conservative amino acid substitutions, (iii) a polypeptide having the sequence of at least 8 contiguous amino acids of SEQ ID NO: 7 with moderately conservative substitutions, (iv) a polypeptide having the sequence of at least 15 contiguous amino acids of SEQ ID NO: 7 with moderately conservative substitutions, or (v) the complement of any of (i) - (iv), wherein the isolated nucleic acid is no more than about 100 kb in length, typically no more than about 75 kb in length, more typically no more than about 50 kb in length. Often, the isolated nucleic acids of this embodiment are no more than about 25 kb in length, often no more than about 15 kb in length, and frequently no more than about 10 kb in length.

### Single Exon Probes

The invention further provides genome-derived single exon probes having portions of no more than one exon of the NHELP1 gene. As further described in commonly owned and copending U.S. patent application serial no. 09/632,366, filed August 3, 2000 ("Methods and Apparatus for High Throughput Detection and Characterization of alternatively Spliced Genes"), the disclosure of which is incorporated herein by reference in its entirety, such single exon probes have particular utility in identifying and characterizing splice variants. In particular, such single exon probes are useful for identifying and discriminating the expression of distinct isoforms of NHELP1.

In a first embodiment, the invention provides an isolated nucleic acid comprising a nucleotide sequence of no more than one portion of SEQ ID NOs: 8 - 23 or the complement of SEQ ID NOs: 8 - 23, wherein the portion comprises at least 17 contiguous nucleotides, 18 contiguous nucleotides, 20 contiguous nucleotides, 24 contiguous nucleotides, 25 contiguous nucleotides, or 50 contiguous nucleotides of any one of SEQ ID NOs: 8 - 23, or their complement. In a further embodiment, the exonic portion comprises the entirety of the referenced SEQ ID NO: or its complement.

In other embodiments, the invention provides isolated single exon probes having the nucleotide sequence of any one of SEQ ID NOs: 24 - 39.

### Transcription Control Nucleic Acids

In another aspect, the present invention provides genome-derived isolated nucleic acids that include nucleic acid sequence elements that control transcription of the NHELP1 gene. These nucleic acids can be used, *inter alia,* to drive expression of heterologous coding regions in recombinant constructs, thus conferring upon such heterologous coding regions the expression pattern of the native NHELP1 gene. These nucleic acids can also be used, conversely, to target heterologous transcription control elements to the NHELP1 genomic locus, altering the expression pattern of the NHELP1 gene itself.

In a first such embodiment, the invention provides an isolated nucleic acid comprising the nucleotide sequence of SEQ ID NO: 40 or its complement, wherein the isolated nucleic acid is no more than about 100 kb in length, typically no more than about 75 kb in length, more typically no more than about 50 kb in length. Often, the isolated nucleic acids of this embodiment are no more than about 25 kb in length, often no more than about 15 kb in length, and frequently no more than about 10 kb in length.

In another embodiment, the invention provides an isolated nucleic acid comprising at least 17, 18, 20, 24, or 25 nucleotides of the sequence of SEQ ID NO: 40 or its complement, wherein the isolated nucleic acid is no more than about 100 kb in length, typically no more than about 75 kb in length, more typically no more than about 50 kb in length. Often, the isolated nucleic acids of this embodiment are no more than about 25 kb in length, often no more than about 15 kb in length, and frequently no more than about 10 kb in length.

### VECTORS AND HOST CELLS

In another aspect, the present invention provides vectors that comprise one or more of the isolated nucleic acids of the present invention, and host cells in which such vectors have been introduced.

The vectors can be used, *inter alia,* for propagating the nucleic acids of the present invention in host cells (cloning vectors), for shuttling the nucleic acids of the present invention between host cells derived from disparate organisms (shuttle vectors), for inserting the nucleic acids of the present invention into host cell chromosomes (insertion vectors), for expressing sense or antisense RNA transcripts of the nucleic acids of the present invention *in vitro* or within a host cell, and for expressing polypeptides encoded by the nucleic acids of the present invention, alone or as fusions to heterologous polypeptides. Vectors of the present invention will often be suitable for several such uses.

Vectors are by now well-known in the art, and are described, *inter alia,* in Jones *et al.* (eds.), Vectors: Cloning Applications : Essential Techniques (Essential Techniques Series), John Wiley & Son Ltd 1998 (ISBN: 047196266X); Jones *et al.* (eds.), Vectors: Expression Systems : Essential Techniques (Essential Techniques Series), John Wiley & Son Ltd, 1998 (ISBN:0471962678); Gacesa *et al.,* Vectors: Essential Data, John Wiley & Sons, 1995 (ISBN: 0471948411); Cid-Arregui (eds.), Viral Vectors: Basic Science and Gene Therapy, Eaton Publishing Co., 2000 (ISBN: 188129935X); Sambrook *et al.,* Molecular Cloning: A Laboratory Manual (3^{rd} ed.), Cold Spring Harbor Laboratory Press, 2001 (ISBN: 0879695773); Ausubel *et al.* (eds.), Short Protocols in Molecular Biology: A Compendium of Methods from Current Protocols in Molecular Biology (4^{th} ed.), John Wiley & Sons, 1999 (ISBN: 047132938X), the disclosures of which are incorporated herein by reference in their entireties. Furthermore, an enormous variety of vectors are available commercially. Use of existing vectors and modifications thereof being well within the skill in the art, only basic features need be described here.

Typically, vectors are derived from virus, plasmid, prokaryotic or eukaryotic chromosomal elements, or some combination thereof, and include at least one origin of replication, at least one site for insertion of heterologous nucleic acid, typically in the form of a polylinker with multiple, tightly clustered, single cutting restriction sites, and at least one selectable marker, although some integrative vectors will lack an origin that is functional in the host to be chromosomally modified, and some vectors will lack selectable markers. Vectors of the present invention will further include at least one nucleic acid of the present invention inserted into the vector in at least one location.

Where present, the origin of replication and selectable markers are chosen based upon the desired host cell or host cells; the host cells, in turn, are selected based upon the desired application.

For example, prokaryotic cells, typically *E. coli,* are typically chosen for cloning. In such case, vector replication is predicated on the replication strategies of coliform-infecting phage - such as phage lambda, M13, T7, T3 and P1 - or on the replication origin of autonomously replicating episomes, notably the ColE1 plasmid and later derivatives, including pBR322 and the pUC series plasmids. Where *E. coli* is used as host, selectable markers are, analogously, chosen for selectivity in gram negative bacteria: *e.g.,* typical markers confer resistance to antibiotics, such as ampicillin, tetracycline, chloramphenicol, kanamycin, streptomycin, zeocin; auxotrophic markers can also be used.

As another example, yeast cells, typically *S*. *cerevisiae,* are chosen, *inter alia,* for eukaryotic genetic studies, due to the ease of targeting genetic changes by homologous recombination and to the ready ability to complement genetic defects using recombinantly expressed proteins, for identification of interacting protein components, *e.g.* through use of a two-hybrid system, and for protein expression. Vectors of the present invention for use in yeast will typically, but not invariably, contain an origin of replication suitable for use in yeast and a selectable marker that is functional in yeast.

Integrative YIp vectors do not replicate autonomously, but integrate, typically in single copy, into the yeast genome at low frequencies and thus replicate as part of the host cell chromosome; these vectors lack an origin of replication that is functional in yeast, although they typically have at least one origin of replication suitable for propagation of the vector in bacterial cells. YEp vectors, in contrast, replicate episomally and autonomously due to presence of the yeast 2 micron plasmid origin (2 µm ori). The YCp yeast centromere plasmid vectors are autonomously replicating vectors containing centromere sequences, CEN, and autonomously replicating sequences, ARS; the ARS sequences are believed to correspond to the natural replication origins of yeast chromosomes. YACs are based on yeast linear plasmids, denoted YLp, containing homologous or heterologous DNA sequences that function as telomeres (TEL) *in vivo,* as well as containing yeast ARS (origins of replication) and CEN (centromeres) segments.

Selectable markers in yeast vectors include a variety of auxotrophic markers, the most common of which are (in *Saccharomyces cerevisiae)* URA3, HIS3, LEU2, TRP1 and LYS2, which complement specific auxotrophic mutations, such as ura3-52, his3-D1, leu2-D1, trp1-D1 and lys2-201. The URA3 and LYS2 yeast genes further permit negative selection based on specific inhibitors, 5-fluoro-orotic acid (FOA) and α-aminoadipic acid (αAA), respectively, that prevent growth of the prototrophic strains but allows growth of the ura3 and lys2 mutants, respectively. Other selectable markers confer resistance to, *e.g.,* zeocin.

As yet another example, insect cells are often chosen for high efficiency protein expression. Where the host cells are from *Spodoptera frugiperda - e.g.,* Sf9 and Sf21 cell lines, and expresSF™ cells (Protein Sciences Corp., Meriden, CT, USA) - the vector replicative strategy is typically based upon the baculovirus life cycle. Typically, baculovirus transfer vectors are used to replace the wild-type AcMNPV polyhedrin gene with a heterologous gene of interest. Sequences that flank the polyhedrin gene in the wild-type genome are positioned 5' and 3' of the expression cassette on the transfer vectors. Following cotransfection with AcMNPV DNA, a homologous recombination event occurs between these sequences resulting in a recombinant virus carrying the gene of interest and the polyhedrin or p10 promoter. Selection can be based upon visual screening for lacZ fusion activity.

As yet another example, mammalian cells are often chosen for expression of proteins intended as pharmaceutical agents, and are also chosen as host cells for screening of potential agonist and antagonists of a protein or a physiological pathway.

Where mammalian cells are chosen as host cells, vectors intended for autonomous extrachromosomal replication will typically include a viral origin, such as the SV40 origin (for replication in cell lines expressing the large T-antigen, such as COS1 and COS7 cells), the papillomavirus origin, or the EBV origin for long term episomal replication (for use, *e.g*., in 293-EBNA cells, which constitutively express the EBV EBNA-1 gene product and adenovirus E1A). Vectors intended for integration, and thus replication as part of the mammalian chromosome, can, but need not, include an origin of replication functional in mammalian cells, such as the SV40 origin. Vectors based upon viruses, such as adenovirus, adeno-associated virus, vaccinia virus, and various mammalian retroviruses, will typically replicate according to the viral replicative strategy.

Selectable markers for use in mammalian cells include resistance to neomycin (G418), blasticidin, hygromycin and to zeocin, and selection based upon the purine salvage pathway using HAT medium.

Plant cells can also be used for expression, with the vector replicon typically derived from a plant virus *(e.g.,* cauliflower mosaic virus, CaMV; tobacco mosaic virus, TMV) and selectable markers chosen for suitability in plants.

For propagation of nucleic acids of the present invention that are larger than can readily be accomodated in vectors derived from plasmids or virus, the invention further provides artificial chromosomes - BACs, YACs, PACs, and HACs - that comprise NHELP1 nucleic acids, often genomic nucleic acids.

The BAC system is based on the well-characterized *E*. *coli* F-factor, a low copy plasmid that exists in a supercoiled circular form in host cells. The structural features of the F-factor allow stable maintenance of individual human DNA clones as well as easy manipulation of the cloned DNA. *See* Shizuya *et al., Keio J. Med.* 50(1):26-30 (2001); Shizuya *et al., Proc. Natl. Acad. Sci. USA* 89(18):8794-7 (1992).

YACs are based on yeast linear plasmids, denoted YLp, containing homologous or heterologous DNA sequences that function as telomeres (TEL) *in vivo,* as well as containing yeast ARS (origins of replication) and CEN (centromeres) segments.

HACs are human artifical chromosomes. Kuroiwa *et al*., *Nature Biotechnol.* 18(10):1086-90 (2000); Henning *et al*., *Proc. Natl. Acad. Sci. USA* 96(2):592-7 (1999); Harrington *et al., Nature Genet.* 15(4):345-55 (1997). In one version, long synthetic arrays of alpha satellite DNA are combined with telomeric DNA and genomic DNA to generate linear microchromosomes that are mitotically and cytogenetically stable in the absence of selection.

PACs are P1-derived artificial chromosomes. Sternberg, *Proc. Natl. Acad. Sci. USA* 87(1):103-7 (1990); Sternberg *et al., New Biol.* 2(2):151-62 (1990); Pierce *et al., Proc. Natl Acad. Sci. USA* 89 (6) :2056-60 (1992).

Vectors of the present invention will also often include elements that permit *in vitro* transcription of RNA from the inserted heterologous nucleic acid. Such vectors typically include a phage promoter, such as that from T7, T3, or SP6, flanking the nucleic acid insert. Often two different such promoters flank the inserted nucleic acid, permitting separate *in vitro* production of both sense and antisense strands.

Expression vectors of the present invention - that is, those vectors that will drive expression of polypeptides from the inserted heterologous nucleic acid - will often include a variety of other genetic elements operatively linked to the protein-encoding heterologous nucleic acid insert, typically genetic elements that drive transcription, such as promoters and enhancer elements, those that facilitate RNA processing, such as transcription termination and/or polyadenylation signals, and those that facilitate translation, such as ribosomal consensus sequences.

For example, vectors for expressing proteins of the present invention in prokaryotic cells, typically *E. coli,* will include a promoter, often a phage promoter, such as phage lambda pL promoter, the trc promoter, a hybrid derived from the trp and lac promoters, the bacteriophage T7 promoter (in *E. coli* cells engineered to express the T7 polymerase), or the araBAD operon. Often, such prokaryotic expression vectors will further include transcription terminators, such as the aspA terminator, and elements that facilitate translation, such as a consensus ribosome binding site and translation termination codon, Schomer *et al*., *Proc. Natl. Acad. Sci. USA* 83:8506-8510 (1986).

As another example, vectors for expressing proteins of the present invention in yeast cells, typically *S. cerevisiae,* will include a yeast promoter, such as the CYC1 promoter, the GAL1 promoter, ADH1 promoter, or the GPD promoter, and will typically have elements that facilitate transcription termination, such as the transcription termination signals from the CYC1 or ADH1 gene.

As another example, vectors for expressing proteins of the present invention in mammalian cells will include a promoter active in mammalian cells. Such promoters are often drawn from mammalian viruses - such as the enhancer-promoter sequences from the immediate early gene of the human cytomegalovirus (CMV), the enhancer-promoter sequences from the Rous sarcoma virus long terminal repeat (RSV LTR), and the enhancer-promoter from SV40. Often, expression is enhanced by incorporation of polyadenylation sites, such as the late SV40 polyadenylation site and the polyadenylation signal and transcription termination sequences from the bovine growth hormone (BGH) gene, and ribosome binding sites. Furthermore, vectors can include introns, such as intron II of rabbit β-globin gene and the SV40 splice elements.

Vector-drive protein expression can be constitutive or inducible.

Inducible vectors include either naturally inducible promoters, such as the trc promoter, which is regulated by the lac operon, and the pL promoter, which is regulated by tryptophan, the MMTV-LTR promoter, which is inducible by dexamethasone, or can contain synthetic promoters and/or additional elements that confer inducible control on adjacent promoters. Examples of inducible synthetic promoters are the hybrid Plac/ara-1 promoter and the PLtetO-1 promoter. The PltetO-1 promoter takes advantage of the high expression levels from the PL promoter of phage lambda, but replaces the lambda repressor sites with two copies of operator 2 of the Tn10 tetracycline resistance operon, causing this promoter to be tightly repressed by the Tet repressor protein and induced in response to tetracycline (Tc) and Tc derivatives such as anhydrotetracycline.

As another example of inducible elements, hormone response elements, such as the glucocorticoid response element (GRE) and the estrogen response element (ERE), can confer hormone inducibility where vectors are used for expression in cells having the respective hormone receptors. To reduce background levels of expression, elements responsive to ecdysone, an insect hormone, can be used instead, with coexpression of the ecdysone receptor.

Expression vectors can be designed to fuse the expressed polypeptide to small protein tags that facilitate purification and/or visualization.

For example, proteins of the present invention can be expressed with a polyhistidine tag that facilitates purification of the fusion protein by immobilized metal affinity chromatography, for example using NiNTA resin (Qiagen Inc., Valencia, CA, USA) or TALON™ resin (cobalt immobilized affinity chromatography medium, Clontech Labs, Palo Alto, CA, USA). As another example, the fusion protein can include a chitin-binding tag and self-excising intein, permitting chitin-based purification with self-removal of the fused tag (IMPACT™ system, New England Biolabs, Inc., Beverley, MA, USA). Alternatively, the fusion protein can include a calmodulin-binding peptide tag, permitting purification by calmodulin affinity resin (Stratagene, La Jolla, CA, USA), or a specifically excisable fragment of the biotin carboxylase carrier protein, permitting purification of *in vivo* biotinylated protein using an avidin resin and subsequent tag removal (Promega, Madison, WI, USA). As another useful alternative, the proteins of the present invention can be expressed as a fusion to glutathione-S-transferase, the affinity and specificity of binding to glutathione permitting purification using glutathione affinity resins, such as Glutathione-Superflow Resin (Clontech Laboratories, Palo Alto, CA, USA), with subsequent elution with free glutathione.

Other tags include, for example, the Xpress epitope, detectable by anti-Xpress antibody (Invitrogen, Carlsbad, CA, USA), a myc tag, detectable by anti-myc tag antibody, the V5 epitope, detectable by anti-V5 antibody (Invitrogen, Carlsbad, CA, USA), FLAG® epitope, detectable by anti-FLAG® antibody (Stratagene, La Jolla, CA, USA), and the HA epitope.

For secretion of expressed proteins, vectors can include appropriate sequences that encode secretion signals, such as leader peptides. For example, the pSecTag2 vectors (Invitrogen, Carlsbad, CA, USA) are 5.2 kb mammalian expression vectors that carry the secretion signal from the V-J2-C region of the mouse Ig kappa-chain for efficient secretion of recombinant proteins from a variety of mammalian cell lines.

Expression vectors can also be designed to fuse proteins encoded by the heterologous nucleic acid insert to polypeptides larger than purification and/or identification tags. Useful protein fusions include those that permit display of the encoded protein on the surface of a phage or cell, fusions to intrinsically fluorescent proteins, such as those that have a green fluorescent protein (GFP)-like chromophore, fusions to the IgG Fc region, and fusions for use in two hybrid systems.

Vectors for phage display fuse the encoded polypeptide to, *e.g.,* the gene III protein (pIII) or gene VIII protein (pVIII) for display on the surface of filamentous phage, such as M13. *See* Barbas *et al.,* Phage Display: A Laboratory Manual, Cold Spring Harbor Laboratory Press (2001) (ISBN 0-87969-546-3); Kay *et al.* (eds.), Phage Display of Peptides and Proteins: A Laboratory Manual, San Diego: Academic Press, Inc., 1996; Abelson *et al.* (eds.), Combinatorial Chemistry, Methods in Enzymology vol. 267, Academic Press (May 1996).

Vectors for yeast display, *e.g.* the pYD1 yeast display vector (Invitrogen, Carlsbad, CA, USA), use the α-agglutinin yeast adhesion receptor to display recombinant protein on the surface of *S. cerevisiae.* Vectors for mammalian display, *e.g*., the pDisplay™ vector (Invitrogen, Carlsbad, CA, USA), target recombinant proteins using an N-terminal cell surface targeting signal and a C-terminal transmembrane anchoring domain of platelet derived growth factor receptor.

A wide variety of vectors now exist that fuse proteins encoded by heterologous nucleic acids to the chromophore of the substrate-independent, intrinsically fluorescent green fluorescent protein from *Aequorea victoria* ("GFP") and its variants. These proteins are intrinsically fluorescent: the GFP-like chromophore is entirely encoded by its amino acid sequence and can fluoresce without requirement for cofactor or substrate.

Structurally, the GFP-like chromophore comprises an 11-stranded β-barrel (β-can) with a central α-helix, the central α-helix having a conjugated π-resonance system that includes two aromatic ring systems and the bridge between them. The π-resonance system is created by autocatalytic cyclization among amino acids; cyclization proceeds through an imidazolinone intermediate, with subsequent dehydrogenation by molecular oxygen at the Cα-Cβ bond of a participating tyrosine.

The GFP-like chromophore can be selected from GFP-like chromophores found in naturally occurring proteins, such as *A*. *victoria* GFP (GenBank accession number AAA27721), *Renilla reniformis* GFP, FP583 (GenBank accession no. AF168419) (DsRed), FP593 (AF272711), FP483 (AF168420), FP484 (AF168424), FP595 (AF246709), FP486 (AF168421), FP538 (AF168423), and FP506 (AF168422), and need include only so much of the native protein as is needed to retain the chromophore's intrinsic fluorescence. Methods for determining the minimal domain required for fluorescence are known in the art. Li *et al*.,"Deletions of the *Aequorea victoria* Green Fluorescent Protein Define the Minimal Domain Required for Fluorescence," J. *Biol. Chem.* 272:28545-28549 (1997).

Alternatively, the GFP-like chromophore can be selected from GFP-like chromophores modified from those found in nature. Typically, such modifications are made to improve recombinant production in heterologous expression systems (with or without change in protein sequence), to alter the excitation and/or emission spectra of the native protein, to facilitate purification, to facilitate or as a consequence of cloning, or are a fortuitous consequence of research investigation.

The methods for engineering such modified GFP-like chromophores and testing them for fluorescence activity, both alone and as part of protein fusions, are well-known in the art. Early results of these efforts are reviewed in Heim *et al., Curr. Biol.* 6:178-182 (1996), incorporated herein by reference in its entirety; a more recent review, with tabulation of useful mutations, is found in Palm *et al.,* "Spectral Variants of Green Fluorescent Protein," in Green Fluorescent Proteins, Conn (ed.), *Methods Enzymol.* vol. 302, pp. 378 - 394 (1999), incorporated herein by reference in its entirety. A variety of such modified chromophores are now commercially available and can readily be used in the fusion proteins of the present invention.

For example, EGFP ("enhanced GFP"), Cormack *et al., Gene* 173:33-38 (1996); U.S. Pat. Nos. 6,090,919 and 5,804,387, is a red-shifted, human codon-optimized variant of GFP that has been engineered for brighter fluorescence, higher expression in mammalian cells, and for an excitation spectrum optimized for use in flow cytometers. EGFP can usefully contribute a GFP-like chromophore to the fusion proteins of the present invention. A variety of EGFP vectors, both plasmid and viral, are available commercially (Clontech Labs, Palo Alto, CA, USA), including vectors for bacterial expression, vectors for N-terminal protein fusion expression, vectors for expression of C-terminal protein fusions, and for bicistronic expression.

Toward the other end of the emission spectrum, EBFP ("enhanced blue fluorescent protein") and BFP2 contain four amino acid substitutions that shift the emission from green to blue, enhance the brightness of fluorescence and improve solubility of the protein, Heim *et al., Curr. Biol.* 6:178-182 (1996); Cormack *et al., Gene* 173:33-38 (1996). EBFP is optimized for expression in mammalian cells whereas BFP2, which retains the original jellyfish codons, can be expressed in bacteria; as is further discussed below, the host cell of production does not affect the utility of the resulting fusion protein. The GFP-like chromophores from EBFP and BFP2 can usefully be included in the fusion proteins of the present invention, and vectors containing these blue-shifted variants are available from Clontech Labs (Palo Alto, CA, USA).

Analogously, EYFP ("enhanced yellow fluorescent protein"), also available from Clontech Labs, contains four amino acid substitutions, different from EBFP, Ormö *et al*., *Science* 273:1392-1395 (1996), that shift the emission from green to yellowish-green. Citrine, an improved yellow fluorescent protein mutant, is described in Heikal *et al*., *Proc. Natl. Acad. Sci. USA* 97:11996-12001 (2000). ECFP ("enhanced cyan fluorescent protein") (Clontech Labs, Palo Alto, CA, USA) contains six amino acid substitutions, one of which shifts the emission spectrum from green to cyan. Heim *et al., Curr. Biol.* 6:178-182 (1996); Miyawaki *et al., Nature* 388:882-887 (1997). The GFP-like chromophore of each of these GFP variants can usefully be included in the fusion proteins of the present invention.

The GFP-like chromophore can also be drawn from other modified GFPs, including those described in U.S. Pat. Nos. 6,124,128; 6,096,865; 6,090,919; 6,066,476; 6,054,321; 6,027,881; 5,968,750; 5,874,304; 5,804,387; 5,777,079; 5,741,668; and 5,625,048, the disclosures of which are incorporated herein by reference in their entireties. See also Conn (ed.), Green Fluorescent Protein, *Methods in Enzymol.* Vol. 302, pp 378-394 (1999), incorporated herein by reference in its entirety. A variety of such modified chromophores are now commercially available and can readily be used in the fusion proteins of the present invention.

Fusions to the IgG Fc region increase serum half life of protein pharmaceutical products through interaction with the FcRn receptor (also denominated the FcRp receptor and the Brambell receptor, FcRb), further described in international patent application nos. WO 97/43316, WO 97/34631, WO 96/32478, WO 96/18412.

For long-term, high-yield recombinant production of the proteins, protein fusions, and protein fragments of the present invention, stable expression is particularly useful.

Stable expression is readily achieved by integration into the host cell genome of vectors having selectable markers, followed by selection for integrants.

For example, the pUB6/V5-His A, B, and C vectors (Invitrogen, Carlsbad, CA, USA) are designed for high-level stable expression of heterologous proteins in a wide range of mammalian tissue types and cell lines. pUB6/V5-His uses the promoter/enhancer sequence from the human ubiquitin C gene to drive expression of recombinant proteins: expression levels in 293, CHO, and NIH3T3 cells are comparable to levels from the CMV and human EF-1a promoters. The bsd gene permits rapid selection of stably transfected mammalian cells with the potent antibiotic blasticidin.

Replication incompetent retroviral vectors, typically derived from Moloney murine leukemia virus, prove particularly useful for creating stable transfectants having integrated provirus. The highly efficient transduction machinery of retroviruses, coupled with the availability of a variety of packaging cell lines - such as RetroPack™ PT 67, EcoPack2™ -293, AmphoPack-293, GP2-293 cell lines (all available from Clontech Laboratories, Palo Alto, CA, USA) - allow a wide host range to be infected with high efficiency; varying the multiplicity of infection readily adjusts the copy number of the integrated provirus. Retroviral vectors are available with a variety of selectable markers, such as resistance to neomycin, hygromycin, and puromycin, permitting ready selection of stable integrants.

The present invention further includes host cells comprising the vectors of the present invention, either present episomally within the cell or integrated, in whole or in part, into the host cell chromosome.

Among other considerations, some of which are described above, a host cell strain may be chosen for its ability to process the expressed protein in the desired fashion. Such post-translational modifications of the polypeptide include, but are not limited to, acetylation, carboxylation, glycosylation, phosphorylation, lipidation, and acylation, and it is an aspect of the present invention to provide NHELP1 proteins with such post-translational modifications.

As noted earlier, host cells can be prokaryotic or eukaryotic. Representative examples of appropriate host cells include, but are not limited to, bacterial cells, such as *E*. *coli, Caulobacter crescentus, Streptomyces* species, and *Salmonella typhimurium;* yeast cells, such as *Saccharomyces cerevisiae, Schizosaccharomyces pombe, Pichia pastoris, Pichia methanolica;* insect cell lines, such as those from *Spodoptera frugiperda - e.g.,* Sf9 and Sf21 cell lines, and expresSF™ cells (Protein Sciences Corp., Meriden, CT, USA) - *Drosophila* S2 cells, and *Trichoplusia ni* High Five® Cells (Invitrogen, Carlsbad, CA, USA); and mammalian cells. Typical mammalian cells include COS1 and COS7 cells, chinese hamster ovary (CHO) cells, NIH 3T3 cells, 293 cells, HEPG2 cells, HeLa cells, L cells, murine ES cell lines (*e.g.,* from strains 129/SV, C57/BL6, DBA-1, 129/SVJ), K562, Jurkat cells, and BW5147. Other mammalian cell lines are well known and readily available from the American Type Culture Collection (ATCC) (Manassas, VA, USA) and the National Institute of General medical Sciences (NIGMS) Human Genetic Cell Repository at the Coriell Cell Repositories (Camden, NJ, USA).

Methods for introducing the vectors and nucleic acids of the present invention into the host cells are well known in the art; the choice of technique will depend primarily upon the specific vector to be introduced and the host cell chosen.

For example, phage lambda vectors will typically be packaged using a packaging extract *(e.g.,* Gigapack® packaging extract, Stratagene, La Jolla, CA, USA), and the packaged virus used to infect *E. coli.* Plasmid vectors will typically be introduced into chemically competent or electrocompetent bacterial cells.

*E. coli* cells can be rendered chemically competent by treatment, *e.g.*, with CaCl₂, or a solution of Mg²⁺, Mn²⁺, Ca²⁺, Rb⁺ or K⁺, dimethyl sulfoxide, dithiothreitol, and hexamine cobalt (III), Hanahan, *J. Mol. Biol.* 166(4):557-80 (1983), and vectors introduced by heat shock. A wide variety of chemically competent strains are also available commercially (*e.g*., Epicurian Coli® XL10-Gold® Ultracompetent Cells (Stratagene, La Jolla, CA, USA); DH5α competent cells (Clontech Laboratories, Palo Alto, CA, USA); TOP10 Chemically Competent E. coli Kit (Invitrogen, Carlsbad, CA, USA)).

Bacterial cells can be rendered electrocompetent - that is, competent to take up exogenous DNA by electroporation - by various pre-pulse treatments; vectors are introduced by electroporation followed by subsequent outgrowth in selected media. An extensive series of protocols is provided online in Electroprotocols (BioRad, Richmond, CA, USA) (http://www.bio-rad.com/LifeScience/pdf/New_Gene_Pulser.pdf).

Vectors can be introduced into yeast cells by spheroplasting, treatment with lithium salts, electroporation, or protoplast fusion.

Spheroplasts are prepared by the action of hydrolytic enzymes - a snail-gut extract, usually denoted Glusulase, or Zymolyase, an enzyme from *Arthrobacter luteus -* to remove portions of the cell wall in the presence of osmotic stabilizers, typically 1 M sorbitol. DNA is added to the spheroplasts, and the mixture is co-precipitated with a solution of polyethylene glycol (PEG) and Ca²⁺. Subsequently, the cells are resuspended in a solution of sorbitol, mixed with molten agar and then layered on the surface of a selective plate containing sorbitol. For lithium-mediated transformation, yeast cells are treated with lithium acetate, which apparently permeabilizes the cell wall, DNA is added and the cells are co-precipitated with PEG. The cells are exposed to a brief heat shock, washed free of PEG and lithium acetate, and subsequently spread on plates containing ordinary selective medium. Increased frequencies of transformation are obtained by using specially-prepared single-stranded carrier DNA and certain organic solvents. Schiestl *et al., Curr. Genet.* 16(5-6):339-46 (1989). For electroporation, freshly-grown yeast cultures are typically washed, suspended in an osmotic protectant, such as sorbitol, mixed with DNA, and the cell suspension pulsed in an electroporation device. Subsequently, the cells are spread on the surface of plates containing selective media. Becker *et al., Methods Enzymol.* 194:182-7 (1991). The efficiency of transformation by electroporation can be increased over 100-fold by using PEG, single-stranded carrier DNA and cells that are in late log-phase of growth. Larger constructs, such as YACs, can be introduced by protoplast fusion.

Mammalian and insect cells can be directly infected by packaged viral vectors, or transfected by chemical or electrical means.

For chemical transfection, DNA can be coprecipitated with CaPO₄ or introduced using liposomal and nonliposomal lipid-based agents. Commercial kits are available for CaPO₄ transfection (CalPhos™ Mammalian Transfection Kit, Clontech Laboratories, Palo Alto, CA, USA), and lipid-mediated transfection can be practiced using commercial reagents, such as LIPOFECTAMINE™ 2000, LIPOFECTAMINE™ Reagent, CELLFECTIN® Reagent, and LIPOFECTIN® Reagent (Invitrogen, Carlsbad, CA, USA), DOTAP Liposomal Transfection Reagent, FuGENE 6, X-tremeGENE Q2, DOSPER, (Roche Molecular Biochemicals, Indianapolis, IN USA), Effectene™, PolyFect®, Superfect® (Qiagen, Inc., Valencia, CA, USA). Protocols for electroporating mammalian cells can be found online in Electroprotocols (Bio-Rad, Richmond, CA, USA) (http://www.bio-rad.com/LifeScience/pdf/New_Gene_Pulser.pdf). *See also,* Norton *et al.* (eds.), Gene Transfer Methods: Introducing DNA into Living Cells and Organisms, BioTechniques Books, Eaton Publishing Co. (2000) (ISBN 1-881299-34-1), incorporated herein by reference in its entirety.

Other transfection techniques include transfection by particle embardment. *See, e.g.,* Cheng *et al., Proc. Natl. Acad. Sci. USA* 90(10):4455-9 (1993); Yang *et al., Proc. Natl. Acad. Sci. USA* 87(24):9568-72 (1990).

### PROTEINS

In another aspect, the present invention provides NHELP1 proteins, various fragments thereof suitable for use as antigens (*e.g*., for epitope mapping) and for use as immunogens (e.g., for raising antibodies or as vaccines), fusions of NHELP1 polypeptides and fragments to heterologous polypeptides, and conjugates of the proteins, fragments, and fusions of the present invention to other moieties (*e.g.,* to carrier proteins, to fluorophores).

FIG. 3 presents the predicted amino acid sequences encoded by the NHELP1 cDNA clone. The amino acid sequence is further presented in SEQ ID NO: 3.

Unless otherwise indicated, amino acid sequences of the proteins of the present invention were determined as a predicted translation from a nucleic acid sequence. Accordingly, any amino acid sequence presented herein may contain errors due to errors in the nucleic acid sequence, as described in detail above. Furthermore, single nucleotide polymorphisms (SNPs) occur frequently in eukaryotic genomes - more than 1.4 million SNPs have already identified in the human genome, International Human Genome Sequencing Consortium, *Nature* 409:860 - 921 (2001) - and the sequence determined from one individual of a species may differ from other allelic forms present within the population. Small deletions and insertions can often be found that do not alter the function of the protein.

Accordingly, it is an aspect of the present invention to provide proteins not only identical in sequence to those described with particularity herein, but also to provide isolated proteins at least about 65% identical in sequence to those described with particularity herein, typically at least about 70%, 75%, 80%, 85%, or 90% identical in sequence to those described with particularity herein, usefully at least about 91%, 92%, 93%, 94%, or 95% identical in sequence to those described with particularity herein, usefully at least about 96%, 97%, 98%, or 99% identical in sequence to those described with particularity herein, and, most conservatively, at least about 99.5%, 99.6%, 99.7%, 99.8% and 99.9% identical in sequence to those described with particularity herein. These sequence variants can be naturally occurring or can result from human intervention by way of random or directed mutagenesis.

For purposes herein, percent identity of two amino acid sequences is determined using the procedure of Tatiana *et al.,* "Blast 2 sequences - a new tool for comparing protein and nucleotide sequences", *FEMS Microbiol Lett.* 174:247-250 (1999), which procedure is effectuated by the computer program BLAST 2 SEQUENCES, available online at
http://www.ncbi.nlm.nih.gov/blast/bl2seq/bl2.html,
To assess percent identity of amino acid sequences, the BLASTP module of BLAST 2 SEQUENCES is used with default values of (i) BLOSUM62 matrix, Henikoff *et al., Proc. Natl. Acad. Sci USA* 89(22):10915-9 (1992); (ii) open gap 11 and extension gap 1 penalties; and (iii) gap x_dropoff 50 expect 10 word size 3 filter, and both sequences are entered in their entireties.

As is well known, amino acid substitutions occur frequently among natural allelic variants, with conservative substitutions often occasioning only *de minimis* change in protein function.

Accordingly, it is an aspect of the present invention to provide proteins not only identical in sequence to those described with particularity herein, but also to provide isolated proteins having the sequence of NHELP1 proteins, or portions thereof, with conservative amino acid substitutions. It is a further aspect to provide isolated proteins having the sequence of NHELP1 proteins, and portions thereof, with moderately conservative amino acid substitutions. These conservatively-substituted and moderately conservatively-substituted variants can be naturally occurring or can result from human intervention.

Although there are a variety of metrics for calling conservative amino acid substitutions, based primarily on either observed changes among evolutionarily related proteins or on predicted chemical similarity, for purposes herein a conservative replacement is any change having a positive value in the PAM250 log-likelihood matrix reproduced herein below *(see* Gonnet *et al., Science* 256(5062):1443-5 (1992)):

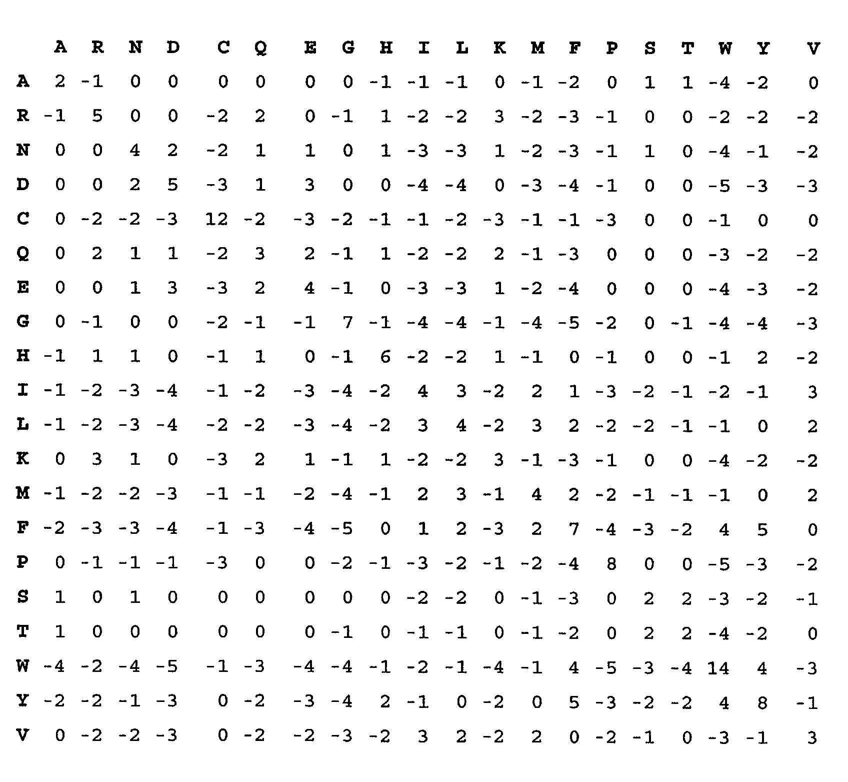

For purposes herein, a "moderately conservative" replacement is any change having a nonnegative value in the PAM250 log-likelihood matrix reproduced herein above.

As is also well known in the art, relatedness of proteins can also be characterized using a functional test, the ability of the encoding nucleic acids to base-pair to one another at defined hybridization stringencies.

It is, therefore, another aspect of the invention to provide isolated proteins not only identical in sequence to those described with particularity herein, but also to provide isolated proteins ("hybridization related proteins") that are encoded by nucleic acids that hybridize under high stringency conditions (as defined herein above) to all or to a portion of various of the isolated nucleic acids of the present invention ("reference nucleic acids"). It is a further aspect of the invention to provide isolated proteins ("hybridization related proteins") that are encoded by nucleic acids that hybridize under moderate stringency conditions (as defined herein above) to all or to a portion of various of the isolated nucleic acids of the present invention ("reference nucleic acids").

The hybridization related proteins can be alternative isoforms, homologues, paralogues, and orthologues of the NHELP1 protein of the present invention. Particularly useful orthologues are those from other primate species, such as chimpanzee, rhesus macaque monkey, baboon, orangutan, and gorilla, from rodents, such as rats, mice, guinea pigs; from lagomorphs, such as rabbits, and from domestic livestock, such as cow, pig, sheep, horse, and goat.

Relatedness of proteins can also be characterized using a second functional test, the ability of a first protein competitively to inhibit the binding of a second protein to an antibody.

It is, therefore, another aspect of the present invention to provide isolated proteins not only identical in sequence to those described with particularity herein, but also to provide isolated proteins ("cross-reactive proteins") that competitively inhibit the binding of antibodies to all or to a portion of various of the isolated NHELP1 proteins of the present invention ("reference proteins"). Such competitive inhibition can readily be determined using immunoassays well known in the art.

Among the proteins of the present invention that differ in amino acid sequence from those described with particularity herein - including those that have deletions and insertions causing up to 10% non-identity, those having conservative or moderately conservative substitutions, hybridization related proteins, and cross-reactive proteins - those that substantially retain one or more NHELP1 activities are particularly useful. As described above, those activities include Na+/H+ exchange.

Residues that are tolerant of change while retaining function can be identified by altering the protein at known residues using methods known in the art, such as alanine scanning mutagenesis, Cunningham *et al*., *Science* 244(4908):1081-5 (1989); transposon linker scanning mutagenesis, Chen *et al*., *Gene* 263(1-2):39-48 (2001); combinations of homolog- and alanine-scanning mutagenesis, Jin *et al.,* J. Mol. Biol. 226(3):851-65 (1992); combinatorial alanine scanning, Weiss *et al.,* Proc. Natl. Acad. Sci USA 97(16):8950-4 (2000), followed by functional assay. Transposon linker scanning kits are available commercially (New England Biolabs, Beverly, MA, USA, catalog. no. E7-102S; EZ::TN™ In-Frame Linker Insertion Kit, catalogue no. EZI04KM, Epicentre Technologies Corporation, Madison, WI, USA).

As further described below, the isolated proteins of the present invention can readily be used as specific immunogens to raise antibodies that specifically recognize NHELP1 proteins, their isoforms, homologues, paralogues, and/or orthologues. The antibodies, in turn, can be used, *inter alia,* specifically to assay for the NHELP1 proteins of the present invention - *e.g.* by ELISA for detection of protein fluid samples, such as serum, by immunohistochemistry or laser scanning cytometry, for detection of protein in tissue samples, or by flow cytometry, for detection of intracellular protein in cell suspensions - for specific antibody-mediated isolation and/or purification of NHELP1 proteins, as for example by immunoprecipitation, and for use as specific agonists or antagonists of NHELP1 action.

The isolated proteins of the present invention are also immediately available for use as specific standards in assays used to determine the concentration and/or amount specifically of the NHELP1 proteins of the present invention. As is well known, ELISA kits for detection and quantitation of protein analytes typically include isolated and purified protein of known concentration for use as a measurement standard (*e.g*., the human interferon-γ OptEIA kit, catalog no. 555142, Pharmingen, San Diego, CA, USA includes human recombinant gamma interferon, baculovirus produced).

The isolated proteins of the present invention are also immediately available for use as specific biomolecule capture probes for surface-enhanced laser desorption ionization (SELDI) detection of protein-protein interactions, WO 98/59362; WO 98/59360; WO 98/59361; and Merchant *et al., Electrophoresis* 21(6):1164-77 (2000), the disclosures of which are incorporated herein by reference in their entireties. Analogously, the isolated proteins of the present invention are also immediately available for use as specific biomolecule capture probes on BIACORE surface plasmon resonance probes. . *See* Weinberger *et al*., *Pharmacogenomics* 1(4):395-416 (2000); Malmqvist, *Biochem. Soc. Trans.* 27(2):335-40 (1999).

The isolated proteins of the present invention are also useful as a therapeutic supplement in patients having a specific deficiency in NHELP1 production.

In another aspect, the invention also provides fragments of various of the proteins of the present invention. The protein fragments are useful, *inter alia,* as antigenic and immunogenic fragments of NHELP1.

By "fragments" of a protein is here intended isolated proteins (equally, polypeptides, peptides, oligopeptides), however obtained, that have an amino acid sequence identical to a portion of the reference amino acid sequence, which portion is at least 6 amino acids and less than the entirety of the reference nucleic acid. As so defined, "fragments" need not be obtained by physical fragmentation of the reference protein, although such provenance is not thereby precluded.

Fragments of at least 6 contiguous amino acids are useful in mapping B cell and T cell epitopes of the reference protein. *See, e.g.,* Geysen *et al*., "Use of peptide synthesis to probe viral antigens for epitopes to a resolution of a single amino acid," *Proc. Natl. Acad. Sci. USA* 81:3998-4002 (1984) and U.S. Pat. Nos. 4,708,871 and 5,595,915, the disclosures of which are incorporated herein by reference in their entireties. Because the fragment need not itself be immunogenic, part of an immunodominant epitope, nor even recognized by native antibody, to be useful in such epitope mapping, all fragments of at least 6 amino acids of the proteins of the present invention have utility in such a study.

Fragments of at least 8 contiguous amino acids, often at least 15 contiguous amino acids, have utility as immunogens for raising antibodies that recognize the proteins of the present invention. *See, e.g.,* Lerner, "Tapping the immunological repertoire to produce antibodies of predetermined specificity," Nature 299:592-596 (1982); Shinnick *et al*., "Synthetic peptide immunogens as vaccines," *Annu. Rev. Microbiol.* 37:425-46 (1983); Sutcliffe *et al*., "Antibodies that react with predetermined sites on proteins," *Science* 219:660-6 (1983), the disclosures of which are incorporated herein by reference in their entireties. As further described in the above-cited references, virtually all 8-mers, conjugated to a carrier, such as a protein, prove immunogenic - that is, prove capable of eliciting antibody for the conjugated peptide; accordingly, all fragments of at least 8 amino acids of the proteins of the present invention have utility as immunogens.

Fragments of at least 8, 9, 10 or 12 contiguous amino acids are also useful as competitive inhibitors of binding of the entire protein, or a portion thereof, to antibodies (as in epitope mapping), and to natural binding partners, such as subunits in a multimeric complex or to receptors or ligands of the subject protein; this competitive inhibition permits identification and separation of molecules that bind specifically to the protein of interest, U.S. Pat. Nos. 5,539,084 and 5,783,674, incorporated herein by reference in their entireties.

The protein, or protein fragment, of the present invention is thus at least 6 amino acids in length, typically at least 8, 9, 10 or 12 amino acids in length, and often at least 15 amino acids in length. Often, the protein or the present invention, or fragment thereof, is at least 20 amino acids in length, even 25 amino acids, 30 amino acids, 35 amino acids, or 50 amino acids or more in length. Of course, larger fragments having at least 75 amino acids, 100 amino acids, or even 150 amino acids are also useful, and at times preferred.

The present invention further provides fusions of each of the proteins and protein fragments of the present invention to heterologous polypeptides.

By fusion is here intended that the protein or protein fragment of the present invention is linearly contiguous to the heterologous polypeptide in a peptide-bonded polymer of amino acids or amino acid analogues; by "heterologous polypeptide" is here intended a polypeptide that does not naturally occur in contiguity with the protein or protein fragment of the present invention. As so defined, the fusion can consist entirely of a plurality of fragments of the NHELP1 protein in altered arrangement; in such case, any of the NHELP1 fragments can be considered heterologous to the other NHELP1 fragments in the fusion protein. More typically, however, the heterologous polypeptide is not drawn from the NHELP1 protein itself.

The fusion proteins of the present invention will include at least one fragment of the protein of the present invention, which fragment is at least 6, typically at least 8, often at least 15, and usefully at least 16, 17, 18, 19, or 20 amino acids long. The fragment of the protein of the present to be included in the fusion can usefully be at least 25 amino acids long, at least 50 amino acids long, and can be at least 75, 100, or even 150 amino acids long. Fusions that include the entirety of the proteins of the present invention have particular utility.

The heterologous polypeptide included within the fusion protein of the present invention is at least 6 amino acids in length, often at least 8 amino acids in length, and usefully at least 15, 20, and 25 amino acids in length. Fusions that include larger polypeptides, such as the IgG Fc region, and even entire proteins (such as GFP chromophore-containing proteins), have particular utility.

As described above in the description of vectors and expression vectors of the present invention, which discussion is incorporated herein by reference in its entirety, heterologous polypeptides to be included in the fusion proteins of the present invention can usefully include those designed to facilitate purification and/or visualization of recombinantly-expressed proteins. Although purification tags can also be incorporated into fusions that are chemically synthesized, chemical synthesis typically provides sufficient purity that further purification by HPLC suffices; however, visualization tags as above described retain their utility even when the protein is produced by chemical synthesis, and when so included render the fusion proteins of the present invention useful as directly detectable markers of NHELP1 presence.

As also discussed above, heterologous polypeptides to be included in the fusion proteins of the present invention can usefully include those that facilitate secretion of recombinantly expressed proteins - into the periplasmic space or extracellular milieu for prokaryotic hosts, into the culture medium for eukaryotic cells - through incorporation of secretion signals and/or leader sequences.

Other useful protein fusions of the present invention include those that permit use of the protein of the present invention as bait in a yeast two-hybrid system. *See* Bartel *et al.* (eds.), The Yeast Two-Hybrid System, Oxford University Press (1997) (ISBN: 0195109384); Zhu *et al.,* Yeast Hybrid Technologies, Eaton Publishing, (2000) (ISBN 1-881299-15-5); Fields *et al., Trends Genet.* 10(8):286-92 (1994); Mendelsohn *et al., Curr. Opin. Biotechnol.* 5(5):482-6 (1994); Luban *et al., Curr. Opin. Biotechnol.* 6(1):59-64 (1995); Allen *et al.,* Trends Biochem. Sci. 20(12):511-6 (1995); Drees, *Curr. Opin. Chem. Biol.* 3(1):64-70 (1999); Topcu *et al., Pharm. Res.* 17(9):1049-55 (2000); Fashena et al., Gene 250(1-2):1-14 (2000), the disclosures of which are incorporated herein by reference in their entireties. Typically, such fusion is to either E. *coli* LexA or yeast GAL4 DNA binding domains. Related bait plasmids are available that express the bait fused to a nuclear localization signal.

Other useful protein fusions include those that permit display of the encoded protein on the surface of a phage or cell, fusions to intrinsically fluorescent proteins, such as green fluorescent protein (GFP), and fusions to the IgG Fc region, as described above, which discussion is incorporated here by reference in its entirety.

The proteins and protein fragments of the present invention can also usefully be fused to protein toxins, such as Pseudomonas exotoxin A, diphtheria toxin, shiga toxin A, anthrax toxin lethal factor, ricin, in order to effect ablation of cells that bind or take up the proteins of the present invention.

The isolated proteins, protein fragments, and protein fusions of the present invention can be composed of natural amino acids linked by native peptide bonds, or can contain any or all of nonnatural amino acid analogues, nonnative bonds, and post-synthetic (post translational) modifications, either throughout the length of the protein or localized to one or more portions thereof.

As is well known in the art, when the isolated protein is used, *e.g.,* for epitope mapping, the range of such nonnatural analogues, nonnative inter-residue bonds, or post-synthesis modifications will be limited to those that permit binding of the peptide to antibodies. When used as an immunogen for the preparation of antibodies in a non-human host, such as a mouse, the range of such nonnatural analogues, nonnative inter-residue bonds, or post-synthesis modifications will be limited to those that do not interfere with the immunogenicity of the protein. When the isolated protein is used as a therapeutic agent, such as a vaccine or for replacement therapy, the range of such changes will be limited to those that do not confer toxicity upon the isolated protein.

Non-natural amino acids can be incorporated during solid phase chemical synthesis or by recombinant techniques, although the former is typically more common.

Solid phase chemical synthesis of peptides is well established in the art. Procedures are described, inter alia, in Chan *et al.* (eds.), Fmoc Solid Phase Peptide Synthesis: A Practical Approach (Practical Approach Series), Oxford Univ. Press (March 2000) (ISBN: 0199637245); Jones, Amino Acid and Peptide Synthesis (Oxford Chemistry Primers, No 7), Oxford Univ. Press (August 1992) (ISBN: 0198556683); and Bodanszky, Principles of Peptide Synthesis (Springer Laboratory), Springer Verlag (December 1993) (ISBN: 0387564314), the disclosures of which are incorporated herein by reference in their entireties.

For example, D-enantiomers of natural amino acids can readily be incorporated during chemical peptide synthesis: peptides assembled from D-amino acids are more resistant to proteolytic attack; incorporation of D-enantiomers can also be used to confer specific three dimensional conformations on the peptide. Other amino acid analogues commonly added during chemical synthesis include ornithine, norleucine, phosphorylated amino acids (typically phosphoserine, phosphothreonine, phosphotyrosine), L-malonyltyrosine, a non-hydrolyzable analog of phosphotyrosine (Kole *et al*., *Biochem. Biophys. Res. Com.* 209:817-821 (1995)), and various halogenated phenylalanine derivatives.

Amino acid analogues having detectable labels are also usefully incorporated during synthesis to provide a labeled polypeptide.

Biotin, for example (indirectly detectable through interaction with avidin, streptavidin, neutravidin, captavidin, or anti-biotin antibody), can be added using biotinoyl--(9-fluorenylmethoxycarbonyl)-L-lysine (FMOC biocytin) (Molecular Probes, Eugene, OR, USA). (Biotin can also be added enzymatically by incorporation into a fusion protein of a *E*. *coli* BirA substrate peptide.)

The FMOC and tBOC derivatives of dabcyl-L-lysine (Molecular Probes, Inc., Eugene, OR, USA) can be used to incorporate the dabcyl chromophore at selected sites in the peptide sequence during synthesis. The aminonaphthalene derivative EDANS, the most common fluorophore for pairing with the dabcyl quencher in fluorescence resonance energy transfer (FRET) systems, can be introduced during automated synthesis of peptides by using EDANS--FMOC-L-glutamic acid or the corresponding tBOC derivative (both from Molecular Probes, Inc., Eugene, OR, USA). Tetramethylrhodamine fluorophores can be incorporated during automated FMOC synthesis of peptides using (FMOC)--TMR-L-lysine (Molecular Probes, Inc. Eugene, OR, USA).

Other useful amino acid analogues that can be incorporated during chemical synthesis include aspartic acid, glutamic acid, lysine, and tyrosine analogues having allyl side-chain protection (Applied Biosystems, Inc., Foster City, CA, USA); the allyl side chain permits synthesis of cyclic, branched-chain, sulfonated, glycosylated, and phosphorylated peptides.

A large number of other FMOC-protected non-natural amino acid analogues capable of incorporation during chemical synthesis are available commercially, including, *e.g*., Fmoc-2-aminobicyclo[2.2.1]heptane-2-carboxylic acid, Fmoc-3-endo-aminobicyclo[2.2.1]heptane-2-endo-carboxylic acid, Fmoc-3-exo-aminobicyclo[2.2.1]heptane-2-exo-carboxylic acid, Fmoc-3-endo-amino-bicyclo[2.2.1]hept-5-ene-2-endo-carboxylic acid, Fmoc-3-exo-amino-bicyclo[2.2.1]hept-5-ene-2-exo-carboxylic acid, Fmoc-cis-2-amino-1-cyclohexanecarboxylic acid, Fmoc-trans-2-amino-1-cyclohexanecarboxylic acid, Fmoc-1-amino-1-cyclopentanecarboxylic acid, Fmoc-cis-2-amino-1-cyclopentanecarboxylic acid, Fmoc-1-amino-1-cyclopropanecarboxylic acid, Fmoc-D-2-amino-4-(ethylthio)butyric acid, Fmoc-L-2-amino-4-(ethylthio)butyric acid, Fmoc-L-buthionine, Fmoc-S-methyl-L-Cysteine, Fmoc-2-aminobenzoic acid (anthranillic acid), Fmoc-3-aminobenzoic acid, Fmoc-4-aminobenzoic acid, Fmoc-2-aminobenzophenone-2'-carboxylic acid, Fmoc-N-(4-aminobenzoyl)-b-alanine, Fmoc-2-amino-4,5-dimethoxybenzoic acid, Fmoc-4-aminohippuric acid, Fmoc-2-amino-3-hydroxybenzoic acid, Fmoc-2-amino-5-hydroxybenzoic acid, Fmoc-3-amino-4-hydroxybenzoic acid, Fmoc-4-amino-3-hydroxybenzoic acid, Fmoc-4-amino-2-hydroxybenzoic acid, Fmoc-5-amino-2-hydroxybenzoic acid, Fmoc-2-amino-3-methoxybenzoic acid, Fmoc-4-amino-3-methoxybenzoic acid, Fmoc-2-amino-3-methylbenzoic acid, Fmoc-2-amino-5-methylbenzoic acid, Fmoc-2-amino-6-methylbenzoic acid, Fmoc-3-amino-2-methylbenzoic acid, Fmoc-3-amino-4-methylbenzoic acid, Fmoc-4-amino-3-methylbenzoic acid, Fmoc-3-amino-2-naphtoic acid, Fmoc-D,L-3-amino-3-phenylpropionic acid, Fmoc-L-Methyldopa, Fmoc-2-amino-4,6-dimethyl-3-pyridinecarboxylic acid, Fmoc-D,L-?-amino-2-thiophenacetic acid, Fmoc-4-(carboxymethyl)piperazine, Fmoc-4-carboxypiperazine, Fmoc-4-(carboxymethyl)homopiperazine, Fmoc-4-phenyl-4-piperidinecarboxylic acid, Fmoc-L-1,2,3,4-tetrahydronorharman-3-carboxylic acid, Fmoc-L-thiazolidine-4-carboxylic acid, all available from The Peptide Laboratory (Richmond, CA, USA).

Non-natural residues can also be added biosynthetically by engineering a suppressor tRNA, typically one that recognizes the UAG stop codon, by chemical aminoacylation with the desired unnatural amino acid and. Conventional site-directed mutagenesis is used to introduce the chosen stop codon UAG at the site of interest in the protein gene. When the acylated suppressor tRNA and the mutant gene are combined in an *in vitro* transcription/translation system, the unnatural amino acid is incorporated in response to the UAG codon to give a protein containing that amino acid at the specified position. Liu *et al*., *Proc. Natl Acad. Sci. USA* 96(9):4780-5 (1999); Wang *et al., Science* 292 (5516) :498-500 (2001).

The isolated proteins, protein fragments and fusion proteins of the present invention can also include nonnative inter-residue bonds, including bonds that lead to circular and branched forms.

The isolated proteins and protein fragments of the present invention can also include post-translational and post-synthetic modifications, either throughout the length of the protein or localized to one or more portions thereof.

For example, when produced by recombinant expression in eukaryotic cells, the isolated proteins, fragments, and fusion proteins of the present invention will typically include N-linked and/or O-linked glycosylation, the pattern of which will reflect both the availability of glycosylation sites on the protein sequence and the identity of the host cell. Further modification of glycosylation pattern can be performed enzymatically.

As another example, recombinant polypeptides of the invention may also include an initial modified methionine residue, in some cases resulting from host-mediated processes.

When the proteins, protein fragments, and protein fusions of the present invention are produced by chemical synthesis, post-synthetic modification can be performed before deprotection and cleavage from the resin or after deprotection and cleavage. Modification before deprotection and cleavage of the synthesized protein often allows greater control, *e.g.* by allowing targeting of the modifying moiety to the N-terminus of a resin-bound synthetic peptide.

Useful post-synthetic (and post-translational) modifications include conjugation to detectable labels, such as fluorophores.

A wide variety of amine-reactive and thiol-reactive fluorophore derivatives have been synthesized that react under nondenaturing conditions with N-terminal amino groups and epsilon amino groups of lysine residues, on the one hand, and with free thiol groups of cysteine residues, on the other.

Kits are available commercially that permit conjugation of proteins to a variety of amine-reactive or thiol-reactive fluorophores: Molecular Probes, Inc. (Eugene, OR, USA), *e.g.,* offers kits for conjugating proteins to Alexa Fluor 350, Alexa Fluor 430, Fluorescein-EX, Alexa Fluor 488, Oregon Green 488, Alexa Fluor 532, Alexa Fluor 546, Alexa Fluor 546, Alexa Fluor 568, Alexa Fluor 594, and Texas Red-X.

A wide variety of other amine-reactive and thiol-reactive fluorophores are available commercially (Molecular Probes, Inc., Eugene, OR, USA), including Alexa Fluor® 350, Alexa Fluor® 488, Alexa Fluor® 532, Alexa Fluor® 546, Alexa Fluor® 568, Alexa Fluor® 594, Alexa Fluor® 647 (monoclonal antibody labeling kits available from Molecular Probes, Inc., Eugene, OR, USA), BODIPY dyes, such as BODIPY 493/503, BODIPY FL, BODIPY R6G, BODIPY 530/550, BODIPY TMR, BODIPY 558/568, BODIPY 558/568, BODIPY 564/570, BODIPY 576/589, BODIPY 581/591, BODIPY TR, BODIPY 630/650, BODIPY 650/665, Cascade Blue, Cascade Yellow, Dansyl, lissamine rhodamine B, Marina Blue, Oregon Green 488, Oregon Green 514, Pacific Blue, rhodamine 6G, rhodamine green, rhodamine red, tetramethylrhodamine, Texas Red (available from Molecular Probes, Inc., Eugene, OR, USA).

The polypeptides of the present invention can also be conjugated to fluorophores, other proteins, and other macromolecules, using bifunctional linking reagents.

Common homobifunctional reagents include, *e.g.,* APG, AEDP, BASED, BMB, BMDB, BMH, BMOE, BM[PEO]3, BM[PEO]4, BS3, BSOCOES, DFDNB, DMA, DMP, DMS, DPDPB, DSG, DSP (Lomant's Reagent), DSS, DST, DTBP, DTME, DTSSP, EGS, HBVS, Sulfo-BSOCOES, Sulfo-DST, Sulfo-EGS (all available from Pierce, Rockford, IL, USA); common heterobifunctional cross-linkers include ABH, AMAS, ANB-NOS, APDP, ASBA, BMPA, BMPH, BMPS, EDC, EMCA, EMCH, EMCS, KMUA, KMUH, GMBS, LC-SMCC, LC-SPDP, MBS, M2C2H, MPBH, MSA, NHS-ASA, PDPH, PMPI, SADP, SAED, SAND, SANPAH, SASD, SATP, SBAP, SFAD, SIA, SIAB, SMCC, SMPB, SMPH, SMPT, SPDP, Sulfo-EMCS, Sulfo-GMBS, Sulfo-HSAB, Sulfo-KMUS, Sulfo-LC-SPDP, Sulfo-MBS, Sulfo-NHS-LC-ASA, Sulfo-SADP, Sulfo-SANPAH, Sulfo-SIAB, Sulfo-SMCC, Sulfo-SMPB, Sulfo-LC-SMPT, SVSB, TFCS (all available Pierce, Rockford, IL, USA).

The proteins, protein fragments, and protein fusions of the present invention can be conjugated, using such cross-linking reagents, to fluorophores that are not amine- or thiol-reactive.

Other labels that usefully can be conjugated to the proteins, protein fragments, and fusion proteins of the present invention include radioactive labels, echosonographic contrast reagents, and MRI contrast agents.

The proteins, protein fragments, and protein fusions of the present invention can also usefully be conjugated using cross-linking agents to carrier proteins, such as KLH, bovine thyroglobulin, and even bovine serum albumin (BSA), to increase immunogenicity for raising anti-NHELP1 antibodies.

The proteins, protein fragments, and protein fusions of the present invention can also usefully be conjugated to polyethylene glycol (PEG); PEGylation increases the serum half life of proteins administered intravenously for replacement therapy. Delgado *et al., Crit. Rev. Ther. Drug Carrier Syst.* 9(3-4):249-304 (1992); Scott *et al., Curr. Pharm. Des.* 4(6):423-38 (1998); DeSantis *et al., Curr. Opin. Biotechnol.* 10(4):324-30 (1999), incorporated herein by reference in their entireties. PEG monomers can be attached to the protein directly or through a linker, with PEGylation using PEG monomers activated with tresyl chloride (2,2,2-trifluoroethanesulphonyl chloride) permitting direct attachment under mild conditions.

The isolated proteins of the present invention, including fusions thereof, can be produced by recombinant expression, typically using the expression vectors of the present invention as above-described or, if fewer than about 100 amino acids, by chemical synthesis (typically, solid phase synthesis), and, on occasion, by *in vitro* translation.

Production of the isolated proteins of the present invention can optionally be followed by purification. Purification of recombinantly expressed proteins is now well within the skill in the art. *See, e.g*., Thorner et *al.* (eds.), Applications of Chimeric Genes and Hybrid Proteins, Part A: Gene Expression and Protein Purification (Methods in Enzymology, Volume 326), Academic Press (2000), (ISBN: 0121822273); Harbin (ed.), Cloning, Gene Expression and Protein Purification : Experimental Procedures and Process Rationale, Oxford Univ. Press (2001) (ISBN: 0195132947); Marshak *et al*., Strategies for Protein Purification and Characterization: A Laboratory Course Manual, Cold Spring Harbor Laboratory Press (1996) (ISBN: 0-87969-385-1); and Roe (ed.), Protein Purification Applications, Oxford University Press (2001), the disclosures of which are incorporated herein by reference in their entireties, and thus need not be detailed here.

Briefly, however, if purification tags have been fused through use of an expression vector that appends such tag, purification can be effected, at least in part, by means appropriate to the tag, such as use of immobilized metal affinity chromatography for polyhistidine tags. Other techniques common in the art include ammonium sulfate fractionation, immunoprecipitation, fast protein liquid chromatography (FPLC), high performance liquid chromatography (HPLC), and preparative gel electrophoresis.

Purification of chemically-synthesized peptides can readily be effected, *e.g*., by HPLC.

Accordingly, it is an aspect of the present invention to provide the isolated proteins of the present invention in pure or substantially pure form.

A purified protein of the present invention is an isolated protein, as above described, that is present at a concentration of at least 95%, as measured on a weight basis (w/w) with respect to total protein in a composition. Such purities can often be obtained during chemical synthesis without further purification, as, *e.g.,* by HPLC. Purified proteins of the present invention can be present at a concentration (measured on a weight basis with respect to total protein in a composition) of 96%, 97%, 98%, and even 99%. The proteins of the present invention can even be present at levels of 99.5%, 99.6%, and even 99.7%, 99.8%, or even 99.9% following purification, as by HPLC.

Although high levels of purity are particularly useful when the isolated proteins of the present invention are used as therapeutic agents - such as vaccines, or for replacement therapy - the isolated proteins of the present invention are also useful at lower purity. For example, partially purified proteins of the present invention can be used as immunogens to raise antibodies in laboratory animals.

Thus, in another aspect, the present invention provides the isolated proteins of the present invention in substantially purified form. A "substantially purified protein" of the present invention is an isolated protein, as above described, present at a concentration of at least 70%, measured on a weight basis with respect to total protein in a composition. Usefully, the substantially purified protein is present at a concentration, measured on a weight basis with respect to total protein in a composition, of at least 75%, 80%, or even at least 85%, 90%, 91%, 92%, 93%, 94%, 94.5% or even at least 94.9%.

In preferred embodiments, the purified and substantially purified proteins of the present invention are in compositions that lack detectable ampholytes, acrylamide monomers, bis-acrylamide monomers, and polyacrylamide.

The proteins, fragments, and fusions of the present invention can usefully be attached to a substrate. The substrate can porous or solid, planar or non-planar; the bond can be covalent or noncovalent.

For example, the proteins, fragments, and fusions of the present invention can usefully be bound to a porous substrate, commonly a membrane, typically comprising nitrocellulose, polyvinylidene fluoride (PVDF), or cationically derivatized, hydrophilic PVDF; so bound, the proteins, fragments, and fusions of the present invention can be used to detect and quantify antibodies, *e.g.* in serum, that bind specifically to the immobilized protein of the present invention.

As another example, the proteins, fragments, and fusions of the present invention can usefully be bound to a substantially nonporous substrate, such as plastic, to detect and quantify antibodies, *e.g.* in serum, that bind specifically to the immobilized protein of the present invention. Such plastics include polymethylacrylic, polyethylene, polypropylene, polyacrylate, polymethylmethacrylate, polyvinylchloride, polytetrafluoroethylene, polystyrene, polycarbonate, polyacetal, polysulfone, celluloseacetate, cellulosenitrate, nitrocellulose, or mixtures thereof; when the assay is performed in standard microtiter dish, the plastic is typically polystyrene.

The proteins, fragments, and fusions of the present invention can also be attached to a substrate suitable for use as a surface enhanced laser desorption ionization source; so attached, the protein, fragment, or fusion of the present invention is useful for binding and then detecting secondary proteins that bind with sufficient affinity or avidity to the surface-bound protein to indicate biologic interaction therebetween. The proteins, fragments, and fusions of the present invention can also be attached to a substrate suitable for use in surface plasmon resonance detection; so attached, the protein, fragment, or fusion of the present invention is useful for binding and then detecting secondary proteins that bind with sufficient affinity or avidity to the surface-bound protein to indicate biological interaction therebetween.

### NHELP1 Proteins

In a first series of protein embodiments, the invention provides an isolated NHELP1 polypeptide having an amino acid sequence in SEQ ID NO: 3, which are full length NHELP1 proteins. When used as immunogens, the full length proteins of the present invention can be used, *inter alia,* to elicit antibodies that bind to a variety of epitopes of the NHELP1 protein.

The invention further provides fragments of the above-described polypeptides, particularly fragments having at least 6 amino acids, typically at least 8 amino acids, often at least 15 amino acids, and even the entirety of the sequence given in SEQ ID NO: 3.

The invention further provides fragments of at least 6 amino acids, typically at least 8 amino acids, often at least 15 amino acids, and even the entirety of the sequence given in SEQ ID NO: 7.

As described above, the invention further provides proteins that differ in sequence from those described with particularity in the above-referenced SEQ ID NOs., whether by way of insertion or deletion, by way of conservative or moderately conservative substitutions, as hybridization related proteins, or as cross-hybridizing proteins, with those that substantially retain a NHELP1 activity particularly useful.

The invention further provides fusions of the proteins and protein fragments herein described to heterologous polypeptides.

### ANTIBODIES AND ANTIBODY-PRODUCING CELLS

In another aspect, the invention provides antibodies, including fragments and derivatives thereof, that bind specifically to NHELP1 proteins and protein fragments of the present invention or to one or more of the proteins and protein fragments encoded by the isolated NHELP1 nucleic acids of the present invention. The antibodies of the present invention can be specific for all of linear epitopes, discontinuous epitopes, or conformational epitopes of such proteins or protein fragments, either as present on the protein in its native conformation or, in some cases, as present on the proteins as denatured, as, *e.g* ., by solubilization in SDS.

In other embodiments, the invention provides antibodies, including fragments and derivatives thereof, the binding of which can be competitively inhibited by one or more of the NHELP1 proteins and protein fragments of the present invention, or by one or more of the proteins and protein fragments encoded by the isolated NHELP1 nucleic acids of the present invention.

As used herein, the term "antibody" refers to a polypeptide, at least a portion of which is encoded by at least one immunoglobulin gene, which can bind specifically to a first molecular species, and to fragments or derivatives thereof that remain capable of such specific binding.

By "bind specifically" and "specific binding" is here intended the ability of the antibody to bind to a first molecular species in preference to binding to other molecular species with which the antibody and first molecular species are admixed. An antibody is said specifically to "recognize" a first molecular species when it can bind specifically to that first molecular species.

As is well known in the art, the degree to which an antibody can discriminate as among molecular species in a mixture will depend, in part, upon the conformational relatedness of the species in the mixture; typically, the antibodies of the present invention will discriminate over adventitious binding to non-NHELP1 proteins by at least two-fold, more typically by at least 5-fold, typically by more than 10-fold, 25-fold, 50-fold, 75-fold, and often by more than 100-fold, and on occasion by more than 500-fold or 1000-fold. When used to detect the proteins or protein fragments of the present invention, the antibody of the present invention is sufficiently specific when it can be used to determine the presence of the protein of the present invention in samples derived from human brain, adult liver, adrenal, bone marrow, fetal liver, testis and prostate.

Typically, the affinity or avidity of an antibody (or antibody multimer, as in the case of an IgM pentamer) of the present invention for a protein or protein fragment of the present invention will be at least about 1 x 10⁻⁶ molar (M), typically at least about 5 x 10⁻⁷ M, usefully at least about 1 x 10⁻⁷ M, with affinities and avidities of at least 1 x 10⁻⁸ M, 5 x 10⁻⁹ M, and 1 x 10⁻¹⁰ M proving especially useful.

The antibodies of the present invention can be naturally-occurring forms, such as IgG, IgM, IgD, IgE, and IgA, from any mammalian species.

Human antibodies can, but will infrequently, be drawn directly from human donors or human cells. In such case, antibodies to the proteins of the present invention will typically have resulted from fortuitous immunization, such as autoimmune immunization, with the protein or protein fragments of the present invention. Such antibodies will typically, but will not invariably, be polyclonal.

Human antibodies are more frequently obtained using transgenic animals that express human immunoglobulin genes, which transgenic animals can be affirmatively immunized with the protein immunogen of the present invention. Human Ig-transgenic mice capable of producing human antibodies and methods of producing human antibodies therefrom upon specific immunization are described, *inter alia,* in U.S. Patent Nos. 6,162,963; 6,150,584; 6,114,598; 6,075,181; 5,939,598; 5,877,397; 5,874,299; 5,814,318; 5,789,650; 5,770,429; 5,661,016; 5,633,425; 5,625,126; 5,569,825; 5,545,807; 5,545,806, and 5,591,669, the disclosures of which are incorporated herein by reference in their entireties. Such antibodies are typically monoclonal, and are typically produced using techniques developed for production of murine antibodies.

Human antibodies are particularly useful, and often preferred, when the antibodies of the present invention are to be administered to human beings as *in vivo* diagnostic or therapeutic agents, since recipient immune response to the administered antibody will often be substantially less than that occasioned by administration of an antibody derived from another species, such as mouse.

IgG, IgM, IgD, IgE and IgA antibodies of the present invention are also usefully obtained from other mammalian species, including rodents - typically mouse, but also rat, guinea pig, and hamster - lagomorphs, typically rabbits, and also larger mammals, such as sheep, goats, cows, and horses. In such cases, as with the transgenic human-antibody-producing non-human mammals, fortuitous immunization is not required, and the non-human mammal is typically affirmatively immunized, according to standard immunization protocols, with the protein or protein fragment of the present invention.

As discussed above, virtually all fragments of 8 or more contiguous amino acids of the proteins of the present invention can be used effectively as immunogens when conjugated to a carrier, typically a protein such as bovine thyroglobulin, keyhole limpet hemocyanin, or bovine serum albumin, conveniently using a bifunctional linker such as those described elsewhere above, which discussion is incorporated by reference here.

Immunogenicity can also be conferred by fusion of the proteins and protein fragments of the present invention to other moieties.

For example, peptides of the present invention can be produced by solid phase synthesis on a branched polylysine core matrix; these multiple antigenic peptides (MAPs) provide high purity, increased avidity, accurate chemical definition and improved safety in vaccine development. Tam *et al.,* Proc. Natl. Acad. Sci. USA 85:5409-5413 (1988); Posnett et al., *J. Biol. Chem.* 263, 1719-1725 (1988).

Protocols for immunizing non-human mammals are well-established in the art, Harlow *et al.* (eds.), Antibodies: A Laboratory Manual, Cold Spring Harbor Laboratory (1998) (ISBN: 0879693142); Coligan *et al.* (eds.), Current Protocols in Immunology, John Wiley & Sons, Inc. (2001) (ISBN: 0-471-52276-7); Zola, Monoclonal Antibodies : Preparation and Use of Monoclonal Antibodies and Engineered Antibody Derivatives (Basics: From Background to Bench), Springer Verlag (2000) (ISBN: 0387915907), the disclosures of which are incorporated herein by reference, and often include multiple immunizations, either with or without adjuvants such as Freund's complete adjuvant and Freund's incomplete adjuvant.

Antibodies from nonhuman mammals can be polyclonal or monoclonal, with polyclonal antibodies having certain advantages in immunohistochemical detection of the proteins of the present invention and monoclonal antibodies having advantages in identifying and distinguishing particular epitopes of the proteins of the present invention.

Following immunization, the antibodies of the present invention can be produced using any art-accepted technique. Such techniques are well known in the art, Coligan *et al.* (eds.), Current Protocols in Immunology, John Wiley & Sons, Inc. (2001) (ISBN: 0-471-52276-7); Zola, Monoclonal Antibodies : Preparation and Use of Monoclonal Antibodies and Engineered Antibody Derivatives (Basics: From Background to Bench), Springer Verlag (2000) (ISBN: 0387915907); Howard *et al.* (eds.), Basic Methods in Antibody Production and Characterization, CRC Press (2000) (ISBN: 0849394457); Harlow *et al.* (eds.), Antibodies: A Laboratory Manual, Cold Spring Harbor Laboratory (1998) (ISBN: 0879693142); Davis (ed.), Monoclonal Antibody Protocols, Vol. 45, Humana Press (1995) (ISBN: 0896033082); Delves (ed.), Antibody Production: Essential Techniques, John Wiley & Son Ltd (1997) (ISBN: 0471970107); Kenney, Antibody Solution: An Antibody Methods Manual, Chapman & Hall (1997) (ISBN: 0412141914), incorporated herein by reference in their entireties, and thus need not be detailed here.

Briefly, however, such techniques include, *inter alia,* production of monoclonal antibodies by hybridomas and expression of antibodies or fragments or derivatives thereof from host cells engineered to express immunoglobulin genes or fragments thereof.
These two methods of production are not mutually exclusive: genes encoding antibodies specific for the proteins or protein fragments of the present invention can be cloned from hybridomas and thereafter expressed in other host cells. Nor need the two necessarily be performed together: *e.g*., genes encoding antibodies specific for the proteins and protein fragments of the present invention can be cloned directly from B cells known to be specific for the desired protein, as further described in U.S. Pat. No. 5,627,052, the disclosure of which is incorporated herein by reference in its entirety, or from antibody-displaying phage.

Recombinant expression in host cells is particularly useful when fragments or derivatives of the antibodies of the present invention are desired.

Host cells for recombinant antibody production - either whole antibodies, antibody fragments, or antibody derivatives - can be prokaryotic or eukaryotic.

Prokaryotic hosts are particularly useful for producing phage displayed antibodies of the present invention.

The technology of phage-displayed antibodies, in which antibody variable region fragments are fused, for example, to the gene III protein (pIII) or gene VIII protein (pVIII) for display on the surface of filamentous phage, such as M13, is by now well-established, Sidhu, *Curr. Opin. Biotechnol.* 11(6):610-6 (2000); Griffiths *et al., Curr. Opin. Biotechnol.* 9(1):102-8 (1998); Hoogenboom *et al., Immunotechnology,* 4(1):1-20 (1998); Rader *et al., Current Opinion in Biotechnology* 8:503-508 (1997); Aujame *et al., Human Antibodies* 8:155-168 (1997); Hoogenboom, *Trends in Biotechnol.* 15:62-70 (1997); de Kruif *et al.,* 17:453-455 (1996); Barbas *et al*., *Trends in Biotechnol.* 14:230-234 (1996); Winter *et al*., *Ann. Rev. Immunol.* 433-455 (1994), and techniques and protocols required to generate, propagate, screen (pan), and use the antibody fragments from such libraries have recently been compiled, Barbas *et al.,* Phage Display: A Laboratory Manual, Cold Spring Harbor Laboratory Press (2001) (ISBN 0-87969-546-3); Kay *et al.* (eds.), Phage Display of Peptides and Proteins: A Laboratory Manual, Academic Press, Inc. (1996); Abelson *et al.* (eds.), Combinatorial Chemistry, Methods in Enzymology vol. 267, Academic Press (May 1996), the disclosures of which are incorporated herein by reference in their entireties.

Typically, phage-displayed antibody fragments are scFv fragments or Fab fragments; when desired, full length antibodies can be produced by cloning the variable regions from the displaying phage into a complete antibody and expressing the full length antibody in a further prokaryotic or a eukaryotic host cell.

Eukaryotic cells are also useful for expression of the antibodies, antibody fragments, and antibody derivatives of the present invention.

For example, antibody fragments of the present invention can be produced in *Pichia pastoris,* Takahashi *et al., Biosci. Biotechnol. Biochem.* 64(10):2138-44 (2000); Freyre *et al.,* J. Biotechnol. 76(2-3):157-63 (2000); Fischer *et al., Biotechnol. Appl. Biochem.* 30 (Pt 2):117-20 (1999); Pennell *et al., Res. Immunol.* 149(6):599-603 (1998); Eldin *et al., J. Immunol. Methods.* 201(1):67-75 (1997); and in *Saccharomyces cerevisiae,* Frenken *et al., Res. Immunol.* 149(6):589-99 (1998); Shusta et al., *Nature Biotechnol.* 16(8):773-7 (1998), the disclosures of which are incorporated herein by reference in their entireties.

Antibodies, including antibody fragments and derivatives, of the present invention can also be produced in insect cells, Li *et al., Protein Expr. Purif.* 21(1):121-8 (2001); Ailor *et al., Biotechnol. Bioeng.* 58(2-3):196-203 (1998); Hsu *et al., Biotechnol. Prog.* 13(1):96-104 (1997); Edelman *et al., Immunology* 91(1):13-9 (1997); and Nesbit *et al., J. Immunol.* *Methods.* 151(1-2):201-8 (1992), the disclosures of which are incorporated herein by reference in their entireties.

Antibodies and fragments and derivatives thereof of the present invention can also be produced in plant cells, Giddings *et al*., *Nature Biotechnol.* 18(11):1151-5 (2000); Gavilondo *et al*., *Biotechniques* 29(1):128-38 (2000); Fischer *et al*., *J. Biol. Regul. Homeost. Agents* 14(2):83-92 (2000); Fischer *et al., Biotechnol. Appl. Biochem.* 30 (Pt 2):113-6 (1999); Fischer *et al., Biol. Chem.* 380(7-8):825-39 (1999); Russell, *Curr. Top. Microbiol. Immunol.* 240:119-38 (1999); and Ma *et al*., *Plant Physiol.* 109(2):341-6 (1995), the disclosures of which are incorporated herein by reference in their entireties.

Mammalian cells useful for recombinant expression of antibodies, antibody fragments, and antibody derivatives of the present invention include CHO cells, COS cells, 293 cells, and myeloma cells.

Verma *et al., J. Immunol. Methods* 216(1-2):165-81 (1998), review and compare bacterial, yeast, insect and mammalian expression systems for expression of antibodies.

Antibodies of the present invention can also be prepared by cell free translation, as further described in Merk *et al.,* J. Biochem. (Tokyo). 125(2):328-33 (1999) and Ryabova *et al*., *Nature Biotechnol.* 15(1):79-84 (1997), and in the milk of transgenic animals, as further described in Pollock *et al*., *J. Immunol. Methods* 231(1-2):147-57 (1999), the disclosures of which are incorporated herein by reference in their entireties.

The invention further provides antibody fragments that bind specifically to one or more of the proteins and protein fragments of the present invention, to one or more of the proteins and protein fragments encoded by the isolated nucleic acids of the present invention, or the binding of which can be competitively inhibited by one or more of the proteins and protein fragments of the present invention or one or more of the proteins and protein fragments encoded by the isolated nucleic acids of the present invention.

Among such useful fragments are Fab, Fab', Fv, F(ab)'₂, and single chain Fv (scFv) fragments. Other useful fragments are described in Hudson, *Curr. Opin. Biotechnol.* 9(4):395-402 (1998).

It is also an aspect of the present invention to provide antibody derivatives that bind specifically to one or more of the proteins and protein fragments of the present invention, to one or more of the proteins and protein fragments encoded by the isolated nucleic acids of the present invention, or the binding of which can be competitively inhibited by one or more of the proteins and protein fragments of the present invention or one or more of the proteins and protein fragments encoded by the isolated nucleic acids of the present invention.

Among such useful derivatives are chimeric, primatized, and humanized antibodies; such derivatives are less immunogenic in human beings, and thus more suitable for in vivo administration, than are unmodified antibodies from non-human mammalian species.

Chimeric antibodies typically include heavy and/or light chain variable regions (including both CDR and framework residues) of immunoglobulins of one species, typically mouse, fused to constant regions of another species, typically human. *See, e.g.,* U.S. Pat. No. 5,807,715; Morrison *et al., Proc. Natl. Acad. Sci USA*.81(21):6851-5 (1984); Sharon *et al*., *Nature* 309(5966):364-7 (1984); Takeda *et al*., *Nature* 314(6010):452-4 (1985), the disclosures of which are incorporated herein by reference in their entireties. Primatized and humanized antibodies typically include heavy and/or light chain CDRs from a murine antibody grafted into a non-human primate or human antibody V region framework, usually further comprising a human constant region, Riechmann *et al., Nature* 332(6162):323-7 (1988); Co *et al., Nature* 351(6326):501-2 (1991); U.S. Pat. Nos. 6,054,297; 5,821,337; 5,770,196; 5,766,886; 5,821,123; 5,869,619; 6,180,377; 6,013,256; 5,693,761; and 6,180,370, the disclosures of which are incorporated herein by reference in their entireties.

Other useful antibody derivatives of the invention include heteromeric antibody complexes and antibody fusions, such as diabodies (bispecific antibodies), single-chain diabodies, and intrabodies.

The antibodies of the present invention, including fragments and derivatives thereof, can usefully be labeled. It is, therefore, another aspect of the present invention to provide labeled antibodies that bind specifically to one or more of the proteins and protein fragments of the present invention, to one or more of the proteins and protein fragments encoded by the isolated nucleic acids of the present invention, or the binding of which can be competitively inhibited by one or more of the proteins and protein fragments of the present invention or one or more of the proteins and protein fragments encoded by the isolated nucleic acids of the present invention.

The choice of label depends, in part, upon the desired use.

For example, when the antibodies of the present invention are used for immunohistochemical staining of tissue samples, the label can usefully be an enzyme that catalyzes production and local deposition of a detectable product.

Enzymes typically conjugated to antibodies to permit their immunohistochemical visualization are well known, and include alkaline phosphatase, β-galactosidase, glucose oxidase, horseradish peroxidase (HRP), and urease. Typical substrates for production and deposition of visually detectable products include o-nitrophenyl-beta-D-galactopyranoside (ONPG); o-phenylenediamine dihydrochloride (OPD); p-nitrophenyl phosphate (PNPP); p-nitrophenyl-beta-D-galactopryanoside (PNPG); 3',3'-diaminobenzidine (DAB); 3-amino-9-ethylcarbazole (AEC); 4-chloro-1-naphthol (CN); 5-bromo-4-chloro-3-indolyl-phosphate (BCIP); ABTS®; BluoGal; iodonitrotetrazolium (INT); nitroblue tetrazolium chloride (NBT); phenazine methosulfate (PMS); phenolphthalein monophosphate (PMP); tetramethyl benzidine (TMB); tetranitroblue tetrazolium (TNBT); X-Gal; X-Gluc; and X-Glucoside.

Other substrates can be used to produce products for local deposition that are luminescent. For example, in the presence of hydrogen peroxide (H₂O₂), horseradish peroxidase (HRP) can catalyze the oxidation of cyclic diacylhydrazides, such as luminol. Immediately following the oxidation, the luminol is in an excited state (intermediate reaction product), which decays to the ground state by emitting light. Strong enhancement of the light emission is produced by enhancers, such as phenolic compounds. Advantages include high sensitivity, high resolution, and rapid detection without radioactivity and requiring only small amounts of antibody. *See, e.g.,* Thorpe *et al*., *Methods Enzymol.* 133:331-53 (1986); Kricka *et al*., *J. Immunoassay* 17(1):67-83 (1996); and Lundqvist *et al*., *J. Biolumin. Chemilumin.* 10(6):353-9 (1995), the disclosures of which are incorporated herein by reference in their entireties. Kits for such enhanced chemiluminescent detection (ECL) are available commercially.

The antibodies can also be labeled using colloidal gold.

As another example, when the antibodies of the present invention are used, *e.g.,* for flow cytometric detection, for scanning laser cytometric detection, or for fluorescent immunoassay, they can usefully be labeled with fluorophores.

There are a wide variety of fluorophore labels that can usefully be attached to the antibodies of the present invention.

For flow cytometric applications, both for extracellular detection and for intracellular detection, common useful fluorophores can be fluorescein isothiocyanate (FITC), allophycocyanin (APC), R-phycoerythrin (PE), peridinin chlorophyll protein (PerCP), Texas Red, Cy3, Cy5, fluorescence resonance energy tandem fluorophores such as PerCP-Cy5.5, PE-Cy5, PE-Cy5.5, PE-Cy7, PE-Texas Red, and APC-Cy7.

Other fluorophores include, *inter alia,* Alexa Fluor® 350, Alexa Fluor® 488, Alexa Fluor® 532, Alexa Fluor® 546, Alexa Fluor® 568, Alexa Fluor® 594, Alexa Fluor® 647 (monoclonal antibody labeling kits available from Molecular Probes, Inc., Eugene, OR, USA), BODIPY dyes, such as BODIPY 493/503, BODIPY FL, BODIPY R6G, BODIPY 530/550, BODIPY TMR, BODIPY 558/568, BODIPY 558/568, BODIPY 564/570, BODIPY 576/589, BODIPY 581/591, BODIPY TR, BODIPY 630/650, BODIPY 650/665, Cascade Blue, Cascade Yellow, Dansyl, lissamine rhodamine B, Marina Blue, Oregon Green 488, Oregon Green 514, Pacific Blue, rhodamine 6G, rhodamine green, rhodamine red, tetramethylrhodamine, Texas Red (available from Molecular Probes, Inc., Eugene, OR, USA), and Cy2, Cy3, Cy3.5, Cy5, Cy5.5, Cy7, all of which are also useful for fluorescently labeling the antibodies of the present invention.

For secondary detection using labeled avidin, streptavidin, captavidin or neutravidin, the antibodies of the present invention can usefully be labeled with biotin.

When the antibodies of the present invention are used, *e.g.*, for western blotting applications, they can usefully be labeled with radioisotopes, such as ³³P, ³²P, ³⁵S, ³H, and ¹²⁵I.

As another example, when the antibodies of the present invention are used for radioimmunotherapy, the label can usefully be ²²⁸Th, ²²⁷Ac, ²²⁵Ac, ²²³Ra, ²¹³Bi, ²¹²Pb, ²¹²Bi, ²¹¹At, ²⁰³Pb, ¹⁹⁴Os, ¹⁸⁸Re, ¹⁸⁶Re, ¹⁵³Sm, ¹⁴⁹Tb, ¹³¹I, ¹²⁵I, ¹¹¹In, ¹⁰⁵Rh, ^{99m}Tc, ⁹⁷Ru, ⁹⁰Y, ⁹⁰Sr, ⁸⁸Y, ⁷²Se, ⁶⁷Cu, or ⁴⁷Sc.

As another example, when the antibodies of the present invention are to be used for *in vivo* diagnostic use, they can be rendered detectable by conjugation to MRI contrast agents, such as gadolinium diethylenetriaminepentaacetic acid (DTPA), Lauffer *et al*., *Radiology* 207(2) :529-38 (1998), or by radioisotopic labeling

As would be understood, use of the labels described above is not restricted to the application as for which they were mentioned.

The antibodies of the present invention, including fragments and derivatives thereof, can also be conjugated to toxins, in order to target the toxin's ablative action to cells that display and/or express the proteins of the present invention. Commonly, the antibody in such immunotoxins is conjugated to Pseudomonas exotoxin A, diphtheria toxin, shiga toxin A, anthrax toxin lethal factor, or ricin. *See* Hall (ed.), Immunotoxin Methods and Protocols (Methods in Molecular Biology, Vol 166), Humana Press (2000) (ISBN:0896037754); and Frankel *et al.* (eds.), Clinical Applications of Immunotoxins, Springer-Verlag New York, Incorporated (1998) (ISBN:3540640975), the disclosures of which are incorporated herein by reference in their entireties, for review.

The antibodies of the present invention can usefully be attached to a substrate, and it is, therefore, another aspect of the invention to provide antibodies that bind specifically to one or more of the proteins and protein fragments of the present invention, to one or more of the proteins and protein fragments encoded by the isolated nucleic acids of the present invention, or the binding of which can be competitively inhibited by one or more of the proteins and protein fragments of the present invention or one or more of the proteins and protein fragments encoded by the isolated nucleic acids of the present invention, attached to a substrate.

Substrates can be porous or nonporous, planar or nonplanar.

For example, the antibodies of the present invention can usefully be conjugated to filtration media, such as NHS-activated Sepharose or CNBr-activated Sepharose for purposes of immunoaffinity chromatography.

For example, the antibodies of the present invention can usefully be attached to paramagnetic microspheres, typically by biotin-streptavidin interaction, which microsphere can then be used for isolation of cells that express or display the proteins of the present invention. As another example, the antibodies of the present invention can usefully be attached to the surface of a microtiter plate for ELISA.

As noted above, the antibodies of the present invention can be produced in prokaryotic and eukaryotic cells. It is, therefore, another aspect of the present invention to provide cells that express the antibodies of the present invention, including hybridoma cells, B cells, plasma cells, and host cells recombinantly modified to express the antibodies of the present invention.

In yet a further aspect, the present invention provides aptamers evolved to bind specifically to one or more of the proteins and protein fragments of the present invention, to one or more of the proteins and protein fragments encoded by the isolated nucleic acids of the present invention, or the binding of which can be competitively inhibited by one or more of the proteins and protein fragments of the present invention or one or more of the proteins and protein fragments encoded by the isolated nucleic acids of the present invention.

### NHELP1 Antibodies

In a first series of antibody embodiments, the invention provides antibodies, both polyclonal and monoclonal, and fragments and derivatives thereof, that bind specifically to a polypeptide having an amino acid sequence in SEQ ID NO: 3, which are full length NHELP1 proteins.

Such antibodies are useful in *in vitro* immunoassays, such as ELISA, western blot or immunohistochemical assay of disease tissue or cells. Such antibodies are also useful in isolating and purifying NHELP1 proteins, including related cross-reactive proteins, by immunoprecipitation, immunoaffinity chromatography, or magnetic bead-mediated purification.

In another series of antibody embodiments, the invention provides antibodies, both polyclonal and monoclonal, and fragments and derivatives thereof, the specific binding of which can be competitively inhibited by the isolated proteins and polypeptides of the present invention.

In other embodiments, the invention further provides the above-described antibodies detectably labeled, and in yet other embodiments, provides the above-described antibodies attached to a substrate.

### PHARMACEUTICAL COMPOSITIONS

NHELP1 is important for Na⁺/H⁺ exchange; defects in NHELP1 expression, activity, distribution, localization, and/or solubility are a cause of human disease, which disease can manifest as a disorder of brain, adrenal, bone marrow, liver, testis or prostate function. Accordingly, pharmaceutical compositions comprising nucleic acids, proteins, and antibodies of the present invention, as well as mimetics, agonists, antagonists, or inhibitors of NHELP1 activity, can be administered as therapeutics for treatment of NHELP1 defects.

Thus, in another aspect, the invention provides pharmaceutical compositions comprising the nucleic acids, nucleic acid fragments, proteins, protein fusions, protein fragments, antibodies, antibody derivatives, antibody fragments, mimetics, agonists, antagonists, and inhibitors of the present invention.

Such a composition typically contains from about 0.1 to 90% by weight of a therapeutic agent of the invention formulated in and/or with a pharmaceutically acceptable carrier or excipient.

Pharmaceutical formulation is a well-established art, and is further described in Gennaro (ed.), Remington: The Science and Practice of Pharmacy, 20^{th} ed., Lippincott, Williams & Wilkins (2000) (ISBN: 0683306472); Ansel *et al.,* Pharmaceutical Dosage Forms and Drug Delivery Systems, 7^{th} ed., Lippincott Williams & Wilkins Publishers (1999) (ISBN: 0683305727); and Kibbe (ed.), Handbook of Pharmaceutical Excipients American Pharmaceutical Association, 3^{rd} ed. (2000) (ISBN: 091733096X), the disclosures of which are incorporated herein by reference in their entireties, and thus need not be described in detail herein.

Briefly, however, formulation of the pharmaceutical compositions of the present invention will depend upon the route chosen for administration. The pharmaceutical compositions utilized in this invention can be administered by various routes including both enteral and parenteral routes, including oral, intravenous, intramuscular, subcutaneous, inhalation, topical, sublingual, rectal, intra-arterial, intramedullary, intrathecal, intraventricular, transmucosal, transdermal, intranasal, intraperitoneal, intrapulmonary, and intrauterine.

Oral dosage forms can be formulated as tablets, pills, dragees, capsules, liquids, gels, syrups, slurries, suspensions, and the like, for ingestion by the patient.

Solid formulations of the compositions for oral administration can contain suitable carriers or excipients, such as carbohydrate or protein fillers, such as sugars, including lactose, sucrose, mannitol, or sorbitol; starch from corn, wheat, rice, potato, or other plants; cellulose, such as methyl cellulose, hydroxypropylmethyl-cellulose, sodium carboxymethylcellulose, or microcrystalline cellulose; gums including arabic and tragacanth; proteins such as gelatin and collagen; inorganics, such as kaolin, calcium carbonate, dicalcium phosphate, sodium chloride; and other agents such as acacia and alginic acid.

Agents that facilitate disintegration and/or solubilization can be added, such as the cross-linked polyvinyl pyrrolidone, agar, alginic acid, or a salt thereof, such as sodium alginate, microcrystalline cellulose, corn starch, sodium starch glycolate, and alginic acid.

Tablet binders that can be used include acacia, methylcellulose, sodium carboxymethylcellulose, polyvinylpyrrolidone (Povidone™), hydroxypropyl methylcellulose, sucrose, starch and ethylcellulose.

Lubricants that can be used include magnesium stearates, stearic acid, silicone fluid, talc, waxes, oils, and colloidal silica.

Fillers, agents that facilitate disintegration and/or solubilization, tablet binders and lubricants, including the aforementioned, can be used singly or in combination.

Solid oral dosage forms need not be uniform throughout.

For example, dragee cores can be used in conjunction with suitable coatings, such as concentrated sugar solutions, which can also contain gum arabic, talc, polyvinylpyrrolidone, carbopol gel, polyethylene glycol, and/or titanium dioxide, lacquer solutions, and suitable organic solvents or solvent mixtures.

Oral dosage forms of the present invention include push-fit capsules made of gelatin, as well as soft, sealed capsules made of gelatin and a coating, such as glycerol or sorbitol. Push-fit capsules can contain active ingredients mixed with a filler or binders, such as lactose or starches, lubricants, such as talc or magnesium stearate, and, optionally, stabilizers. In soft capsules, the active compounds can be dissolved or suspended in suitable liquids, such as fatty oils, liquid, or liquid polyethylene glycol with or without stabilizers.

Additionally, dyestuffs or pigments can be added to the tablets or dragee coatings for product identification or to characterize the quantity of active compound, *i.e*., dosage.

Liquid formulations of the pharmaceutical compositions for oral (enteral) administration are prepared in water or other aqueous vehicles and can contain various suspending agents such as methylcellulose, alginates, tragacanth, pectin, kelgin, carrageenan, acacia, polyvinylpyrrolidone, and polyvinyl alcohol. The liquid formulations can also include solutions, emulsions, syrups and elixirs containing, together with the active compound(s), wetting agents, sweeteners, and coloring and flavoring agents.

The pharmaceutical compositions of the present invention can also be formulated for parenteral administration.

For intravenous injection, water soluble versions of the compounds of the present invention are formulated in, or if provided as a lyophilate, mixed with, a physiologically acceptable fluid vehicle, such as 5% dextrose ("D5"), physiologically buffered saline, 0.9% saline, Hanks' solution, or Ringer's solution.

Intramuscular preparations, *e.g.* a sterile formulation of a suitable soluble salt form of the compounds of the present invention, can be dissolved and administered in a pharmaceutical excipient such as Water-for-Injection, 0.9% saline, or 5% glucose solution. Alternatively, a suitable insoluble form of the compound can be prepared and administered as a suspension in an aqueous base or a pharmaceutically acceptable oil base, such as an ester of a long chain fatty acid (*e.g*., ethyl oleate), fatty oils such as sesame oil, triglycerides, or liposomes.

Parenteral formulations of the compositions can contain various carriers such as vegetable oils, dimethylacetamide, dimethylformamide, ethyl lactate, ethyl carbonate, isopropyl myristate, ethanol, polyols (glycerol, propylene glycol, liquid polyethylene glycol, and the like).

Aqueous injection suspensions can also contain substances that increase the viscosity of the suspension, such as sodium carboxymethyl cellulose, sorbitol, or dextran. Non-lipid polycationic amino polymers can also be used for delivery. Optionally, the suspension can also contain suitable stabilizers or agents that increase the solubility of the compounds to allow for the preparation of highly concentrated solutions.

Pharmaceutical compositions of the present invention can also be formulated to permit injectable, long-term, deposition.

The pharmaceutical compositions of the present invention can be administered topically.

A topical semi-solid ointment formulation typically contains a concentration of the active ingredient from about 1 to 20%, *e.g*., 5 to 10%, in a carrier such as a pharmaceutical cream base. Various formulations for topical use include drops, tinctures, lotions, creams, solutions, and ointments containing the active ingredient and various supports and vehicles. In other transdermal formulations, typically in patch-delivered formulations, the pharmaceutically active compound is formulated with one or more skin penetrants, such as 2-N-methyl-pyrrolidone (NMP) or Azone.

Inhalation formulations can also readily be formulated. For inhalation, various powder and liquid formulations can be prepared.

The pharmaceutically active compound in the pharmaceutical compositions of the present inention can be provided as the salt of a variety of acids, including but not limited to hydrochloric, sulfuric, acetic, lactic, tartaric, malic, and succinic acid. Salts tend to be more soluble in aqueous or other protonic solvents than are the corresponding free base forms.

After pharmaceutical compositions have been prepared, they are packaged in an appropriate container and labeled for treatment of an indicated condition.

The active compound will be present in an amount effective to achieve the intended purpose. The determination of an effective dose is well within the capability of those skilled in the art.

A "therapeutically effective dose" refers to that amount of active ingredient - for example NHELP1 protein, fusion protein, or fragments thereof, antibodies specific for NHELP1, agonists, antagonists or inhibitors of NHELP1 - which ameliorates the signs or symptoms of the disease or prevents progression thereof; as would be understood in the medical arts, cure, although desired, is not required.

The therapeutically effective dose of the pharmaceutical agents of the present invention can be estimated initially by *in vitro* tests, such as cell culture assays, followed by assay in model animals, usually mice, rats, rabbits, dogs, or pigs. The animal model can also be used to determine an initial useful concentration range and route of administration.

For example, the ED50 (the dose therapeutically effective in 50% of the population) and LD50 (the dose lethal to 50% of the population) can be determined in one or more cell culture of animal model systems. The dose ratio of toxic to therapeutic effects is the therapeutic index, which can be expressed as LD50/ED50. Pharmaceutical compositions that exhibit large therapeutic indices are particularly useful.

The data obtained from cell culture assays and animal studies is used in formulating an initial dosage range for human use, and preferably provides a range of circulating concentrations that includes the ED50 with little or no toxicity. After administration, or between successive administrations, the circulating concentration of active agent varies within this range depending upon pharmacokinetic factors well known in the art, such as the dosage form employed, sensitivity of the patient, and the route of administration.

The exact dosage will be determined by the practitioner, in light of factors specific to the subject requiring treatment. Factors that can be taken into account by the practitioner include the severity of the disease state, general health of the subject, age, weight, gender of the subject, diet, time and frequency of administration, drug combination(s), reaction sensitivities, and tolerance/response to therapy. Long-acting pharmaceutical compositions can be administered every 3 to 4 days, every week, or once every two weeks depending on half-life and clearance rate of the particular formulation.

Normal dosage amounts may vary from 0.1 to 100,000 micrograms, up to a total dose of about 1 g, depending upon the route of administration. Where the therapeutic agent is a protein or antibody of the present invention, the therapeutic protein or antibody agent typically is administered at a daily dosage of 0.01 mg to 30 mg/kg of body weight of the patient (e.g., 1mg/kg to 5 mg/kg). The pharmaceutical formulation can be administered in multiple doses per day, if desired, to achieve the total desired daily dose.

Guidance as to particular dosages and methods of delivery is provided in the literature and generally available to practitioners in the art. Those skilled in the art will employ different formulations for nucleotides than for proteins or their inhibitors. Similarly, delivery of polynucleotides or polypeptides will be specific to particular cells, conditions, locations, etc.

Conventional methods, known to those of ordinary skill in the art of medicine, can be used to administer the pharmaceutical formulation(s) of the present invention to the patient. The pharmaceutical compositions of the present invention can be administered alone, or in combination with other therapeutic agents or interventions.

### THERAPEUTIC METHODS

The present invention further provides methods of treating subjects having defects in NHELP1 - *e.g.,* in expression, activity, distribution, localization, and/or solubility of NHELP1 - which can manifest as a disorder of brain, adrenal, bone marrow, liver, testis or prostate function. As used herein, "treating" includes all medically-acceptable types of therapeutic intervention, including palliation and prophylaxis (prevention) of disease.

In one embodiment of the therapeutic methods of the present invention, a therapeutically effective amount of a pharmaceutical composition comprising NHELP1 protein, fusion, fragment or derivative thereof is administered to a subject with a clinically-significant NHELP1 defect.

Protein compositions are administered, for example, to complement a deficiency in native NHELP1. In other embodiments, protein compositions are administered as a vaccine to elicit a humoral and/or cellular immune response to NHELP1. The immune response can be used to modulate activity of NHELP1 or, depending on the immunogen, to immunize against aberrant or aberrantly expressed forms, such as mutant or inappropriately expressed isoforms. In yet other embodiments, protein fusions having a toxic moiety are administered to ablate cells that aberrantly accumulate NHELP1.

In another embodiment of the therapeutic methods of the present invention, a therapeutically effective amount of a pharmaceutical composition comprising nucleic acid of the present invention is administered. The nucleic acid can be delivered in a vector that drives expression of NHELP1 protein, fusion, or fragment thereof, or without such vector.

Nucleic acid compositions that can drive expression of NHELP1 are administered, for example, to complement a deficiency in native NHELP1, or as DNA vaccines. Expression vectors derived from virus, replication deficient retroviruses, adenovirus, adeno-associated (AAV) virus, herpes virus, or vaccinia virus can be used - *see, e.g.,* Cid-Arregui (ed.), Viral Vectors: Basic Science and Gene Therapy, Eaton Publishing Co., 2000 (ISBN: 188129935X) - as can plasmids.

Antisense nucleic acid compositions, or vectors that drive expression of NHELP1 antisense nucleic acids, are administered to downregulate transcription and/or translation of NHELP1 in circumstances in which excessive production, or production of aberrant protein, is the pathophysiologic basis of disease.

Antisense compositions useful in therapy can have sequence that is complementary to coding or to noncoding regions of the NHELP1 gene. For example, oligonucleotides derived from the transcription initiation site, *e.g.,* between positions -10 and +10 from the start site, are particularly useful.

Catalytic antisense compositions, such as ribozymes, that are capable of sequence-specific hybridization to NHELP1 transcripts, are also useful in therapy. *See, e.g.,* Phylactou, *Adv. Drug Deliv. Rev.* 44(2-3):97-108 (2000); Phylactou *et al., Hum. Mol.* Genet. 7(10):1649-53 (1998); Rossi, *Ciba Found. Symp.* 209:195-204 (1997); and Sigurdsson *et al., Trends Biotechnol.* 13(8):286-9 (1995), the disclosures of which are incorporated herein by reference in their entireties.

Other nucleic acids useful in the therapeutic methods of the present invention are those that are capable of triplex helix formation in or near the NHELP1 genomic locus. Such triplexing oligonucleotides are able to inhibit transcription, Intody *et al.*, Nucleic Acids Res. 28(21):4283-90 (2000); McGuffie *et al*., *Cancer Res.* 60(14):3790-9 (2000), the disclosures of which are incorporated herein by reference, and pharmaceutical compositions comprising such triplex forming oligos (TFOs) are administered in circumstances in which excessive production, or production of aberrant protein, is a pathophysiologic basis of disease.

In another embodiment of the therapeutic methods of the present invention, a therapeutically effective amount of a pharmaceutical composition comprising an antibody (including fragment or derivative thereof) of the present invention is administered. As is well known, antibody compositions are administered, for example, to antagonize activity of NHELP1, or to target therapeutic agents to sites of NHELP1 presence and/or accumulation.

In another embodiment of the therapeutic methods of the present invention, a pharmaceutical composition comprising a non-antibody antagonist of NHELP1 is administered. Antagonists of NHELP1 can be produced using methods generally known in the art. In particular, purified NHELP1 can be used to screen libraries of pharmaceutical agents, often combinatorial libraries of small molecules, to identify those that specifically bind and antagonize at least one activity of NHELP1.

In other embodiments a pharmaceutical composition comprising an agonist of NHELP1 is administered. Agonists can be identified using methods analogous to those used to identify antagonists.

In still other therapeutic methods of the present invention, pharmaceutical compositions comprising host cells that express NHELP1, fusions, or fragments thereof can be administered. In such cases, the cells are typically autologous, so as to circumvent xenogeneic or allotypic rejection, and are administered to complement defects in NHELP1 production or activity.

In other embodiments, pharmaceutical compositions comprising the NHELP1 proteins, nucleic acids, antibodies, antagonists, and agonists of the present invention can be administered in combination with other appropriate therapeutic agents. Selection of the appropriate agents for use in combination therapy can be made by one of ordinary skill in the art according to conventional pharmaceutical principles. The combination of therapeutic agents or approaches can act additively or synergistically to effect the treatment or prevention of the various disorders described above, providing greater therapeutic efficacy and/or permitting use of the pharmaceutical compositions of the present invention using lower dosages, reducing the potential for adverse side effects.

### TRANSGENIC ANIMALS AND CELLS

In another aspect, the invention provides transgenic cells and non-human organisms comprising NHELP1 isoform nucleic acids, and transgenic cells and non-human organisms with targeted disruption of the endogenous orthologue of the human NHELP1 gene.

The cells can be embryonic stem cells or somatic cells. The transgenic non-human organisms can be chimeric, nonchimeric heterozygotes, and nonchimeric homozygotes.

### DIAGNOSTIC METHODS

The nucleic acids of the present invention can be used as nucleic acid probes to assess the levels of NHELP1 mRNA in brain, adrenal, bone marrow, liver, testis and prostate, and antibodies of the present invention can be used to assess the expression levels of NHELP1 proteins in brain, adrenal, bone marrow, liver, testis and prostate to diagnose cancer.

The following examples are offered for purpose of illustration, not limitation.

### EXAMPLE 1

### Identification and Characterization of cDNAs Encoding NHELP1 Proteins

Predicating our gene discovery efforts on use of genome-derived single exon probes and hybridization to genome-derived single exon microarrays - an approach that we have previously demonstrated will readily identify novel genes that have proven refractory to mRNA-based identification efforts - we identified an exon in raw human genomic sequence that is particularly expressed in human brain, adrenal, bone marrow, liver, testis and prostate.

Briefly, bioinformatic algorithms were applied to human genomic sequence data to identify putative exons. Each of the predicted exons was amplified from genomic DNA, typically centering the putative coding sequence within a larger amplicon that included flanking noncoding sequence. These genome-derived single exon probes were arrayed on a support and expression of the bioinformatically predicted exons assessed through a series of simultaneous two-color hybridizations to the genome-derived single exon microarrays.

The approach and procedures are further described in detail in Penn *et al.,* "Mining the Human Genome using Microarrays of Open Reading Frames," *Nature Genetics* 26:315-318 (2000); commonly owned and copending U.S. patent application nos. 09/864,761, filed May 23, 2001, 09/774,203, filed January 29, 2001, and 09/632,366, filed August 3, 2000, the disclosures of which are incorporated herein by reference in their entireties.

Using a graphical display particularly designed to facilitate computerized query of the resulting exon-specific expression data, as further described in commonly owned and copending U.S. patent application no. 09/864,761, filed May 23, 2001, 09/774,203, filed January 29, 2001 and 09/632,366, filed August 3, 2000, the disclosures of which are incorporated herein by reference in their entireties, one exon was identified that is expressed in all the human tissues tested; subsequent analysis revealed that the exon belong to a novel gene.

Table 1 summarizes the microarray expression data obtained using genome-derived single exon probe corresponding to exon 9. The probe was completely sequenced on both strands prior to its use on a genome-derived single exon microarray; sequencing confirmed the exact chemical structure of the probe. An added benefit of sequencing is that it placed us in possession of a set of single base-incremented fragments of the sequenced nucleic acid, starting from the sequencing primer's 3' OH. (Since the single exon probes were first obtained by PCR amplification from genomic DNA, we were of course additionally in possession of an even larger set of single base incremented fragments of each of the single exon probes, each fragment corresponding to an extension product from one of the two amplification primers.)

Signals are normalized values measured and calculated as further described in commonly owned and copending U.S. patent application nos. 09/864,761, filed May 23, 2001, 09/774,203, filed January 29, 2001, 09/632,366, filed August 3, 2000, and U.S. provisional patent application no. 60/207,456, filed May 26, 2000, the disclosures of which are incorporated herein by reference in their entireties.

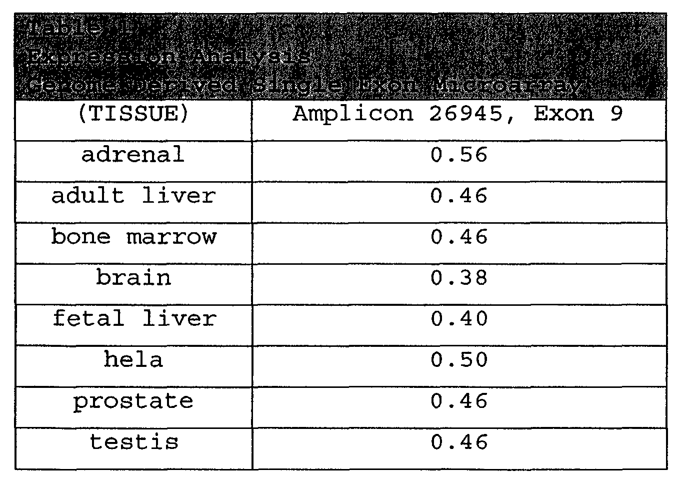

As shown in Table 1, significant expression of exon 9 was seen in brain, adrenal, bone marrow, liver, testis and prostate, as well as a cell line, hela.

Marathon-Ready™ brain cDNA (Clontech Laboratories, Palo Alto, CA, USA) was used as a substrate for standard RACE (rapid amplification of cDNA ends) to obtain a cDNA clone that spans 2.1 kilobases and appears to contain the entire coding region of the gene to which the exon contributes; for reasons described below, we termed this cDNA NHELP1. Marathon-Ready™ cDNAs are adaptor-ligated double stranded cDNAs suitable for 3' and 5' RACE. Chenchik *et al., BioTechniques* 21:526-532 (1996); Chenchik *et al., CLONTECHniques* X(1):5-8 (January 1995). RACE techniques are described, *inter alia,* in the Marathon-Ready™ cDNA User Manual (Clontech Labs., Palo Alto, CA, USA, March 30, 2000, Part No. PT1156-1 (PR03517)), Ausubel *et al.* (eds.), Short Protocols in Molecular Biology : A Compendium of Methods from Current Protocols in Molecular Biology, 4^{th} edition (April 1999), John Wiley & Sons (ISBN: 047132938X) and Sambrook *et al.* (eds.), Molecular Cloning: A Laboratory Manual (3rd ed.), Cold Spring Harbor Laboratory Press (2000) (ISBN: 0879695773), the disclosures of which are incorporated herein by reference in their entireties.

The NHELP1 cDNA was sequenced on both strands using a MegaBACE™ sequencer (Amersham Biosciences, Sunnyvale, CA, USA). Sequencing both strands provided us with the exact chemical structure of the cDNA, which is shown in FIG. 3 and further presented in the SEQUENCE LISTING as SEQ ID NO: 1, and placed us in actual physical possession of the entire set of single-base incremented fragments of the sequenced clone, starting at the 5' and 3' termini.

As shown in FIG. 3, the NHELP1 cDNA spans 2078 nucleotides and contains an open reading frame from nucleotide 50 through and including nt 1987 (inclusive of termination codon), predicting a protein of 645 amino acids with a (posttranslationally unmodified) molecular weight of 72.6 kD. The clone appears full length, with the reading frame opening starting with a methionine and terminating with a stop codon.

BLAST query of genomic sequence identified 7 BACS, spanning at least 80k, that constitute the minimum set of clones encompassing the cDNA sequence. Based upon the known origin of the BACs (GenBank accession numbers AC013592.5, AC013641.4, AC013805.4, AC016934.18, AC026673.18, AC073242.4, AC073358.9), the NHELP1 gene can be mapped to human chromosome 3q23.

Comparison of the cDNA and genomic sequences identified 16 exons. Exon organization is listed in Table 2.

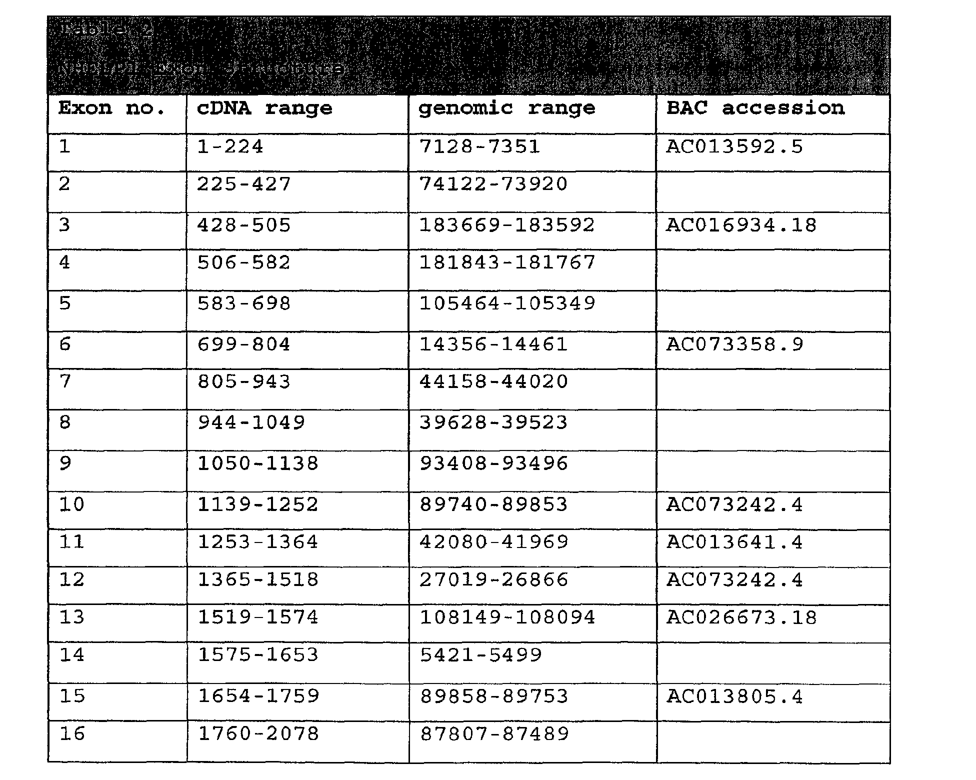

FIG. 2 schematizes the exon organization of the NHELP1 clone.

At the top are shown the seven bacterial artificial chromosomes (BACs), with GenBank accession numbers, that span the NHELP1 locus. The genome-derived single-exon probe first used to demonstrate expression from this locus is shown below the BACs and labeled "592". The 592 bp probe includes sequence drawn from exon 9, with additional sequence from introns 8 and 9.

As shown in FIG. 2, NHELP1, encoding a protein of 645 amino acids, comprises exons 1 - 16. Predicted molecular weight, prior to any post_translational modification, is 72.6 kD. The clone appears full length, with the reading frame opening starting with a methionine and terminating with a stop codon.

As further discussed in the examples herein, expression of NHELP1 was assessed using hybridization to genome-derived single exon microarrays. Microarray analysis of exon nine showed expression in all tissues tested, including fetal liver, adult liver, brain, prostate, adrenal gland, testis and bone marrow.

The sequence of the NHELP1 cDNA was used as a BLAST query into the GenBank nr and dbEst databases. The nr database includes all non-redundant GenBank coding sequence translations, sequences derived from the 3-dimensional structures in the Brookhaven Protein Data Bank (PDB), sequences from SwissProt, sequences from the protein information resource (PIR), and sequences from protein research foundation (PRF). The dbEst (database of expressed sequence tags) includes ESTs, short, single pass read cDNA (mRNA) sequences, and cDNA sequences from differential display experiments and RACE experiments.

BLAST search identified multiple human and mouse ESTs, one EST from rat (BF404144.1) and one from zebrafish (AW566665.1) as having sequence closely related to NHELP1.

Globally, the human NHELP1 protein resembles the human sodium/hydrogen exchanger isoform 7 (GenBank accession: AAK54508.1, the NHELP1 protein with 59% amino acid identity and 71% amino acid similarity over 653 amino acids). NHELP1 aslo resembles the human sodium/hydrogen exchanger isoform 6 (GenBank accession: AAC39643.1, the NHELP1 protein with 60% amino acid identity and 73% amino acid similarity over 636 amino acids).

Motif searches using Pfam (http://pfam.wustl.edu), SMART (http://smart.embl- heidelberg.de), and PROSITE pattern and profile databases (http://www.expasy.ch/prosite), identified one known domains shared with human sodium/hydrogen exchangers.

FIG. 1 shows the domain structure of NHELP1.

As schematized in FIG. 1, the newly isolated gene product shares certain protein domains and an overall structural organization with other human sodium/hydrogen exchangers. The shared structural features strongly imply that NHELP1 plays a role similar to that of other human sodium/hydrogen exchangers in maintaining cation ion homeostasis.

Like the other human sodium/hydrogen exchanger isoforms, NHELP1 contains a Na_H_Exchanger domain. In NHELP1, the Na_H_Exchanger motif ocurrs at amino acids 128-454 (http://www.ncbi.nlm.nih.gov/Structure/cdd/wrpsb.cgi/). Other signatures of the newly isolated NHELP1 proteins were identified by searching the PROSITE database (http://www.expasy.ch/tools/scnpsitl.html). These include three N-glycosylation sites (96 - 99, 352 - 355, 516 - 519), four protein kinase C phosphorylation sites (9 - 11, 149 - 151, 258 - 260 and 522 - 524), one tyrosine kinase phosphorylation site (6 - 14), a leucine zipper (311 - 332), a amidation site, seven Casein kinase II phosphorylation sites, and eleven *N*-myristoylation sites.

Possession of the genomic sequence permitted search for promoter and other control sequences for the NHELP1 gene. A putative transcriptional control region, inclusive of promoter and downstream elements, was defined as 1 kb around the transcription start site, itself defined as the first nucleotide of the NHELP1 cDNA clone. The region, drawn from sequence of BAC AC013592.5, has the sequence given in SEQ ID NO: 40, which lists 1000 nucleotides before the transcription start site.

Transcription factor binding sites were identified using a web based program (http://motif.genome.ad.jp/), including binding sites for CdxA (681 - 687 and 710 - 716), AP-1 (600 - 608), as well as c-Ets-1 (870 - 879, with numbering according to SEQ ID NO: 40), amongst others.

We have thus identified a newly described human gene, NHELP1, which shares certain protein domains and an overall structural organization with human sodium/hydrogen exchanger isoforms. The shared structural features strongly imply that the NHELP1 protein plays a role similar to other sodium/hydrogen exchangers, in maintaining cation ion homeostasis, making the NHELP1 proteins and nucleic acids clinically useful diagnostic markers and potential therapeutic agents for cancer and AIDS.

### EXAMPLE 2

### Preparation and Labeling of Useful Fragments of NHELP1

Useful fragments of NHELP1 are produced by PCR, using standard techniques, or solid phase chemical synthesis using an automated nucleic acid synthesizer. Each fragment is sequenced, confirming the exact chemical structure thereof.

The exact chemical structure of preferred fragments is provided in the attached SEQUENCE LISTING, the disclosure of which is incorporated herein by reference in its entirety. The following summary identifies the fragments whose structures are more fully described in the SEQUENCE LISTING:
SEQ ID NO: 1 (nt, full length NHELP1 cDNA)
SEQ ID NO: 2 (nt, cDNA ORF)
SEQ ID NO: 3 (aa, full length NHELP1 protein)
SEQ ID NO: 4 (nt, (nt 1 - 1528) portion of NHELP1)
SEQ ID NO: 5 (nt, 5' UT portion of SEQ ID NO: 4)
SEQ ID NO: 6 (nt, coding region of SEQ ID NO: 4)
SEQ ID NO: 7 (aa, (aa 1 - 493) CDS entirely within portion of NHELP1)
SEQ ID NOs: 8 - 23 (nt, exons 1 - 16 (from genomic sequence))
SEQ ID NOs: 24 - 39 (nt, 500 bp genomic amplicon centered about exons 1 - 16)
SEQ ID NO: 40 (nt, 1000 bp putative promoter)
SEQ ID NOs: 41 - 1552 (nt, 17-mers scanning (nt 1 - 1528) portion of NHELP1)
SEQ ID NOs: 1553 - 3056 (nt, 25-mers scanning (nt 1 - 1528) portion of NHELP1)

Upon confirmation of the exact structure, each of the above-described nucleic acids of confirmed structure is recognized to be immediately useful as a NHELP1-specific probe.

For use as labeled nucleic acid probes, the above-described NHELP1 nucleic acids are separately labeled by random priming. As is well known in the art of molecular biology, random priming places the investigator in possession of a near-complete set of labeled fragments of the template of varying length and varying starting nucleotide.

The labeled probes are used to identify the NHELP1 gene on a Southern blot, and are used to measure expression of NHELP1 mRNA on a northern blot and by RT-PCR, using standard techniques.

### EXAMPLE 3

### Production of NHELP1 Protein

The full length NHELP1 cDNA clone is cloned into the mammalian expression vector pcDNA3.1/HISA (Invitrogen, Carlsbad, CA, USA), transfected into COS7 cells, transfectants selected with G418, and protein expression in transfectants confirmed by detection of the anti-Xpress™ epitope according to manufacturer's instructions. Protein is purified using immobilized metal affinity chromatography and vector-encoded protein sequence is then removed with enterokinase, per manufacturer's instructions, followed by gel filtration and/or HPLC.

Following epitope tag removal, NHELP1 protein is present at a concentration of at least 70%, measured on a weight basis with respect to total protein (i.e., w/w), and is free of acrylamide monomers, bis acrylamide monomers, polyacrylamide and ampholytes. Further HPLC purification provides NHELP1 protein at a concentration of at least 95%, measured on a weight basis with respect to total protein (i.e., w/w).

### EXAMPLE 4

### Production of Anti-NHELP1 Antibody

Purified proteins prepared as in Example 3 are conjugated to carrier proteins and used to prepare murine monoclonal antibodies by standard techniques. Initial screening with the unconjugated purified proteins, followed by competitive inhibition screening using peptide fragments of the NHELP1, identifies monoclonal antibodies with specificity for NHELP1.

### EXAMPLE 5

### Use of NHELP1 Probes and Antibodies for Diagnosis of Cancer

After informed consent is obtained, samples are drawn from disease tissue or cells and tested for NHELP1 mRNA levels by standard techniques and tested additionally for NHELP1 protein levels using anti-NHELP1 antibodies in a standard ELISA.

### EXAMPLE 6

### Use of NHELP1 Nucleic Acids, Proteins, and Antibodies in Therapy

Once over-expression of NHELP1 is detected in patients, NHELP1 antisense RNA or NHELP1-specific antibody is introduced into disease cells to reduce the amount of the protein.

Once mutations of NHELP1 have been detected in patients, normal NHELP1 is reintroduced into the patient's disease cells by introduction of expression vectors that drive NHELP1 expression or by introducing NHELP1 proteins into cells. Antibodies for the mutated forms of NHELP1 are used to block the function of the abnormal forms of the protein.

### EXAMPLE 7

### NHELP1 Disease Associations

Diseases that map to the NHELP1 chromosomal region are shown in Table 3. Mutations of NHELP1 might lead to the disease(s) listed below. Alternatively, mutations of NHELP1 might lead to some other human disorder(s) as well.

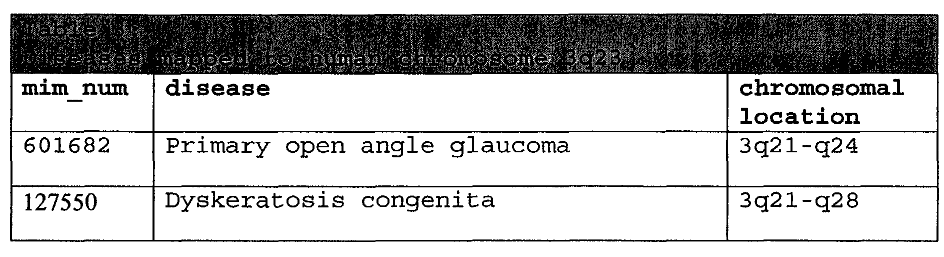

All patents, patent publications, and other published references mentioned herein are hereby incorporated by reference in their entireties as if each had been individually and specifically incorporated by reference herein. While preferred illustrative embodiments of the present invention are described, one skilled in the art will appreciate that the present invention can be practiced by other than the described embodiments, which are presented for purposes of illustration only and not by way of limitation. The present invention is limited only by the claims that follow.

## Claims

1. An isolated nucleic acid that encodes a Na+/H+ exchanger protein, comprising:
(a) a nucleotide sequence selected from the group consisting of:
(i) SEQ_ID_NO: 1;
(ii) the complement of the sequences set forth in (i);
(iii) the nucleotide sequence of SEQ_ID_NO: 2;
(iv) a degenerate variant of the sequences set forth in (iii); and
(v) the complement of the sequences set forth in (iii) and (iv); or
(b) a nucleotide sequence selected from the group consisting of:
(i) a nucleotide sequence that encodes a polypeptide having the sequence of SEQ_ID_No: 3;
(ii) a nucleotide sequence that encodes a polypeptide having the sequence of SEQ_ID_No: 3, with conservative amino acid substitutions; and
(iii) the complement of the sequences set forth in (i) and (ii),
wherein said isolated nucleic acid comprising a nucleotide sequence selected from group (b) is no more than about 100 kb in length.

2. The isolated nucleic acid of claim 1 wherein said nucleic acid, or the complement of said nucleic acid, encodes a polypeptide.

3. The isolated nucleic acid of either of claims 1 or 2, wherein said nucleic acid, or the complement of said nucleic acid, is expressed in brain, adrenal, bone marrow, liver, testis and prostate, and/or a cell line, hela.

4. A nucleic acid probe, comprising:
(a) the nucleic acid of claim 1; or
(b) at least 17 contiguous nucleotides of SEQ_ID_NO: 4,
wherein said probe according to (b) is no longer than about 100 kb in length.

5. The probe of claim 4, wherein said probe is detectably labeled.

6. The probe of either of claims 4 or 5, attached to a substrate.

7. A microarray, wherein at least one probe of said array is a probe according to claim 4.

8. The isolated nucleic acid molecule of any of claims 1 - 3, wherein said nucleic acid molecule is operably linked to one or more expression control elements.

9. A replicable vector comprising a nucleic acid molecule of any of claims 1 - 3 or 8.

10. A host cell transformed to contain the nucleic acid molecule of any one of claims 1 - 3 or 8 or 9, or the progeny thereof.

11. A method for producing a polypeptide, the method comprising: culturing the host cell of claim 10 under conditions in which the protein encoded by said nucleic acid molecule is expressed.

12. An isolated polypeptide produced by the method of claim 11.

13. An isolated polypeptide, comprising:
(a) an amino acid sequence of SEQ_ID_NO: 3;
(b) an amino acid sequence having at least 65% amino acid sequence identity to that of (a);
(c) an amino acid sequence according to (a) in which at least 95% of deviations from the sequence of (a) are conservative substitutions; or
(d) a fragment of at least 8 contiguous amino acids of any of (a) - (c).

14. A fusion protein, said fusion protein comprising a polypeptide of claim 13 fused to a heterologous amino acid sequence.

15. An isolated antibody, or antigen-binding fragment or derivative thereof, the binding of which can be competitively inhibited by a polypeptide of claim 13.

16. A transgenic non-human animal modified to contain the nucleic acid molecule of any one of claims 1 - 3 or 8 or 9.

17. A transgenic non-human animal unable to express the endogenous orthologue of the nucleic acid molecule of any one of claims 1 - 3 or 8 or 9.

18. A method of identifying agents that modulate the expression of NHELP1, the method comprising:
contacting a cell or tissue sample believed to express NHELP1 with a chemical or biological agent, and then
comparing the amount of NHELP1 expression in said cell or tissue sample with that of a control,
changes in the amount relative to control identifying an agent that modulates expression of NHELP1.

19. A method of identifying agonists and antagonists of NHELP1, the method comprising:
contacting a cell or tissue sample believed to express NHELP1 with a chemical or biological agent, and then
comparing the activity of NHELP1 with that of a control,
increased activity relative to a control identifying an agonist, decreased activity relative to a control identifying an antagonist.

20. A purified agonist of the polypeptide of claim 13.

21. A purified antagonist of the polypeptide of claim 13.

22. A method of identifying a specific binding partner for a polypeptide according to claim 13, the method comprising:
contacting said polypeptide to a potential binding partner; and
determining if the potential binding partner binds to said polypeptide.

23. The method of claim 22, wherein said contacting is performed *in vivo.*

24. A purified binding partner of the polypeptide of claim 13.

25. A method for detecting a target nucleic acid in a sample, said target being a nucleic acid according to any one of claims 1 - 3 or 8 or 9, the method comprising:
a) hybridizing the sample with a probe comprising at least 17 contiguous nucleotides of a sequence complementary to said target nucleic acid in said sample under high stringency hybridization conditions, and
b) detecting the presence or absence, and optionally the amount, of said binding.

26. A method of diagnosing a disease caused by mutation in NHELP1, comprising:
detecting said mutation in a sample of nucleic acids that derives from a subject suspected to have said disease.

27. A method of diagnosing or monitoring a disease caused by altered expression of NHELP1, comprising:
determining the level of expression of NHELP1 in a sample of nucleic acids or proteins that derives from a subject suspected to have said disease,
alterations from a normal level of expression providing diagnostic and/or monitoring information.

28. A diagnostic composition comprising the nucleic acid of any of claims 1 - 3, said nucleic acid being detectably labeled.

29. The diagnostic composition of claim 28, wherein said composition is further suitable for *in vivo* administration.

30. A diagnostic composition comprising the polypeptide of claim 13, said polypeptide being detectably labeled.

31. The diagnostic composition of claim 30, wherein said composition is further suitable for *in vivo* administration.

32. A diagnostic composition comprising the antibody, or antigen-binding fragment or derivative thereof, of claim 15.

33. The diagnostic composition of claim 32, wherein said antibody or antigen-binding fragment or derivative thereof is detectably labeled.

34. The diagnostic composition of claim 33, wherein said composition is further suitable for *in vivo* administration.

35. A pharmaceutical composition comprising the nucleic acid of any one of claims 1 - 3 or 8 or 9 and a pharmaceutically acceptable excipient.

36. A pharmaceutical composition comprising the polypeptide of claim 13 and a pharmaceutically acceptable excipient.

37. A pharmaceutical composition comprising the antibody or antigen-binding fragment or derivative thereof of claim 15 and a pharmaceutically acceptable excipient.

38. A pharmaceutical composition comprising the agonist of claim 20 and a pharmaceutically acceptable excipient.

39. A pharmaceutical composition comprising the antagonist of claim 21 and a pharmaceutically acceptable excipient.

40. Nucleic acid of any one of claims 1 - 3 or 8 or 9 for use in therapy.

41. Use of the nucleic acid of any one of claims 1 - 3 or 8 or 9 for the manufacture of a medicament for the treatment or prevention of a disorder associated with decreased expression or activity of human NHELP1.

42. Polypeptide of claim 13 for use in therapy.

43. Use of the polypeptide of claim 13 for the manufacture of a medicament for the treatment or prevention of a disorder associated with decreased expression of activity of human NHELP1.

44. Use of the agonist of claim 20 for the manufacture of a medicament for the treatment or prevention of a disorder associated with decreased expression or activity of human NHELP1.

45. Antibody or antigen-binding fragment or derivative thereof of claim 15 for use in therapy.

46. Use of the antibody or antigen-binding fragment or derivative thereof of claim 15 for the manufacture of a medicament for the treatment or prevention of a disorder associated with increased expression or activity of human NHELP1.

47. Use of the antagonist of claim 21 for the manufacture of a medicament for the treatment or prevention of a disorder associated with increased expression or activity of human NHELP1.

48. A method of modulating the expression of a nucleic acid according to any of claims 1 - 3 or 8 or 9, the method comprising:
administering an effective amount of an agent which modulates the expression of a nucleic acid according to any of claims 1 - 3 or 8 or 9.

49. A method of modulating at least one activity of a polypeptide according to claim 13, the method comprising:
administering an effective amount of an agent which modulates at least one activity of a polypeptide according to claim 13.


<110>
<120> HUMAN SODIUM-HYDROGEN EXCHANGER LIKE PROTEIN 1

<130> AEOMICA-34

<150> PCT/US01/00666
<151> 2001-01-30

<160> 3056

<170> Aeomica Sequence Listing Engine

<210> 1
<211> 2078
<212> DNA
<213> Homo sapiens

<400> 1
atagccctta tccaggtttt tatctaagga atcccaagaa gactggggaa tggagagaca 60
gtcaagggtt atgtcagaaa aggatgagta tcagtttcaa catcagggag cggtggagct 120
gcttgtcttc aattttttgc tcatccttac cattttgaca atctggttat ttaaaaatca 180
tcgattccgc ttcttgcatg aaactggagg agcaatggtg tatggcctta taatgggact 240
aattttacga tatgctacag caccaactga tattgaaagt ggaactgtct atgactgtgt 300
aaaactaact ttcagtccat caactctgct ggttaatatc actgaccaag tttatgaata 360
taaatacaaa agagaaataa gtcagcacaa catcaatcct catcaaggaa atgctatact 420
tgaaaagatg acatttgatc cagaaatctt cttcaatgtt ttactgccac caattatatt 480
tcatgcagga tatagtctaa agaagagaca cttttttcaa aacttaggat ctattttaac 540
gtatgccttc ttgggaactg ccatctcctg catcgtcata gggttaatta tgtatggttt 600
tgtgaaggct atgatacatg ctggccagct gaaaaatgga gactttcatt tcactgactg 660
tttatttttt ggttcactga tgtctgctac agatccagtg acagtgctgg ccattttcca 720
tgaactgcac gtcgaccctg acctgtacac actcttgttt ggagagagtg tgttgaatga 780
tgcagtggcc atagtcctta catattctat atccatttac agtcccaagg agaatccaaa 840
tgcatttgat gccgcagcat tcttccagtc tgtggggaat ttcctgggaa tcttcgctgg 900
ctcatttgca atggggtctg cgtatgccat catcacagca ctgttgacca aatttaccaa 960
gctgtgtgag ttcccgatgc tggaaaccgg cctgtttttc ctgctttctt ggagtgcctt 1020
cctgtctgcc gaggctgccg gcctaacagg gatagttgct gttctcttct gtggagtcac 1080
acaagcacat tatacctaca acaatctgtc ttcggattcc aaaataagaa ctaaacagtt 1140
gtttgaattt atgaactttt tggcggagaa cgtcatcttc tgttacatgg gcctggcact 1200
gttcacgttc cagaatcata tctttaatgc tctttttata cttggagcct ttctagcaat 1260
ttttgttgcc agagcctgca acatatatcc cctctccttc ctcctgaatc taggccgaaa 1320
acagaagatc ccctggaact ttcagcacat gatgatgttt tcaggtttgc gaggagcgat 1380
cgcatttgcc ttagctattc ggaacacaga atctcagccc aaacaaatga tgtttaccac 1440
tacgctgctc ctcgtgttct tcactgtctg ggtatttgga ggaggaacaa cccccatgtt 1500
gacttggctt cagatcagag ttggcgtgga cctggatgaa aatctgaagg aggacccctc 1560
ctcacaacac caggaagcaa ataacttgga taaaaacatg acgaaagcag agagtgctcg 1620
gctcttcaga atgtggtata gctttgacca caagtatctg aaaccaattt taacccactc 1680
tggtcctccg ctgactacaa cattacctga atggtgtggt ccgatttcca ggctgcttac 1740
cagtcctcaa gcctatgggg aacagctaaa agaggatgat gtggaatgca ttgtaaacca 1800
ggatgaacta gccataaatt accaggagca agcctcctca ccctgcagtc ctcctgcaag 1860
gctaggtctg gaccagaaag cttcacccca gacgccaggc aaggaaaaca tttatgaggg 1920
agacctcggc ctgggaggct atgaactcaa gcttgagcaa actttgggtc aatcccagtt 1980
gaattaattg gcatgaagag tacagatgta atcacaagta atgcaagact cactgaggaa 2040
tacaagccaa gctgatgagg cagtacaggg gagaggct 2078

<210> 2
<211> 1938
<212> DNA
<213> Homo sapiens

<400> 2
atggagagac agtcaagggt tatgtcagaa aaggatgagt atcagtttca acatcaggga 60

gcggtggagc tgcttgtctt caattttttg ctcatcctta ccattttgac aatctggtta 120
tttaaaaatc atcgattccg cttcttgcat gaaactggag gagcaatggt gtatggcctt 180
ataatgggac taattttacg atatgctaca gcaccaactg atattgaaag tggaactgtc 240
tatgactgtg taaaactaac tttcagtcca tcaactctgc tggttaatat cactgaccaa 300
gtttatgaat ataaatacaa aagagaaata agtcagcaca acatcaatcc tcatcaagga 360
aatgctatac ttgaaaagat gacatttgat ccagaaatct tcttcaatgt tttactgcca 420
ccaattatat ttcatgcagg atatagtcta aagaagagac acttttttca aaacttagga 480
tctattttaa cgtatgcctt cttgggaact gccatctcct gcatcgtcat agggttaatt 540
atgtatggtt ttgtgaaggc tatgatacat gctggccagc tgaaaaatgg agactttcat 600
ttcactgact gtttattttt tggttcactg atgtctgcta cagatccagt gacagtgctg 660
gccattttcc atgaactgca cgtcgaccct gacctgtaca cactcttgtt tggagagagt 720
gtgttgaatg atgcagtggc catagtcctt acatattcta tatccattta cagtcccaag 780
gagaatccaa atgcatttga tgccgcagca ttcttccagt ctgtggggaa tttcctggga 840
atcttcgctg gctcatttgc aatggggtct gcgtatgcca tcatcacagc actgttgacc 900
aaatttacca agctgtgtga gttcccgatg ctggaaaccg gcctgttttt cctgctttct 960
tggagtgcct tcctgtctgc cgaggctgcc ggcctaacag ggatagttgc tgttctcttc 1020
tgtggagtca cacaagcaca ttatacctac aacaatctgt cttcggattc caaaataaga 1080
actaaacagt tgtttgaatt tatgaacttt ttggcggaga acgtcatctt ctgttacatg 1140
ggcctggcac tgttcacgtt ccagaatcat atctttaatg ctctttttat acttggagcc 1200
tttctagcaa tttttgttgc cagagcctgc aacatatatc ccctctcctt cctcctgaat 1260
ctaggccgaa aacagaagat cccctggaac tttcagcaca tgatgatgtt ttcaggtttg 1320
cgaggagcga tcgcatttgc cttagctatt cggaacacag aatctcagcc caaacaaatg 1380
atgtttacca ctacgctgct cctcgtgttc ttcactgtct gggtatttgg aggaggaaca 1440
acccccatgt tgacttggct tcagatcaga gttggcgtgg acctggatga aaatctgaag 1500
gaggacccct cctcacaaca ccaggaagca aataacttgg ataaaaacat gacgaaagca 1560
gagagtgctc ggctcttcag aatgtggtat agctttgacc acaagtatct gaaaccaatt 1620
ttaacccact ctggtcctcc gctgactaca acattacctg aatggtgtgg tccgatttcc 1680
aggctgctta ccagtcctca agcctatggg gaacagctaa aagaggatga tgtggaatgc 1740
attgtaaacc aggatgaact agccataaat taccaggagc aagcctcctc accctgcagt 1800
cctcctgcaa ggctaggtct ggaccagaaa gcttcacccc agacgccagg caaggaaaac 1860
atttatgagg gagacctcgg cctgggaggc tatgaactca agcttgagca aactttgggt 1920
caatcccagt tgaattaa 1938

<210> 3
<211> 645
<212> PRT
<213> Homo sapiens

<400> 3
Met Glu Arg Gln Ser Arg Val Met Ser Glu Lys Asp Glu Tyr Gln Phe
1               5                   10                  15

Gln His Gln Gly Ala Val Glu Leu Leu Val Phe Asn Phe Leu Leu Ile
            20                  25                  30

Leu Thr Ile Leu Thr Ile Trp Leu Phe Lys Asn His Arg Phe Arg Phe
        35                  40                  45

Leu His Glu Thr Gly Gly Ala Met Val Tyr Gly Leu Ile Met Gly Leu
    50                  55                  60

Ile Leu Arg Tyr Ala Thr Ala Pro Thr Asp Ile Glu Ser Gly Thr Val
65                  70                  75                  80

Tyr Asp Cys Val Lys Leu Thr Phe Ser Pro Ser Thr Leu Leu Val Asn
                85                  90                  95

Ile Thr Asp Gln Val Tyr Glu Tyr Lys Tyr Lys Arg Glu Ile Ser Gln
            100                 105                 110

His Asn Ile Asn Pro His Gln Gly Asn Ala Ile Leu Glu Lys Met Thr

115                 120                 125

Phe Asp Pro Glu Ile Phe Phe Asn Val Leu Leu Pro Pro Ile Ile Phe
    130                 135                 140

His Ala Gly Tyr Ser Leu Lys Lys Arg His Phe Phe Gln Asn Leu Gly
145                 150                 155                 160

Ser Ile Leu Thr Tyr Ala Phe Leu Gly Thr Ala Ile Ser Cys Ile Val
                165                 170                 175

Ile Gly Leu Ile Met Tyr Gly Phe Val Lys Ala Met Ile His Ala Gly
            180                 185                 190

Gln Leu Lys Asn Gly Asp Phe His Phe Thr Asp Cys Leu Phe Phe Gly
        195                 200                 205

Ser Leu Met Ser Ala Thr Asp Pro Val Thr Val Leu Ala Ile Phe His
    210                 215                 220

Glu Leu His Val Asp Pro Asp Leu Tyr Thr Leu Leu Phe Gly Glu Ser
225                 230                 235                 240

Val Leu Asn Asp Ala Val Ala Ile Val Leu Thr Tyr Ser Ile Ser Ile
                245                 250                 255

Tyr Ser Pro Lys Glu Asn Pro Asn Ala Phe Asp Ala Ala Ala Phe Phe
            260                 265                 270

Gln Ser Val Gly Asn Phe Leu Gly Ile Phe Ala Gly Ser Phe Ala Met
        275                 280                 285

Gly Ser Ala Tyr Ala Ile Ile Thr Ala Leu Leu Thr Lys Phe Thr Lys
    290                 295                 300

Leu Cys Glu Phe Pro Met Leu Glu Thr Gly Leu Phe Phe Leu Leu Ser
305                 310                 315                 320

Trp Ser Ala Phe Leu Ser Ala Glu Ala Ala Gly Leu Thr Gly Ile Val
                325                 330                 335

Ala Val Leu Phe Cys Gly Val Thr Gln Ala His Tyr Thr Tyr Asn Asn
            340                 345                 350

Leu Ser Ser Asp Ser Lys Ile Arg Thr Lys Gln Leu Phe Glu Phe Met
        355                 360                 365

Asn Phe Leu Ala Glu Asn Val Ile Phe Cys Tyr Met Gly Leu Ala Leu
    370                 375                 380

Phe Thr Phe Gln Asn His Ile Phe Asn Ala Leu Phe Ile Leu Gly Ala
385                 390                 395                 400

Phe Leu Ala Ile Phe Val Ala Arg Ala Cys Asn Ile Tyr Pro Leu Ser
                405                 410                 415

Phe Leu Leu Asn Leu Gly Arg Lys Gln Lys Ile Pro Trp Asn Phe Gln
            420                 425                 430

His Met Met Met Phe Ser Gly Leu Arg Gly Ala Ile Ala Phe Ala Leu
        435                 440                 445

Ala Ile Arg Asn Thr Glu Ser Gln Pro Lys Gln Met Met Phe Thr Thr
    450                 455                 460

Thr Leu Leu Leu Val Phe Phe Thr Val Trp Val Phe Gly Gly Gly Thr
465                 470                 475                 480

Thr Pro Met Leu Thr Trp Leu Gln Ile Arg Val Gly Val Asp Leu Asp
                485                 490                 495

Glu Asn Leu Lys Glu Asp Pro Ser Ser Gln His Gln Glu Ala Asn Asn
            500                 505                 510

Leu Asp Lys Asn Met Thr Lys Ala Glu Ser Ala Arg Leu Phe Arg Met
        515                 520                 525

Trp Tyr Ser Phe Asp His Lys Tyr Leu Lys Pro Ile Leu Thr His Ser
    530                 535                 540

Gly Pro Pro Leu Thr Thr Thr Leu Pro Glu Trp Cys Gly Pro Ile Ser
545                 550                 555                 560

Arg Leu Leu Thr Ser Pro Gln Ala Tyr Gly Glu Gln Leu Lys Glu Asp
                565                 570                 575

Asp Val Glu Cys Ile Val Asn Gln Asp Glu Leu Ala Ile Asn Tyr Gln
            580                 585                 590

Glu Gln Ala Ser Ser Pro Cys Ser Pro Pro Ala Arg Leu Gly Leu Asp
        595                 600                 605

Gln Lys Ala Ser Pro Gln Thr Pro Gly Lys Glu Asn Ile Tyr Glu Gly
    610                 615                 620

Asp Leu Gly Leu Gly Gly Tyr Glu Leu Lys Leu Glu Gln Thr Leu Gly
625                 630                 635                 640

Gln Ser Gln Leu Asn
                645

<210> 4
<211> 1528
<212> DNA
<213> Homo sapiens

<400> 4
atagccctta tccaggtttt tatctaagga atcccaagaa gactggggaa tggagagaca 60
gtcaagggtt atgtcagaaa aggatgagta tcagtttcaa catcagggag cggtggagct 120
gcttgtcttc aattttttgc tcatccttac cattttgaca atctggttat ttaaaaatca 180
tcgattccgc ttcttgcatg aaactggagg agcaatggtg tatggcctta taatgggact 240
aattttacga tatgctacag caccaactga tattgaaagt ggaactgtct atgactgtgt 300
aaaactaact ttcagtccat caactctgct ggttaatatc actgaccaag tttatgaata 360
taaatacaaa agagaaataa gtcagcacaa catcaatcct catcaaggaa atgctatact 420
tgaaaagatg acatttgatc cagaaatctt cttcaatgtt ttactgccac caattatatt 480
tcatgcagga tatagtctaa agaagagaca cttttttcaa aacttaggat ctattttaac 540
gtatgccttc ttgggaactg ccatctcctg catcgtcata gggttaatta tgtatggttt 600
tgtgaaggct atgatacatg ctggccagct gaaaaatgga gactttcatt tcactgactg 660
tttatttttt ggttcactga tgtctgctac agatccagtg acagtgctgg ccattttcca 720
tgaactgcac gtcgaccctg acctgtacac actcttgttt ggagagagtg tgttgaatga 780
tgcagtggcc atagtcctta catattctat atccatttac agtcccaagg agaatccaaa 840
tgcatttgat gccgcagcat tcttccagtc tgtggggaat ttcctgggaa tcttcgctgg 900

ctcatttgca atggggtctg cgtatgccat catcacagca ctgttgacca aatttaccaa 960
gctgtgtgag ttcccgatgc tggaaaccgg cctgtttttc ctgctttctt ggagtgcctt 1020
cctgtctgcc gaggctgccg gcctaacagg gatagttgct gttctcttct gtggagtcac 1080
acaagcacat tatacctaca acaatctgtc ttcggattcc aaaataagaa ctaaacagtt 1140
gtttgaattt atgaactttt tggcggagaa cgtcatcttc tgttacatgg gcctggcact 1200
gttcacgttc cagaatcata tctttaatgc tctttttata cttggagcct ttctagcaat 1260
ttttgttgcc agagcctgca acatatatcc cctctccttc ctcctgaatc taggccgaaa 1320
acagaagatc ccctggaact ttcagcacat gatgatgttt tcaggtttgc gaggagcgat 1380
cgcatttgcc ttagctattc ggaacacaga atctcagccc aaacaaatga tgtttaccac 1440
tacgctgctc ctcgtgttct tcactgtctg ggtatttgga ggaggaacaa cccccatgtt 1500
gacttggctt cagatcagag ttggcgtg 1528

<210> 5
<211> 49
<212> DNA
<213> Homo sapiens

<400> 5
atagccctta tccaggtttt tatctaagga atcccaagaa gactgggga 49

<210> 6
<211> 1479
<212> DNA
<213> Homo sapiens

<400> 6
atggagagac agtcaagggt tatgtcagaa aaggatgagt atcagtttca acatcaggga 60
gcggtggagc tgcttgtctt caattttttg ctcatcctta ccattttgac aatctggtta 120
tttaaaaatc atcgattccg cttcttgcat gaaactggag gagcaatggt gtatggcctt 180
ataatgggac taattttacg atatgctaca gcaccaactg atattgaaag tggaactgtc 240
tatgactgtg taaaactaac tttcagtcca tcaactctgc tggttaatat cactgaccaa 300
gtttatgaat ataaatacaa aagagaaata agtcagcaca acatcaatcc tcatcaagga 360
aatgctatac ttgaaaagat gacatttgat ccagaaatct tcttcaatgt tttactgcca 420
ccaattatat ttcatgcagg atatagtcta aagaagagac acttttttca aaacttagga 480
tctattttaa cgtatgcctt cttgggaact gccatctcct gcatcgtcat agggttaatt 540
atgtatggtt ttgtgaaggc tatgatacat gctggccagc tgaaaaatgg agactttcat 600
ttcactgact gtttattttt tggttcactg atgtctgcta cagatccagt gacagtgctg 660
gccattttcc atgaactgca cgtcgaccct gacctgtaca cactcttgtt tggagagagt 720
gtgttgaatg atgcagtggc catagtcctt acatattcta tatccattta cagtcccaag 780
gagaatccaa atgcatttga tgccgcagca ttcttccagt ctgtggggaa tttcctggga 840
atcttcgctg gctcatttgc aatggggtct gcgtatgcca tcatcacagc actgttgacc 900
aaatttacca agctgtgtga gttcccgatg ctggaaaccg gcctgttttt cctgctttct 960
tggagtgcct tcctgtctgc cgaggctgcc ggcctaacag ggatagttgc tgttctcttc 1020
tgtggagtca cacaagcaca ttatacctac aacaatctgt cttcggattc caaaataaga 1080
actaaacagt tgtttgaatt tatgaacttt ttggcggaga acgtcatctt ctgttacatg 1140
ggcctggcac tgttcacgtt ccagaatcat atctttaatg ctctttttat acttggagcc 1200
tttctagcaa tttttgttgc cagagcctgc aacatatatc ccctctcctt cctcctgaat 1260
ctaggccgaa aacagaagat cccctggaac tttcagcaca tgatgatgtt ttcaggtttg 1320
cgaggagcga tcgcatttgc cttagctatt cggaacacag aatctcagcc caaacaaatg 1380
atgtttacca ctacgctgct cctcgtgttc ttcactgtct gggtatttgg aggaggaaca 1440
acccccatgt tgacttggct tcagatcaga gttggcgtg 1479

<210> 7
<211> 493
<212> PRT
<213> Homo sapiens

<400> 7
Met Glu Arg Gln Ser Arg Val Met Ser Glu Lys Asp Glu Tyr Gln Phe
1               5                   10                  15

Gln His Gln Gly Ala Val Glu Leu Leu Val Phe Asn Phe Leu Leu Ile
            20                  25                  30

Leu Thr Ile Leu Thr Ile Trp Leu Phe Lys Asn His Arg Phe Arg Phe
        35                  40                  45

Leu His Glu Thr Gly Gly Ala Met Val Tyr Gly Leu Ile Met Gly Leu
    50                  55                  60

Ile Leu Arg Tyr Ala Thr Ala Pro Thr Asp Ile Glu Ser Gly Thr Val
65                  70                  75                  80

Tyr Asp Cys Val Lys Leu Thr Phe Ser Pro Ser Thr Leu Leu Val Asn
                85                  90                  95

Ile Thr Asp Gln Val Tyr Glu Tyr Lys Tyr Lys Arg Glu Ile Ser Gln
            100                 105                 110

His Asn Ile Asn Pro His Gln Gly Asn Ala Ile Leu Glu Lys Met Thr
        115                 120                 125

Phe Asp Pro Glu Ile Phe Phe Asn Val Leu Leu Pro Pro Ile Ile Phe
    130                 135                 140

His Ala Gly Tyr Ser Leu Lys Lys Arg His Phe Phe Gln Asn Leu Gly
145                 150                 155                 160

Ser Ile Leu Thr Tyr Ala Phe Leu Gly Thr Ala Ile Ser Cys Ile Val
                165                 170                 175

Ile Gly Leu Ile Met Tyr Gly Phe Val Lys Ala Met Ile His Ala Gly
            180                 185                 190

Gln Leu Lys Asn Gly Asp Phe His Phe Thr Asp Cys Leu Phe Phe Gly
        195                 200                 205

Ser Leu Met Ser Ala Thr Asp Pro Val Thr Val Leu Ala Ile Phe His
    210                 215                 220

Glu Leu His Val Asp Pro Asp Leu Tyr Thr Leu Leu Phe Gly Glu Ser
225                 230                 235                 240

Val Leu Asn Asp Ala Val Ala Ile Val Leu Thr Tyr Ser Ile Ser Ile
                245                 250                 255

Tyr Ser Pro Lys Glu Asn Pro Asn Ala Phe Asp Ala Ala Ala Phe Phe
            260                 265                 270

Gln Ser Val Gly Asn Phe Leu Gly Ile Phe Ala Gly Ser Phe Ala Met
        275                 280                 285

Gly Ser Ala Tyr Ala Ile Ile Thr Ala Leu Leu Thr Lys Phe Thr Lys
    290                 295                 300

Leu Cys Glu Phe Pro Met Leu Glu Thr Gly Leu Phe Phe Leu Leu Ser
305                 310                 315                 320

Trp Ser Ala Phe Leu Ser Ala Glu Ala Ala Gly Leu Thr Gly Ile Val
                325                 330                 335

Ala Val Leu Phe Cys Gly Val Thr Gln Ala His Tyr Thr Tyr Asn Asn

340                 345                 350

Leu Ser Ser Asp Ser Lys Ile Arg Thr Lys Gln Leu Phe Glu Phe Met
        355                 360                 365

Asn Phe Leu Ala Glu Asn Val Ile Phe Cys Tyr Met Gly Leu Ala Leu
    370                 375                 380

Phe Thr Phe Gln Asn His Ile Phe Asn Ala Leu Phe Ile Leu Gly Ala
385                 390                 395                 400

Phe Leu Ala Ile Phe Val Ala Arg Ala Cys Asn Ile Tyr Pro Leu Ser
                405                 410                 415

Phe Leu Leu Asn Leu Gly Arg Lys Gln Lys Ile Pro Trp Asn Phe Gln
            420                 425                 430

His Met Met Met Phe Ser Gly Leu Arg Gly Ala Ile Ala Phe Ala Leu
        435                 440                 445

Ala Ile Arg Asn Thr Glu Ser Gln Pro Lys Gln Met Met Phe Thr Thr
    450                 455                 460

Thr Leu Leu Leu Val Phe Phe Thr Val Trp Val Phe Gly Gly Gly Thr
465                 470                 475                 480

Thr Pro Met Leu Thr Trp Leu Gln Ile Arg Val Gly Val
                485                 490

<210> 8
<211> 224
<212> DNA
<213> Homo sapiens

<400> 8
atagccctta tccaggtttt tatctaagga atcccaagaa gactggggaa tggagagaca 60
gtcaagggtt atgtcagaaa aggatgagta tcagtttcaa catcagggag cggtggagct 120
gcttgtcttc aattttttgc tcatccttac cattttgaca atctggttat ttaaaaatca 180
tcgattccgc ttcttgcatg aaactggagg agcaatggtg tatg                   224

<210> 9
<211> 203
<212> DNA
<213> Homo sapiens

<400> 9
gccttataat gggactaatt ttacgatatg ctacagcacc aactgatatt gaaagtggaa 60
ctgtctatga ctgtgtaaaa ctaactttca gtccatcaac tctgctggtt aatatcactg 120
accaagttta tgaatataaa tacaaaagag aaataagtca gcacaacatc aatcctcatc 180
aaggaaatgc tatacttgaa aag                                          203

<210> 10
<211> 78
<212> DNA
<213> Homo sapiens

<400> 10
atgacatttg atccagaaat cttcttcaat gttttactgc caccaattat atttcatgca 60
ggatatagtc taaagaag                                                78

<210> 11

<211> 77
<212> DNA
<213> Homo sapiens

<400> 11
agacactttt ttcaaaactt aggatctatt ttaacgtatg ccttcttggg aactgccatc 60
tcctgcatcg tcatagg 77

<210> 12
<211> 116
<212> DNA
<213> Homo sapiens

<400> 12
gttaattatg tatggttttg tgaaggctat gatacatgct ggccagctga aaaatggaga 60
ctttcatttc actgactgtt tattttttgg ttcactgatg tctgctacag atccag 116

<210> 13
<211> 106
<212> DNA
<213> Homo sapiens

<400> 13
tgacagtgct ggccattttc catgaactgc acgtcgaccc tgacctgtac acactcttgt 60
ttggagagag tgtgttgaat gatgcagtgg ccatagtcct tacata 106

<210> 14
<211> 139
<212> DNA
<213> Homo sapiens

<400> 14
ttctatatcc atttacagtc ccaaggagaa tccaaatgca tttgatgccg cagcattctt 60
ccagtctgtg gggaatttcc tgggaatctt cgctggctca tttgcaatgg ggtctgcgta 120
tgccatcatc acagcactg 139

<210> 15
<211> 106
<212> DNA
<213> Homo sapiens

<400> 15
ttgaccaaat ttaccaagct gtgtgagttc ccgatgctgg aaaccggcct gtttttcctg 60
ctttcttgga gtgccttcct gtctgccgag gctgccggcc taacag 106

<210> 16
<211> 89
<212> DNA
<213> Homo sapiens

<400> 16
ggatagttgc tgttctcttc tgtggagtca cacaagcaca ttatacctac aacaatctgt 60
cttcggattc caaaataaga actaaacag 89

<210> 17
<211> 114
<212> DNA
<213> Homo sapiens

<400> 17
ttgtttgaat ttatgaactt tttggcggag aacgtcatct tctgttacat gggcctggca 60

ctgttcacgt tccagaatca tatctttaat gctcttttta tacttggagc cttt 114

<210> 18
<211> 112
<212> DNA
<213> Homo sapiens

<400> 18
ctagcaattt ttgttgccag agcctgcaac atatatcccc tctccttcct cctgaatcta 60
ggccgaaaac agaagatccc ctggaacttt cagcacatga tgatgttttc ag 112

<210> 19
<211> 154
<212> DNA
<213> Homo sapiens

<400> 19
gtttgcgagg agcgatcgca tttgccttag ctattcggaa cacagaatct cagcccaaac 60
aaatgatgtt taccactacg ctgctcctcg tgttcttcac tgtctgggta tttggaggag 120
gaacaacccc catgttgact tggcttcaga tcag 154

<210> 20
<211> 56
<212> DNA
<213> Homo sapiens

<400> 20
agttggcgtg gacctggatg aaaatctgaa ggaggacccc tcctcacaac accagg 56

<210> 21
<211> 79
<212> DNA
<213> Homo sapiens

<400> 21
aagcaaataa cttggataaa aacatgacga aagcagagag tgctcggctc ttcagaatgt 60
ggtatagctt tgaccacaa 79

<210> 22
<211> 106
<212> DNA
<213> Homo sapiens

<400> 22
gtatctgaaa ccaattttaa cccactctgg tcctccgctg actacaacat tacctgaatg 60
gtgtggtccg atttccaggc tgcttaccag tcctcaagcc tatggg 106

<210> 23
<211> 319
<212> DNA
<213> Homo sapiens

<400> 23
gaacagctaa aagaggatga tgtggaatgc attgtaaacc aggatgaact agccataaat 60
taccaggagc aagcctcctc accctgcagt cctcctgcaa ggctaggtct ggaccagaaa 120
gcttcacccc agacgccagg caaggaaaac atttatgagg gagacctcgg cctgggaggc 180
tatgaactca agcttgagca aactttgggt caatcccagt tgaattaatt ggcatgaaga 240
gtacagatgt aatcacaagt aatgcaagac tcactgagga atacaagcca agctgatgag 300
gcagtacagg ggagaggct 319

<210> 24

<211> 500
<212> DNA
<213> Homo sapiens

<400> 24
agagagaaac aggaagttgt aactagaagc catctgaata ctaagccagg gcagaatgct 60
tgtgaagtag caactaaagt ggcagtgttt cttctgaaat tctcaggcag tcagactgtc 120
ttaggcaaat cttgataaaa tagcctttat ccaggttttt atctaaggaa tcccaagaag 180
actggggaat ggagagacag tcaagggtta tgtcagaaaa ggatgagtat cagtttcaac 240
atcagggagc ggtggagctg cttgtcttca attttttgct catccttacc attttgacaa 300
tctggttatt taaaaatcat cgattccgct tcttgcatga aactggagga gcaatggtgt 360
atggtgagtg cataaattgt gagattgatg tgaaacttgt tgcttgagcg taattagaga 420
cttgtgctca gatagcaaag taccgaaact ggagaaaatg tgctgattga aatgtcatta 480
gtttgatgaa aggtgctgca 500

<210> 25
<211> 500
<212> DNA
<213> Homo sapiens

<400> 25
tccatgcaaa agtgaacact tttctagacc aggcaataac ccaacacttt atcctgtcac 60
ttctgaaggt tccaaatcat ataggtttcc aatagtggca ttttagattg ccttcctcca 120
gtaccattat ttattctttt ttttttaggc cttataatgg gactaatttt acgatatgct 180
acagcaccaa ctgatattga aagtggaact gtctatgact gtgtaaaact aactttcagt 240
ccatcaactc tgctggttaa tatcactgac caagtttatg aatataaata caaaagagaa 300
ataagtcagc acaacatcaa tcctcatcaa ggaaatgcta tacttgaaaa ggtaaggatc 360
ctgtctggta ttatatattt gttttacagt ataaataatt gtatcatatt tgagccttta 420
tactttttat ataatgcatt cacacacaca tatatgccta ttttaacacc actcttttgg 480
agacgttaat tgactaagga 500

<210> 26
<211> 500
<212> DNA
<213> Homo sapiens

<400> 26
gtatgtagat tttaccttta gaagctacag ttatactgta tttatgaatt tccaaatttt 60
cattttttcc taccttcatc tctgccaagt taatagttaa tatgcctcac aaaggcttag 120
caaaagcaat tacaactgtt ttttgaaaca tgttttttta atgacccaaa ggagcatcat 180
tctaactatc ctttttttcc tttctggcag atgacatttg atccagaaat cttcttcaat 240
gttttactgc caccaattat atttcatgca ggatatagtc taaagaaggt aaatatatag 300
tgatcatttt cttcttttat cattaagtag agaattttac cagcactaat gagaaactgt 360
tatttttatg ggcttctcta tttggtaaga gtatcattat cttaaatttg tgatgcagat 420
tcaaacctga catgcttaga attctagact tcttctaaac ctgttctgtt gaatacaggc 480
cattaaccac atgtaactat 500

<210> 27
<211> 500
<212> DNA
<213> Homo sapiens

<400> 27
tttttttctg cttcttgact tttccctcca gtcctttgca atgataatca gttaagtccc 60
tattagcaca ataaggccag gaacgaggct ccaaaaccaa aatttgccta aacagtgagg 120
ggtctgtgaa ctgcatacat ttatttttat tttcttttgc actaagaaaa aattctgatg 180
tactctatta aatatctttt tttctcccca gagacacttt tttcaaaact taggatctat 240
tttaacgtat gccttcttgg gaactgccat ctcctgcatc gtcatagggt aagtgacatt 300
cggagctcaa gttgcaggtg gctgtggggt ctgtgatctg tgtgagggat ctaacacttc 360
caggattctt gctggctggg aaaattgtct tttttttagt atatcacata tttgtatgtt 420
ttttctgact taattccacg gcttctgaca aatacaaggc ttcaaatcaa agcaaactag 480

aggattgctg gactttctct 500

<210> 28
<211> 500
<212> DNA
<213> Homo sapiens

<400> 28
tttggtaaaa ttgtctttca tagccaagag attccagtgt acagagacct agcacggctg 60
tcatttggca ggatggtggc aatttgtgca tgatacgtat tcaggaaaaa cattagcatg 120
tggcttatga tagcatgggt ttacacaggg ttcatcttga tgtttgaagg tcatgttttt 180
ctcttcaaca ggttaattat gtatggtttt gtgaaggcta tgatacatgc tggccagctg 240
aaaaatggag actttcattt cactgactgt ttattttttg gttcactgat gtctgctaca 300
gatccaggta attgctcaaa taatatattt tcttctttta agaatcaaac atttaaattg 360
aatgtttctc cccaagacca tcacctgcgt ctacataata ataatttcta tttttcttca 420
cctgcagttg acatatctgt tagtaattac aaaagaaaag aaaaggggaa tattttgata 480
gtgctaaaga aattggttag 500

<210> 29
<211> 500
<212> DNA
<213> Homo sapiens

<400> 29
attttatctt taagcagtag ggagccaaga gaagcctctt aagcatcaga tattttttga 60
ataaaggtct ggtgttagta tagtcattgg atttgagaag atgagcattt cattaatgtt 120
tttgagtggt gaccatagcc aaggaaaact ccatgcctgc atccaagcta acctgcatgt 180
ttgtgtgtgt cttcctagtg acagtgctgg ccattttcca tgaactgcac gtcgaccctg 240
acctgtacac actcttgttt ggagagagtg tgttgaatga tgcagtggcc atagtcctta 300
catagtaagt accagaactt ggcctatgtt tctttaatca tgtgaaatat gctttcactt 360
gtatcttttc tcttatgtca cagagatacg ggcagtctct agcttttttc aaggaattgt 420
tatcacagaa aatttccata atgggcacaa ttcattaggc atctaatttc taatactatt 480
aatgggtttt atgtttccag 500

<210> 30
<211> 500
<212> DNA
<213> Homo sapiens

<400> 30
aggaagcaga aagatttctg cattctccaa ctctcccctt ctgtatctgt tttcccctaa 60
ctcttccaac cagcacccaa cattacaggg aaagatacct ctccccaaag tgggtgcgaa 120
atcctatctg ggctatgaga tgaaagtcac taactaacct ccaccctctt ctcttttcag 180
ttctatatcc atttacagtc ccaaggagaa tccaaatgca tttgatgccg cagcattctt 240
ccagtctgtg gggaatttcc tgggaatctt cgctggctca tttgcaatgg ggtctgcgta 300
tgccatcatc acagcactga tatcctttgt ctgtatgtgg aaagctggga ctgtcaagaa 360
cagtcagtat ccatggaata tttctgatga tggacaacct ccaggcccat aaaaggctag 420
atagtctggt ccaagtttca tacggcacca ctggtgccat cttggttctc agtcacccct 480
ggaggcatag gttggtacca 500

<210> 31
<211> 500
<212> DNA
<213> Homo sapiens

<400> 31
cccccggtga agcatggaac tcgtgctggc cttcaaagta ggtcccagtg agaattatgg 60
ccttccagtc aggtctgaat atcagttctt ttactttgct ctagagctat catcaccaga 120
tcagttttta ttctcagcca agaaggagat gtagctgtaa aagctgttgt tgtttccctt 180
aacttatctt cctcacttga ccaaatttac caagctgtgt gagttcccga tgctggaaac 240
cggcctgttt ttcctgcttt cttggagtgc cttcctgtct gccgaggctg ccggcctaac 300

aggtcagtgc ttcatgctgc acagccaact ggatgtgtga atatgtgtgt ggctctggtg 360
tgtgcccctc cctgacctag caggaggtga aatgaagtct ctcttaaata gtgcatgtga 420
atggtttggg tcagaatcag cctctcttgg gtacgctgga gagaaaaaat aatagtaatt 480
catccatttt gtaccaaaca 500

<210> 32
<211> 500
<212> DNA
<213> Homo sapiens

<400> 32
tttttcttta ggtttaatcc ttcaggaccc tgtttaaacc ctgtttaaag caagtgcaag 60
atttacctag gttgcattat catggtaact actagcagtt ttaactctac tgacaccaag 120
tctattttcc aactggaata gccctaacct ttgacttgga tggaaaagaa aaattggttt 180
tgactgatct gtttttttaa tttcagggat agttgctgtt ctcttctgtg gagtcacaca 240
agcacattat acctacaaca atctgtcttc ggattccaaa ataagaacta aacaggtaaa 300
agaaaaaatt tactacagac ttggtctctt ttctcagtga aatggttatg tagcaattct 360
ggcagtatag tcagaaagag tttcattagt aaaggaagaa tgtaagctta aggcttcatt 420
acgagttgga tatttataga tttttttccc tccaggataa ccattaacaa ttctcttggt 480
aagcaaaaat atagttataa 500

<210> 33
<211> 500
<212> DNA
<213> Homo sapiens

<400> 33
tgatgtggct gtggcatctg tccctgggtc aagagccagg gcctccatcc tccttcagag 60
gctaagcaca cggccaaagg ggtggccttc cagataaagg agacccagtc agttgtcaaa 120
tacagttacc agaaagtaag tgcactcaaa cctgagttat aattaatggt tttttgtttt 180
atgttttgtt tcagttgttt gaatttatga actttttggc ggagaacgtc atcttctgtt 240
acatgggcct ggcactgttc acgttccaga atcatatctt taatgctctt tttatacttg 300
gagcctttgt atcctttgaa atacagaaca tggggtgatt agagaagata acgaatgact 360
tacttttacg gattattctg ttaaatcatt aaagcacttt cctaaagtcc actgcaaggc 420
tcagacacaa ggcagatacg tgatttctag ttaggggagg aaaacatcct aagaaattag 480
gacctccatt aaataattat 500

<210> 34
<211> 500
<212> DNA
<213> Homo sapiens

<400> 34
tattatttct gggaagaact tgactttgaa atataaattt tggaagcaag ttggacaaat 60
gtgaattgac atgtttattt ctggttttgg ggtcataatg aagagaagct tgaggacata 120
atatttaagc tcaaggattc aaaccatcat tgcagcaaca ctttcctcaa tgtttccctg 180
tgcttatcta cagctagcaa tttttgttgc cagagcctgc aacatatatc ccctctcctt 240
cctcctgaat ctaggccgaa aacagaagat cccctggaac tttcagcaca tgatgatgtt 300
ttcaggtatg tgaactatgt tatatgtaaa gtgctgctgg ttttctcaca attttacaga 360
acatgaaaaa ccatgagaaa atttagaacc cttttgggga aacaacttct ctttatccca 420
ttgtacctaa cactatgctg attctccgta agtgctgagc aacagcagga tttctctttc 480
caagtgggaa atgaagttag 500

<210> 35
<211> 500
<212> DNA
<213> Homo sapiens

<400> 35
gtacacagtg tctttctgtt ttatttctta caactcttta tgaatctata attatctcaa 60
aataaaaaga ttaatttaaa aaaattgtgt cagcaaattg taaagatgaa tccattgaaa 120

tgaaaaagac acctgcaagg catttatttt ctcctttggt ttttcctgca aggtttgcga 180
ggagcgatcg catttgcctt agctattcgg aacacagaat ctcagcccaa acaaatgatg 240
tttaccacta cgctgctcct cgtgttcttc actgtctggg tatttggagg aggaacaacc 300
cccatgttga cttggcttca gatcaggtga gtgacaccgt ctagcaaagg aaatcattat 360
gaggcatggc tgcgctgttc aatttctggt attgctgact tagtcaatag tacagtggca 420
ggtcctaccc taactttccc agcagtcaag ttgaccttca ctcataaggt gaaacaatat 480
gacatagtac caaccacact 500

<210> 36
<211> 500
<212> DNA
<213> Homo sapiens

<400> 36
tgctgttgat gcttaatttc tccagcatcc tgaggcccag ctgttcaagt tcttattttg 60
gtataaatct gaggttcttt tgattcaaga agacgtagga tagttttctg ccaaatcttt 120
tctcagattc acattgtgtt tgggttaggt catctgacac acaagactgt ctcattcctt 180
ctgcagcagg ggattgacca gtttttggtt ttgtttaaca gagttggcgt ggacctggat 240
gaaaatctga aggaggaccc ctcctcacaa caccaggcaa gtcaaagaaa ccaatgcaca 300
ttatatagag atatgattgt tctgttccat aggctgttcc atgtttgtcc agcatgtgac 360
caaggagcta tttctaaacg gagcttcctc tgctgataaa acaaatgaga ttacaataaa 420
atacaaggga attcagggtg ttgataactt ctagcatatg aatgcttaga actagcatgt 480
acttgcaagc ctctctatgg 500

<210> 37
<211> 500
<212> DNA
<213> Homo sapiens

<400> 37
aatgttgaaa tactccaaaa gggtctcatc cacatagcaa gatttatgag ctaatttgcc 60
tggaaactgc ttcaccattt agatgaaaat gcctatactt agaaaggttt tttttaaatg 120
tagttaaatt taacttggtt tgacatgtat ttttatatta aaatcttttt gactatttac 180
ccaatgcctt tttctattgt ttctccccag gaagcaaata acttggataa aaacatgacg 240
aaagcagaga gtgctcggct cttcagaatg tggtatagct ttgaccacaa gtatcctttg 300
ataataacga tctcacagat cctgctgcag gcagagaatt actttaagct cttaatcatt 360
gaagtgtgca ctcctgctct tcaagaaaca ccaaaaggta aagtgaattc acagccaaac 420
ttataaaaag gaatgatttc ataaaataaa aagcagggta gtccatactt aagtgtttca 480
gagagctaaa gtgaaacttc 500

<210> 38
<211> 500
<212> DNA
<213> Homo sapiens

<400> 38
ggctatcagg aaggctggaa aagtatgtgt aacaaagatt ataaacagca atttttgcag 60
ggcaaatttc ttcctaagtg agggagtagg ggatttaaag gcaaacgtag ctgtctcagc 120
atacaaaggt ttgtgcagcg taagattcct aaatcctaac tccctgttga caagtattcc 180
ttaacaggcc tcccaggtat ctgaaaccaa ttttaaccca ctctggtcct ccgctgacta 240
caacattacc tgaatggtgt ggtccgattt ccaggctgct taccagtcct caagcctatg 300
gggtaagtaa atctcaggcc tctagggtga gggaatatcc cttgtgggac agacttggtg 360
aatatcgatg agcccaggaa cctcaaaaag cctgctactt gcaggattga ggaggaagct 420
tttttttttt tttttttttt ttttgagacg gagtcttgct ctgttgccca ggctggagtg 480
cagtggcgtg atctcagctc 500

<210> 39
<211> 500
<212> DNA
<213> Homo sapiens

<400> 39
attctgttta aaaaaaaaaa aaaattgtag gactgctatt gttttctgcc ttcatcatga 60
agtgacacct ctctctcttt tcccaaccag gaacagctaa aagaggatga tgtggaatgc 120
attgtaaacc aggatgaact agccataaat taccaggagc aagcctcctc accctgcagt 180
cctcctgcaa ggctaggtct ggaccagaaa gcttcacccc agacgccagg caaggaaaac 240
atttatgagg gagacctcgg cctgggaggc tatgaactca agcttgagca aactttgggt 300
caatcccagt tgaattaatt ggcatgaaga gtacagatgt aatcacaagt aatgcaagac 360
tcactgagga atacaagcca agctgatgag gcagtacagg ggagaggctg gaaaacatat 420
taagagcata aattggagag aatcaaagcc ttgtcacatg gatcctctgg tgcctgaaga 480
aatgagattt tattatccct 500

<210> 40
<211> 1000
<212> DNA
<213> Homo sapiens

<400> 40
tctgtgggtc ctggatgccg agaccaggaa ccctacaaag agaaatccat gcatgtggcg 60
ttgctgagga tggctgcgtg agtttctctt tcgaagtaat gtgttttgtt tttttctttt 120
tttgtctcaa cgtatccaaa cagtacagtt gtagagatgg ggatggaatt ttatttgtct 180
tttgctttca ttgttttctc ttgttaaaag aaaaacaatt gatttagagt ttaaaggctg 240
ctctgcaact atcaattata taaagccaaa tagagtcata tactaaatcc tttcttgctt 300
ctcaactaaa aaaaaaaaaa aaaaatcctg tgaaatcact ttaggtttgc ttaaccaatg 360
tacagtcatg atttgagagc ataagaaacc ctataagaaa ccctatgcct taagtgaatc 420
ccattgtgag atacttgagc tatgaggcgg aagtttcagg ctaagcagtg taatatcttt 480
tcaggaggct aatatgctga tccatttaga gcacagaggc tgattgataa ctgtgctatc 540
aatccccatc actttggcag gtcttggtta gagaggaaag atcttgcatt taggtgaagt 600
tgagtcagga attggctttt tgaacttaga aaacgttgct agtgtaaggc tacctttttt 660
attattaatc tacagttcat atttatgcag cttaagaaga cttatttgga ataataagac 720
cgtgattcaa gatcttagca tcattgcttg cagatttgtc cagtatgaaa gatattttca 780
tgagatgaat cacttaaaga cattaagagt cagtgatgaa accacacact ctcgagtttg 840
aaaagagaca gggctgcttt aagagagaaa caggaagttg taactagaag ccatctgaat 900
actaagccag ggcagaatgc ttgtgaagta gcaactaaag tggcagtgtt tcttctgaaa 960
ttctcaggca gtcagactgt cttaggcaaa tcttgataaa 1000

<210> 41
<211> 17
<212> DNA
<213> Homo sapiens

<400> 41
atagccctta tccaggt 17

<210> 42
<211> 17
<212> DNA
<213> Homo sapiens

<400> 42
tagcccttat ccaggtt 17

<210> 43
<211> 17
<212> DNA
<213> Homo sapiens

<400> 43
agcccttatc caggttt 17

<210> 44
<211> 17

<212> DNA
<213> Homo sapiens

<400> 44
gcccttatcc aggtttt                                                    17

<210> 45
<211> 17
<212> DNA
<213> Homo sapiens

<400> 45
cccttatcca ggttttt                                                    17

<210> 46
<211> 17
<212> DNA
<213> Homo sapiens

<400> 46
ccttatccag gttttta                                                    17

<210> 47
<211> 17
<212> DNA
<213> Homo sapiens

<400> 47
cttatccagg tttttat                                                    17

<210> 48
<211> 17
<212> DNA
<213> Homo sapiens

<400> 48
ttatccaggt ttttatc                                                    17

<210> 49
<211> 17
<212> DNA
<213> Homo sapiens

<400> 49
tatccaggtt tttatct                                                    17

<210> 50
<211> 17
<212> DNA
<213> Homo sapiens

<400> 50
atccaggttt ttatcta                                                    17

<210> 51
<211> 17
<212> DNA
<213> Homo sapiens

<400> 51
tccaggtttt tatctaa                                                    17

<210> 52
<211> 17
<212> DNA
<213> Homo sapiens

<400> 52
ccaggttttt atctaag                                                    17

<210> 53
<211> 17
<212> DNA
<213> Homo sapiens

<400> 53
caggttttta tctaagg                                                    17

<210> 54
<211> 17
<212> DNA
<213> Homo sapiens

<400> 54
aggtttttat ctaagga                                                    17

<210> 55
<211> 17
<212> DNA
<213> Homo sapiens

<400> 55
ggtttttatc taaggaa                                                    17

<210> 56
<211> 17
<212> DNA
<213> Homo sapiens

<400> 56
gtttttatct aaggaat                                                    17

<210> 57
<211> 17
<212> DNA
<213> Homo sapiens

<400> 57
tttttatcta aggaatc                                                    17

<210> 58
<211> 17
<212> DNA
<213> Homo sapiens

<400> 58
ttttatctaa ggaatcc                                                    17

<210> 59
<211> 17
<212> DNA
<213> Homo sapiens

<400> 59
tttatctaag gaatccc                                                    17

<210> 60
<211> 17
<212> DNA
<213> Homo sapiens

<400> 60
ttatctaagg aatccca                                                    17

<210> 61
<211> 17
<212> DNA
<213> Homo sapiens

<400> 61
tatctaagga atcccaa                                                    17

<210> 62
<211> 17
<212> DNA
<213> Homo sapiens

<400> 62
atctaaggaa tcccaag                                                    17

<210> 63
<211> 17
<212> DNA
<213> Homo sapiens

<400> 63
tctaaggaat cccaaga                                                    17

<210> 64
<211> 17
<212> DNA
<213> Homo sapiens

<400> 64
ctaaggaatc ccaagaa                                                    17

<210> 65
<211> 17
<212> DNA
<213> Homo sapiens

<400> 65
taaggaatcc caagaag                                                    17

<210> 66
<211> 17
<212> DNA
<213> Homo sapiens

<400> 66
aaggaatccc aagaaga                                                    17

<210> 67

<211> 17
<212> DNA
<213> Homo sapiens

<400> 67
aggaatccca agaagac                                                    17

<210> 68
<211> 17
<212> DNA
<213> Homo sapiens

<400> 68
ggaatcccaa gaagact                                                    17

<210> 69
<211> 17
<212> DNA
<213> Homo sapiens

<400> 69
gaatcccaag aagactg                                                    17

<210> 70
<211> 17
<212> DNA
<213> Homo sapiens

<400> 70
aatcccaaga agactgg                                                    17

<210> 71
<211> 17
<212> DNA
<213> Homo sapiens

<400> 71
atcccaagaa gactggg                                                    17

<210> 72
<211> 17
<212> DNA
<213> Homo sapiens

<400> 72
tcccaagaag actgggg                                                    17

<210> 73
<211> 17
<212> DNA
<213> Homo sapiens

<400> 73
cccaagaaga ctgggga                                                    17

<210> 74
<211> 17
<212> DNA
<213> Homo sapiens

<400> 74

ccaagaagac tggggaa                                                    17

<210> 75
<211> 17
<212> DNA
<213> Homo sapiens

<400> 75
caagaagact ggggaat                                                    17

<210> 76
<211> 17
<212> DNA
<213> Homo sapiens

<400> 76
aagaagactg gggaatg                                                    17

<210> 77
<211> 17
<212> DNA
<213> Homo sapiens

<400> 77
agaagactgg ggaatgg                                                    17

<210> 78
<211> 17
<212> DNA
<213> Homo sapiens

<400> 78
gaagactggg gaatgga                                                    17

<210> 79
<211> 17
<212> DNA
<213> Homo sapiens

<400> 79
aagactgggg aatggag                                                    17

<210> 80
<211> 17
<212> DNA
<213> Homo sapiens

<400> 80
agactgggga atggaga                                                    17

<210> 81
<211> 17
<212> DNA
<213> Homo sapiens

<400> 81
gactggggaa tggagag                                                    17

<210> 82
<211> 17
<212> DNA

<213> Homo sapiens

<400> 82
actggggaat ggagaga                                                    17

<210> 83
<211> 17
<212> DNA
<213> Homo sapiens

<400> 83
ctggggaatg gagagac                                                    17

<210> 84
<211> 17
<212> DNA
<213> Homo sapiens

<400> 84
tggggaatgg agagaca                                                    17

<210> 85
<211> 17
<212> DNA
<213> Homo sapiens

<400> 85
ggggaatgga gagacag                                                    17

<210> 86
<211> 17
<212> DNA
<213> Homo sapiens

<400> 86
gggaatggag agacagt                                                    17

<210> 87
<211> 17
<212> DNA
<213> Homo sapiens

<400> 87
ggaatggaga gacagtc                                                    17

<210> 88
<211> 17
<212> DNA
<213> Homo sapiens

<400> 88
gaatggagag acagtca                                                    17

<210> 89
<211> 17
<212> DNA
<213> Homo sapiens

<400> 89
aatggagaga cagtcaa                                                    17

<210> 90
<211> 17
<212> DNA
<213> Homo sapiens

<400> 90
atggagagac agtcaag                                                    17

<210> 91
<211> 17
<212> DNA
<213> Homo sapiens

<400> 91
tggagagaca gtcaagg                                                    17

<210> 92
<211> 17
<212> DNA
<213> Homo sapiens

<400> 92
ggagagacag tcaaggg                                                    17

<210> 93
<211> 17
<212> DNA
<213> Homo sapiens

<400> 93
gagagacagt caagggt                                                    17

<210> 94
<211> 17
<212> DNA
<213> Homo sapiens

<400> 94
agagacagtc aagggtt                                                    17

<210> 95
<211> 17
<212> DNA
<213> Homo sapiens

<400> 95
gagacagtca agggtta                                                    17

<210> 96
<211> 17
<212> DNA
<213> Homo sapiens

<400> 96
agacagtcaa gggttat                                                    17

<210> 97
<211> 17
<212> DNA
<213> Homo sapiens

<400> 97
gacagtcaag ggttatg                                                    17

<210> 98
<211> 17
<212> DNA
<213> Homo sapiens

<400> 98
acagtcaagg gttatgt                                                    17

<210> 99
<211> 17
<212> DNA
<213> Homo sapiens

<400> 99
cagtcaaggg ttatgtc                                                    17

<210> 100
<211> 17
<212> DNA
<213> Homo sapiens

<400> 100
agtcaagggt tatgtca                                                    17

<210> 101
<211> 17
<212> DNA
<213> Homo sapiens

<400> 101
gtcaagggtt atgtcag                                                    17

<210> 102
<211> 17
<212> DNA
<213> Homo sapiens

<400> 102
tcaagggtta tgtcaga                                                    17

<210> 103
<211> 17
<212> DNA
<213> Homo sapiens

<400> 103
caagggttat gtcagaa                                                    17

<210> 104
<211> 17
<212> DNA
<213> Homo sapiens

<400> 104
aagggttatg tcagaaa                                                    17

<210> 105
<211> 17

<212> DNA
<213> Homo sapiens

<400> 105
agggttatgt cagaaaa                                                    17

<210> 106
<211> 17
<212> DNA
<213> Homo sapiens

<400> 106
gggttatgtc agaaaag                                                    17

<210> 107
<211> 17
<212> DNA
<213> Homo sapiens

<400> 107
ggttatgtca gaaaagg                                                    17

<210> 108
<211> 17
<212> DNA
<213> Homo sapiens

<400> 108
gttatgtcag aaaagga                                                    17

<210> 109
<211> 17
<212> DNA
<213> Homo sapiens

<400> 109
ttatgtcaga aaaggat                                                    17

<210> 110
<211> 17
<212> DNA
<213> Homo sapiens

<400> 110
tatgtcagaa aaggatg                                                    17

<210> 111
<211> 17
<212> DNA
<213> Homo sapiens

<400> 111
atgtcagaaa aggatga                                                    17

<210> 112
<211> 17
<212> DNA
<213> Homo sapiens

<400> 112
tgtcagaaaa ggatgag                                                    17

<210> 113
<211> 17
<212> DNA
<213> Homo sapiens

<400> 113
gtcagaaaag gatgagt                                                    17

<210> 114
<211> 17
<212> DNA
<213> Homo sapiens

<400> 114
tcagaaaagg atgagta                                                    17

<210> 115
<211> 17
<212> DNA
<213> Homo sapiens

<400> 115
cagaaaagga tgagtat                                                    17

<210> 116
<211> 17
<212> DNA
<213> Homo sapiens

<400> 116
agaaaaggat gagtatc                                                    17

<210> 117
<211> 17
<212> DNA
<213> Homo sapiens

<400> 117
gaaaaggatg agtatca                                                    17

<210> 118
<211> 17
<212> DNA
<213> Homo sapiens

<400> 118
aaaaggatga gtatcag                                                    17

<210> 119
<211> 17
<212> DNA
<213> Homo sapiens

<400> 119
aaaggatgag tatcagt                                                    17

<210> 120
<211> 17
<212> DNA
<213> Homo sapiens

<400> 120
aaggatgagt atcagtt                                                    17

<210> 121
<211> 17
<212> DNA
<213> Homo sapiens

<400> 121
aggatgagta tcagttt                                                    17

<210> 122
<211> 17
<212> DNA
<213> Homo sapiens

<400> 122
ggatgagtat cagtttc                                                    17

<210> 123
<211> 17
<212> DNA
<213> Homo sapiens

<400> 123
gatgagtatc agtttca                                                    17

<210> 124
<211> 17
<212> DNA
<213> Homo sapiens

<400> 124
atgagtatca gtttcaa                                                    17

<210> 125
<211> 17
<212> DNA
<213> Homo sapiens

<400> 125
tgagtatcag tttcaac                                                    17

<210> 126
<211> 17
<212> DNA
<213> Homo sapiens

<400> 126
gagtatcagt ttcaaca                                                    17

<210> 127
<211> 17
<212> DNA
<213> Homo sapiens

<400> 127
agtatcagtt tcaacat                                                    17

<210> 128

<211> 17
<212> DNA
<213> Homo sapiens

<400> 128
gtatcagttt caacatc                                                    17

<210> 129
<211> 17
<212> DNA
<213> Homo sapiens

<400> 129
tatcagtttc aacatca                                                    17

<210> 130
<211> 17
<212> DNA
<213> Homo sapiens

<400> 130
atcagtttca acatcag                                                    17

<210> 131
<211> 17
<212> DNA
<213> Homo sapiens

<400> 131
tcagtttcaa catcagg                                                    17

<210> 132
<211> 17
<212> DNA
<213> Homo sapiens

<400> 132
cagtttcaac atcaggg                                                    17

<210> 133
<211> 17
<212> DNA
<213> Homo sapiens

<400> 133
agtttcaaca tcaggga                                                    17

<210> 134
<211> 17
<212> DNA
<213> Homo sapiens

<400> 134
gtttcaacat cagggag                                                    17

<210> 135
<211> 17
<212> DNA
<213> Homo sapiens

<400> 135

tttcaacatc agggagc                                                    17

<210> 136
<211> 17
<212> DNA
<213> Homo sapiens

<400> 136
ttcaacatca gggagcg                                                    17

<210> 137
<211> 17
<212> DNA
<213> Homo sapiens

<400> 137
tcaacatcag ggagcgg                                                    17

<210> 138
<211> 17
<212> DNA
<213> Homo sapiens

<400> 138
caacatcagg gagcggt                                                    17

<210> 139
<211> 17
<212> DNA
<213> Homo sapiens

<400> 139
aacatcaggg agcggtg                                                    17

<210> 140
<211> 17
<212> DNA
<213> Homo sapiens

<400> 140
acatcaggga gcggtgg                                                    17

<210> 141
<211> 17
<212> DNA
<213> Homo sapiens

<400> 141
catcagggag cggtgga                                                    17

<210> 142
<211> 17
<212> DNA
<213> Homo sapiens

<400> 142
atcagggagc ggtggag                                                    17

<210> 143
<211> 17
<212> DNA

<213> Homo sapiens

<400> 143
tcagggagcg gtggagc                                                    17

<210> 144
<211> 17
<212> DNA
<213> Homo sapiens

<400> 144
cagggagcgg tggagct                                                    17

<210> 145
<211> 17
<212> DNA
<213> Homo sapiens

<400> 145
agggagcggt ggagctg                                                    17

<210> 146
<211> 17
<212> DNA
<213> Homo sapiens

<400> 146
gggagcggtg gagctgc                                                    17

<210> 147
<211> 17
<212> DNA
<213> Homo sapiens

<400> 147
ggagcggtgg agctgct                                                    17

<210> 148
<211> 17
<212> DNA
<213> Homo sapiens

<400> 148
gagcggtgga gctgctt                                                    17

<210> 149
<211> 17
<212> DNA
<213> Homo sapiens

<400> 149
agcggtggag ctgcttg                                                    17

<210> 150
<211> 17
<212> DNA
<213> Homo sapiens

<400> 150
gcggtggagc tgcttgt                                                    17

<210> 151
<211> 17
<212> DNA
<213> Homo sapiens

<400> 151
cggtggagct gcttgtc                                                    17

<210> 152
<211> 17
<212> DNA
<213> Homo sapiens

<400> 152
ggtggagctg cttgtct                                                    17

<210> 153
<211> 17
<212> DNA
<213> Homo sapiens

<400> 153
gtggagctgc ttgtctt                                                    17

<210> 154
<211> 17
<212> DNA
<213> Homo sapiens

<400> 154
tggagctgct tgtcttc                                                    17

<210> 155
<211> 17
<212> DNA
<213> Homo sapiens

<400> 155
ggagctgctt gtcttca                                                    17

<210> 156
<211> 17
<212> DNA
<213> Homo sapiens

<400> 156
gagctgcttg tcttcaa                                                    17

<210> 157
<211> 17
<212> DNA
<213> Homo sapiens

<400> 157
agctgcttgt cttcaat                                                    17

<210> 158
<211> 17
<212> DNA
<213> Homo sapiens

<400> 158
gctgcttgtc ttcaatt                                                    17

<210> 159
<211> 17
<212> DNA
<213> Homo sapiens

<400> 159
ctgcttgtct tcaattt                                                    17

<210> 160
<211> 17
<212> DNA
<213> Homo sapiens

<400> 160
tgcttgtctt caatttt                                                    17

<210> 161
<211> 17
<212> DNA
<213> Homo sapiens

<400> 161
gcttgtcttc aattttt                                                    17

<210> 162
<211> 17
<212> DNA
<213> Homo sapiens

<400> 162
   cttgtcttca atttttt                                                   17

<210> 163
<211> 17
<212> DNA
<213> Homo sapiens

<400> 163
   ttgtcttcaa ttttttg                                                     17

<210> 164
<211> 17
<212> DNA
<213> Homo sapiens

<400> 164
   tgtcttcaat tttttgc                                                     17

<210> 165
<211> 17
<212> DNA
<213> Homo sapiens

<400> 165
gtcttcaatt ttttgct                                                    17

<210> 166
<211> 17

<212> DNA
<213> Homo sapiens

<400> 166
tcttcaattt tttgctc                                                    17

<210> 167
<211> 17
<212> DNA
<213> Homo sapiens

<400> 167
cttcaatttt ttgctca                                                    17

<210> 168
<211> 17
<212> DNA
<213> Homo sapiens

<400> 168
ttcaattttt tgctcat                                                    17

<210> 169
<211> 17
<212> DNA
<213> Homo sapiens

<400> 169
tcaatttttt gctcatc                                                    17

<210> 170
<211> 17
<212> DNA
<213> Homo sapiens

<400> 170
caattttttg ctcatcc                                                    17

<210> 171
<211> 17
<212> DNA
<213> Homo sapiens

<400> 171
aattttttgc tcatcct                                                    17

<210> 172
<211> 17
<212> DNA
<213> Homo sapiens

<400> 172
attttttgct catcctt                                                    17

<210> 173
<211> 17
<212> DNA
<213> Homo sapiens

<400> 173
ttttttgctc atcctta                                                    17

<210> 174
<211> 17
<212> DNA
<213> Homo sapiens

<400> 174
tttttgctca tccttac                                                      17

<210> 175
<211> 17
<212> DNA
<213> Homo sapiens

<400> 175
ttttgctcat ccttacc                                                      17

<210> 176
<211> 17
<212> DNA
<213> Homo sapiens

<400> 176
tttgctcatc cttacca                                                      17

<210> 177
<211> 17
<212> DNA
<213> Homo sapiens

<400> 177
ttgctcatcc ttaccat                                                      17

<210> 178
<211> 17
<212> DNA
<213> Homo sapiens

<400> 178
tgctcatcct taccatt                                                      17

<210> 179
<211> 17
<212> DNA
<213> Homo sapiens

<400> 179
gctcatcctt accattt                                                      17

<210> 180
<211> 17
<212> DNA
<213> Homo sapiens

<400> 180
ctcatcctta ccatttt                                                      17

<210> 181
<211> 17
<212> DNA
<213> Homo sapiens

<400> 181
tcatccttac cattttg                                                    17

<210> 182
<211> 17
<212> DNA
<213> Homo sapiens

<400> 182
catccttacc attttga                                                    17

<210> 183
<211> 17
<212> DNA
<213> Homo sapiens

<400> 183
atccttacca ttttgac                                                    17

<210> 184
<211> 17
<212> DNA
<213> Homo sapiens

<400> 184
tccttaccat tttgaca                                                    17

<210> 185
<211> 17
<212> DNA
<213> Homo sapiens

<400> 185
ccttaccatt ttgacaa                                                    17

<210> 186
<211> 17
<212> DNA
<213> Homo sapiens

<400> 186
cttaccattt tgacaat                                                    17

<210> 187
<211> 17
<212> DNA
<213> Homo sapiens

<400> 187
ttaccatttt gacaatc                                                    17

<210> 188
<211> 17
<212> DNA
<213> Homo sapiens

<400> 188
taccattttg acaatct                                                    17

<210> 189

<211> 17
<212> DNA
<213> Homo sapiens

<400> 189
accattttga caatctg                                                    17

<210> 190
<211> 17
<212> DNA
<213> Homo sapiens

<400> 190
ccattttgac aatctgg                                                    17

<210> 191
<211> 17
<212> DNA
<213> Homo sapiens

<400> 191
cattttgaca atctggt                                                    17

<210> 192
<211> 17
<212> DNA
<213> Homo sapiens

<400> 192
attttgacaa tctggtt                                                    17

<210> 193
<211> 17
<212> DNA
<213> Homo sapiens

<400> 193
ttttgacaat ctggtta                                                    17

<210> 194
<211> 17
<212> DNA
<213> Homo sapiens

<400> 194
tttgacaatc tggttat                                                    17

<210> 195
<211> 17
<212> DNA
<213> Homo sapiens

<400> 195
ttgacaatct ggttatt                                                    17

<210> 196
<211> 17
<212> DNA
<213> Homo sapiens

<400> 196

tgacaatctg gttattt                                                    17

<210> 197
<211> 17
<212> DNA
<213> Homo sapiens

<400> 197
gacaatctgg ttattta                                                    17

<210> 198
<211> 17
<212> DNA
<213> Homo sapiens

<400> 198
acaatctggt tatttaa                                                    17

<210> 199
<211> 17
<212> DNA
<213> Homo sapiens

<400> 199
caatctggtt atttaaa                                                    17

<210> 200
<211> 17
<212> DNA
<213> Homo sapiens

<400> 200
aatctggtta tttaaaa                                                    17

<210> 201
<211> 17
<212> DNA
<213> Homo sapiens

<400> 201
atctggttat ttaaaaa                                                    17

<210> 202
<211> 17
<212> DNA
<213> Homo sapiens

<400> 202
tctggttatt taaaaat                                                    17

<210> 203
<211> 17
<212> DNA
<213> Homo sapiens

<400> 203
ctggttattt aaaaatc                                                    17

<210> 204
<211> 17
<212> DNA

<213> Homo sapiens

<400> 204
tggttattta aaaatca                                                    17

<210> 205
<211> 17
<212> DNA
<213> Homo sapiens

<400> 205
ggttatttaa aaatcat                                                    17

<210> 206
<211> 17
<212> DNA
<213> Homo sapiens

<400> 206
gttatttaaa aatcatc                                                    17

<210> 207
<211> 17
<212> DNA
<213> Homo sapiens

<400> 207
ttatttaaaa atcatcg                                                    17

<210> 208
<211> 17
<212> DNA
<213> Homo sapiens

<400> 208
tatttaaaaa tcatcga                                                    17

<210> 209
<211> 17
<212> DNA
<213> Homo sapiens

<400> 209
atttaaaaat catcgat                                                    17

<210> 210
<211> 17
<212> DNA
<213> Homo sapiens

<400> 210
tttaaaaatc atcgatt                                                    17

<210> 211
<211> 17
<212> DNA
<213> Homo sapiens

<400> 211
ttaaaaatca tcgattc                                                    17

<210> 212
<211> 17
<212> DNA
<213> Homo sapiens

<400> 212
taaaaatcat cgattcc                                                    17

<210> 213
<211> 17
<212> DNA
<213> Homo sapiens

<400> 213
aaaaatcatc gattccg                                                    17

<210> 214
<211> 17
<212> DNA
<213> Homo sapiens

<400> 214
aaaatcatcg attccgc                                                    17

<210> 215
<211> 17
<212> DNA
<213> Homo sapiens

<400> 215
aaatcatcga ttccgct                                                    17

<210> 216
<211> 17
<212> DNA
<213> Homo sapiens

<400> 216
aatcatcgat tccgctt                                                    17

<210> 217
<211> 17
<212> DNA
<213> Homo sapiens

<400> 217
atcatcgatt ccgcttc                                                    17

<210> 218
<211> 17
<212> DNA
<213> Homo sapiens

<400> 218
tcatcgattc cgcttct                                                    17

<210> 219
<211> 17
<212> DNA
<213> Homo sapiens

<400> 219
catcgattcc gcttctt                                                    17

<210> 220
<211> 17
<212> DNA
<213> Homo sapiens

<400> 220
atcgattccg cttcttg                                                    17

<210> 221
<211> 17
<212> DNA
<213> Homo sapiens

<400> 221
tcgattccgc ttcttgc                                                    17

<210> 222
<211> 17
<212> DNA
<213> Homo sapiens

<400> 222
cgattccgct tcttgca                                                    17

<210> 223
<211> 17
<212> DNA
<213> Homo sapiens

<400> 223
gattccgctt cttgcat                                                    17

<210> 224
<211> 17
<212> DNA
<213> Homo sapiens

<400> 224
attccgcttc ttgcatg                                                    17

<210> 225
<211> 17
<212> DNA
<213> Homo sapiens

<400> 225
ttccgcttct tgcatga                                                    17

<210> 226
<211> 17
<212> DNA
<213> Homo sapiens

<400> 226
tccgcttctt gcatgaa                                                    17

<210> 227
<211> 17

<212> DNA
<213> Homo sapiens

<400> 227
ccgcttcttg catgaaa                                                    17

<210> 228
<211> 17
<212> DNA
<213> Homo sapiens

<400> 228
cgcttcttgc atgaaac                                                    17

<210> 229
<211> 17
<212> DNA
<213> Homo sapiens

<400> 229
gcttcttgca tgaaact                                                    17

<210> 230
<211> 17
<212> DNA
<213> Homo sapiens

<400> 230
cttcttgcat gaaactg                                                    17

<210> 231
<211> 17
<212> DNA
<213> Homo sapiens

<400> 231
ttcttgcatg aaactgg                                                    17

<210> 232
<211> 17
<212> DNA
<213> Homo sapiens

<400> 232
tcttgcatga aactgga                                                    17

<210> 233
<211> 17
<212> DNA
<213> Homo sapiens

<400> 233
cttgcatgaa actggag                                                    17

<210> 234
<211> 17
<212> DNA
<213> Homo sapiens

<400> 234
ttgcatgaaa ctggagg                                                    17

<210> 235
<211> 17
<212> DNA
<213> Homo sapiens

<400> 235
tgcatgaaac tggagga                                                    17

<210> 236
<211> 17
<212> DNA
<213> Homo sapiens

<400> 236
gcatgaaact ggaggag                                                    17

<210> 237
<211> 17
<212> DNA
<213> Homo sapiens

<400> 237
catgaaactg gaggagc                                                    17

<210> 238
<211> 17
<212> DNA
<213> Homo sapiens

<400> 238
atgaaactgg aggagca                                                    17

<210> 239
<211> 17
<212> DNA
<213> Homo sapiens

<400> 239
tgaaactgga ggagcaa                                                    17

<210> 240
<211> 17
<212> DNA
<213> Homo sapiens

<400> 240
gaaactggag gagcaat                                                    17

<210> 241
<211> 17
<212> DNA
<213> Homo sapiens

<400> 241
aaactggagg agcaatg                                                    17

<210> 242
<211> 17
<212> DNA
<213> Homo sapiens

<400> 242
aactggagga gcaatgg                                                    17

<210> 243
<211> 17
<212> DNA
<213> Homo sapiens

<400> 243
actggaggag caatggt                                                    17

<210> 244
<211> 17
<212> DNA
<213> Homo sapiens

<400> 244
ctggaggagc aatggtg                                                    17

<210> 245
<211> 17
<212> DNA
<213> Homo sapiens

<400> 245
tggaggagca atggtgt                                                    17

<210> 246
<211> 17
<212> DNA
<213> Homo sapiens

<400> 246
ggaggagcaa tggtgta                                                    17

<210> 247
<211> 17
<212> DNA
<213> Homo sapiens

<400> 247
gaggagcaat ggtgtat                                                    17

<210> 248
<211> 17
<212> DNA
<213> Homo sapiens

<400> 248
aggagcaatg gtgtatg                                                    17

<210> 249
<211> 17
<212> DNA
<213> Homo sapiens

<400> 249
ggagcaatgg tgtatgg                                                    17

<210> 250

<211> 17
<212> DNA
<213> Homo sapiens

<400> 250
gagcaatggt gtatggc                                                    17

<210> 251
<211> 17
<212> DNA
<213> Homo sapiens

<400> 251
agcaatggtg tatggcc                                                    17

<210> 252
<211> 17
<212> DNA
<213> Homo sapiens

<400> 252
gcaatggtgt atggcct                                                    17

<210> 253
<211> 17
<212> DNA
<213> Homo sapiens

<400> 253
caatggtgta tggcctt                                                    17

<210> 254
<211> 17
<212> DNA
<213> Homo sapiens

<400> 254
aatggtgtat ggcctta                                                    17

<210> 255
<211> 17
<212> DNA
<213> Homo sapiens

<400> 255
atggtgtatg gccttat                                                    17

<210> 256
<211> 17
<212> DNA
<213> Homo sapiens

<400> 256
tggtgtatgg ccttata                                                    17

<210> 257
<211> 17
<212> DNA
<213> Homo sapiens

<400> 257

ggtgtatggc cttataa                                                    17

<210> 258
<211> 17
<212> DNA
<213> Homo sapiens

<400> 258
gtgtatggcc ttataat                                                    17

<210> 259
<211> 17
<212> DNA
<213> Homo sapiens

<400> 259
tgtatggcct tataatg                                                    17

<210> 260
<211> 17
<212> DNA
<213> Homo sapiens

<400> 260
gtatggcctt ataatgg                                                    17

<210> 261
<211> 17
<212> DNA
<213> Homo sapiens

<400> 261
tatggcctta taatggg                                                    17

<210> 262
<211> 17
<212> DNA
<213> Homo sapiens

<400> 262
atggccttat aatggga                                                    17

<210> 263
<211> 17
<212> DNA
<213> Homo sapiens

<400> 263
tggccttata atgggac                                                    17

<210> 264
<211> 17
<212> DNA
<213> Homo sapiens

<400> 264
ggccttataa tgggact                                                    17

<210> 265
<211> 17
<212> DNA

<213> Homo sapiens

<400> 265
gccttataat gggacta                                                    17

<210> 266
<211> 17
<212> DNA
<213> Homo sapiens

<400> 266
ccttataatg ggactaa                                                    17

<210> 267
<211> 17
<212> DNA
<213> Homo sapiens

<400> 267
cttataatgg gactaat                                                    17

<210> 268
<211> 17
<212> DNA
<213> Homo sapiens

<400> 268
ttataatggg actaatt                                                    17

<210> 269
<211> 17
<212> DNA
<213> Homo sapiens

<400> 269
tataatggga ctaattt                                                    17

<210> 270
<211> 17
<212> DNA
<213> Homo sapiens

<400> 270
ataatgggac taatttt                                                    17

<210> 271
<211> 17
<212> DNA
<213> Homo sapiens

<400> 271
taatgggact aatttta                                                    17

<210> 272
<211> 17
<212> DNA
<213> Homo sapiens

<400> 272
aatgggacta attttac                                                    17

<210> 273
<211> 17
<212> DNA
<213> Homo sapiens

<400> 273
atgggactaa ttttacg                                                    17

<210> 274
<211> 17
<212> DNA
<213> Homo sapiens

<400> 274
tgggactaat tttacga                                                    17

<210> 275
<211> 17
<212> DNA
<213> Homo sapiens

<400> 275
gggactaatt ttacgat                                                    17

<210> 276
<211> 17
<212> DNA
<213> Homo sapiens

<400> 276
ggactaattt tacgata                                                    17

<210> 277
<211> 17
<212> DNA
<213> Homo sapiens

<400> 277
gactaatttt acgatat                                                    17

<210> 278
<211> 17
<212> DNA
<213> Homo sapiens

<400> 278
actaatttta cgatatg                                                    17

<210> 279
<211> 17
<212> DNA
<213> Homo sapiens

<400> 279
ctaattttac gatatgc                                                    17

<210> 280
<211> 17
<212> DNA
<213> Homo sapiens

<400> 280
taattttacg atatgct                                                    17

<210> 281
<211> 17
<212> DNA
<213> Homo sapiens

<400> 281
aattttacga tatgcta                                                    17

<210> 282
<211> 17
<212> DNA
<213> Homo sapiens

<400> 282
attttacgat atgctac                                                    17

<210> 283
<211> 17
<212> DNA
<213> Homo sapiens

<400> 283
ttttacgata tgctaca                                                    17

<210> 284
<211> 17
<212> DNA
<213> Homo sapiens

<400> 284
tttacgatat gctacag                                                    17

<210> 285
<211> 17
<212> DNA
<213> Homo sapiens

<400> 285
ttacgatatg ctacagc                                                    17

<210> 286
<211> 17
<212> DNA
<213> Homo sapiens

<400> 286
tacgatatgc tacagca                                                    17

<210> 287
<211> 17
<212> DNA
<213> Homo sapiens

<400> 287
acgatatgct acagcac                                                    17

<210> 288
<211> 17

<212> DNA
<213> Homo sapiens

<400> 288
cgatatgcta cagcacc                                                    17

<210> 289
<211> 17
<212> DNA
<213> Homo sapiens

<400> 289
gatatgctac agcacca                                                    17

<210> 290
<211> 17
<212> DNA
<213> Homo sapiens

<400> 290
atatgctaca gcaccaa                                                    17

<210> 291
<211> 17
<212> DNA
<213> Homo sapiens

<400> 291
tatgctacag caccaac                                                    17

<210> 292
<211> 17
<212> DNA
<213> Homo sapiens

<400> 292
atgctacagc accaact                                                    17

<210> 293
<211> 17
<212> DNA
<213> Homo sapiens

<400> 293
tgctacagca ccaactg                                                    17

<210> 294
<211> 17
<212> DNA
<213> Homo sapiens

<400> 294
gctacagcac caactga                                                    17

<210> 295
<211> 17
<212> DNA
<213> Homo sapiens

<400> 295
ctacagcacc aactgat                                                    17

<210> 296
<211> 17
<212> DNA
<213> Homo sapiens

<400> 296
tacagcacca actgata                                                    17

<210> 297
<211> 17
<212> DNA
<213> Homo sapiens

<400> 297
acagcaccaa ctgatat                                                    17

<210> 298
<211> 17
<212> DNA
<213> Homo sapiens

<400> 298
cagcaccaac tgatatt                                                    17

<210> 299
<211> 17
<212> DNA
<213> Homo sapiens

<400> 299
agcaccaact gatattg                                                    17

<210> 300
<211> 17
<212> DNA
<213> Homo sapiens

<400> 300
gcaccaactg atattga                                                    17

<210> 301
<211> 17
<212> DNA
<213> Homo sapiens

<400> 301
caccaactga tattgaa                                                    17

<210> 302
<211> 17
<212> DNA
<213> Homo sapiens

<400> 302
accaactgat attgaaa                                                    17

<210> 303
<211> 17
<212> DNA
<213> Homo sapiens

<400> 303
ccaactgata ttgaaag                                                    17

<210> 304
<211> 17
<212> DNA
<213> Homo sapiens

<400> 304
caactgatat tgaaagt                                                    17

<210> 305
<211> 17
<212> DNA
<213> Homo sapiens

<400> 305
aactgatatt gaaagtg                                                    17

<210> 306
<211> 17
<212> DNA
<213> Homo sapiens

<400> 306
actgatattg aaagtgg                                                    17

<210> 307
<211> 17
<212> DNA
<213> Homo sapiens

<400> 307
ctgatattga aagtgga                                                    17

<210> 308
<211> 17
<212> DNA
<213> Homo sapiens

<400> 308
tgatattgaa agtggaa                                                    17

<210> 309
<211> 17
<212> DNA
<213> Homo sapiens

<400> 309
gatattgaaa gtggaac                                                    17

<210> 310
<211> 17
<212> DNA
<213> Homo sapiens

<400> 310
atattgaaag tggaact                                                    17

<210> 311

<211> 17
<212> DNA
<213> Homo sapiens

<400> 311
tattgaaagt ggaactg                                                    17

<210> 312
<211> 17
<212> DNA
<213> Homo sapiens

<400> 312
attgaaagtg gaactgt                                                    17

<210> 313
<211> 17
<212> DNA
<213> Homo sapiens

<400> 313
ttgaaagtgg aactgtc                                                    17

<210> 314
<211> 17
<212> DNA
<213> Homo sapiens

<400> 314
tgaaagtgga actgtct                                                    17

<210> 315
<211> 17
<212> DNA
<213> Homo sapiens

<400> 315
gaaagtggaa ctgtcta                                                    17

<210> 316
<211> 17
<212> DNA
<213> Homo sapiens

<400> 316
aaagtggaac tgtctat                                                    17

<210> 317
<211> 17
<212> DNA
<213> Homo sapiens

<400> 317
aagtggaact gtctatg                                                    17

<210> 318
<211> 17
<212> DNA
<213> Homo sapiens

<400> 318

agtggaactg tctatga                                                    17

<210> 319
<211> 17
<212> DNA
<213> Homo sapiens

<400> 319
gtggaactgt ctatgac                                                    17

<210> 320
<211> 17
<212> DNA
<213> Homo sapiens

<400> 320
tggaactgtc tatgact                                                    17

<210> 321
<211> 17
<212> DNA
<213> Homo sapiens

<400> 321
ggaactgtct atgactg                                                    17

<210> 322
<211> 17
<212> DNA
<213> Homo sapiens

<400> 322
gaactgtcta tgactgt                                                    17

<210> 323
<211> 17
<212> DNA
<213> Homo sapiens

<400> 323
aactgtctat gactgtg                                                    17

<210> 324
<211> 17
<212> DNA
<213> Homo sapiens

<400> 324
actgtctatg actgtgt                                                    17

<210> 325
<211> 17
<212> DNA
<213> Homo sapiens

<400> 325
ctgtctatga ctgtgta                                                    17

<210> 326
<211> 17
<212> DNA

<213> Homo sapiens

<400> 326
tgtctatgac tgtgtaa                                                    17

<210> 327
<211> 17
<212> DNA
<213> Homo sapiens

<400> 327
gtctatgact gtgtaaa                                                    17

<210> 328
<211> 17
<212> DNA
<213> Homo sapiens

<400> 328
tctatgactg tgtaaaa                                                    17

<210> 329
<211> 17
<212> DNA
<213> Homo sapiens

<400> 329
ctatgactgt gtaaaac                                                    17

<210> 330
<211> 17
<212> DNA
<213> Homo sapiens

<400> 330
tatgactgtg taaaact                                                    17

<210> 331
<211> 17
<212> DNA
<213> Homo sapiens

<400> 331
atgactgtgt aaaacta                                                    17

<210> 332
<211> 17
<212> DNA
<213> Homo sapiens

<400> 332
tgactgtgta aaactaa                                                    17

<210> 333
<211> 17
<212> DNA
<213> Homo sapiens

<400> 333
gactgtgtaa aactaac                                                    17

<210> 334
<211> 17
<212> DNA
<213> Homo sapiens

<400> 334
actgtgtaaa actaact                                                    17

<210> 335
<211> 17
<212> DNA
<213> Homo sapiens

<400> 335
ctgtgtaaaa ctaactt                                                    17

<210> 336
<211> 17
<212> DNA
<213> Homo sapiens

<400> 336
tgtgtaaaac taacttt                                                    17

<210> 337
<211> 17
<212> DNA
<213> Homo sapiens

<400> 337
gtgtaaaact aactttc                                                    17

<210> 338
<211> 17
<212> DNA
<213> Homo sapiens

<400> 338
tgtaaaacta actttca                                                    17

<210> 339
<211> 17
<212> DNA
<213> Homo sapiens

<400> 339
gtaaaactaa ctttcag                                                    17

<210> 340
<211> 17
<212> DNA
<213> Homo sapiens

<400> 340
taaaactaac tttcagt                                                    17

<210> 341
<211> 17
<212> DNA
<213> Homo sapiens

<400> 341
aaaactaact ttcagtc                                                    17

<210> 342
<211> 17
<212> DNA
<213> Homo sapiens

<400> 342
aaactaactt tcagtcc                                                    17

<210> 343
<211> 17
<212> DNA
<213> Homo sapiens

<400> 343
aactaacttt cagtcca                                                    17

<210> 344
<211> 17
<212> DNA
<213> Homo sapiens

<400> 344
actaactttc agtccat                                                    17

<210> 345
<211> 17
<212> DNA
<213> Homo sapiens

<400> 345
ctaactttca gtccatc                                                    17

<210> 346
<211> 17
<212> DNA
<213> Homo sapiens

<400> 346
taactttcag tccatca                                                    17

<210> 347
<211> 17
<212> DNA
<213> Homo sapiens

<400> 347
aactttcagt ccatcaa                                                    17

<210> 348
<211> 17
<212> DNA
<213> Homo sapiens

<400> 348
actttcagtc catcaac                                                    17

<210> 349
<211> 17

<212> DNA
<213> Homo sapiens

<400> 349
ctttcagtcc atcaact                                                    17

<210> 350
<211> 17
<212> DNA
<213> Homo sapiens

<400> 350
tttcagtcca tcaactc                                                    17

<210> 351
<211> 17
<212> DNA
<213> Homo sapiens

<400> 351
ttcagtccat caactct                                                    17

<210> 352
<211> 17
<212> DNA
<213> Homo sapiens

<400> 352
tcagtccatc aactctg                                                    17

<210> 353
<211> 17
<212> DNA
<213> Homo sapiens

<400> 353
cagtccatca actctgc                                                    17

<210> 354
<211> 17
<212> DNA
<213> Homo sapiens

<400> 354
agtccatcaa ctctgct                                                    17

<210> 355
<211> 17
<212> DNA
<213> Homo sapiens

<400> 355
gtccatcaac tctgctg                                                    17

<210> 356
<211> 17
<212> DNA
<213> Homo sapiens

<400> 356
tccatcaact ctgctgg                                                    17

<210> 357
<211> 17
<212> DNA
<213> Homo sapiens

<400> 357
ccatcaactc tgctggt                                                    17

<210> 358
<211> 17
<212> DNA
<213> Homo sapiens

<400> 358
catcaactct gctggtt                                                    17

<210> 359
<211> 17
<212> DNA
<213> Homo sapiens

<400> 359
atcaactctg ctggtta                                                    17

<210> 360
<211> 17
<212> DNA
<213> Homo sapiens

<400> 360
tcaactctgc tggttaa                                                    17

<210> 361
<211> 17
<212> DNA
<213> Homo sapiens

<400> 361
caactctgct ggttaat                                                    17

<210> 362
<211> 17
<212> DNA
<213> Homo sapiens

<400> 362
aactctgctg gttaata                                                    17

<210> 363
<211> 17
<212> DNA
<213> Homo sapiens

<400> 363
actctgctgg ttaatat                                                    17

<210> 364
<211> 17
<212> DNA
<213> Homo sapiens

<400> 364
ctctgctggt taatatc                                                    17

<210> 365
<211> 17
<212> DNA
<213> Homo sapiens

<400> 365
tctgctggtt aatatca                                                    17

<210> 366
<211> 17
<212> DNA
<213> Homo sapiens

<400> 366
ctgctggtta atatcac                                                    17

<210> 367
<211> 17
<212> DNA
<213> Homo sapiens

<400> 367
tgctggttaa tatcact                                                    17

<210> 368
<211> 17
<212> DNA
<213> Homo sapiens

<400> 368
gctggttaat atcactg                                                    17

<210> 369
<211> 17
<212> DNA
<213> Homo sapiens

<400> 369
ctggttaata tcactga                                                    17

<210> 370
<211> 17
<212> DNA
<213> Homo sapiens

<400> 370
tggttaatat cactgac                                                    17

<210> 371
<211> 17
<212> DNA
<213> Homo sapiens

<400> 371
ggttaatatc actgacc                                                    17

<210> 372

<211> 17
<212> DNA
<213> Homo sapiens

<400> 372
gttaatatca ctgacca                                                    17

<210> 373
<211> 17
<212> DNA
<213> Homo sapiens

<400> 373
ttaatatcac tgaccaa                                                    17

<210> 374
<211> 17
<212> DNA
<213> Homo sapiens

<400> 374
taatatcact gaccaag                                                    17

<210> 375
<211> 17
<212> DNA
<213> Homo sapiens

<400> 375
aatatcactg accaagt                                                    17

<210> 376
<211> 17
<212> DNA
<213> Homo sapiens

<400> 376
atatcactga ccaagtt                                                    17

<210> 377
<211> 17
<212> DNA
<213> Homo sapiens

<400> 377
tatcactgac caagttt                                                    17

<210> 378
<211> 17
<212> DNA
<213> Homo sapiens

<400> 378
atcactgacc aagttta                                                    17

<210> 379
<211> 17
<212> DNA
<213> Homo sapiens

<400> 379

tcactgacca agtttat                                                    17

<210> 380
<211> 17
<212> DNA
<213> Homo sapiens

<400> 380
cactgaccaa gtttatg                                                    17

<210> 381
<211> 17
<212> DNA
<213> Homo sapiens

<400> 381
actgaccaag tttatga                                                    17

<210> 382
<211> 17
<212> DNA
<213> Homo sapiens

<400> 382
ctgaccaagt ttatgaa                                                    17

<210> 383
<211> 17
<212> DNA
<213> Homo sapiens

<400> 383
tgaccaagtt tatgaat                                                    17

<210> 384
<211> 17
<212> DNA
<213> Homo sapiens

<400> 384
gaccaagttt atgaata                                                    17

<210> 385
<211> 17
<212> DNA
<213> Homo sapiens

<400> 385
accaagttta tgaatat                                                    17

<210> 386
<211> 17
<212> DNA
<213> Homo sapiens

<400> 386
ccaagtttat gaatata                                                    17

<210> 387
<211> 17
<212> DNA

<213> Homo sapiens

<400> 387
caagtttatg aatataa                                                    17

<210> 388
<211> 17
<212> DNA
<213> Homo sapiens

<400> 388
aagtttatga atataaa                                                    17

<210> 389
<211> 17
<212> DNA
<213> Homo sapiens

<400> 389
agtttatgaa tataaat                                                    17

<210> 390
<211> 17
<212> DNA
<213> Homo sapiens

<400> 390
gtttatgaat ataaata                                                    17

<210> 391
<211> 17
<212> DNA
<213> Homo sapiens

<400> 391
tttatgaata taaatac                                                    17

<210> 392
<211> 17
<212> DNA
<213> Homo sapiens

<400> 392
ttatgaatat aaataca                                                    17

<210> 393
<211> 17
<212> DNA
<213> Homo sapiens

<400> 393
tatgaatata aatacaa                                                    17

<210> 394
<211> 17
<212> DNA
<213> Homo sapiens

<400> 394
atgaatataa atacaaa                                                    17

<210> 395
<211> 17
<212> DNA
<213> Homo sapiens

<400> 395
tgaatataaa tacaaaa                                                    17

<210> 396
<211> 17
<212> DNA
<213> Homo sapiens

<400> 396
gaatataaat acaaaag                                                    17

<210> 397
<211> 17
<212> DNA
<213> Homo sapiens

<400> 397
aatataaata caaaaga                                                    17

<210> 398
<211> 17
<212> DNA
<213> Homo sapiens

<400> 398
atataaatac aaaagag                                                    17

<210> 399
<211> 17
<212> DNA
<213> Homo sapiens

<400> 399
tataaataca aaagaga                                                    17

<210> 400
<211> 17
<212> DNA
<213> Homo sapiens

<400> 400
ataaatacaa aagagaa                                                    17

<210> 401
<211> 17
<212> DNA
<213> Homo sapiens

<400> 401
taaatacaaa agagaaa                                                    17

<210> 402
<211> 17
<212> DNA
<213> Homo sapiens

<400> 402
aaatacaaaa gagaaat                                                    17

<210> 403
<211> 17
<212> DNA
<213> Homo sapiens

<400> 403
aatacaaaag agaaata                                                    17

<210> 404
<211> 17
<212> DNA
<213> Homo sapiens

<400> 404
atacaaaaga gaaataa                                                    17

<210> 405
<211> 17
<212> DNA
<213> Homo sapiens

<400> 405
tacaaaagag aaataag                                                    17

<210> 406
<211> 17
<212> DNA
<213> Homo sapiens

<400> 406
acaaaagaga aataagt                                                    17

<210> 407
<211> 17
<212> DNA
<213> Homo sapiens

<400> 407
caaaagagaa ataagtc                                                    17

<210> 408
<211> 17
<212> DNA
<213> Homo sapiens

<400> 408
aaaagagaaa taagtca                                                    17

<210> 409
<211> 17
<212> DNA
<213> Homo sapiens

<400> 409
aaagagaaat aagtcag                                                    17

<210> 410
<211> 17

<212> DNA
<213> Homo sapiens

<400> 410
aagagaaata agtcagc                                                    17

<210> 411
<211> 17
<212> DNA
<213> Homo sapiens

<400> 411
agagaaataa gtcagca                                                    17

<210> 412
<211> 17
<212> DNA
<213> Homo sapiens

<400> 412
gagaaataag tcagcac                                                    17

<210> 413
<211> 17
<212> DNA
<213> Homo sapiens

<400> 413
agaaataagt cagcaca                                                    17

<210> 414
<211> 17
<212> DNA
<213> Homo sapiens

<400> 414
gaaataagtc agcacaa                                                    17

<210> 415
<211> 17
<212> DNA
<213> Homo sapiens

<400> 415
aaataagtca gcacaac                                                    17

<210> 416
<211> 17
<212> DNA
<213> Homo sapiens

<400> 416
aataagtcag cacaaca                                                    17

<210> 417
<211> 17
<212> DNA
<213> Homo sapiens

<400> 417
ataagtcagc acaacat                                                    17

<210> 418
<211> 17
<212> DNA
<213> Homo sapiens

<400> 418
taagtcagca caacatc                                                    17

<210> 419
<211> 17
<212> DNA
<213> Homo sapiens

<400> 419
aagtcagcac aacatca                                                    17

<210> 420
<211> 17
<212> DNA
<213> Homo sapiens

<400> 420
agtcagcaca acatcaa                                                    17

<210> 421
<211> 17
<212> DNA
<213> Homo sapiens

<400> 421
gtcagcacaa catcaat                                                    17

<210> 422
<211> 17
<212> DNA
<213> Homo sapiens

<400> 422
tcagcacaac atcaatc                                                    17

<210> 423
<211> 17
<212> DNA
<213> Homo sapiens

<400> 423
cagcacaaca tcaatcc                                                    17

<210> 424
<211> 17
<212> DNA
<213> Homo sapiens

<400> 424
agcacaacat caatcct                                                    17

<210> 425
<211> 17
<212> DNA
<213> Homo sapiens

<400> 425
gcacaacatc aatcctc                                                    17

<210> 426
<211> 17
<212> DNA
<213> Homo sapiens

<400> 426
cacaacatca atcctca                                                    17

<210> 427
<211> 17
<212> DNA
<213> Homo sapiens

<400> 427
acaacatcaa tcctcat                                                    17

<210> 428
<211> 17
<212> DNA
<213> Homo sapiens

<400> 428
caacatcaat cctcatc                                                    17

<210> 429
<211> 17
<212> DNA
<213> Homo sapiens

<400> 429
aacatcaatc ctcatca                                                    17

<210> 430
<211> 17
<212> DNA
<213> Homo sapiens

<400> 430
acatcaatcc tcatcaa                                                    17

<210> 431
<211> 17
<212> DNA
<213> Homo sapiens

<400> 431
catcaatcct catcaag                                                    17

<210> 432
<211> 17
<212> DNA
<213> Homo sapiens

<400> 432
atcaatcctc atcaagg                                                    17

<210> 433

<211> 17
<212> DNA
<213> Homo sapiens

<400> 433
tcaatcctca tcaagga                                                    17

<210> 434
<211> 17
<212> DNA
<213> Homo sapiens

<400> 434
caatcctcat caaggaa                                                    17

<210> 435
<211> 17
<212> DNA
<213> Homo sapiens

<400> 435
aatcctcatc aaggaaa                                                    17

<210> 436
<211> 17
<212> DNA
<213> Homo sapiens

<400> 436
atcctcatca aggaaat                                                    17

<210> 437
<211> 17
<212> DNA
<213> Homo sapiens

<400> 437
tcctcatcaa ggaaatg                                                    17

<210> 438
<211> 17
<212> DNA
<213> Homo sapiens

<400> 438
cctcatcaag gaaatgc                                                    17

<210> 439
<211> 17
<212> DNA
<213> Homo sapiens

<400> 439
ctcatcaagg aaatgct                                                    17

<210> 440
<211> 17
<212> DNA
<213> Homo sapiens

<400> 440

tcatcaagga aatgcta 17

<210> 441
<211> 17
<212> DNA
<213> Homo sapiens

<400> 441
catcaaggaa atgctat 17

<210> 442
<211> 17
<212> DNA
<213> Homo sapiens

<400> 442
atcaaggaaa tgctata 17

<210> 443
<211> 17
<212> DNA
<213> Homo sapiens

<400> 443
tcaaggaaat gctatac 17

<210> 444
<211> 17
<212> DNA
<213> Homo sapiens

<400> 444
caaggaaatg ctatact 17

<210> 445
<211> 17
<212> DNA
<213> Homo sapiens

<400> 445
aaggaaatgc tatactt 17

<210> 446
<211> 17
<212> DNA
<213> Homo sapiens

<400> 446
aggaaatgct atacttg 17

<210> 447
<211> 17
<212> DNA
<213> Homo sapiens

<400> 447
ggaaatgcta tacttga 17

<210> 448
<211> 17
<212> DNA

<213> Homo sapiens

<400> 448
gaaatgctat acttgaa                                                    17

<210> 449
<211> 17
<212> DNA
<213> Homo sapiens

<400> 449
aaatgctata cttgaaa                                                    17

<210> 450
<211> 17
<212> DNA
<213> Homo sapiens

<400> 450
aatgctatac ttgaaaa                                                    17

<210> 451
<211> 17
<212> DNA
<213> Homo sapiens

<400> 451
atgctatact tgaaaag                                                    17

<210> 452
<211> 17
<212> DNA
<213> Homo sapiens

<400> 452
tgctatactt gaaaaga                                                    17

<210> 453
<211> 17
<212> DNA
<213> Homo sapiens

<400> 453
gctatacttg aaaagat                                                    17

<210> 454
<211> 17
<212> DNA
<213> Homo sapiens

<400> 454
ctatacttga aaagatg                                                    17

<210> 455
<211> 17
<212> DNA
<213> Homo sapiens

<400> 455
tatacttgaa aagatga                                                    17

<210> 456
<211> 17
<212> DNA
<213> Homo sapiens

<400> 456
atacttgaaa agatgac                                                    17

<210> 457
<211> 17
<212> DNA
<213> Homo sapiens

<400> 457
tacttgaaaa gatgaca                                                    17

<210> 458
<211> 17
<212> DNA
<213> Homo sapiens

<400> 458
acttgaaaag atgacat                                                    17

<210> 459
<211> 17
<212> DNA
<213> Homo sapiens

<400> 459
cttgaaaaga tgacatt                                                    17

<210> 460
<211> 17
<212> DNA
<213> Homo sapiens

<400> 460
ttgaaaagat gacattt                                                    17

<210> 461
<211> 17
<212> DNA
<213> Homo sapiens

<400> 461
tgaaaagatg acatttg                                                    17

<210> 462
<211> 17
<212> DNA
<213> Homo sapiens

<400> 462
gaaaagatga catttga                                                    17

<210> 463
<211> 17
<212> DNA
<213> Homo sapiens

<400> 463
aaaagatgac atttgat                                                    17

<210> 464
<211> 17
<212> DNA
<213> Homo sapiens

<400> 464
aaagatgaca tttgatc                                                    17

<210> 465
<211> 17
<212> DNA
<213> Homo sapiens

<400> 465
aagatgacat ttgatcc                                                    17

<210> 466
<211> 17
<212> DNA
<213> Homo sapiens

<400> 466
agatgacatt tgatcca                                                    17

<210> 467
<211> 17
<212> DNA
<213> Homo sapiens

<400> 467
gatgacattt gatccag                                                    17

<210> 468
<211> 17
<212> DNA
<213> Homo sapiens

<400> 468
atgacatttg atccaga                                                    17

<210> 469
<211> 17
<212> DNA
<213> Homo sapiens

<400> 469
tgacatttga tccagaa                                                    17

<210> 470
<211> 17
<212> DNA
<213> Homo sapiens

<400> 470
gacatttgat ccagaaa                                                    17

<210> 471
<211> 17

<212> DNA
<213> Homo sapiens

<400> 471
acatttgatc cagaaat                                                    17

<210> 472
<211> 17
<212> DNA
<213> Homo sapiens

<400> 472
catttgatcc agaaatc                                                    17

<210> 473
<211> 17
<212> DNA
<213> Homo sapiens

<400> 473
atttgatcca gaaatct                                                    17

<210> 474
<211> 17
<212> DNA
<213> Homo sapiens

<400> 474
tttgatccag aaatctt                                                    17

<210> 475
<211> 17
<212> DNA
<213> Homo sapiens

<400> 475
ttgatccaga aatcttc                                                    17

<210> 476
<211> 17
<212> DNA
<213> Homo sapiens

<400> 476
tgatccagaa atcttct                                                    17

<210> 477
<211> 17
<212> DNA
<213> Homo sapiens

<400> 477
gatccagaaa tcttctt                                                    17

<210> 478
<211> 17
<212> DNA
<213> Homo sapiens

<400> 478
atccagaaat cttcttc                                                    17

<210> 479
<211> 17
<212> DNA
<213> Homo sapiens

<400> 479
tccagaaatc ttcttca                                                    17

<210> 480
<211> 17
<212> DNA
<213> Homo sapiens

<400> 480
ccagaaatct tcttcaa                                                    17

<210> 481
<211> 17
<212> DNA
<213> Homo sapiens

<400> 481
cagaaatctt cttcaat                                                    17

<210> 482
<211> 17
<212> DNA
<213> Homo sapiens

<400> 482
agaaatcttc ttcaatg                                                    17

<210> 483
<211> 17
<212> DNA
<213> Homo sapiens

<400> 483
gaaatcttct tcaatgt                                                    17

<210> 484
<211> 17
<212> DNA
<213> Homo sapiens

<400> 484
aaatcttctt caatgtt                                                    17

<210> 485
<211> 17
<212> DNA
<213> Homo sapiens

<400> 485
aatcttcttc aatgttt                                                    17

<210> 486
<211> 17
<212> DNA
<213> Homo sapiens

<400> 486
atcttcttca atgtttt                                                    17

<210> 487
<211> 17
<212> DNA
<213> Homo sapiens

<400> 487
tcttcttcaa tgtttta                                                    17

<210> 488
<211> 17
<212> DNA
<213> Homo sapiens

<400> 488
cttcttcaat gttttac                                                    17

<210> 489
<211> 17
<212> DNA
<213> Homo sapiens

<400> 489
ttcttcaatg ttttact                                                    17

<210> 490
<211> 17
<212> DNA
<213> Homo sapiens

<400> 490
tcttcaatgt tttactg                                                    17

<210> 491
<211> 17
<212> DNA
<213> Homo sapiens

<400> 491
cttcaatgtt ttactgc                                                    17

<210> 492
<211> 17
<212> DNA
<213> Homo sapiens

<400> 492
ttcaatgttt tactgcc                                                    17

<210> 493
<211> 17
<212> DNA
<213> Homo sapiens

<400> 493
tcaatgtttt actgcca                                                    17

<210> 494

<211> 17
<212> DNA
<213> Homo sapiens

<400> 494
caatgtttta ctgccac                                                    17

<210> 495
<211> 17
<212> DNA
<213> Homo sapiens

<400> 495
aatgttttac tgccacc                                                    17

<210> 496
<211> 17
<212> DNA
<213> Homo sapiens

<400> 496
atgttttact gccacca                                                    17

<210> 497
<211> 17
<212> DNA
<213> Homo sapiens

<400> 497
tgttttactg ccaccaa                                                    17

<210> 498
<211> 17
<212> DNA
<213> Homo sapiens

<400> 498
gttttactgc caccaat                                                    17

<210> 499
<211> 17
<212> DNA
<213> Homo sapiens

<400> 499
ttttactgcc accaatt                                                    17

<210> 500
<211> 17
<212> DNA
<213> Homo sapiens

<400> 500
tttactgcca ccaatta                                                    17

<210> 501
<211> 17
<212> DNA
<213> Homo sapiens

<400> 501

ttactgccac caattat 17

<210> 502
<211> 17
<212> DNA
<213> Homo sapiens

<400> 502
tactgccacc aattata 17

<210> 503
<211> 17
<212> DNA
<213> Homo sapiens

<400> 503
actgccacca attatat 17

<210> 504
<211> 17
<212> DNA
<213> Homo sapiens

<400> 504
ctgccaccaa ttatatt 17

<210> 505
<211> 17
<212> DNA
<213> Homo sapiens

<400> 505
tgccaccaat tatattt 17

<210> 506
<211> 17
<212> DNA
<213> Homo sapiens

<400> 506
gccaccaatt atatttc 17

<210> 507
<211> 17
<212> DNA
<213> Homo sapiens

<400> 507
ccaccaatta tatttca 17

<210> 508
<211> 17
<212> DNA
<213> Homo sapiens

<400> 508
caccaattat atttcat 17

<210> 509
<211> 17
<212> DNA

<213> Homo sapiens

<400> 509
accaattata tttcatg                                                    17

<210> 510
<211> 17
<212> DNA
<213> Homo sapiens

<400> 510
ccaattatat ttcatgc                                                    17

<210> 511
<211> 17
<212> DNA
<213> Homo sapiens

<400> 511
caattatatt tcatgca                                                    17

<210> 512
<211> 17
<212> DNA
<213> Homo sapiens

<400> 512
aattatattt catgcag                                                    17

<210> 513
<211> 17
<212> DNA
<213> Homo sapiens

<400> 513
attatatttc atgcagg                                                    17

<210> 514
<211> 17
<212> DNA
<213> Homo sapiens

<400> 514
ttatatttca tgcagga                                                    17

<210> 515
<211> 17
<212> DNA
<213> Homo sapiens

<400> 515
tatatttcat gcaggat                                                    17

<210> 516
<211> 17
<212> DNA
<213> Homo sapiens

<400> 516
atatttcatg caggata                                                    17

<210> 517
<211> 17
<212> DNA
<213> Homo sapiens

<400> 517
tatttcatgc aggatat                                                    17

<210> 518
<211> 17
<212> DNA
<213> Homo sapiens

<400> 518
atttcatgca ggatata                                                    17

<210> 519
<211> 17
<212> DNA
<213> Homo sapiens

<400> 519
tttcatgcag gatatag                                                    17

<210> 520
<211> 17
<212> DNA
<213> Homo sapiens

<400> 520
ttcatgcagg atatagt                                                    17

<210> 521
<211> 17
<212> DNA
<213> Homo sapiens

<400> 521
tcatgcagga tatagtc                                                    17

<210> 522
<211> 17
<212> DNA
<213> Homo sapiens

<400> 522
catgcaggat atagtct                                                    17

<210> 523
<211> 17
<212> DNA
<213> Homo sapiens

<400> 523
atgcaggata tagtcta                                                    17

<210> 524
<211> 17
<212> DNA
<213> Homo sapiens

<400> 524
tgcaggatat agtctaa                                                    17

<210> 525
<211> 17
<212> DNA
<213> Homo sapiens

<400> 525
gcaggatata gtctaaa                                                    17

<210> 526
<211> 17
<212> DNA
<213> Homo sapiens

<400> 526
caggatatag tctaaag                                                    17

<210> 527
<211> 17
<212> DNA
<213> Homo sapiens

<400> 527
aggatatagt ctaaaga                                                    17

<210> 528
<211> 17
<212> DNA
<213> Homo sapiens

<400> 528
ggatatagtc taaagaa                                                    17

<210> 529
<211> 17
<212> DNA
<213> Homo sapiens

<400> 529
gatatagtct aaagaag                                                    17

<210> 530
<211> 17
<212> DNA
<213> Homo sapiens

<400> 530
atatagtcta aagaaga                                                    17

<210> 531
<211> 17
<212> DNA
<213> Homo sapiens

<400> 531
tatagtctaa agaagag                                                    17

<210> 532
<211> 17

<212> DNA
<213> Homo sapiens

<400> 532
atagtctaaa gaagaga                                                    17

<210> 533
<211> 17
<212> DNA
<213> Homo sapiens

<400> 533
tagtctaaag aagagac                                                    17

<210> 534
<211> 17
<212> DNA
<213> Homo sapiens

<400> 534
agtctaaaga agagaca                                                    17

<210> 535
<211> 17
<212> DNA
<213> Homo sapiens

<400> 535
gtctaaagaa gagacac                                                    17

<210> 536
<211> 17
<212> DNA
<213> Homo sapiens

<400> 536
tctaaagaag agacact                                                    17

<210> 537
<211> 17
<212> DNA
<213> Homo sapiens

<400> 537
ctaaagaaga gacactt                                                    17

<210> 538
<211> 17
<212> DNA
<213> Homo sapiens

<400> 538
taaagaagag acacttt                                                    17

<210> 539
<211> 17
<212> DNA
<213> Homo sapiens

<400> 539
aaagaagaga cactttt                                                    17

<210> 540
<211> 17
<212> DNA
<213> Homo sapiens

<400> 540
aagaagagac acttttt                                                    17

<210> 541
<211> 17
<212> DNA
<213> Homo sapiens

<400> 541
agaagagaca ctttttt                                                    17

<210> 542
<211> 17
<212> DNA
<213> Homo sapiens

<400> 542
gaagagacac ttttttc                                                    17

<210> 543
<211> 17
<212> DNA
<213> Homo sapiens

<400> 543
aagagacact tttttca                                                    17

<210> 544
<211> 17
<212> DNA
<213> Homo sapiens

<400> 544
agagacactt ttttcaa                                                    17

<210> 545
<211> 17
<212> DNA
<213> Homo sapiens

<400> 545
gagacacttt tttcaaa                                                    17

<210> 546
<211> 17
<212> DNA
<213> Homo sapiens

<400> 546
agacactttt ttcaaaa                                                    17

<210> 547
<211> 17
<212> DNA
<213> Homo sapiens

<400> 547
gacacttttt tcaaaac                                                    17

<210> 548
<211> 17
<212> DNA
<213> Homo sapiens

<400> 548
acactttttt caaaact                                                    17

<210> 549
<211> 17
<212> DNA
<213> Homo sapiens

<400> 549
   cacttttttc aaaactt                                                     17

<210> 550
<211> 17
<212> DNA
<213> Homo sapiens

<400> 550
   acttttttca aaactta                                                     17

<210> 551
<211> 17
<212> DNA
<213> Homo sapiens

<400> 551
   cttttttcaa aacttag                                                     17

<210> 552
<211> 17
<212> DNA
<213> Homo sapiens

<400> 552
ttttttcaaa acttagg                                                    17

<210> 553
<211> 17
<212> DNA
<213> Homo sapiens

<400> 553
tttttcaaaa cttagga                                                    17

<210> 554
<211> 17
<212> DNA
<213> Homo sapiens

<400> 554
ttttcaaaac ttaggat                                                    17

<210> 555

<211> 17
<212> DNA
<213> Homo sapiens

<400> 555
tttcaaaact taggatc                                                    17

<210> 556
<211> 17
<212> DNA
<213> Homo sapiens

<400> 556
ttcaaaactt aggatct                                                    17

<210> 557
<211> 17
<212> DNA
<213> Homo sapiens

<400> 557
tcaaaactta ggatcta                                                    17

<210> 558
<211> 17
<212> DNA
<213> Homo sapiens

<400> 558
caaaacttag gatctat                                                    17

<210> 559
<211> 17
<212> DNA
<213> Homo sapiens

<400> 559
aaaacttagg atctatt                                                    17

<210> 560
<211> 17
<212> DNA
<213> Homo sapiens

<400> 560
aaacttagga tctattt                                                    17

<210> 561
<211> 17
<212> DNA
<213> Homo sapiens

<400> 561
aacttaggat ctatttt                                                    17

<210> 562
<211> 17
<212> DNA
<213> Homo sapiens

<400> 562

acttaggatc tatttta                                                    17

<210> 563
<211> 17
<212> DNA
<213> Homo sapiens

<400> 563
cttaggatct attttaa                                                    17

<210> 564
<211> 17
<212> DNA
<213> Homo sapiens

<400> 564
ttaggatcta ttttaac                                                    17

<210> 565
<211> 17
<212> DNA
<213> Homo sapiens

<400> 565
taggatctat tttaacg                                                    17

<210> 566
<211> 17
<212> DNA
<213> Homo sapiens

<400> 566
aggatctatt ttaacgt                                                    17

<210> 567
<211> 17
<212> DNA
<213> Homo sapiens

<400> 567
ggatctattt taacgta                                                    17

<210> 568
<211> 17
<212> DNA
<213> Homo sapiens

<400> 568
gatctatttt aacgtat                                                    17

<210> 569
<211> 17
<212> DNA
<213> Homo sapiens

<400> 569
atctatttta acgtatg                                                    17

<210> 570
<211> 17
<212> DNA

<213> Homo sapiens

<400> 570
tctattttaa cgtatgc                                                    17

<210> 571
<211> 17
<212> DNA
<213> Homo sapiens

<400> 571
ctattttaac gtatgcc                                                    17

<210> 572
<211> 17
<212> DNA
<213> Homo sapiens

<400> 572
tattttaacg tatgcct                                                    17

<210> 573
<211> 17
<212> DNA
<213> Homo sapiens

<400> 573
attttaacgt atgcctt                                                    17

<210> 574
<211> 17
<212> DNA
<213> Homo sapiens

<400> 574
ttttaacgta tgccttc                                                    17

<210> 575
<211> 17
<212> DNA
<213> Homo sapiens

<400> 575
tttaacgtat gccttct                                                    17

<210> 576
<211> 17
<212> DNA
<213> Homo sapiens

<400> 576
ttaacgtatg ccttctt                                                    17

<210> 577
<211> 17
<212> DNA
<213> Homo sapiens

<400> 577
taacgtatgc cttcttg                                                    17

<210> 578
<211> 17
<212> DNA
<213> Homo sapiens

<400> 578
aacgtatgcc ttcttgg                                                    17

<210> 579
<211> 17
<212> DNA
<213> Homo sapiens

<400> 579
acgtatgcct tcttggg                                                    17

<210> 580
<211> 17
<212> DNA
<213> Homo sapiens

<400> 580
cgtatgcctt cttggga                                                    17

<210> 581
<211> 17
<212> DNA
<213> Homo sapiens

<400> 581
gtatgccttc ttgggaa                                                    17

<210> 582
<211> 17
<212> DNA
<213> Homo sapiens

<400> 582
tatgccttct tgggaac                                                    17

<210> 583
<211> 17
<212> DNA
<213> Homo sapiens

<400> 583
atgccttctt gggaact                                                    17

<210> 584
<211> 17
<212> DNA
<213> Homo sapiens

<400> 584
tgccttcttg ggaactg                                                    17

<210> 585
<211> 17
<212> DNA
<213> Homo sapiens

<400> 585
gccttcttgg gaactgc                                                    17

<210> 586
<211> 17
<212> DNA
<213> Homo sapiens

<400> 586
ccttcttggg aactgcc                                                    17

<210> 587
<211> 17
<212> DNA
<213> Homo sapiens

<400> 587
cttcttggga actgcca                                                    17

<210> 588
<211> 17
<212> DNA
<213> Homo sapiens

<400> 588
ttcttgggaa ctgccat                                                    17

<210> 589
<211> 17
<212> DNA
<213> Homo sapiens

<400> 589
tcttgggaac tgccatc                                                    17

<210> 590
<211> 17
<212> DNA
<213> Homo sapiens

<400> 590
cttgggaact gccatct                                                    17

<210> 591
<211> 17
<212> DNA
<213> Homo sapiens

<400> 591
ttgggaactg ccatctc                                                    17

<210> 592
<211> 17
<212> DNA
<213> Homo sapiens

<400> 592
tgggaactgc catctcc                                                    17

<210> 593
<211> 17

<212> DNA
<213> Homo sapiens

<400> 593
gggaactgcc atctcct                                                    17

<210> 594
<211> 17
<212> DNA
<213> Homo sapiens

<400> 594
ggaactgcca tctcctg                                                    17

<210> 595
<211> 17
<212> DNA
<213> Homo sapiens

<400> 595
gaactgccat ctcctgc                                                    17

<210> 596
<211> 17
<212> DNA
<213> Homo sapiens

<400> 596
aactgccatc tcctgca                                                    17

<210> 597
<211> 17
<212> DNA
<213> Homo sapiens

<400> 597
actgccatct cctgcat                                                    17

<210> 598
<211> 17
<212> DNA
<213> Homo sapiens

<400> 598
ctgccatctc ctgcatc                                                    17

<210> 599
<211> 17
<212> DNA
<213> Homo sapiens

<400> 599
tgccatctcc tgcatcg                                                    17

<210> 600
<211> 17
<212> DNA
<213> Homo sapiens

<400> 600
gccatctcct gcatcgt                                                    17

<210> 601
<211> 17
<212> DNA
<213> Homo sapiens

<400> 601
ccatctcctg catcgtc                                                    17

<210> 602
<211> 17
<212> DNA
<213> Homo sapiens

<400> 602
catctcctgc atcgtca                                                    17

<210> 603
<211> 17
<212> DNA
<213> Homo sapiens

<400> 603
atctcctgca tcgtcat                                                    17

<210> 604
<211> 17
<212> DNA
<213> Homo sapiens

<400> 604
tctcctgcat cgtcata                                                    17

<210> 605
<211> 17
<212> DNA
<213> Homo sapiens

<400> 605
ctcctgcatc gtcatag                                                    17

<210> 606
<211> 17
<212> DNA
<213> Homo sapiens

<400> 606
tcctgcatcg tcatagg                                                    17

<210> 607
<211> 17
<212> DNA
<213> Homo sapiens

<400> 607
cctgcatcgt cataggg                                                    17

<210> 608
<211> 17
<212> DNA
<213> Homo sapiens

<400> 608
ctgcatcgtc atagggt                                                    17

<210> 609
<211> 17
<212> DNA
<213> Homo sapiens

<400> 609
tgcatcgtca tagggtt                                                    17

<210> 610
<211> 17
<212> DNA
<213> Homo sapiens

<400> 610
gcatcgtcat agggtta                                                    17

<210> 611
<211> 17
<212> DNA
<213> Homo sapiens

<400> 611
catcgtcata gggttaa                                                    17

<210> 612
<211> 17
<212> DNA
<213> Homo sapiens

<400> 612
atcgtcatag ggttaat                                                    17

<210> 613
<211> 17
<212> DNA
<213> Homo sapiens

<400> 613
tcgtcatagg gttaatt                                                    17

<210> 614
<211> 17
<212> DNA
<213> Homo sapiens

<400> 614
cgtcataggg ttaatta                                                    17

<210> 615
<211> 17
<212> DNA
<213> Homo sapiens

<400> 615
gtcatagggt taattat                                                    17

<210> 616

<211> 17
<212> DNA
<213> Homo sapiens

<400> 616
tcatagggtt aattatg                                                    17

<210> 617
<211> 17
<212> DNA
<213> Homo sapiens

<400> 617
catagggtta attatgt                                                    17

<210> 618
<211> 17
<212> DNA
<213> Homo sapiens

<400> 618
atagggttaa ttatgta                                                    17

<210> 619
<211> 17
<212> DNA
<213> Homo sapiens

<400> 619
tagggttaat tatgtat                                                    17

<210> 620
<211> 17
<212> DNA
<213> Homo sapiens

<400> 620
agggttaatt atgtatg                                                    17

<210> 621
<211> 17
<212> DNA
<213> Homo sapiens

<400> 621
gggttaatta tgtatgg                                                    17

<210> 622
<211> 17
<212> DNA
<213> Homo sapiens

<400> 622
ggttaattat gtatggt                                                    17

<210> 623
<211> 17
<212> DNA
<213> Homo sapiens

<400> 623

gttaattatg tatggtt 17

<210> 624
<211> 17
<212> DNA
<213> Homo sapiens

<400> 624
ttaattatgt atggttt 17

<210> 625
<211> 17
<212> DNA
<213> Homo sapiens

<400> 625
taattatgta tggtttt 17

<210> 626
<211> 17
<212> DNA
<213> Homo sapiens

<400> 626
aattatgtat ggttttg 17

<210> 627
<211> 17
<212> DNA
<213> Homo sapiens

<400> 627
attatgtatg gttttgt 17

<210> 628
<211> 17
<212> DNA
<213> Homo sapiens

<400> 628
ttatgtatgg ttttgtg 17

<210> 629
<211> 17
<212> DNA
<213> Homo sapiens

<400> 629
tatgtatggt tttgtga 17

<210> 630
<211> 17
<212> DNA
<213> Homo sapiens

<400> 630
atgtatggtt ttgtgaa 17

<210> 631
<211> 17
<212> DNA

<213> Homo sapiens

<400> 631
tgtatggttt tgtgaag                                                    17

<210> 632
<211> 17
<212> DNA
<213> Homo sapiens

<400> 632
gtatggtttt gtgaagg                                                    17

<210> 633
<211> 17
<212> DNA
<213> Homo sapiens

<400> 633
tatggttttg tgaaggc                                                    17

<210> 634
<211> 17
<212> DNA
<213> Homo sapiens

<400> 634
atggttttgt gaaggct                                                    17

<210> 635
<211> 17
<212> DNA
<213> Homo sapiens

<400> 635
tggttttgtg aaggcta                                                    17

<210> 636
<211> 17
<212> DNA
<213> Homo sapiens

<400> 636
ggttttgtga aggctat                                                    17

<210> 637
<211> 17
<212> DNA
<213> Homo sapiens

<400> 637
gttttgtgaa ggctatg                                                    17

<210> 638
<211> 17
<212> DNA
<213> Homo sapiens

<400> 638
ttttgtgaag gctatga                                                    17

<210> 639
<211> 17
<212> DNA
<213> Homo sapiens

<400> 639
tttgtgaagg ctatgat                                                    17

<210> 640
<211> 17
<212> DNA
<213> Homo sapiens

<400> 640
ttgtgaaggc tatgata                                                    17

<210> 641
<211> 17
<212> DNA
<213> Homo sapiens

<400> 641
tgtgaaggct atgatac                                                    17

<210> 642
<211> 17
<212> DNA
<213> Homo sapiens

<400> 642
gtgaaggcta tgataca                                                    17

<210> 643
<211> 17
<212> DNA
<213> Homo sapiens

<400> 643
tgaaggctat gatacat                                                    17

<210> 644
<211> 17
<212> DNA
<213> Homo sapiens

<400> 644
gaaggctatg atacatg                                                    17

<210> 645
<211> 17
<212> DNA
<213> Homo sapiens

<400> 645
aaggctatga tacatgc                                                    17

<210> 646
<211> 17
<212> DNA
<213> Homo sapiens

<400> 646
aggctatgat acatgct                                                    17

<210> 647
<211> 17
<212> DNA
<213> Homo sapiens

<400> 647
ggctatgata catgctg                                                    17

<210> 648
<211> 17
<212> DNA
<213> Homo sapiens

<400> 648
gctatgatac atgctgg                                                    17

<210> 649
<211> 17
<212> DNA
<213> Homo sapiens

<400> 649
ctatgataca tgctggc                                                    17

<210> 650
<211> 17
<212> DNA
<213> Homo sapiens

<400> 650
tatgatacat gctggcc                                                    17

<210> 651
<211> 17
<212> DNA
<213> Homo sapiens

<400> 651
atgatacatg ctggcca                                                    17

<210> 652
<211> 17
<212> DNA
<213> Homo sapiens

<400> 652
tgatacatgc tggccag                                                    17

<210> 653
<211> 17
<212> DNA
<213> Homo sapiens

<400> 653
gatacatgct ggccagc                                                    17

<210> 654
<211> 17

<212> DNA
<213> Homo sapiens

<400> 654
atacatgctg gccagct                                                    17

<210> 655
<211> 17
<212> DNA
<213> Homo sapiens

<400> 655
tacatgctgg ccagctg                                                    17

<210> 656
<211> 17
<212> DNA
<213> Homo sapiens

<400> 656
acatgctggc cagctga                                                    17

<210> 657
<211> 17
<212> DNA
<213> Homo sapiens

<400> 657
catgctggcc agctgaa                                                    17

<210> 658
<211> 17
<212> DNA
<213> Homo sapiens

<400> 658
atgctggcca gctgaaa                                                    17

<210> 659
<211> 17
<212> DNA
<213> Homo sapiens

<400> 659
tgctggccag ctgaaaa                                                    17

<210> 660
<211> 17
<212> DNA
<213> Homo sapiens

<400> 660
gctggccagc tgaaaaa                                                    17

<210> 661
<211> 17
<212> DNA
<213> Homo sapiens

<400> 661
ctggccagct gaaaaat                                                    17

<210> 662
<211> 17
<212> DNA
<213> Homo sapiens

<400> 662
tggccagctg aaaaatg                                                    17

<210> 663
<211> 17
<212> DNA
<213> Homo sapiens

<400> 663
ggccagctga aaaatgg                                                    17

<210> 664
<211> 17
<212> DNA
<213> Homo sapiens

<400> 664
gccagctgaa aaatgga                                                    17

<210> 665
<211> 17
<212> DNA
<213> Homo sapiens

<400> 665
ccagctgaaa aatggag                                                    17

<210> 666
<211> 17
<212> DNA
<213> Homo sapiens

<400> 666
cagctgaaaa atggaga                                                    17

<210> 667
<211> 17
<212> DNA
<213> Homo sapiens

<400> 667
agctgaaaaa tggagac                                                    17

<210> 668
<211> 17
<212> DNA
<213> Homo sapiens

<400> 668
gctgaaaaat ggagact                                                    17

<210> 669
<211> 17
<212> DNA
<213> Homo sapiens

<400> 669
ctgaaaaatg gagactt                                                    17

<210> 670
<211> 17
<212> DNA
<213> Homo sapiens

<400> 670
tgaaaaatgg agacttt                                                    17

<210> 671
<211> 17
<212> DNA
<213> Homo sapiens

<400> 671
gaaaaatgga gactttc                                                    17

<210> 672
<211> 17
<212> DNA
<213> Homo sapiens

<400> 672
aaaaatggag actttca                                                    17

<210> 673
<211> 17
<212> DNA
<213> Homo sapiens

<400> 673
aaaatggaga ctttcat                                                    17

<210> 674
<211> 17
<212> DNA
<213> Homo sapiens

<400> 674
aaatggagac tttcatt                                                    17

<210> 675
<211> 17
<212> DNA
<213> Homo sapiens

<400> 675
aatggagact ttcattt                                                    17

<210> 676
<211> 17
<212> DNA
<213> Homo sapiens

<400> 676
atggagactt tcatttc                                                    17

<210> 677

<211> 17
<212> DNA
<213> Homo sapiens

<400> 677
tggagacttt catttca                                                    17

<210> 678
<211> 17
<212> DNA
<213> Homo sapiens

<400> 678
ggagactttc atttcac                                                    17

<210> 679
<211> 17
<212> DNA
<213> Homo sapiens

<400> 679
gagactttca tttcact                                                    17

<210> 680
<211> 17
<212> DNA
<213> Homo sapiens

<400> 680
agactttcat ttcactg                                                    17

<210> 681
<211> 17
<212> DNA
<213> Homo sapiens

<400> 681
gactttcatt tcactga                                                    17

<210> 682
<211> 17
<212> DNA
<213> Homo sapiens

<400> 682
actttcattt cactgac                                                    17

<210> 683
<211> 17
<212> DNA
<213> Homo sapiens

<400> 683
ctttcatttc actgact                                                    17

<210> 684
<211> 17
<212> DNA
<213> Homo sapiens

<400> 684

tttcatttca ctgactg                                                    17

<210> 685
<211> 17
<212> DNA
<213> Homo sapiens

<400> 685
ttcatttcac tgactgt                                                    17

<210> 686
<211> 17
<212> DNA
<213> Homo sapiens

<400> 686
tcatttcact gactgtt                                                    17

<210> 687
<211> 17
<212> DNA
<213> Homo sapiens

<400> 687
catttcactg actgttt                                                    17

<210> 688
<211> 17
<212> DNA
<213> Homo sapiens

<400> 688
atttcactga ctgttta                                                    17

<210> 689
<211> 17
<212> DNA
<213> Homo sapiens

<400> 689
tttcactgac tgtttat                                                    17

<210> 690
<211> 17
<212> DNA
<213> Homo sapiens

<400> 690
ttcactgact gtttatt                                                    17

<210> 691
<211> 17
<212> DNA
<213> Homo sapiens

<400> 691
tcactgactg tttattt                                                    17

<210> 692
<211> 17
<212> DNA

<213> Homo sapiens

<400> 692
cactgactgt ttatttt                                                    17

<210> 693
<211> 17
<212> DNA
<213> Homo sapiens

<400> 693
actgactgtt tattttt                                                    17

<210> 694
<211> 17
<212> DNA
<213> Homo sapiens

<400> 694
ctgactgttt attttt t                                                   17

<210> 695
<211> 17
<212> DNA
<213> Homo sapiens

<400> 695
tgactgttta ttttttg                                                    17

<210> 696
<211> 17
<212> DNA
<213> Homo sapiens

<400> 696
gactgtttat tttttgg                                                    17

<210> 697
<211> 17
<212> DNA
<213> Homo sapiens

<400> 697
actgtttatt ttttggt                                                    17

<210> 698
<211> 17
<212> DNA
<213> Homo sapiens

<400> 698
ctgtttattt tttggtt                                                    17

<210> 699
<211> 17
<212> DNA
<213> Homo sapiens

<400> 699
tgtttatttt ttggttc                                                    17

<210> 700
<211> 17
<212> DNA
<213> Homo sapiens

<400> 700
gtttattttt tggttca                                                    17

<210> 701
<211> 17
<212> DNA
<213> Homo sapiens

<400> 701
tttatttttt ggttcac                                                    17

<210> 702
<211> 17
<212> DNA
<213> Homo sapiens

<400> 702
ttattttttg gttcact                                                    17

<210> 703
<211> 17
<212> DNA
<213> Homo sapiens

<400> 703
tattttttgg ttcactg                                                    17

<210> 704
<211> 17
<212> DNA
<213> Homo sapiens

<400> 704
attttttggt tcactga                                                    17

<210> 705
<211> 17
<212> DNA
<213> Homo sapiens

<400> 705
ttttttggtt cactgat                                                    17

<210> 706
<211> 17
<212> DNA
<213> Homo sapiens

<400> 706
tttttggttc actgatg                                                    17

<210> 707
<211> 17
<212> DNA
<213> Homo sapiens

<400> 707
ttttggttca ctgatgt                                                    17

<210> 708
<211> 17
<212> DNA
<213> Homo sapiens

<400> 708
tttggttcac tgatgtc                                                    17

<210> 709
<211> 17
<212> DNA
<213> Homo sapiens

<400> 709
ttggttcact gatgtct                                                    17

<210> 710
<211> 17
<212> DNA
<213> Homo sapiens

<400> 710
tggttcactg atgtctg                                                    17

<210> 711
<211> 17
<212> DNA
<213> Homo sapiens

<400> 711
ggttcactga tgtctgc                                                    17

<210> 712
<211> 17
<212> DNA
<213> Homo sapiens

<400> 712
gttcactgat gtctgct                                                    17

<210> 713
<211> 17
<212> DNA
<213> Homo sapiens

<400> 713
ttcactgatg tctgcta                                                    17

<210> 714
<211> 17
<212> DNA
<213> Homo sapiens

<400> 714
tcactgatgt ctgctac                                                    17

<210> 715
<211> 17

<212> DNA
<213> Homo sapiens

<400> 715
cactgatgtc tgctaca                                                    17

<210> 716
<211> 17
<212> DNA
<213> Homo sapiens

<400> 716
actgatgtct gctacag                                                    17

<210> 717
<211> 17
<212> DNA
<213> Homo sapiens

<400> 717
ctgatgtctg ctacaga                                                    17

<210> 718
<211> 17
<212> DNA
<213> Homo sapiens

<400> 718
tgatgtctgc tacagat                                                    17

<210> 719
<211> 17
<212> DNA
<213> Homo sapiens

<400> 719
gatgtctgct acagatc                                                    17

<210> 720
<211> 17
<212> DNA
<213> Homo sapiens

<400> 720
atgtctgcta cagatcc                                                    17

<210> 721
<211> 17
<212> DNA
<213> Homo sapiens

<400> 721
tgtctgctac agatcca                                                    17

<210> 722
<211> 17
<212> DNA
<213> Homo sapiens

<400> 722
gtctgctaca gatccag                                                    17

<210> 723
<211> 17
<212> DNA
<213> Homo sapiens

<400> 723
tctgctacag atccagt                                                    17

<210> 724
<211> 17
<212> DNA
<213> Homo sapiens

<400> 724
ctgctacaga tccagtg                                                    17

<210> 725
<211> 17
<212> DNA
<213> Homo sapiens

<400> 725
tgctacagat ccagtga                                                    17

<210> 726
<211> 17
<212> DNA
<213> Homo sapiens

<400> 726
gctacagatc cagtgac                                                    17

<210> 727
<211> 17
<212> DNA
<213> Homo sapiens

<400> 727
ctacagatcc agtgaca                                                    17

<210> 728
<211> 17
<212> DNA
<213> Homo sapiens

<400> 728
tacagatcca gtgacag                                                    17

<210> 729
<211> 17
<212> DNA
<213> Homo sapiens

<400> 729
acagatccag tgacagt                                                    17

<210> 730
<211> 17
<212> DNA
<213> Homo sapiens

<400> 730
cagatccagt gacagtg                                                    17

<210> 731
<211> 17
<212> DNA
<213> Homo sapiens

<400> 731
agatccagtg acagtgc                                                    17

<210> 732
<211> 17
<212> DNA
<213> Homo sapiens

<400> 732
gatccagtga cagtgct                                                    17

<210> 733
<211> 17
<212> DNA
<213> Homo sapiens

<400> 733
atccagtgac agtgctg                                                    17

<210> 734
<211> 17
<212> DNA
<213> Homo sapiens

<400> 734
tccagtgaca gtgctgg                                                    17

<210> 735
<211> 17
<212> DNA
<213> Homo sapiens

<400> 735
ccagtgacag tgctggc                                                    17

<210> 736
<211> 17
<212> DNA
<213> Homo sapiens

<400> 736
cagtgacagt gctggcc                                                    17

<210> 737
<211> 17
<212> DNA
<213> Homo sapiens

<400> 737
agtgacagtg ctggcca                                                    17

<210> 738

<211> 17
<212> DNA
<213> Homo sapiens

<400> 738
gtgacagtgc tggccat                                                    17

<210> 739
<211> 17
<212> DNA
<213> Homo sapiens

<400> 739
tgacagtgct ggccatt                                                    17

<210> 740
<211> 17
<212> DNA
<213> Homo sapiens

<400> 740
gacagtgctg gccattt                                                    17

<210> 741
<211> 17
<212> DNA
<213> Homo sapiens

<400> 741
acagtgctgg ccatttt                                                    17

<210> 742
<211> 17
<212> DNA
<213> Homo sapiens

<400> 742
cagtgctggc cattttc                                                    17

<210> 743
<211> 17
<212> DNA
<213> Homo sapiens

<400> 743
agtgctggcc attttcc                                                    17

<210> 744
<211> 17
<212> DNA
<213> Homo sapiens

<400> 744
gtgctggcca ttttcca                                                    17

<210> 745
<211> 17
<212> DNA
<213> Homo sapiens

<400> 745

tgctggccat tttccat                                                    17

<210> 746
<211> 17
<212> DNA
<213> Homo sapiens

<400> 746
gctggccatt ttccatg                                                    17

<210> 747
<211> 17
<212> DNA
<213> Homo sapiens

<400> 747
ctggccattt tccatga                                                    17

<210> 748
<211> 17
<212> DNA
<213> Homo sapiens

<400> 748
tggccatttt ccatgaa                                                    17

<210> 749
<211> 17
<212> DNA
<213> Homo sapiens

<400> 749
ggccattttc catgaac                                                    17

<210> 750
<211> 17
<212> DNA
<213> Homo sapiens

<400> 750
gccattttcc atgaact                                                    17

<210> 751
<211> 17
<212> DNA
<213> Homo sapiens

<400> 751
ccattttcca tgaactg                                                    17

<210> 752
<211> 17
<212> DNA
<213> Homo sapiens

<400> 752
cattttccat gaactgc                                                    17

<210> 753
<211> 17
<212> DNA

<213> Homo sapiens

<400> 753
attttccatg aactgca                                                    17

<210> 754
<211> 17
<212> DNA
<213> Homo sapiens

<400> 754
ttttccatga actgcac                                                    17

<210> 755
<211> 17
<212> DNA
<213> Homo sapiens

<400> 755
tttccatgaa ctgcacg                                                    17

<210> 756
<211> 17
<212> DNA
<213> Homo sapiens

<400> 756
ttccatgaac tgcacgt                                                    17

<210> 757
<211> 17
<212> DNA
<213> Homo sapiens

<400> 757
tccatgaact gcacgtc                                                    17

<210> 758
<211> 17
<212> DNA
<213> Homo sapiens

<400> 758
ccatgaactg cacgtcg                                                    17

<210> 759
<211> 17
<212> DNA
<213> Homo sapiens

<400> 759
catgaactgc acgtcga                                                    17

<210> 760
<211> 17
<212> DNA
<213> Homo sapiens

<400> 760
atgaactgca cgtcgac                                                    17

<210> 761
<211> 17
<212> DNA
<213> Homo sapiens

<400> 761
tgaactgcac gtcgacc                                                    17

<210> 762
<211> 17
<212> DNA
<213> Homo sapiens

<400> 762
gaactgcacg tcgaccc                                                    17

<210> 763
<211> 17
<212> DNA
<213> Homo sapiens

<400> 763
aactgcacgt cgaccct                                                    17

<210> 764
<211> 17
<212> DNA
<213> Homo sapiens

<400> 764
actgcacgtc gaccctg                                                    17

<210> 765
<211> 17
<212> DNA
<213> Homo sapiens

<400> 765
ctgcacgtcg accctga                                                    17

<210> 766
<211> 17
<212> DNA
<213> Homo sapiens

<400> 766
tgcacgtcga ccctgac                                                    17

<210> 767
<211> 17
<212> DNA
<213> Homo sapiens

<400> 767
gcacgtcgac cctgacc                                                    17

<210> 768
<211> 17
<212> DNA
<213> Homo sapiens

<400> 768
cacgtcgacc ctgacct                                                    17

<210> 769
<211> 17
<212> DNA
<213> Homo sapiens

<400> 769
acgtcgaccc tgacctg                                                    17

<210> 770
<211> 17
<212> DNA
<213> Homo sapiens

<400> 770
cgtcgaccct gacctgt                                                    17

<210> 771
<211> 17
<212> DNA
<213> Homo sapiens

<400> 771
gtcgaccctg acctgta                                                    17

<210> 772
<211> 17
<212> DNA
<213> Homo sapiens

<400> 772
tcgaccctga cctgtac                                                    17

<210> 773
<211> 17
<212> DNA
<213> Homo sapiens

<400> 773
cgaccctgac ctgtaca                                                    17

<210> 774
<211> 17
<212> DNA
<213> Homo sapiens

<400> 774
gaccctgacc tgtacac                                                    17

<210> 775
<211> 17
<212> DNA
<213> Homo sapiens

<400> 775
accctgacct gtacaca                                                    17

<210> 776
<211> 17

<212> DNA
<213> Homo sapiens

<400> 776
ccctgacctg tacacac                                                    17

<210> 777
<211> 17
<212> DNA
<213> Homo sapiens

<400> 777
cctgacctgt acacact                                                    17

<210> 778
<211> 17
<212> DNA
<213> Homo sapiens

<400> 778
ctgacctgta cacactc                                                    17

<210> 779
<211> 17
<212> DNA
<213> Homo sapiens

<400> 779
tgacctgtac acactct                                                    17

<210> 780
<211> 17
<212> DNA
<213> Homo sapiens

<400> 780
gacctgtaca cactctt                                                    17

<210> 781
<211> 17
<212> DNA
<213> Homo sapiens

<400> 781
acctgtacac actcttg                                                    17

<210> 782
<211> 17
<212> DNA
<213> Homo sapiens

<400> 782
cctgtacaca ctcttgt                                                    17

<210> 783
<211> 17
<212> DNA
<213> Homo sapiens

<400> 783
ctgtacacac tcttgtt                                                    17

<210> 784
<211> 17
<212> DNA
<213> Homo sapiens

<400> 784
tgtacacact cttgttt                                                    17

<210> 785
<211> 17
<212> DNA
<213> Homo sapiens

<400> 785
gtacacactc ttgtttg                                                    17

<210> 786
<211> 17
<212> DNA
<213> Homo sapiens

<400> 786
tacacactct tgtttgg                                                    17

<210> 787
<211> 17
<212> DNA
<213> Homo sapiens

<400> 787
acacactctt gtttgga                                                    17

<210> 788
<211> 17
<212> DNA
<213> Homo sapiens

<400> 788
cacactcttg tttggag                                                    17

<210> 789
<211> 17
<212> DNA
<213> Homo sapiens

<400> 789
acactcttgt ttggaga                                                    17

<210> 790
<211> 17
<212> DNA
<213> Homo sapiens

<400> 790
cactcttgtt tggagag                                                    17

<210> 791
<211> 17
<212> DNA
<213> Homo sapiens

<400> 791
actcttgttt ggagaga                                                    17

<210> 792
<211> 17
<212> DNA
<213> Homo sapiens

<400> 792
ctcttgtttg gagagag                                                    17

<210> 793
<211> 17
<212> DNA
<213> Homo sapiens

<400> 793
tcttgtttgg agagagt                                                    17

<210> 794
<211> 17
<212> DNA
<213> Homo sapiens

<400> 794
cttgtttgga gagagtg                                                    17

<210> 795
<211> 17
<212> DNA
<213> Homo sapiens

<400> 795
ttgtttggag agagtgt                                                    17

<210> 796
<211> 17
<212> DNA
<213> Homo sapiens

<400> 796
tgtttggaga gagtgtg                                                    17

<210> 797
<211> 17
<212> DNA
<213> Homo sapiens

<400> 797
gtttggagag agtgtgt                                                    17

<210> 798
<211> 17
<212> DNA
<213> Homo sapiens

<400> 798
tttggagaga gtgtgtt                                                    17

<210> 799

<211> 17
<212> DNA
<213> Homo sapiens

<400> 799
ttggagagag tgtgttg                                                    17

<210> 800
<211> 17
<212> DNA
<213> Homo sapiens

<400> 800
tggagagagt gtgttga                                                    17

<210> 801
<211> 17
<212> DNA
<213> Homo sapiens

<400> 801
ggagagagtg tgttgaa                                                    17

<210> 802
<211> 17
<212> DNA
<213> Homo sapiens

<400> 802
gagagagtgt gttgaat                                                    17

<210> 803
<211> 17
<212> DNA
<213> Homo sapiens

<400> 803
agagagtgtg ttgaatg                                                    17

<210> 804
<211> 17
<212> DNA
<213> Homo sapiens

<400> 804
gagagtgtgt tgaatga                                                    17

<210> 805
<211> 17
<212> DNA
<213> Homo sapiens

<400> 805
agagtgtgtt gaatgat                                                    17

<210> 806
<211> 17
<212> DNA
<213> Homo sapiens

<400> 806

gagtgtgttg aatgatg 17

<210> 807
<211> 17
<212> DNA
<213> Homo sapiens

<400> 807
agtgtgttga atgatgc 17

<210> 808
<211> 17
<212> DNA
<213> Homo sapiens

<400> 808
gtgtgttgaa tgatgca 17

<210> 809
<211> 17
<212> DNA
<213> Homo sapiens

<400> 809
tgtgttgaat gatgcag 17

<210> 810
<211> 17
<212> DNA
<213> Homo sapiens

<400> 810
gtgttgaatg atgcagt 17

<210> 811
<211> 17
<212> DNA
<213> Homo sapiens

<400> 811
tgttgaatga tgcagtg 17

<210> 812
<211> 17
<212> DNA
<213> Homo sapiens

<400> 812
gttgaatgat gcagtgg 17

<210> 813
<211> 17
<212> DNA
<213> Homo sapiens

<400> 813
ttgaatgatg cagtggc 17

<210> 814
<211> 17
<212> DNA

<213> Homo sapiens

<400> 814
tgaatgatgc agtggcc                                                    17

<210> 815
<211> 17
<212> DNA
<213> Homo sapiens

<400> 815
gaatgatgca gtggcca                                                    17

<210> 816
<211> 17
<212> DNA
<213> Homo sapiens

<400> 816
aatgatgcag tggccat                                                    17

<210> 817
<211> 17
<212> DNA
<213> Homo sapiens

<400> 817
atgatgcagt ggccata                                                    17

<210> 818
<211> 17
<212> DNA
<213> Homo sapiens

<400> 818
tgatgcagtg gccatag                                                    17

<210> 819
<211> 17
<212> DNA
<213> Homo sapiens

<400> 819
gatgcagtgg ccatagt                                                    17

<210> 820
<211> 17
<212> DNA
<213> Homo sapiens

<400> 820
atgcagtggc catagtc                                                    17

<210> 821
<211> 17
<212> DNA
<213> Homo sapiens

<400> 821
tgcagtggcc atagtcc                                                    17

<210> 822
<211> 17
<212> DNA
<213> Homo sapiens

<400> 822
gcagtggcca tagtcct                                                    17

<210> 823
<211> 17
<212> DNA
<213> Homo sapiens

<400> 823
cagtggccat agtcctt                                                    17

<210> 824
<211> 17
<212> DNA
<213> Homo sapiens

<400> 824
agtggccata gtcctta                                                    17

<210> 825
<211> 17
<212> DNA
<213> Homo sapiens

<400> 825
gtggccatag tccttac                                                    17

<210> 826
<211> 17
<212> DNA
<213> Homo sapiens

<400> 826
tggccatagt ccttaca                                                    17

<210> 827
<211> 17
<212> DNA
<213> Homo sapiens

<400> 827
ggccatagtc cttacat                                                    17

<210> 828
<211> 17
<212> DNA
<213> Homo sapiens

<400> 828
gccatagtcc ttacata                                                    17

<210> 829
<211> 17
<212> DNA
<213> Homo sapiens

<400> 829
ccatagtcct tacatat                                                    17

<210> 830
<211> 17
<212> DNA
<213> Homo sapiens

<400> 830
catagtcctt acatatt                                                    17

<210> 831
<211> 17
<212> DNA
<213> Homo sapiens

<400> 831
atagtcctta catattc                                                    17

<210> 832
<211> 17
<212> DNA
<213> Homo sapiens

<400> 832
tagtccttac atattct                                                    17

<210> 833
<211> 17
<212> DNA
<213> Homo sapiens

<400> 833
agtccttaca tattcta                                                    17

<210> 834
<211> 17
<212> DNA
<213> Homo sapiens

<400> 834
gtccttacat attctat                                                    17

<210> 835
<211> 17
<212> DNA
<213> Homo sapiens

<400> 835
tccttacata ttctata                                                    17

<210> 836
<211> 17
<212> DNA
<213> Homo sapiens

<400> 836
ccttacatat tctatat                                                    17

<210> 837
<211> 17

<212> DNA
<213> Homo sapiens

<400> 837
cttacatatt ctatatc                                                    17

<210> 838
<211> 17
<212> DNA
<213> Homo sapiens

<400> 838
ttacatattc tatatcc                                                    17

<210> 839
<211> 17
<212> DNA
<213> Homo sapiens

<400> 839
tacatattct atatcca                                                    17

<210> 840
<211> 17
<212> DNA
<213> Homo sapiens

<400> 840
acatattcta tatccat                                                    17

<210> 841
<211> 17
<212> DNA
<213> Homo sapiens

<400> 841
catattctat atccatt                                                    17

<210> 842
<211> 17
<212> DNA
<213> Homo sapiens

<400> 842
atattctata tccattt                                                    17

<210> 843
<211> 17
<212> DNA
<213> Homo sapiens

<400> 843
tattctatat ccattta                                                    17

<210> 844
<211> 17
<212> DNA
<213> Homo sapiens

<400> 844
attctatatc catttac                                                    17

<210> 845
<211> 17
<212> DNA
<213> Homo sapiens

<400> 845
ttctatatcc atttaca                                                    17

<210> 846
<211> 17
<212> DNA
<213> Homo sapiens

<400> 846
tctatatcca tttacag                                                    17

<210> 847
<211> 17
<212> DNA
<213> Homo sapiens

<400> 847
ctatatccat ttacagt                                                    17

<210> 848
<211> 17
<212> DNA
<213> Homo sapiens

<400> 848
tatatccatt tacagtc                                                    17

<210> 849
<211> 17
<212> DNA
<213> Homo sapiens

<400> 849
atatccattt acagtcc                                                    17

<210> 850
<211> 17
<212> DNA
<213> Homo sapiens

<400> 850
tatccattta cagtccc                                                    17

<210> 851
<211> 17
<212> DNA
<213> Homo sapiens

<400> 851
atccatttac agtccca                                                    17

<210> 852
<211> 17
<212> DNA
<213> Homo sapiens

<400> 852
tccatttaca gtcccaa                                                    17

<210> 853
<211> 17
<212> DNA
<213> Homo sapiens

<400> 853
ccatttacag tcccaag                                                    17

<210> 854
<211> 17
<212> DNA
<213> Homo sapiens

<400> 854
catttacagt cccaagg                                                    17

<210> 855
<211> 17
<212> DNA
<213> Homo sapiens

<400> 855
atttacagtc ccaagga                                                    17

<210> 856
<211> 17
<212> DNA
<213> Homo sapiens

<400> 856
tttacagtcc caaggag                                                    17

<210> 857
<211> 17
<212> DNA
<213> Homo sapiens

<400> 857
ttacagtccc aaggaga                                                    17

<210> 858
<211> 17
<212> DNA
<213> Homo sapiens

<400> 858
tacagtccca aggagaa                                                    17

<210> 859
<211> 17
<212> DNA
<213> Homo sapiens

<400> 859
acagtcccaa ggagaat                                                    17

<210> 860

<211> 17
<212> DNA
<213> Homo sapiens

<400> 860
cagtcccaag gagaatc                                                    17

<210> 861
<211> 17
<212> DNA
<213> Homo sapiens

<400> 861
agtcccaagg agaatcc                                                    17

<210> 862
<211> 17
<212> DNA
<213> Homo sapiens

<400> 862
gtcccaagga gaatcca                                                    17

<210> 863
<211> 17
<212> DNA
<213> Homo sapiens

<400> 863
tcccaaggag aatccaa                                                    17

<210> 864
<211> 17
<212> DNA
<213> Homo sapiens

<400> 864
cccaaggaga atccaaa                                                    17

<210> 865
<211> 17
<212> DNA
<213> Homo sapiens

<400> 865
ccaaggagaa tccaaat                                                    17

<210> 866
<211> 17
<212> DNA
<213> Homo sapiens

<400> 866
caaggagaat ccaaatg                                                    17

<210> 867
<211> 17
<212> DNA
<213> Homo sapiens

<400> 867

aaggagaatc caaatgc                                                    17

<210> 868
<211> 17
<212> DNA
<213> Homo sapiens

<400> 868
aggagaatcc aaatgca                                                    17

<210> 869
<211> 17
<212> DNA
<213> Homo sapiens

<400> 869
ggagaatcca aatgcat                                                    17

<210> 870
<211> 17
<212> DNA
<213> Homo sapiens

<400> 870
gagaatccaa atgcatt                                                    17

<210> 871
<211> 17
<212> DNA
<213> Homo sapiens

<400> 871
agaatccaaa tgcattt                                                    17

<210> 872
<211> 17
<212> DNA
<213> Homo sapiens

<400> 872
gaatccaaat gcatttg                                                    17

<210> 873
<211> 17
<212> DNA
<213> Homo sapiens

<400> 873
aatccaaatg catttga                                                    17

<210> 874
<211> 17
<212> DNA
<213> Homo sapiens

<400> 874
atccaaatgc atttgat                                                    17

<210> 875
<211> 17
<212> DNA

<213> Homo sapiens

<400> 875
tccaaatgca tttgatg                                                    17

<210> 876
<211> 17
<212> DNA
<213> Homo sapiens

<400> 876
ccaaatgcat ttgatgc                                                    17

<210> 877
<211> 17
<212> DNA
<213> Homo sapiens

<400> 877
caaatgcatt tgatgcc                                                    17

<210> 878
<211> 17
<212> DNA
<213> Homo sapiens

<400> 878
aaatgcattt gatgccg                                                    17

<210> 879
<211> 17
<212> DNA
<213> Homo sapiens

<400> 879
aatgcatttg atgccgc                                                    17

<210> 880
<211> 17
<212> DNA
<213> Homo sapiens

<400> 880
atgcatttga tgccgca                                                    17

<210> 881
<211> 17
<212> DNA
<213> Homo sapiens

<400> 881
tgcatttgat gccgcag                                                    17

<210> 882
<211> 17
<212> DNA
<213> Homo sapiens

<400> 882
gcatttgatg ccgcagc                                                    17

<210> 883
<211> 17
<212> DNA
<213> Homo sapiens

<400> 883
catttgatgc cgcagca                                                    17

<210> 884
<211> 17
<212> DNA
<213> Homo sapiens

<400> 884
atttgatgcc gcagcat                                                    17

<210> 885
<211> 17
<212> DNA
<213> Homo sapiens

<400> 885
tttgatgccg cagcatt                                                    17

<210> 886
<211> 17
<212> DNA
<213> Homo sapiens

<400> 886
ttgatgccgc agcattc                                                    17

<210> 887
<211> 17
<212> DNA
<213> Homo sapiens

<400> 887
tgatgccgca gcattct                                                    17

<210> 888
<211> 17
<212> DNA
<213> Homo sapiens

<400> 888
gatgccgcag cattctt                                                    17

<210> 889
<211> 17
<212> DNA
<213> Homo sapiens

<400> 889
atgccgcagc attcttc                                                    17

<210> 890
<211> 17
<212> DNA
<213> Homo sapiens

<400> 890
tgccgcagca ttcttcc                                                    17

<210> 891
<211> 17
<212> DNA
<213> Homo sapiens

<400> 891
gccgcagcat tcttcca                                                    17

<210> 892
<211> 17
<212> DNA
<213> Homo sapiens

<400> 892
ccgcagcatt cttccag                                                    17

<210> 893
<211> 17
<212> DNA
<213> Homo sapiens

<400> 893
cgcagcattc ttccagt                                                    17

<210> 894
<211> 17
<212> DNA
<213> Homo sapiens

<400> 894
gcagcattct tccagtc                                                    17

<210> 895
<211> 17
<212> DNA
<213> Homo sapiens

<400> 895
cagcattctt ccagtct                                                    17

<210> 896
<211> 17
<212> DNA
<213> Homo sapiens

<400> 896
agcattcttc cagtctg                                                    17

<210> 897
<211> 17
<212> DNA
<213> Homo sapiens

<400> 897
gcattcttcc agtctgt                                                    17

<210> 898
<211> 17

<212> DNA
<213> Homo sapiens

<400> 898
cattcttcca gtctgtg                                                    17

<210> 899
<211> 17
<212> DNA
<213> Homo sapiens

<400> 899
attcttccag tctgtgg                                                    17

<210> 900
<211> 17
<212> DNA
<213> Homo sapiens

<400> 900
ttcttccagt ctgtggg                                                    17

<210> 901
<211> 17
<212> DNA
<213> Homo sapiens

<400> 901
tcttccagtc tgtgggg                                                    17

<210> 902
<211> 17
<212> DNA
<213> Homo sapiens

<400> 902
cttccagtct gtgggga                                                    17

<210> 903
<211> 17
<212> DNA
<213> Homo sapiens

<400> 903
ttccagtctg tggggaa                                                    17

<210> 904
<211> 17
<212> DNA
<213> Homo sapiens

<400> 904
tccagtctgt ggggaat                                                    17

<210> 905
<211> 17
<212> DNA
<213> Homo sapiens

<400> 905
ccagtctgtg gggaatt                                                    17

<210> 906
<211> 17
<212> DNA
<213> Homo sapiens

<400> 906
cagtctgtgg ggaattt                                                    17

<210> 907
<211> 17
<212> DNA
<213> Homo sapiens

<400> 907
agtctgtggg gaatttc                                                    17

<210> 908
<211> 17
<212> DNA
<213> Homo sapiens

<400> 908
gtctgtgggg aatttcc                                                    17

<210> 909
<211> 17
<212> DNA
<213> Homo sapiens

<400> 909
tctgtgggga atttcct                                                    17

<210> 910
<211> 17
<212> DNA
<213> Homo sapiens

<400> 910
ctgtggggaa tttcctg                                                    17

<210> 911
<211> 17
<212> DNA
<213> Homo sapiens

<400> 911
tgtggggaat ttcctgg                                                    17

<210> 912
<211> 17
<212> DNA
<213> Homo sapiens

<400> 912
gtggggaatt tcctggg                                                    17

<210> 913
<211> 17
<212> DNA
<213> Homo sapiens

<400> 913
tggggaattt cctggga                                                    17

<210> 914
<211> 17
<212> DNA
<213> Homo sapiens

<400> 914
ggggaatttc ctgggaa                                                    17

<210> 915
<211> 17
<212> DNA
<213> Homo sapiens

<400> 915
gggaatttcc tgggaat                                                    17

<210> 916
<211> 17
<212> DNA
<213> Homo sapiens

<400> 916
ggaatttcct gggaatc                                                    17

<210> 917
<211> 17
<212> DNA
<213> Homo sapiens

<400> 917
gaatttcctg ggaatct                                                    17

<210> 918
<211> 17
<212> DNA
<213> Homo sapiens

<400> 918
aatttcctgg gaatctt                                                    17

<210> 919
<211> 17
<212> DNA
<213> Homo sapiens

<400> 919
atttcctggg aatcttc                                                    17

<210> 920
<211> 17
<212> DNA
<213> Homo sapiens

<400> 920
tttcctggga atcttcg                                                    17

<210> 921

<211> 17
<212> DNA
<213> Homo sapiens

<400> 921
ttcctgggaa tcttcgc                                                    17

<210> 922
<211> 17
<212> DNA
<213> Homo sapiens

<400> 922
tcctgggaat cttcgct                                                    17

<210> 923
<211> 17
<212> DNA
<213> Homo sapiens

<400> 923
cctgggaatc ttcgctg                                                    17

<210> 924
<211> 17
<212> DNA
<213> Homo sapiens

<400> 924
ctgggaatct tcgctgg                                                    17

<210> 925
<211> 17
<212> DNA
<213> Homo sapiens

<400> 925
tgggaatctt cgctggc                                                    17

<210> 926
<211> 17
<212> DNA
<213> Homo sapiens

<400> 926
gggaatcttc gctggct                                                    17

<210> 927
<211> 17
<212> DNA
<213> Homo sapiens

<400> 927
ggaatcttcg ctggctc                                                    17

<210> 928
<211> 17
<212> DNA
<213> Homo sapiens

<400> 928

gaatcttcgc tggctca                                                    17

<210> 929
<211> 17
<212> DNA
<213> Homo sapiens

<400> 929
aatcttcgct ggctcat                                                    17

<210> 930
<211> 17
<212> DNA
<213> Homo sapiens

<400> 930
atcttcgctg gctcatt                                                    17

<210> 931
<211> 17
<212> DNA
<213> Homo sapiens

<400> 931
tcttcgctgg ctcattt                                                    17

<210> 932
<211> 17
<212> DNA
<213> Homo sapiens

<400> 932
cttcgctggc tcatttg                                                    17

<210> 933
<211> 17
<212> DNA
<213> Homo sapiens

<400> 933
ttcgctggct catttgc                                                    17

<210> 934
<211> 17
<212> DNA
<213> Homo sapiens

<400> 934
tcgctggctc atttgca                                                    17

<210> 935
<211> 17
<212> DNA
<213> Homo sapiens

<400> 935
cgctggctca tttgcaa                                                    17

<210> 936
<211> 17
<212> DNA

<213> Homo sapiens

<400> 936
gctggctcat ttgcaat                                                    17

<210> 937
<211> 17
<212> DNA
<213> Homo sapiens

<400> 937
ctggctcatt tgcaatg                                                    17

<210> 938
<211> 17
<212> DNA
<213> Homo sapiens

<400> 938
tggctcattt gcaatgg                                                    17

<210> 939
<211> 17
<212> DNA
<213> Homo sapiens

<400> 939
ggctcatttg caatggg                                                    17

<210> 940
<211> 17
<212> DNA
<213> Homo sapiens

<400> 940
gctcatttgc aatgggg                                                    17

<210> 941
<211> 17
<212> DNA
<213> Homo sapiens

<400> 941
ctcatttgca atggggt                                                    17

<210> 942
<211> 17
<212> DNA
<213> Homo sapiens

<400> 942
tcatttgcaa tggggtc                                                    17

<210> 943
<211> 17
<212> DNA
<213> Homo sapiens

<400> 943
catttgcaat ggggtct                                                    17

<210> 944
<211> 17
<212> DNA
<213> Homo sapiens

<400> 944
   atttgcaatg gggtctg                                                    17

<210> 945
<211> 17
<212> DNA
<213> Homo sapiens

<400> 945
tttgcaatgg ggtctgc                                                   17

<210> 946
<211> 17
<212> DNA
<213> Homo sapiens

<400> 946
ttgcaatggg gtctgcg                                                   17

<210> 947
<211> 17
<212> DNA
<213> Homo sapiens

<400> 947
tgcaatgggg tctgcgt                                                   17

<210> 948
<211> 17
<212> DNA
<213> Homo sapiens

<400> 948
gcaatggggt ctgcgta                                                   17

<210> 949
<211> 17
<212> DNA
<213> Homo sapiens

<400> 949
caatggggtc tgcgtat                                                   17

<210> 950
<211> 17
<212> DNA
<213> Homo sapiens

<400> 950
aatggggtct gcgtatg                                                   17

<210> 951
<211> 17
<212> DNA
<213> Homo sapiens

<400> 951
atggggtctg cgtatgc                                                    17

<210> 952
<211> 17
<212> DNA
<213> Homo sapiens

<400> 952
tggggtctgc gtatgcc                                                    17

<210> 953
<211> 17
<212> DNA
<213> Homo sapiens

<400> 953
ggggtctgcg tatgcca                                                    17

<210> 954
<211> 17
<212> DNA
<213> Homo sapiens

<400> 954
gggtctgcgt atgccat                                                    17

<210> 955
<211> 17
<212> DNA
<213> Homo sapiens

<400> 955
ggtctgcgta tgccatc                                                    17

<210> 956
<211> 17
<212> DNA
<213> Homo sapiens

<400> 956
gtctgcgtat gccatca                                                    17

<210> 957
<211> 17
<212> DNA
<213> Homo sapiens

<400> 957
tctgcgtatg ccatcat                                                    17

<210> 958
<211> 17
<212> DNA
<213> Homo sapiens

<400> 958
ctgcgtatgc catcatc                                                    17

<210> 959
<211> 17

<212> DNA
<213> Homo sapiens

<400> 959
tgcgtatgcc atcatca                                                    17

<210> 960
<211> 17
<212> DNA
<213> Homo sapiens

<400> 960
gcgtatgcca tcatcac                                                    17

<210> 961
<211> 17
<212> DNA
<213> Homo sapiens

<400> 961
cgtatgccat catcaca                                                    17

<210> 962
<211> 17
<212> DNA
<213> Homo sapiens

<400> 962
gtatgccatc atcacag                                                    17

<210> 963
<211> 17
<212> DNA
<213> Homo sapiens

<400> 963
tatgccatca tcacagc                                                    17

<210> 964
<211> 17
<212> DNA
<213> Homo sapiens

<400> 964
atgccatcat cacagca                                                    17

<210> 965
<211> 17
<212> DNA
<213> Homo sapiens

<400> 965
tgccatcatc acagcac                                                    17

<210> 966
<211> 17
<212> DNA
<213> Homo sapiens

<400> 966
gccatcatca cagcact                                                    17

<210> 967
<211> 17
<212> DNA
<213> Homo sapiens

<400> 967
ccatcatcac agcactg                                                    17

<210> 968
<211> 17
<212> DNA
<213> Homo sapiens

<400> 968
catcatcaca gcactgt                                                    17

<210> 969
<211> 17
<212> DNA
<213> Homo sapiens

<400> 969
atcatcacag cactgtt                                                    17

<210> 970
<211> 17
<212> DNA
<213> Homo sapiens

<400> 970
tcatcacagc actgttg                                                    17

<210> 971
<211> 17
<212> DNA
<213> Homo sapiens

<400> 971
catcacagca ctgttga                                                    17

<210> 972
<211> 17
<212> DNA
<213> Homo sapiens

<400> 972
atcacagcac tgttgac                                                    17

<210> 973
<211> 17
<212> DNA
<213> Homo sapiens

<400> 973
tcacagcact gttgacc                                                    17

<210> 974
<211> 17
<212> DNA
<213> Homo sapiens

<400> 974
cacagcactg ttgacca                                                    17

<210> 975
<211> 17
<212> DNA
<213> Homo sapiens

<400> 975
acagcactgt tgaccaa                                                    17

<210> 976
<211> 17
<212> DNA
<213> Homo sapiens

<400> 976
cagcactgtt gaccaaa                                                    17

<210> 977
<211> 17
<212> DNA
<213> Homo sapiens

<400> 977
agcactgttg accaaat                                                    17

<210> 978
<211> 17
<212> DNA
<213> Homo sapiens

<400> 978
gcactgttga ccaaatt                                                    17

<210> 979
<211> 17
<212> DNA
<213> Homo sapiens

<400> 979
cactgttgac caaattt                                                    17

<210> 980
<211> 17
<212> DNA
<213> Homo sapiens

<400> 980
actgttgacc aaattta                                                    17

<210> 981
<211> 17
<212> DNA
<213> Homo sapiens

<400> 981
ctgttgacca aatttac                                                    17

<210> 982

<211> 17
<212> DNA
<213> Homo sapiens

<400> 982
tgttgaccaa atttacc                                                    17

<210> 983
<211> 17
<212> DNA
<213> Homo sapiens

<400> 983
gttgaccaaa tttacca                                                    17

<210> 984
<211> 17
<212> DNA
<213> Homo sapiens

<400> 984
ttgaccaaat ttaccaa                                                    17

<210> 985
<211> 17
<212> DNA
<213> Homo sapiens

<400> 985
tgaccaaatt taccaag                                                    17

<210> 986
<211> 17
<212> DNA
<213> Homo sapiens

<400> 986
gaccaaattt accaagc                                                    17

<210> 987
<211> 17
<212> DNA
<213> Homo sapiens

<400> 987
accaaattta ccaagct                                                    17

<210> 988
<211> 17
<212> DNA
<213> Homo sapiens

<400> 988
ccaaatttac caagctg                                                    17

<210> 989
<211> 17
<212> DNA
<213> Homo sapiens

<400> 989

caaatttacc aagctgt 17

<210> 990
<211> 17
<212> DNA
<213> Homo sapiens

<400> 990
aaatttacca agctgtg 17

<210> 991
<211> 17
<212> DNA
<213> Homo sapiens

<400> 991
aatttaccaa gctgtgt 17

<210> 992
<211> 17
<212> DNA
<213> Homo sapiens

<400> 992
atttaccaag ctgtgtg 17

<210> 993
<211> 17
<212> DNA
<213> Homo sapiens

<400> 993
tttaccaagc tgtgtga 17

<210> 994
<211> 17
<212> DNA
<213> Homo sapiens

<400> 994
ttaccaagct gtgtgag 17

<210> 995
<211> 17
<212> DNA
<213> Homo sapiens

<400> 995
taccaagctg tgtgagt 17

<210> 996
<211> 17
<212> DNA
<213> Homo sapiens

<400> 996
accaagctgt gtgagtt 17

<210> 997
<211> 17
<212> DNA

<213> Homo sapiens

<400> 997
ccaagctgtg tgagttc                                                    17

<210> 998
<211> 17
<212> DNA
<213> Homo sapiens

<400> 998
caagctgtgt gagttcc                                                    17

<210> 999
<211> 17
<212> DNA
<213> Homo sapiens

<400> 999
aagctgtgtg agttccc                                                    17

<210> 1000
<211> 17
<212> DNA
<213> Homo sapiens

<400> 1000
agctgtgtga gttcccg                                                    17

<210> 1001
<211> 17
<212> DNA
<213> Homo sapiens

<400> 1001
gctgtgtgag ttcccga                                                    17

<210> 1002
<211> 17
<212> DNA
<213> Homo sapiens

<400> 1002
ctgtgtgagt tcccgat                                                    17

<210> 1003
<211> 17
<212> DNA
<213> Homo sapiens

<400> 1003
tgtgtgagtt cccgatg                                                    17

<210> 1004
<211> 17
<212> DNA
<213> Homo sapiens

<400> 1004
gtgtgagttc ccgatgc                                                    17

<210> 1005
<211> 17
<212> DNA
<213> Homo sapiens

<400> 1005
tgtgagttcc cgatgct                                                    17

<210> 1006
<211> 17
<212> DNA
<213> Homo sapiens

<400> 1006
gtgagttccc gatgctg                                                    17

<210> 1007
<211> 17
<212> DNA
<213> Homo sapiens

<400> 1007
tgagttcccg atgctgg                                                    17

<210> 1008
<211> 17
<212> DNA
<213> Homo sapiens

<400> 1008
gagttcccga tgctgga                                                    17

<210> 1009
<211> 17
<212> DNA
<213> Homo sapiens

<400> 1009
agttcccgat gctggaa                                                    17

<210> 1010
<211> 17
<212> DNA
<213> Homo sapiens

<400> 1010
gttcccgatg ctggaaa                                                    17

<210> 1011
<211> 17
<212> DNA
<213> Homo sapiens

<400> 1011
ttcccgatgc tggaaac                                                    17

<210> 1012
<211> 17
<212> DNA
<213> Homo sapiens

<400> 1012
tcccgatgct ggaaacc                                                    17

<210> 1013
<211> 17
<212> DNA
<213> Homo sapiens

<400> 1013
cccgatgctg gaaaccg                                                    17

<210> 1014
<211> 17
<212> DNA
<213> Homo sapiens

<400> 1014
ccgatgctgg aaaccgg                                                    17

<210> 1015
<211> 17
<212> DNA
<213> Homo sapiens

<400> 1015
cgatgctgga aaccggc                                                    17

<210> 1016
<211> 17
<212> DNA
<213> Homo sapiens

<400> 1016
gatgctggaa accggcc                                                    17

<210> 1017
<211> 17
<212> DNA
<213> Homo sapiens

<400> 1017
atgctggaaa ccggcct                                                    17

<210> 1018
<211> 17
<212> DNA
<213> Homo sapiens

<400> 1018
tgctggaaac cggcctg                                                    17

<210> 1019
<211> 17
<212> DNA
<213> Homo sapiens

<400> 1019
gctggaaacc ggcctgt                                                    17

<210> 1020
<211> 17

<212> DNA
<213> Homo sapiens

<400> 1020
ctggaaaccg gcctgtt                                                    17

<210> 1021
<211> 17
<212> DNA
<213> Homo sapiens

<400> 1021
tggaaaccgg cctgttt                                                    17

<210> 1022
<211> 17
<212> DNA
<213> Homo sapiens

<400> 1022
ggaaaccggc ctgtttt                                                    17

<210> 1023
<211> 17
<212> DNA
<213> Homo sapiens

<400> 1023
gaaaccggcc tgttttt                                                    17

<210> 1024
<211> 17
<212> DNA
<213> Homo sapiens

<400> 1024
aaaccggcct gtttttc                                                    17

<210> 1025
<211> 17
<212> DNA
<213> Homo sapiens

<400> 1025
aaccggcctg tttttcc                                                    17

<210> 1026
<211> 17
<212> DNA
<213> Homo sapiens

<400> 1026
accggcctgt ttttcct                                                    17

<210> 1027
<211> 17
<212> DNA
<213> Homo sapiens

<400> 1027
ccggcctgtt tttcctg                                                    17

<210> 1028
<211> 17
<212> DNA
<213> Homo sapiens

<400> 1028
cggcctgttt ttcctgc                                                    17

<210> 1029
<211> 17
<212> DNA
<213> Homo sapiens

<400> 1029
ggcctgtttt tcctgct                                                    17

<210> 1030
<211> 17
<212> DNA
<213> Homo sapiens

<400> 1030
gcctgttttt cctgctt                                                    17

<210> 1031
<211> 17
<212> DNA
<213> Homo sapiens

<400> 1031
cctgtttttc ctgcttt                                                    17

<210> 1032
<211> 17
<212> DNA
<213> Homo sapiens

<400> 1032
ctgtttttcc tgctttc                                                    17

<210> 1033
<211> 17
<212> DNA
<213> Homo sapiens

<400> 1033
tgtttttcct gctttct                                                    17

<210> 1034
<211> 17
<212> DNA
<213> Homo sapiens

<400> 1034
gtttttcctg ctttctt                                                    17

<210> 1035
<211> 17
<212> DNA
<213> Homo sapiens

<400> 1035
tttttcctgc tttcttg                                                    17

<210> 1036
<211> 17
<212> DNA
<213> Homo sapiens

<400> 1036
ttttcctgct ttcttgg                                                    17

<210> 1037
<211> 17
<212> DNA
<213> Homo sapiens

<400> 1037
tttcctgctt tcttgga                                                    17

<210> 1038
<211> 17
<212> DNA
<213> Homo sapiens

<400> 1038
ttcctgcttt cttggag                                                    17

<210> 1039
<211> 17
<212> DNA
<213> Homo sapiens

<400> 1039
tcctgctttc ttggagt                                                    17

<210> 1040
<211> 17
<212> DNA
<213> Homo sapiens

<400> 1040
cctgctttct tggagtg                                                    17

<210> 1041
<211> 17
<212> DNA
<213> Homo sapiens

<400> 1041
ctgctttctt ggagtgc                                                    17

<210> 1042
<211> 17
<212> DNA
<213> Homo sapiens

<400> 1042
tgctttcttg gagtgcc                                                    17

<210> 1043

<211> 17
<212> DNA
<213> Homo sapiens

<400> 1043
gctttcttgg agtgcct                                                    17

<210> 1044
<211> 17
<212> DNA
<213> Homo sapiens

<400> 1044
ctttcttgga gtgcctt                                                    17

<210> 1045
<211> 17
<212> DNA
<213> Homo sapiens

<400> 1045
tttcttggag tgccttc                                                    17

<210> 1046
<211> 17
<212> DNA
<213> Homo sapiens

<400> 1046
ttcttggagt gccttcc                                                    17

<210> 1047
<211> 17
<212> DNA
<213> Homo sapiens

<400> 1047
tcttggagtg ccttcct                                                    17

<210> 1048
<211> 17
<212> DNA
<213> Homo sapiens

<400> 1048
cttggagtgc cttcctg                                                    17

<210> 1049
<211> 17
<212> DNA
<213> Homo sapiens

<400> 1049
ttggagtgcc ttcctgt                                                    17

<210> 1050
<211> 17
<212> DNA
<213> Homo sapiens

<400> 1050

tggagtgcct tcctgtc                                                    17

<210> 1051
<211> 17
<212> DNA
<213> Homo sapiens

<400> 1051
ggagtgcctt cctgtct                                                    17

<210> 1052
<211> 17
<212> DNA
<213> Homo sapiens

<400> 1052
gagtgccttc ctgtctg                                                    17

<210> 1053
<211> 17
<212> DNA
<213> Homo sapiens

<400> 1053
agtgccttcc tgtctgc                                                    17

<210> 1054
<211> 17
<212> DNA
<213> Homo sapiens

<400> 1054
gtgccttcct gtctgcc                                                    17

<210> 1055
<211> 17
<212> DNA
<213> Homo sapiens

<400> 1055
tgccttcctg tctgccg                                                    17

<210> 1056
<211> 17
<212> DNA
<213> Homo sapiens

<400> 1056
gccttcctgt ctgccga                                                    17

<210> 1057
<211> 17
<212> DNA
<213> Homo sapiens

<400> 1057
ccttcctgtc tgccgag                                                    17

<210> 1058
<211> 17
<212> DNA

<213> Homo sapiens

<400> 1058
cttcctgtct gccgagg                                                    17

<210> 1059
<211> 17
<212> DNA
<213> Homo sapiens

<400> 1059
ttcctgtctg ccgaggc                                                    17

<210> 1060
<211> 17
<212> DNA
<213> Homo sapiens

<400> 1060
tcctgtctgc cgaggct                                                    17

<210> 1061
<211> 17
<212> DNA
<213> Homo sapiens

<400> 1061
cctgtctgcc gaggctg                                                    17

<210> 1062
<211> 17
<212> DNA
<213> Homo sapiens

<400> 1062
ctgtctgccg aggctgc                                                    17

<210> 1063
<211> 17
<212> DNA
<213> Homo sapiens

<400> 1063
tgtctgccga ggctgcc                                                    17

<210> 1064
<211> 17
<212> DNA
<213> Homo sapiens

<400> 1064
gtctgccgag gctgccg                                                    17

<210> 1065
<211> 17
<212> DNA
<213> Homo sapiens

<400> 1065
tctgccgagg ctgccgg                                                    17

<210> 1066
<211> 17
<212> DNA
<213> Homo sapiens

<400> 1066
ctgccgaggc tgccggc                                                    17

<210> 1067
<211> 17
<212> DNA
<213> Homo sapiens

<400> 1067
tgccgaggct gccggcc                                                    17

<210> 1068
<211> 17
<212> DNA
<213> Homo sapiens

<400> 1068
gccgaggctg ccggcct                                                    17

<210> 1069
<211> 17
<212> DNA
<213> Homo sapiens

<400> 1069
ccgaggctgc cggccta                                                    17

<210> 1070
<211> 17
<212> DNA
<213> Homo sapiens

<400> 1070
cgaggctgcc ggcctaa                                                    17

<210> 1071
<211> 17
<212> DNA
<213> Homo sapiens

<400> 1071
gaggctgccg gcctaac                                                    17

<210> 1072
<211> 17
<212> DNA
<213> Homo sapiens

<400> 1072
aggctgccgg cctaaca                                                    17

<210> 1073
<211> 17
<212> DNA
<213> Homo sapiens

<400> 1073
ggctgccggc ctaacag                                                    17

<210> 1074
<211> 17
<212> DNA
<213> Homo sapiens

<400> 1074
gctgccggcc taacagg                                                    17

<210> 1075
<211> 17
<212> DNA
<213> Homo sapiens

<400> 1075
ctgccggcct aacaggg                                                    17

<210> 1076
<211> 17
<212> DNA
<213> Homo sapiens

<400> 1076
tgccggccta acaggga                                                    17

<210> 1077
<211> 17
<212> DNA
<213> Homo sapiens

<400> 1077
gccggcctaa cagggat                                                    17

<210> 1078
<211> 17
<212> DNA
<213> Homo sapiens

<400> 1078
ccggcctaac agggata                                                    17

<210> 1079
<211> 17
<212> DNA
<213> Homo sapiens

<400> 1079
cggcctaaca gggatag                                                    17

<210> 1080
<211> 17
<212> DNA
<213> Homo sapiens

<400> 1080
ggcctaacag ggatagt                                                    17

<210> 1081
<211> 17

<212> DNA
<213> Homo sapiens

<400> 1081
gcctaacagg gatagtt                                                    17

<210> 1082
<211> 17
<212> DNA
<213> Homo sapiens

<400> 1082
cctaacaggg atagttg                                                    17

<210> 1083
<211> 17
<212> DNA
<213> Homo sapiens

<400> 1083
ctaacaggga tagttgc                                                    17

<210> 1084
<211> 17
<212> DNA
<213> Homo sapiens

<400> 1084
taacagggat agttgct                                                    17

<210> 1085
<211> 17
<212> DNA
<213> Homo sapiens

<400> 1085
aacagggata gttgctg                                                    17

<210> 1086
<211> 17
<212> DNA
<213> Homo sapiens

<400> 1086
acagggatag ttgctgt                                                    17

<210> 1087
<211> 17
<212> DNA
<213> Homo sapiens

<400> 1087
cagggatagt tgctgtt                                                    17

<210> 1088
<211> 17
<212> DNA
<213> Homo sapiens

<400> 1088
agggatagtt gctgttc                                                    17

<210> 1089
<211> 17
<212> DNA
<213> Homo sapiens

<400> 1089
gggatagttg ctgttct                                                      17

<210> 1090
<211> 17
<212> DNA
<213> Homo sapiens

<400> 1090
ggatagttgc tgttctc                                                      17

<210> 1091
<211> 17
<212> DNA
<213> Homo sapiens

<400> 1091
gatagttgct gttctct                                                      17

<210> 1092
<211> 17
<212> DNA
<213> Homo sapiens

<400> 1092
atagttgctg ttctctt                                                      17

<210> 1093
<211> 17
<212> DNA
<213> Homo sapiens

<400> 1093
tagttgctgt tctcttc                                                      17

<210> 1094
<211> 17
<212> DNA
<213> Homo sapiens

<400> 1094
agttgctgtt ctcttct                                                      17

<210> 1095
<211> 17
<212> DNA
<213> Homo sapiens

<400> 1095
gttgctgttc tcttctg                                                      17

<210> 1096
<211> 17
<212> DNA
<213> Homo sapiens

<400> 1096
ttgctgttct cttctgt                                                    17

<210> 1097
<211> 17
<212> DNA
<213> Homo sapiens

<400> 1097
tgctgttctc ttctgtg                                                    17

<210> 1098
<211> 17
<212> DNA
<213> Homo sapiens

<400> 1098
gctgttctct tctgtgg                                                    17

<210> 1099
<211> 17
<212> DNA
<213> Homo sapiens

<400> 1099
ctgttctctt ctgtgga                                                    17

<210> 1100
<211> 17
<212> DNA
<213> Homo sapiens

<400> 1100
tgttctcttc tgtggag                                                    17

<210> 1101
<211> 17
<212> DNA
<213> Homo sapiens

<400> 1101
gttctcttct gtggagt                                                    17

<210> 1102
<211> 17
<212> DNA
<213> Homo sapiens

<400> 1102
ttctcttctg tggagtc                                                    17

<210> 1103
<211> 17
<212> DNA
<213> Homo sapiens

<400> 1103
tctcttctgt ggagtca                                                    17

<210> 1104

<211> 17
<212> DNA
<213> Homo sapiens

<400> 1104
ctcttctgtg gagtcac                                                    17

<210> 1105
<211> 17
<212> DNA
<213> Homo sapiens

<400> 1105
tcttctgtgg agtcaca                                                    17

<210> 1106
<211> 17
<212> DNA
<213> Homo sapiens

<400> 1106
cttctgtgga gtcacac                                                    17

<210> 1107
<211> 17
<212> DNA
<213> Homo sapiens

<400> 1107
ttctgtggag tcacaca                                                    17

<210> 1108
<211> 17
<212> DNA
<213> Homo sapiens

<400> 1108
tctgtggagt cacacaa                                                    17

<210> 1109
<211> 17
<212> DNA
<213> Homo sapiens

<400> 1109
ctgtggagtc acacaag                                                    17

<210> 1110
<211> 17
<212> DNA
<213> Homo sapiens

<400> 1110
tgtggagtca cacaagc                                                    17

<210> 1111
<211> 17
<212> DNA
<213> Homo sapiens

<400> 1111

gtggagtcac acaagca                                                    17

<210> 1112
<211> 17
<212> DNA
<213> Homo sapiens

<400> 1112
tggagtcaca caagcac                                                    17

<210> 1113
<211> 17
<212> DNA
<213> Homo sapiens

<400> 1113
ggagtcacac aagcaca                                                    17

<210> 1114
<211> 17
<212> DNA
<213> Homo sapiens

<400> 1114
gagtcacaca agcacat                                                    17

<210> 1115
<211> 17
<212> DNA
<213> Homo sapiens

<400> 1115
agtcacacaa gcacatt                                                    17

<210> 1116
<211> 17
<212> DNA
<213> Homo sapiens

<400> 1116
gtcacacaag cacatta                                                    17

<210> 1117
<211> 17
<212> DNA
<213> Homo sapiens

<400> 1117
tcacacaagc acattat                                                    17

<210> 1118
<211> 17
<212> DNA
<213> Homo sapiens

<400> 1118
cacacaagca cattata                                                    17

<210> 1119
<211> 17
<212> DNA

<213> Homo sapiens

<400> 1119
acacaagcac attatac                                                    17

<210> 1120
<211> 17
<212> DNA
<213> Homo sapiens

<400> 1120
cacaagcaca ttatacc                                                    17

<210> 1121
<211> 17
<212> DNA
<213> Homo sapiens

<400> 1121
acaagcacat tatacct                                                    17

<210> 1122
<211> 17
<212> DNA
<213> Homo sapiens

<400> 1122
caagcacatt ataccta                                                    17

<210> 1123
<211> 17
<212> DNA
<213> Homo sapiens

<400> 1123
aagcacatta tacctac                                                    17

<210> 1124
<211> 17
<212> DNA
<213> Homo sapiens

<400> 1124
agcacattat acctaca                                                    17

<210> 1125
<211> 17
<212> DNA
<213> Homo sapiens

<400> 1125
gcacattata cctacaa                                                    17

<210> 1126
<211> 17
<212> DNA
<213> Homo sapiens

<400> 1126
cacattatac ctacaac                                                    17

<210> 1127
<211> 17
<212> DNA
<213> Homo sapiens

<400> 1127
acattatacc tacaaca                                                    17

<210> 1128
<211> 17
<212> DNA
<213> Homo sapiens

<400> 1128
cattatacct acaacaa                                                    17

<210> 1129
<211> 17
<212> DNA
<213> Homo sapiens

<400> 1129
attataccta caacaat                                                    17

<210> 1130
<211> 17
<212> DNA
<213> Homo sapiens

<400> 1130
ttatacctac aacaatc                                                    17

<210> 1131
<211> 17
<212> DNA
<213> Homo sapiens

<400> 1131
tatacctaca acaatct                                                    17

<210> 1132
<211> 17
<212> DNA
<213> Homo sapiens

<400> 1132
atacctacaa caatctg                                                    17

<210> 1133
<211> 17
<212> DNA
<213> Homo sapiens

<400> 1133
tacctacaac aatctgt                                                    17

<210> 1134
<211> 17
<212> DNA
<213> Homo sapiens

<400> 1134
acctacaaca atctgtc                                                    17

<210> 1135
<211> 17
<212> DNA
<213> Homo sapiens

<400> 1135
cctacaacaa tctgtct                                                    17

<210> 1136
<211> 17
<212> DNA
<213> Homo sapiens

<400> 1136
ctacaacaat ctgtctt                                                    17

<210> 1137
<211> 17
<212> DNA
<213> Homo sapiens

<400> 1137
tacaacaatc tgtcttc                                                    17

<210> 1138
<211> 17
<212> DNA
<213> Homo sapiens

<400> 1138
acaacaatct gtcttcg                                                    17

<210> 1139
<211> 17
<212> DNA
<213> Homo sapiens

<400> 1139
caacaatctg tcttcgg                                                    17

<210> 1140
<211> 17
<212> DNA
<213> Homo sapiens

<400> 1140
aacaatctgt cttcgga                                                    17

<210> 1141
<211> 17
<212> DNA
<213> Homo sapiens

<400> 1141
acaatctgtc ttcggat                                                    17

<210> 1142
<211> 17

<212> DNA
<213> Homo sapiens

<400> 1142
caatctgtct tcggatt                                                    17

<210> 1143
<211> 17
<212> DNA
<213> Homo sapiens

<400> 1143
aatctgtctt cggattc                                                    17

<210> 1144
<211> 17
<212> DNA
<213> Homo sapiens

<400> 1144
atctgtcttc ggattcc                                                    17

<210> 1145
<211> 17
<212> DNA
<213> Homo sapiens

<400> 1145
tctgtcttcg gattcca                                                    17

<210> 1146
<211> 17
<212> DNA
<213> Homo sapiens

<400> 1146
ctgtcttcgg attccaa                                                    17

<210> 1147
<211> 17
<212> DNA
<213> Homo sapiens

<400> 1147
tgtcttcgga ttccaaa                                                    17

<210> 1148
<211> 17
<212> DNA
<213> Homo sapiens

<400> 1148
gtcttcggat tccaaaa                                                    17

<210> 1149
<211> 17
<212> DNA
<213> Homo sapiens

<400> 1149
tcttcggatt ccaaaat                                                    17

<210> 1150
<211> 17
<212> DNA
<213> Homo sapiens

<400> 1150
cttcggattc caaaata                                                    17

<210> 1151
<211> 17
<212> DNA
<213> Homo sapiens

<400> 1151
ttcggattcc aaaataa                                                    17

<210> 1152
<211> 17
<212> DNA
<213> Homo sapiens

<400> 1152
tcggattcca aaataag                                                    17

<210> 1153
<211> 17
<212> DNA
<213> Homo sapiens

<400> 1153
cggattccaa aataaga                                                    17

<210> 1154
<211> 17
<212> DNA
<213> Homo sapiens

<400> 1154
ggattccaaa ataagaa                                                    17

<210> 1155
<211> 17
<212> DNA
<213> Homo sapiens

<400> 1155
gattccaaaa taagaac                                                    17

<210> 1156
<211> 17
<212> DNA
<213> Homo sapiens

<400> 1156
attccaaaat aagaact                                                    17

<210> 1157
<211> 17
<212> DNA
<213> Homo sapiens

<400> 1157
ttccaaaata agaacta                                                    17

<210> 1158
<211> 17
<212> DNA
<213> Homo sapiens

<400> 1158
tccaaaataa gaactaa                                                    17

<210> 1159
<211> 17
<212> DNA
<213> Homo sapiens

<400> 1159
ccaaaataag aactaaa                                                    17

<210> 1160
<211> 17
<212> DNA
<213> Homo sapiens

<400> 1160
caaaataaga actaaac                                                    17

<210> 1161
<211> 17
<212> DNA
<213> Homo sapiens

<400> 1161
aaaataagaa ctaaaca                                                    17

<210> 1162
<211> 17
<212> DNA
<213> Homo sapiens

<400> 1162
aaataagaac taaacag                                                    17

<210> 1163
<211> 17
<212> DNA
<213> Homo sapiens

<400> 1163
aataagaact aaacagt                                                    17

<210> 1164
<211> 17
<212> DNA
<213> Homo sapiens

<400> 1164
ataagaacta aacagtt                                                    17

<210> 1165

<211> 17
<212> DNA
<213> Homo sapiens

<400> 1165
taagaactaa acagttg                                                    17

<210> 1166
<211> 17
<212> DNA
<213> Homo sapiens

<400> 1166
aagaactaaa cagttgt                                                    17

<210> 1167
<211> 17
<212> DNA
<213> Homo sapiens

<400> 1167
agaactaaac agttgtt                                                    17

<210> 1168
<211> 17
<212> DNA
<213> Homo sapiens

<400> 1168
gaactaaaca gttgttt                                                    17

<210> 1169
<211> 17
<212> DNA
<213> Homo sapiens

<400> 1169
aactaaacag ttgtttg                                                    17

<210> 1170
<211> 17
<212> DNA
<213> Homo sapiens

<400> 1170
actaaacagt tgtttga                                                    17

<210> 1171
<211> 17
<212> DNA
<213> Homo sapiens

<400> 1171
ctaaacagtt gtttgaa                                                    17

<210> 1172
<211> 17
<212> DNA
<213> Homo sapiens

<400> 1172

taaacagttg tttgaat                                                    17

<210> 1173
<211> 17
<212> DNA
<213> Homo sapiens

<400> 1173
aaacagttgt ttgaatt                                                    17

<210> 1174
<211> 17
<212> DNA
<213> Homo sapiens

<400> 1174
aacagttgtt tgaattt                                                    17

<210> 1175
<211> 17
<212> DNA
<213> Homo sapiens

<400> 1175
acagttgttt gaattta                                                    17

<210> 1176
<211> 17
<212> DNA
<213> Homo sapiens

<400> 1176
cagttgtttg aatttat                                                    17

<210> 1177
<211> 17
<212> DNA
<213> Homo sapiens

<400> 1177
agttgtttga atttatg                                                    17

<210> 1178
<211> 17
<212> DNA
<213> Homo sapiens

<400> 1178
gttgtttgaa tttatga                                                    17

<210> 1179
<211> 17
<212> DNA
<213> Homo sapiens

<400> 1179
ttgtttgaat ttatgaa                                                    17

<210> 1180
<211> 17
<212> DNA

<213> Homo sapiens

<400> 1180
tgtttgaatt tatgaac                                                    17

<210> 1181
<211> 17
<212> DNA
<213> Homo sapiens

<400> 1181
gtttgaattt atgaact                                                    17

<210> 1182
<211> 17
<212> DNA
<213> Homo sapiens

<400> 1182
tttgaattta tgaactt                                                    17

<210> 1183
<211> 17
<212> DNA
<213> Homo sapiens

<400> 1183
ttgaatttat gaacttt                                                    17

<210> 1184
<211> 17
<212> DNA
<213> Homo sapiens

<400> 1184
tgaatttatg aactttt                                                    17

<210> 1185
<211> 17
<212> DNA
<213> Homo sapiens

<400> 1185
gaatttatga acttttt                                                    17

<210> 1186
<211> 17
<212> DNA
<213> Homo sapiens

<400> 1186
aatttatgaa ctttttg                                                    17

<210> 1187
<211> 17
<212> DNA
<213> Homo sapiens

<400> 1187
atttatgaac tttttgg                                                    17

<210> 1188
<211> 17
<212> DNA
<213> Homo sapiens

<400> 1188
tttatgaact ttttggc                                                    17

<210> 1189
<211> 17
<212> DNA
<213> Homo sapiens

<400> 1189
ttatgaactt tttggcg                                                    17

<210> 1190
<211> 17
<212> DNA
<213> Homo sapiens

<400> 1190
tatgaacttt ttggcgg                                                    17

<210> 1191
<211> 17
<212> DNA
<213> Homo sapiens

<400> 1191
atgaactttt tggcgga                                                    17

<210> 1192
<211> 17
<212> DNA
<213> Homo sapiens

<400> 1192
tgaacttttt ggcggag                                                    17

<210> 1193
<211> 17
<212> DNA
<213> Homo sapiens

<400> 1193
gaactttttg gcggaga                                                    17

<210> 1194
<211> 17
<212> DNA
<213> Homo sapiens

<400> 1194
aactttttgg cggagaa                                                    17

<210> 1195
<211> 17
<212> DNA
<213> Homo sapiens

<400> 1195
actttttggc ggagaac                                                    17

<210> 1196
<211> 17
<212> DNA
<213> Homo sapiens

<400> 1196
ctttttggcg gagaacg                                                    17

<210> 1197
<211> 17
<212> DNA
<213> Homo sapiens

<400> 1197
tttttggcgg agaacgt                                                    17

<210> 1198
<211> 17
<212> DNA
<213> Homo sapiens

<400> 1198
ttttggcgga gaacgtc                                                    17

<210> 1199
<211> 17
<212> DNA
<213> Homo sapiens

<400> 1199
tttggcggag aacgtca                                                    17

<210> 1200
<211> 17
<212> DNA
<213> Homo sapiens

<400> 1200
ttggcggaga acgtcat                                                    17

<210> 1201
<211> 17
<212> DNA
<213> Homo sapiens

<400> 1201
tggcggagaa cgtcatc                                                    17

<210> 1202
<211> 17
<212> DNA
<213> Homo sapiens

<400> 1202
ggcggagaac gtcatct                                                    17

<210> 1203
<211> 17

<212> DNA
<213> Homo sapiens

<400> 1203
gcggagaacg tcatctt                                                    17

<210> 1204
<211> 17
<212> DNA
<213> Homo sapiens

<400> 1204
cggagaacgt catcttc                                                    17

<210> 1205
<211> 17
<212> DNA
<213> Homo sapiens

<400> 1205
ggagaacgtc atcttct                                                    17

<210> 1206
<211> 17
<212> DNA
<213> Homo sapiens

<400> 1206
gagaacgtca tcttctg                                                    17

<210> 1207
<211> 17
<212> DNA
<213> Homo sapiens

<400> 1207
agaacgtcat cttctgt                                                    17

<210> 1208
<211> 17
<212> DNA
<213> Homo sapiens

<400> 1208
gaacgtcatc ttctgtt                                                    17

<210> 1209
<211> 17
<212> DNA
<213> Homo sapiens

<400> 1209
aacgtcatct tctgtta                                                    17

<210> 1210
<211> 17
<212> DNA
<213> Homo sapiens

<400> 1210
acgtcatctt ctgttac                                                    17

<210> 1211
<211> 17
<212> DNA
<213> Homo sapiens

<400> 1211
cgtcatcttc tgttaca                                                    17

<210> 1212
<211> 17
<212> DNA
<213> Homo sapiens

<400> 1212
gtcatcttct gttacat                                                    17

<210> 1213
<211> 17
<212> DNA
<213> Homo sapiens

<400> 1213
tcatcttctg ttacatg                                                    17

<210> 1214
<211> 17
<212> DNA
<213> Homo sapiens

<400> 1214
catcttctgt tacatgg                                                    17

<210> 1215
<211> 17
<212> DNA
<213> Homo sapiens

<400> 1215
atcttctgtt acatggg                                                    17

<210> 1216
<211> 17
<212> DNA
<213> Homo sapiens

<400> 1216
tcttctgtta catgggc                                                    17

<210> 1217
<211> 17
<212> DNA
<213> Homo sapiens

<400> 1217
cttctgttac atgggcc                                                    17

<210> 1218
<211> 17
<212> DNA
<213> Homo sapiens

<400> 1218
ttctgttaca tgggcct                                                    17

<210> 1219
<211> 17
<212> DNA
<213> Homo sapiens

<400> 1219
tctgttacat gggcctg                                                    17

<210> 1220
<211> 17
<212> DNA
<213> Homo sapiens

<400> 1220
ctgttacatg ggcctgg                                                    17

<210> 1221
<211> 17
<212> DNA
<213> Homo sapiens

<400> 1221
tgttacatgg gcctggc                                                    17

<210> 1222
<211> 17
<212> DNA
<213> Homo sapiens

<400> 1222
gttacatggg cctggca                                                    17

<210> 1223
<211> 17
<212> DNA
<213> Homo sapiens

<400> 1223
ttacatgggc ctggcac                                                    17

<210> 1224
<211> 17
<212> DNA
<213> Homo sapiens

<400> 1224
tacatgggcc tggcact                                                    17

<210> 1225
<211> 17
<212> DNA
<213> Homo sapiens

<400> 1225
acatgggcct ggcactg                                                    17

<210> 1226

<211> 17
<212> DNA
<213> Homo sapiens

<400> 1226
catgggcctg gcactgt                                                    17

<210> 1227
<211> 17
<212> DNA
<213> Homo sapiens

<400> 1227
atgggcctgg cactgtt                                                    17

<210> 1228
<211> 17
<212> DNA
<213> Homo sapiens

<400> 1228
tgggcctggc actgttc                                                    17

<210> 1229
<211> 17
<212> DNA
<213> Homo sapiens

<400> 1229
gggcctggca ctgttca                                                    17

<210> 1230
<211> 17
<212> DNA
<213> Homo sapiens

<400> 1230
ggcctggcac tgttcac                                                    17

<210> 1231
<211> 17
<212> DNA
<213> Homo sapiens

<400> 1231
gcctggcact gttcacg                                                    17

<210> 1232
<211> 17
<212> DNA
<213> Homo sapiens

<400> 1232
cctggcactg ttcacgt                                                    17

<210> 1233
<211> 17
<212> DNA
<213> Homo sapiens

<400> 1233

ctggcactgt tcacgtt 17

<210> 1234
<211> 17
<212> DNA
<213> Homo sapiens

<400> 1234
tggcactgtt cacgttc 17

<210> 1235
<211> 17
<212> DNA
<213> Homo sapiens

<400> 1235
ggcactgttc acgttcc 17

<210> 1236
<211> 17
<212> DNA
<213> Homo sapiens

<400> 1236
gcactgttca cgttcca 17

<210> 1237
<211> 17
<212> DNA
<213> Homo sapiens

<400> 1237
cactgttcac gttccag 17

<210> 1238
<211> 17
<212> DNA
<213> Homo sapiens

<400> 1238
actgttcacg ttccaga 17

<210> 1239
<211> 17
<212> DNA
<213> Homo sapiens

<400> 1239
ctgttcacgt tccagaa 17

<210> 1240
<211> 17
<212> DNA
<213> Homo sapiens

<400> 1240
tgttcacgtt ccagaat 17

<210> 1241
<211> 17
<212> DNA

<213> Homo sapiens

<400> 1241
gttcacgttc cagaatc                                                    17

<210> 1242
<211> 17
<212> DNA
<213> Homo sapiens

<400> 1242
ttcacgttcc agaatca                                                    17

<210> 1243
<211> 17
<212> DNA
<213> Homo sapiens

<400> 1243
tcacgttcca gaatcat                                                    17

<210> 1244
<211> 17
<212> DNA
<213> Homo sapiens

<400> 1244
cacgttccag aatcata                                                    17

<210> 1245
<211> 17
<212> DNA
<213> Homo sapiens

<400> 1245
acgttccaga atcatat                                                    17

<210> 1246
<211> 17
<212> DNA
<213> Homo sapiens

<400> 1246
cgttccagaa tcatatc                                                    17

<210> 1247
<211> 17
<212> DNA
<213> Homo sapiens

<400> 1247
gttccagaat catatct                                                    17

<210> 1248
<211> 17
<212> DNA
<213> Homo sapiens

<400> 1248
ttccagaatc atatctt                                                    17

<210> 1249
<211> 17
<212> DNA
<213> Homo sapiens

<400> 1249
tccagaatca tatcttt                                                    17

<210> 1250
<211> 17
<212> DNA
<213> Homo sapiens

<400> 1250
ccagaatcat atcttta                                                    17

<210> 1251
<211> 17
<212> DNA
<213> Homo sapiens

<400> 1251
cagaatcata tctttaa                                                    17

<210> 1252
<211> 17
<212> DNA
<213> Homo sapiens

<400> 1252
agaatcatat ctttaat                                                    17

<210> 1253
<211> 17
<212> DNA
<213> Homo sapiens

<400> 1253
gaatcatatc tttaatg                                                    17

<210> 1254
<211> 17
<212> DNA
<213> Homo sapiens

<400> 1254
aatcatatct ttaatgc                                                    17

<210> 1255
<211> 17
<212> DNA
<213> Homo sapiens

<400> 1255
atcatatctt taatgct                                                    17

<210> 1256
<211> 17
<212> DNA
<213> Homo sapiens

<400> 1256
tcatatcttt aatgctc                                                    17

<210> 1257
<211> 17
<212> DNA
<213> Homo sapiens

<400> 1257
catatcttta atgctct                                                    17

<210> 1258
<211> 17
<212> DNA
<213> Homo sapiens

<400> 1258
atatctttaa tgctctt                                                    17

<210> 1259
<211> 17
<212> DNA
<213> Homo sapiens

<400> 1259
tatctttaat gctcttt                                                    17

<210> 1260
<211> 17
<212> DNA
<213> Homo sapiens

<400> 1260
atctttaatg ctctttt                                                    17

<210> 1261
<211> 17
<212> DNA
<213> Homo sapiens

<400> 1261
tctttaatgc tcttttt                                                    17

<210> 1262
<211> 17
<212> DNA
<213> Homo sapiens

<400> 1262
ctttaatgct cttttta                                                    17

<210> 1263
<211> 17
<212> DNA
<213> Homo sapiens

<400> 1263
tttaatgctc tttttat                                                    17

<210> 1264
<211> 17

<212> DNA
<213> Homo sapiens

<400> 1264
ttaatgctct ttttata                                                    17

<210> 1265
<211> 17
<212> DNA
<213> Homo sapiens

<400> 1265
taatgctctt tttatac                                                    17

<210> 1266
<211> 17
<212> DNA
<213> Homo sapiens

<400> 1266
aatgctcttt ttatact                                                    17

<210> 1267
<211> 17
<212> DNA
<213> Homo sapiens

<400> 1267
atgctctttt tatactt                                                    17

<210> 1268
<211> 17
<212> DNA
<213> Homo sapiens

<400> 1268
tgctcttttt atacttg                                                    17

<210> 1269
<211> 17
<212> DNA
<213> Homo sapiens

<400> 1269
gctcttttta tacttgg                                                    17

<210> 1270
<211> 17
<212> DNA
<213> Homo sapiens

<400> 1270
ctctttttat acttgga                                                    17

<210> 1271
<211> 17
<212> DNA
<213> Homo sapiens

<400> 1271
tctttttata cttggag                                                    17

<210> 1272
<211> 17
<212> DNA
<213> Homo sapiens

<400> 1272
ctttttatac ttggagc                                                    17

<210> 1273
<211> 17
<212> DNA
<213> Homo sapiens

<400> 1273
tttttatact tggagcc                                                    17

<210> 1274
<211> 17
<212> DNA
<213> Homo sapiens

<400> 1274
ttttatactt ggagcct                                                    17

<210> 1275
<211> 17
<212> DNA
<213> Homo sapiens

<400> 1275
tttatacttg gagcctt                                                    17

<210> 1276
<211> 17
<212> DNA
<213> Homo sapiens

<400> 1276
ttatacttgg agccttt                                                    17

<210> 1277
<211> 17
<212> DNA
<213> Homo sapiens

<400> 1277
tatacttgga gcctttc                                                    17

<210> 1278
<211> 17
<212> DNA
<213> Homo sapiens

<400> 1278
atacttggag cctttct                                                    17

<210> 1279
<211> 17
<212> DNA
<213> Homo sapiens

<400> 1279
tacttggagc ctttcta                                                    17

<210> 1280
<211> 17
<212> DNA
<213> Homo sapiens

<400> 1280
acttggagcc tttctag                                                    17

<210> 1281
<211> 17
<212> DNA
<213> Homo sapiens

<400> 1281
cttggagcct ttctagc                                                    17

<210> 1282
<211> 17
<212> DNA
<213> Homo sapiens

<400> 1282
ttggagcctt tctagca                                                    17

<210> 1283
<211> 17
<212> DNA
<213> Homo sapiens

<400> 1283
tggagccttt ctagcaa                                                    17

<210> 1284
<211> 17
<212> DNA
<213> Homo sapiens

<400> 1284
ggagcctttc tagcaat                                                    17

<210> 1285
<211> 17
<212> DNA
<213> Homo sapiens

<400> 1285
gagcctttct agcaatt                                                    17

<210> 1286
<211> 17
<212> DNA
<213> Homo sapiens

<400> 1286
agcctttcta gcaattt                                                    17

<210> 1287

<211> 17
<212> DNA
<213> Homo sapiens

<400> 1287
gcctttctag caatttt                                                    17

<210> 1288
<211> 17
<212> DNA
<213> Homo sapiens

<400> 1288
cctttctagc aattttt                                                    17

<210> 1289
<211> 17
<212> DNA
<213> Homo sapiens

<400> 1289
ctttctagca atttttg                                                    17

<210> 1290
<211> 17
<212> DNA
<213> Homo sapiens

<400> 1290
tttctagcaa tttttgt                                                    17

<210> 1291
<211> 17
<212> DNA
<213> Homo sapiens

<400> 1291
ttctagcaat ttttgtt                                                    17

<210> 1292
<211> 17
<212> DNA
<213> Homo sapiens

<400> 1292
tctagcaatt tttgttg                                                    17

<210> 1293
<211> 17
<212> DNA
<213> Homo sapiens

<400> 1293
ctagcaattt ttgttgc                                                    17

<210> 1294
<211> 17
<212> DNA
<213> Homo sapiens

<400> 1294

tagcaatttt tgttgcc 17

<210> 1295
<211> 17
<212> DNA
<213> Homo sapiens

<400> 1295
agcaattttt gttgcca 17

<210> 1296
<211> 17
<212> DNA
<213> Homo sapiens

<400> 1296
gcaatttttg ttgccag 17

<210> 1297
<211> 17
<212> DNA
<213> Homo sapiens

<400> 1297
caatttttgt tgccaga 17

<210> 1298
<211> 17
<212> DNA
<213> Homo sapiens

<400> 1298
aatttttgtt gccagag 17

<210> 1299
<211> 17
<212> DNA
<213> Homo sapiens

<400> 1299
atttttgttg ccagagc 17

<210> 1300
<211> 17
<212> DNA
<213> Homo sapiens

<400> 1300
tttttgttgc cagagcc 17

<210> 1301
<211> 17
<212> DNA
<213> Homo sapiens

<400> 1301
ttttgttgcc agagcct 17

<210> 1302
<211> 17
<212> DNA

<213> Homo sapiens

<400> 1302
tttgttgcca gagcctg                                                    17

<210> 1303
<211> 17
<212> DNA
<213> Homo sapiens

<400> 1303
ttgttgccag agcctgc                                                    17

<210> 1304
<211> 17
<212> DNA
<213> Homo sapiens

<400> 1304
tgttgccaga gcctgca                                                    17

<210> 1305
<211> 17
<212> DNA
<213> Homo sapiens

<400> 1305
gttgccagag cctgcaa                                                    17

<210> 1306
<211> 17
<212> DNA
<213> Homo sapiens

<400> 1306
ttgccagagc ctgcaac                                                    17

<210> 1307
<211> 17
<212> DNA
<213> Homo sapiens

<400> 1307
tgccagagcc tgcaaca                                                    17

<210> 1308
<211> 17
<212> DNA
<213> Homo sapiens

<400> 1308
gccagagcct gcaacat                                                    17

<210> 1309
<211> 17
<212> DNA
<213> Homo sapiens

<400> 1309
ccagagcctg caacata                                                    17

<210> 1310
<211> 17
<212> DNA
<213> Homo sapiens

<400> 1310
cagagcctgc aacatat                                                    17

<210> 1311
<211> 17
<212> DNA
<213> Homo sapiens

<400> 1311
agagcctgca acatata                                                    17

<210> 1312
<211> 17
<212> DNA
<213> Homo sapiens

<400> 1312
gagcctgcaa catatat                                                    17

<210> 1313
<211> 17
<212> DNA
<213> Homo sapiens

<400> 1313
agcctgcaac atatatc                                                    17

<210> 1314
<211> 17
<212> DNA
<213> Homo sapiens

<400> 1314
gcctgcaaca tatatcc                                                    17

<210> 1315
<211> 17
<212> DNA
<213> Homo sapiens

<400> 1315
cctgcaacat atatccc                                                    17

<210> 1316
<211> 17
<212> DNA
<213> Homo sapiens

<400> 1316
ctgcaacata tatcccc                                                    17

<210> 1317
<211> 17
<212> DNA
<213> Homo sapiens

<400> 1317
tgcaacatat atcccct                                                    17

<210> 1318
<211> 17
<212> DNA
<213> Homo sapiens

<400> 1318
gcaacatata tcccctc                                                    17

<210> 1319
<211> 17
<212> DNA
<213> Homo sapiens

<400> 1319
caacatatat cccctct                                                    17

<210> 1320
<211> 17
<212> DNA
<213> Homo sapiens

<400> 1320
aacatatatc ccctctc                                                    17

<210> 1321
<211> 17
<212> DNA
<213> Homo sapiens

<400> 1321
acatatatcc cctctcc                                                    17

<210> 1322
<211> 17
<212> DNA
<213> Homo sapiens

<400> 1322
catatatccc ctctcct                                                    17

<210> 1323
<211> 17
<212> DNA
<213> Homo sapiens

<400> 1323
atatatcccc tctcctt                                                    17

<210> 1324
<211> 17
<212> DNA
<213> Homo sapiens

<400> 1324
tatatcccct ctccttc                                                    17

<210> 1325
<211> 17

<212> DNA
<213> Homo sapiens

<400> 1325
atatcccctc tccttcc                                                    17

<210> 1326
<211> 17
<212> DNA
<213> Homo sapiens

<400> 1326
tatcccctct ccttcct                                                    17

<210> 1327
<211> 17
<212> DNA
<213> Homo sapiens

<400> 1327
atcccctctc cttcctc                                                    17

<210> 1328
<211> 17
<212> DNA
<213> Homo sapiens

<400> 1328
tcccctctcc ttcctcc                                                    17

<210> 1329
<211> 17
<212> DNA
<213> Homo sapiens

<400> 1329
cccctctcct tcctcct                                                    17

<210> 1330
<211> 17
<212> DNA
<213> Homo sapiens

<400> 1330
ccctctcctt cctcctg                                                    17

<210> 1331
<211> 17
<212> DNA
<213> Homo sapiens

<400> 1331
cctctccttc ctcctga                                                    17

<210> 1332
<211> 17
<212> DNA
<213> Homo sapiens

<400> 1332
ctctccttcc tcctgaa                                                    17

<210> 1333
<211> 17
<212> DNA
<213> Homo sapiens

<400> 1333
tctccttcct cctgaat                                                    17

<210> 1334
<211> 17
<212> DNA
<213> Homo sapiens

<400> 1334
ctccttcctc ctgaatc                                                    17

<210> 1335
<211> 17
<212> DNA
<213> Homo sapiens

<400> 1335
tccttcctcc tgaatct                                                    17

<210> 1336
<211> 17
<212> DNA
<213> Homo sapiens

<400> 1336
ccttcctcct gaatcta                                                    17

<210> 1337
<211> 17
<212> DNA
<213> Homo sapiens

<400> 1337
cttcctcctg aatctag                                                    17

<210> 1338
<211> 17
<212> DNA
<213> Homo sapiens

<400> 1338
ttcctcctga atctagg                                                    17

<210> 1339
<211> 17
<212> DNA
<213> Homo sapiens

<400> 1339
tcctcctgaa tctaggc                                                    17

<210> 1340
<211> 17
<212> DNA
<213> Homo sapiens

<400> 1340
cctcctgaat ctaggcc                                                    17

<210> 1341
<211> 17
<212> DNA
<213> Homo sapiens

<400> 1341
ctcctgaatc taggccg                                                    17

<210> 1342
<211> 17
<212> DNA
<213> Homo sapiens

<400> 1342
tcctgaatct aggccga                                                    17

<210> 1343
<211> 17
<212> DNA
<213> Homo sapiens

<400> 1343
cctgaatcta ggccgaa                                                    17

<210> 1344
<211> 17
<212> DNA
<213> Homo sapiens

<400> 1344
ctgaatctag gccgaaa                                                    17

<210> 1345
<211> 17
<212> DNA
<213> Homo sapiens

<400> 1345
tgaatctagg ccgaaaa                                                    17

<210> 1346
<211> 17
<212> DNA
<213> Homo sapiens

<400> 1346
gaatctaggc cgaaaac                                                    17

<210> 1347
<211> 17
<212> DNA
<213> Homo sapiens

<400> 1347
aatctaggcc gaaaaca                                                    17

<210> 1348

<211> 17
<212> DNA
<213> Homo sapiens

<400> 1348
atctaggccg aaaacag                                                    17

<210> 1349
<211> 17
<212> DNA
<213> Homo sapiens

<400> 1349
tctaggccga aaacaga                                                    17

<210> 1350
<211> 17
<212> DNA
<213> Homo sapiens

<400> 1350
ctaggccgaa aacagaa                                                    17

<210> 1351
<211> 17
<212> DNA
<213> Homo sapiens

<400> 1351
taggccgaaa acagaag                                                    17

<210> 1352
<211> 17
<212> DNA
<213> Homo sapiens

<400> 1352
aggccgaaaa cagaaga                                                    17

<210> 1353
<211> 17
<212> DNA
<213> Homo sapiens

<400> 1353
ggccgaaaac agaagat                                                    17

<210> 1354
<211> 17
<212> DNA
<213> Homo sapiens

<400> 1354
gccgaaaaca gaagatc                                                    17

<210> 1355
<211> 17
<212> DNA
<213> Homo sapiens

<400> 1355

ccgaaaacag aagatcc                                                    17

<210> 1356
<211> 17
<212> DNA
<213> Homo sapiens

<400> 1356
cgaaaacaga agatccc                                                    17

<210> 1357
<211> 17
<212> DNA
<213> Homo sapiens

<400> 1357
gaaaacagaa gatcccc                                                    17

<210> 1358
<211> 17
<212> DNA
<213> Homo sapiens

<400> 1358
aaaacagaag atcccct                                                    17

<210> 1359
<211> 17
<212> DNA
<213> Homo sapiens

<400> 1359
aaacagaaga tcccctg                                                    17

<210> 1360
<211> 17
<212> DNA
<213> Homo sapiens

<400> 1360
aacagaagat cccctgg                                                    17

<210> 1361
<211> 17
<212> DNA
<213> Homo sapiens

<400> 1361
acagaagatc ccctgga                                                    17

<210> 1362
<211> 17
<212> DNA
<213> Homo sapiens

<400> 1362
cagaagatcc cctggaa                                                    17

<210> 1363
<211> 17
<212> DNA

<213> Homo sapiens

<400> 1363
agaagatccc ctggaac                                                    17

<210> 1364
<211> 17
<212> DNA
<213> Homo sapiens

<400> 1364
gaagatcccc tggaact                                                    17

<210> 1365
<211> 17
<212> DNA
<213> Homo sapiens

<400> 1365
aagatcccct ggaactt                                                    17

<210> 1366
<211> 17
<212> DNA
<213> Homo sapiens

<400> 1366
agatcccctg gaacttt                                                    17

<210> 1367
<211> 17
<212> DNA
<213> Homo sapiens

<400> 1367
gatcccctgg aactttc                                                    17

<210> 1368
<211> 17
<212> DNA
<213> Homo sapiens

<400> 1368
atcccctgga actttca                                                    17

<210> 1369
<211> 17
<212> DNA
<213> Homo sapiens

<400> 1369
tcccctggaa ctttcag                                                    17

<210> 1370
<211> 17
<212> DNA
<213> Homo sapiens

<400> 1370
cccctggaac tttcagc                                                    17

<210> 1371
<211> 17
<212> DNA
<213> Homo sapiens

<400> 1371
ccctggaact ttcagca                                                    17

<210> 1372
<211> 17
<212> DNA
<213> Homo sapiens

<400> 1372
cctggaactt tcagcac                                                    17

<210> 1373
<211> 17
<212> DNA
<213> Homo sapiens

<400> 1373
ctggaacttt cagcaca                                                    17

<210> 1374
<211> 17
<212> DNA
<213> Homo sapiens

<400> 1374
tggaactttc agcacat                                                    17

<210> 1375
<211> 17
<212> DNA
<213> Homo sapiens

<400> 1375
ggaactttca gcacatg                                                    17

<210> 1376
<211> 17
<212> DNA
<213> Homo sapiens

<400> 1376
gaactttcag cacatga                                                    17

<210> 1377
<211> 17
<212> DNA
<213> Homo sapiens

<400> 1377
aactttcagc acatgat                                                    17

<210> 1378
<211> 17
<212> DNA
<213> Homo sapiens

<400> 1378
actttcagca catgatg                                                    17

<210> 1379
<211> 17
<212> DNA
<213> Homo sapiens

<400> 1379
ctttcagcac atgatga                                                    17

<210> 1380
<211> 17
<212> DNA
<213> Homo sapiens

<400> 1380
tttcagcaca tgatgat                                                    17

<210> 1381
<211> 17
<212> DNA
<213> Homo sapiens

<400> 1381
ttcagcacat gatgatg                                                    17

<210> 1382
<211> 17
<212> DNA
<213> Homo sapiens

<400> 1382
tcagcacatg atgatgt                                                    17

<210> 1383
<211> 17
<212> DNA
<213> Homo sapiens

<400> 1383
cagcacatga tgatgtt                                                    17

<210> 1384
<211> 17
<212> DNA
<213> Homo sapiens

<400> 1384
agcacatgat gatgttt                                                    17

<210> 1385
<211> 17
<212> DNA
<213> Homo sapiens

<400> 1385
gcacatgatg atgtttt                                                    17

<210> 1386
<211> 17

<212> DNA
<213> Homo sapiens

<400> 1386
cacatgatga tgttttc                                                    17

<210> 1387
<211> 17
<212> DNA
<213> Homo sapiens

<400> 1387
acatgatgat gttttca                                                    17

<210> 1388
<211> 17
<212> DNA
<213> Homo sapiens

<400> 1388
catgatgatg ttttcag                                                    17

<210> 1389
<211> 17
<212> DNA
<213> Homo sapiens

<400> 1389
atgatgatgt tttcagg                                                    17

<210> 1390
<211> 17
<212> DNA
<213> Homo sapiens

<400> 1390
tgatgatgtt ttcaggt                                                    17

<210> 1391
<211> 17
<212> DNA
<213> Homo sapiens

<400> 1391
gatgatgttt tcaggtt                                                    17

<210> 1392
<211> 17
<212> DNA
<213> Homo sapiens

<400> 1392
atgatgtttt caggttt                                                    17

<210> 1393
<211> 17
<212> DNA
<213> Homo sapiens

<400> 1393
tgatgttttc aggtttg                                                    17

<210> 1394
<211> 17
<212> DNA
<213> Homo sapiens

<400> 1394
gatgttttca ggtttgc                                                    17

<210> 1395
<211> 17
<212> DNA
<213> Homo sapiens

<400> 1395
atgttttcag gtttgcg                                                    17

<210> 1396
<211> 17
<212> DNA
<213> Homo sapiens

<400> 1396
tgttttcagg tttgcga                                                    17

<210> 1397
<211> 17
<212> DNA
<213> Homo sapiens

<400> 1397
gttttcaggt ttgcgag                                                    17

<210> 1398
<211> 17
<212> DNA
<213> Homo sapiens

<400> 1398
ttttcaggtt tgcgagg                                                    17

<210> 1399
<211> 17
<212> DNA
<213> Homo sapiens

<400> 1399
tttcaggttt gcgagga                                                    17

<210> 1400
<211> 17
<212> DNA
<213> Homo sapiens

<400> 1400
ttcaggtttg cgaggag                                                    17

<210> 1401
<211> 17
<212> DNA
<213> Homo sapiens

<400> 1401
tcaggtttgc gaggagc                                                    17

<210> 1402
<211> 17
<212> DNA
<213> Homo sapiens

<400> 1402
caggtttgcg aggagcg                                                    17

<210> 1403
<211> 17
<212> DNA
<213> Homo sapiens

<400> 1403
aggtttgcga ggagcga                                                    17

<210> 1404
<211> 17
<212> DNA
<213> Homo sapiens

<400> 1404
ggtttgcgag gagcgat                                                    17

<210> 1405
<211> 17
<212> DNA
<213> Homo sapiens

<400> 1405
gtttgcgagg agcgatc                                                    17

<210> 1406
<211> 17
<212> DNA
<213> Homo sapiens

<400> 1406
tttgcgagga gcgatcg                                                    17

<210> 1407
<211> 17
<212> DNA
<213> Homo sapiens

<400> 1407
ttgcgaggag cgatcgc                                                    17

<210> 1408
<211> 17
<212> DNA
<213> Homo sapiens

<400> 1408
tgcgaggagc gatcgca                                                    17

<210> 1409

<211> 17
<212> DNA
<213> Homo sapiens

<400> 1409
gcgaggagcg atcgcat                                                    17

<210> 1410
<211> 17
<212> DNA
<213> Homo sapiens

<400> 1410
cgaggagcga tcgcatt                                                    17

<210> 1411
<211> 17
<212> DNA
<213> Homo sapiens

<400> 1411
gaggagcgat cgcattt                                                    17

<210> 1412
<211> 17
<212> DNA
<213> Homo sapiens

<400> 1412
aggagcgatc gcatttg                                                    17

<210> 1413
<211> 17
<212> DNA
<213> Homo sapiens

<400> 1413
ggagcgatcg catttgc                                                    17

<210> 1414
<211> 17
<212> DNA
<213> Homo sapiens

<400> 1414
gagcgatcgc atttgcc                                                    17

<210> 1415
<211> 17
<212> DNA
<213> Homo sapiens

<400> 1415
agcgatcgca tttgcct                                                    17

<210> 1416
<211> 17
<212> DNA
<213> Homo sapiens

<400> 1416

gcgatcgcat ttgcctt                                                    17

<210> 1417
<211> 17
<212> DNA
<213> Homo sapiens

<400> 1417
cgatcgcatt tgcctta                                                    17

<210> 1418
<211> 17
<212> DNA
<213> Homo sapiens

<400> 1418
gatcgcattt gccttag                                                    17

<210> 1419
<211> 17
<212> DNA
<213> Homo sapiens

<400> 1419
atcgcatttg ccttagc                                                    17

<210> 1420
<211> 17
<212> DNA
<213> Homo sapiens

<400> 1420
tcgcatttgc cttagct                                                    17

<210> 1421
<211> 17
<212> DNA
<213> Homo sapiens

<400> 1421
cgcatttgcc ttagcta                                                    17

<210> 1422
<211> 17
<212> DNA
<213> Homo sapiens

<400> 1422
gcatttgcct tagctat                                                    17

<210> 1423
<211> 17
<212> DNA
<213> Homo sapiens

<400> 1423
catttgcctt agctatt                                                    17

<210> 1424
<211> 17
<212> DNA

<213> Homo sapiens

<400> 1424
atttgcctta gctattc                                                    17

<210> 1425
<211> 17
<212> DNA
<213> Homo sapiens

<400> 1425
tttgccttag ctattcg                                                    17

<210> 1426
<211> 17
<212> DNA
<213> Homo sapiens

<400> 1426
ttgccttagc tattcgg                                                    17

<210> 1427
<211> 17
<212> DNA
<213> Homo sapiens

<400> 1427
tgccttagct attcgga                                                    17

<210> 1428
<211> 17
<212> DNA
<213> Homo sapiens

<400> 1428
gccttagcta ttcggaa                                                    17

<210> 1429
<211> 17
<212> DNA
<213> Homo sapiens

<400> 1429
ccttagctat tcggaac                                                    17

<210> 1430
<211> 17
<212> DNA
<213> Homo sapiens

<400> 1430
cttagctatt cggaaca                                                    17

<210> 1431
<211> 17
<212> DNA
<213> Homo sapiens

<400> 1431
ttagctattc ggaacac                                                    17

<210> 1432
<211> 17
<212> DNA
<213> Homo sapiens

<400> 1432
tagctattcg gaacaca                                                    17

<210> 1433
<211> 17
<212> DNA
<213> Homo sapiens

<400> 1433
agctattcgg aacacag                                                    17

<210> 1434
<211> 17
<212> DNA
<213> Homo sapiens

<400> 1434
gctattcgga acacaga                                                    17

<210> 1435
<211> 17
<212> DNA
<213> Homo sapiens

<400> 1435
ctattcggaa cacagaa                                                    17

<210> 1436
<211> 17
<212> DNA
<213> Homo sapiens

<400> 1436
tattcggaac acagaat                                                    17

<210> 1437
<211> 17
<212> DNA
<213> Homo sapiens

<400> 1437
attcggaaca cagaatc                                                    17

<210> 1438
<211> 17
<212> DNA
<213> Homo sapiens

<400> 1438
ttcggaacac agaatct                                                    17

<210> 1439
<211> 17
<212> DNA
<213> Homo sapiens

<400> 1439
tcggaacaca gaatctc                                                    17

<210> 1440
<211> 17
<212> DNA
<213> Homo sapiens

<400> 1440
cggaacacag aatctca                                                    17

<210> 1441
<211> 17
<212> DNA
<213> Homo sapiens

<400> 1441
ggaacacaga atctcag                                                    17

<210> 1442
<211> 17
<212> DNA
<213> Homo sapiens

<400> 1442
gaacacagaa tctcagc                                                    17

<210> 1443
<211> 17
<212> DNA
<213> Homo sapiens

<400> 1443
aacacagaat ctcagcc                                                    17

<210> 1444
<211> 17
<212> DNA
<213> Homo sapiens

<400> 1444
acacagaatc tcagccc                                                    17

<210> 1445
<211> 17
<212> DNA
<213> Homo sapiens

<400> 1445
cacagaatct cagccca                                                    17

<210> 1446
<211> 17
<212> DNA
<213> Homo sapiens

<400> 1446
acagaatctc agcccaa                                                    17

<210> 1447
<211> 17

<212> DNA
<213> Homo sapiens

<400> 1447
cagaatctca gcccaaa                                                    17

<210> 1448
<211> 17
<212> DNA
<213> Homo sapiens

<400> 1448
agaatctcag cccaaac                                                    17

<210> 1449
<211> 17
<212> DNA
<213> Homo sapiens

<400> 1449
gaatctcagc ccaaaca                                                    17

<210> 1450
<211> 17
<212> DNA
<213> Homo sapiens

<400> 1450
aatctcagcc caaacaa                                                    17

<210> 1451
<211> 17
<212> DNA
<213> Homo sapiens

<400> 1451
atctcagccc aaacaaa                                                    17

<210> 1452
<211> 17
<212> DNA
<213> Homo sapiens

<400> 1452
tctcagccca aacaaat                                                    17

<210> 1453
<211> 17
<212> DNA
<213> Homo sapiens

<400> 1453
ctcagcccaa acaaatg                                                    17

<210> 1454
<211> 17
<212> DNA
<213> Homo sapiens

<400> 1454
tcagcccaaa caaatga                                                    17

<210> 1455
<211> 17
<212> DNA
<213> Homo sapiens

<400> 1455
cagcccaaac aaatgat                                                    17

<210> 1456
<211> 17
<212> DNA
<213> Homo sapiens

<400> 1456
agcccaaaca aatgatg                                                    17

<210> 1457
<211> 17
<212> DNA
<213> Homo sapiens

<400> 1457
gcccaaacaa atgatgt                                                    17

<210> 1458
<211> 17
<212> DNA
<213> Homo sapiens

<400> 1458
cccaaacaaa tgatgtt                                                    17

<210> 1459
<211> 17
<212> DNA
<213> Homo sapiens

<400> 1459
ccaaacaaat gatgttt                                                    17

<210> 1460
<211> 17
<212> DNA
<213> Homo sapiens

<400> 1460
caaacaaatg atgttta                                                    17

<210> 1461
<211> 17
<212> DNA
<213> Homo sapiens

<400> 1461
aaacaaatga tgtttac                                                    17

<210> 1462
<211> 17
<212> DNA
<213> Homo sapiens

<400> 1462
aacaaatgat gtttacc                                                    17

<210> 1463
<211> 17
<212> DNA
<213> Homo sapiens

<400> 1463
acaaatgatg tttacca                                                    17

<210> 1464
<211> 17
<212> DNA
<213> Homo sapiens

<400> 1464
caaatgatgt ttaccac                                                    17

<210> 1465
<211> 17
<212> DNA
<213> Homo sapiens

<400> 1465
aaatgatgtt taccact                                                    17

<210> 1466
<211> 17
<212> DNA
<213> Homo sapiens

<400> 1466
aatgatgttt accacta                                                    17

<210> 1467
<211> 17
<212> DNA
<213> Homo sapiens

<400> 1467
atgatgttta ccactac                                                    17

<210> 1468
<211> 17
<212> DNA
<213> Homo sapiens

<400> 1468
tgatgtttac cactacg                                                    17

<210> 1469
<211> 17
<212> DNA
<213> Homo sapiens

<400> 1469
gatgtttacc actacgc                                                    17

<210> 1470

<211> 17
<212> DNA
<213> Homo sapiens

<400> 1470
atgtttacca ctacgct                                                    17

<210> 1471
<211> 17
<212> DNA
<213> Homo sapiens

<400> 1471
tgtttaccac tacgctg                                                    17

<210> 1472
<211> 17
<212> DNA
<213> Homo sapiens

<400> 1472
gtttaccact acgctgc                                                    17

<210> 1473
<211> 17
<212> DNA
<213> Homo sapiens

<400> 1473
tttaccacta cgctgct                                                    17

<210> 1474
<211> 17
<212> DNA
<213> Homo sapiens

<400> 1474
ttaccactac gctgctc                                                    17

<210> 1475
<211> 17
<212> DNA
<213> Homo sapiens

<400> 1475
taccactacg ctgctcc                                                    17

<210> 1476
<211> 17
<212> DNA
<213> Homo sapiens

<400> 1476
accactacgc tgctcct                                                    17

<210> 1477
<211> 17
<212> DNA
<213> Homo sapiens

<400> 1477

ccactacgct gctcctc                                                    17

<210> 1478
<211> 17
<212> DNA
<213> Homo sapiens

<400> 1478
cactacgctg ctcctcg                                                    17

<210> 1479
<211> 17
<212> DNA
<213> Homo sapiens

<400> 1479
actacgctgc tcctcgt                                                    17

<210> 1480
<211> 17
<212> DNA
<213> Homo sapiens

<400> 1480
ctacgctgct cctcgtg                                                    17

<210> 1481
<211> 17
<212> DNA
<213> Homo sapiens

<400> 1481
tacgctgctc ctcgtgt                                                    17

<210> 1482
<211> 17
<212> DNA
<213> Homo sapiens

<400> 1482
acgctgctcc tcgtgtt                                                    17

<210> 1483
<211> 17
<212> DNA
<213> Homo sapiens

<400> 1483
cgctgctcct cgtgttc                                                    17

<210> 1484
<211> 17
<212> DNA
<213> Homo sapiens

<400> 1484
gctgctcctc gtgttct                                                    17

<210> 1485
<211> 17
<212> DNA

<213> Homo sapiens

<400> 1485
ctgctcctcg tgttctt                                                    17

<210> 1486
<211> 17
<212> DNA
<213> Homo sapiens

<400> 1486
tgctcctcgt gttcttc                                                    17

<210> 1487
<211> 17
<212> DNA
<213> Homo sapiens

<400> 1487
gctcctcgtg ttcttca                                                    17

<210> 1488
<211> 17
<212> DNA
<213> Homo sapiens

<400> 1488
ctcctcgtgt tcttcac                                                    17

<210> 1489
<211> 17
<212> DNA
<213> Homo sapiens

<400> 1489
tcctcgtgtt cttcact                                                    17

<210> 1490
<211> 17
<212> DNA
<213> Homo sapiens

<400> 1490
cctcgtgttc ttcactg                                                    17

<210> 1491
<211> 17
<212> DNA
<213> Homo sapiens

<400> 1491
ctcgtgttct tcactgt                                                    17

<210> 1492
<211> 17
<212> DNA
<213> Homo sapiens

<400> 1492
tcgtgttctt cactgtc                                                    17

<210> 1493
<211> 17
<212> DNA
<213> Homo sapiens

<400> 1493
cgtgttcttc actgtct                                                    17

<210> 1494
<211> 17
<212> DNA
<213> Homo sapiens

<400> 1494
gtgttcttca ctgtctg                                                    17

<210> 1495
<211> 17
<212> DNA
<213> Homo sapiens

<400> 1495
tgttcttcac tgtctgg                                                    17

<210> 1496
<211> 17
<212> DNA
<213> Homo sapiens

<400> 1496
gttcttcact gtctggg                                                    17

<210> 1497
<211> 17
<212> DNA
<213> Homo sapiens

<400> 1497
ttcttcactg tctgggt                                                    17

<210> 1498
<211> 17
<212> DNA
<213> Homo sapiens

<400> 1498
tcttcactgt ctgggta                                                    17

<210> 1499
<211> 17
<212> DNA
<213> Homo sapiens

<400> 1499
cttcactgtc tgggtat                                                    17

<210> 1500
<211> 17
<212> DNA
<213> Homo sapiens

<400> 1500
ttcactgtct gggtatt                                                    17

<210> 1501
<211> 17
<212> DNA
<213> Homo sapiens

<400> 1501
tcactgtctg ggtattt                                                    17

<210> 1502
<211> 17
<212> DNA
<213> Homo sapiens

<400> 1502
cactgtctgg gtatttg                                                    17

<210> 1503
<211> 17
<212> DNA
<213> Homo sapiens

<400> 1503
actgtctggg tatttgg                                                    17

<210> 1504
<211> 17
<212> DNA
<213> Homo sapiens

<400> 1504
ctgtctgggt atttgga                                                    17

<210> 1505
<211> 17
<212> DNA
<213> Homo sapiens

<400> 1505
tgtctgggta tttggag                                                    17

<210> 1506
<211> 17
<212> DNA
<213> Homo sapiens

<400> 1506
gtctgggtat ttggagg                                                    17

<210> 1507
<211> 17
<212> DNA
<213> Homo sapiens

<400> 1507
tctgggtatt tggagga                                                    17

<210> 1508
<211> 17

<212> DNA
<213> Homo sapiens

<400> 1508
ctgggtattt ggaggag                                                    17

<210> 1509
<211> 17
<212> DNA
<213> Homo sapiens

<400> 1509
tgggtatttg gaggagg                                                    17

<210> 1510
<211> 17
<212> DNA
<213> Homo sapiens

<400> 1510
gggtatttgg aggagga                                                    17

<210> 1511
<211> 17
<212> DNA
<213> Homo sapiens

<400> 1511
ggtatttgga ggaggaa                                                    17

<210> 1512
<211> 17
<212> DNA
<213> Homo sapiens

<400> 1512
gtatttggag gaggaac                                                    17

<210> 1513
<211> 17
<212> DNA
<213> Homo sapiens

<400> 1513
tatttggagg aggaaca                                                    17

<210> 1514
<211> 17
<212> DNA
<213> Homo sapiens

<400> 1514
atttggagga ggaacaa                                                    17

<210> 1515
<211> 17
<212> DNA
<213> Homo sapiens

<400> 1515
tttggaggag gaacaac                                                    17

<210> 1516
<211> 17
<212> DNA
<213> Homo sapiens

<400> 1516
ttggaggagg aacaacc                                                    17

<210> 1517
<211> 17
<212> DNA
<213> Homo sapiens

<400> 1517
tggaggagga acaaccc                                                    17

<210> 1518
<211> 17
<212> DNA
<213> Homo sapiens

<400> 1518
ggaggaggaa caacccc                                                    17

<210> 1519
<211> 17
<212> DNA
<213> Homo sapiens

<400> 1519
gaggaggaac aaccccc                                                    17

<210> 1520
<211> 17
<212> DNA
<213> Homo sapiens

<400> 1520
aggaggaaca accccca                                                    17

<210> 1521
<211> 17
<212> DNA
<213> Homo sapiens

<400> 1521
ggaggaacaa cccccat                                                    17

<210> 1522
<211> 17
<212> DNA
<213> Homo sapiens

<400> 1522
gaggaacaac ccccatg                                                    17

<210> 1523
<211> 17
<212> DNA
<213> Homo sapiens

<400> 1523
aggaacaacc cccatgt                                                    17

<210> 1524
<211> 17
<212> DNA
<213> Homo sapiens

<400> 1524
ggaacaaccc ccatgtt                                                    17

<210> 1525
<211> 17
<212> DNA
<213> Homo sapiens

<400> 1525
gaacaacccc catgttg                                                    17

<210> 1526
<211> 17
<212> DNA
<213> Homo sapiens

<400> 1526
aacaaccccc atgttga                                                    17

<210> 1527
<211> 17
<212> DNA
<213> Homo sapiens

<400> 1527
acaaccccca tgttgac                                                    17

<210> 1528
<211> 17
<212> DNA
<213> Homo sapiens

<400> 1528
caacccccat gttgact                                                    17

<210> 1529
<211> 17
<212> DNA
<213> Homo sapiens

<400> 1529
aacccccatg ttgactt                                                    17

<210> 1530
<211> 17
<212> DNA
<213> Homo sapiens

<400> 1530
acccccatgt tgacttg                                                    17

<210> 1531

<211> 17
<212> DNA
<213> Homo sapiens

<400> 1531
cccccatgtt gacttgg                                                    17

<210> 1532
<211> 17
<212> DNA
<213> Homo sapiens

<400> 1532
ccccatgttg acttggc                                                    17

<210> 1533
<211> 17
<212> DNA
<213> Homo sapiens

<400> 1533
cccatgttga cttggct                                                    17

<210> 1534
<211> 17
<212> DNA
<213> Homo sapiens

<400> 1534
ccatgttgac ttggctt                                                    17

<210> 1535
<211> 17
<212> DNA
<213> Homo sapiens

<400> 1535
catgttgact tggcttc                                                    17

<210> 1536
<211> 17
<212> DNA
<213> Homo sapiens

<400> 1536
atgttgactt ggcttca                                                    17

<210> 1537
<211> 17
<212> DNA
<213> Homo sapiens

<400> 1537
tgttgacttg gcttcag                                                    17

<210> 1538
<211> 17
<212> DNA
<213> Homo sapiens

<400> 1538

gttgacttgg cttcaga                                                    17

<210> 1539
<211> 17
<212> DNA
<213> Homo sapiens

<400> 1539
ttgacttggc ttcagat                                                    17

<210> 1540
<211> 17
<212> DNA
<213> Homo sapiens

<400> 1540
tgacttggct tcagatc                                                    17

<210> 1541
<211> 17
<212> DNA
<213> Homo sapiens

<400> 1541
gacttggctt cagatca                                                    17

<210> 1542
<211> 17
<212> DNA
<213> Homo sapiens

<400> 1542
acttggcttc agatcag                                                    17

<210> 1543
<211> 17
<212> DNA
<213> Homo sapiens

<400> 1543
cttggcttca gatcaga                                                    17

<210> 1544
<211> 17
<212> DNA
<213> Homo sapiens

<400> 1544
ttggcttcag atcagag                                                    17

<210> 1545
<211> 17
<212> DNA
<213> Homo sapiens

<400> 1545
tggcttcaga tcagagt                                                    17

<210> 1546
<211> 17
<212> DNA

<213> Homo sapiens

<400> 1546
ggcttcagat cagagtt                                                    17

<210> 1547
<211> 17
<212> DNA
<213> Homo sapiens

<400> 1547
gcttcagatc agagttg                                                    17

<210> 1548
<211> 17
<212> DNA
<213> Homo sapiens

<400> 1548
cttcagatca gagttgg                                                    17

<210> 1549
<211> 17
<212> DNA
<213> Homo sapiens

<400> 1549
ttcagatcag agttggc                                                    17

<210> 1550
<211> 17
<212> DNA
<213> Homo sapiens

<400> 1550
tcagatcaga gttggcg                                                    17

<210> 1551
<211> 17
<212> DNA
<213> Homo sapiens

<400> 1551
cagatcagag ttggcgt                                                    17

<210> 1552
<211> 17
<212> DNA
<213> Homo sapiens

<400> 1552
agatcagagt tggcgtg                                                    17

<210> 1553
<211> 25
<212> DNA
<213> Homo sapiens

<400> 1553
atagccctta tccaggtttt tatct                                           25

<210> 1554
<211> 25
<212> DNA
<213> Homo sapiens

<400> 1554
tagcccttat ccaggttttt atcta                                          25

<210> 1555
<211> 25
<212> DNA
<213> Homo sapiens

<400> 1555
agcccttatc caggttttta tctaa                                          25

<210> 1556
<211> 25
<212> DNA
<213> Homo sapiens

<400> 1556
gcccttatcc aggtttttat ctaag                                          25

<210> 1557
<211> 25
<212> DNA
<213> Homo sapiens

<400> 1557
cccttatcca ggtttttatc taagg                                          25

<210> 1558
<211> 25
<212> DNA
<213> Homo sapiens

<400> 1558
ccttatccag gtttttatct aagga                                          25

<210> 1559
<211> 25
<212> DNA
<213> Homo sapiens

<400> 1559
cttatccagg tttttatcta aggaa                                          25

<210> 1560
<211> 25
<212> DNA
<213> Homo sapiens

<400> 1560
ttatccaggt ttttatctaa ggaat                                          25

<210> 1561
<211> 25
<212> DNA
<213> Homo sapiens

<400> 1561
tatccaggtt tttatctaag gaatc                                          25

<210> 1562
<211> 25
<212> DNA
<213> Homo sapiens

<400> 1562
atccaggttt ttatctaagg aatcc                                          25

<210> 1563
<211> 25
<212> DNA
<213> Homo sapiens

<400> 1563
tccaggtttt tatctaagga atccc                                          25

<210> 1564
<211> 25
<212> DNA
<213> Homo sapiens

<400> 1564
ccaggttttt atctaaggaa tccca                                          25

<210> 1565
<211> 25
<212> DNA
<213> Homo sapiens

<400> 1565
caggttttta tctaaggaat cccaa                                          25

<210> 1566
<211> 25
<212> DNA
<213> Homo sapiens

<400> 1566
aggtttttat ctaaggaatc ccaag                                          25

<210> 1567
<211> 25
<212> DNA
<213> Homo sapiens

<400> 1567
ggtttttatc taaggaatcc caaga                                          25

<210> 1568
<211> 25
<212> DNA
<213> Homo sapiens

<400> 1568
gtttttatct aaggaatccc aagaa                                          25

<210> 1569
<211> 25

<212> DNA
<213> Homo sapiens

<400> 1569
tttttatcta aggaatccca agaag                                          25

<210> 1570
<211> 25
<212> DNA
<213> Homo sapiens

<400> 1570
ttttatctaa ggaatcccaa gaaga                                          25

<210> 1571
<211> 25
<212> DNA
<213> Homo sapiens

<400> 1571
tttatctaag gaatcccaag aagac                                          25

<210> 1572
<211> 25
<212> DNA
<213> Homo sapiens

<400> 1572
ttatctaagg aatcccaaga agact                                          25

<210> 1573
<211> 25
<212> DNA
<213> Homo sapiens

<400> 1573
tatctaagga atcccaagaa gactg                                          25

<210> 1574
<211> 25
<212> DNA
<213> Homo sapiens

<400> 1574
atctaaggaa tcccaagaag actgg                                          25

<210> 1575
<211> 25
<212> DNA
<213> Homo sapiens

<400> 1575
tctaaggaat cccaagaaga ctggg                                          25

<210> 1576
<211> 25
<212> DNA
<213> Homo sapiens

<400> 1576
ctaaggaatc ccaagaagac tgggg                                          25

<210> 1577
<211> 25
<212> DNA
<213> Homo sapiens

<400> 1577
taaggaatcc caagaagact gggga                                            25

<210> 1578
<211> 25
<212> DNA
<213> Homo sapiens

<400> 1578
aaggaatccc aagaagactg gggaa                                            25

<210> 1579
<211> 25
<212> DNA
<213> Homo sapiens

<400> 1579
aggaatccca agaagactgg ggaat                                            25

<210> 1580
<211> 25
<212> DNA
<213> Homo sapiens

<400> 1580
ggaatcccaa gaagactggg gaatg                                            25

<210> 1581
<211> 25
<212> DNA
<213> Homo sapiens

<400> 1581
gaatcccaag aagactgggg aatgg                                            25

<210> 1582
<211> 25
<212> DNA
<213> Homo sapiens

<400> 1582
aatcccaaga agactgggga atgga                                            25

<210> 1583
<211> 25
<212> DNA
<213> Homo sapiens

<400> 1583
atcccaagaa gactggggaa tggag                                            25

<210> 1584
<211> 25
<212> DNA
<213> Homo sapiens

<400> 1584
tcccaagaag actggggaat ggaga                                          25

<210> 1585
<211> 25
<212> DNA
<213> Homo sapiens

<400> 1585
cccaagaaga ctggggaatg gagag                                          25

<210> 1586
<211> 25
<212> DNA
<213> Homo sapiens

<400> 1586
ccaagaagac tggggaatgg agaga                                          25

<210> 1587
<211> 25
<212> DNA
<213> Homo sapiens

<400> 1587
caagaagact ggggaatgga gagac                                          25

<210> 1588
<211> 25
<212> DNA
<213> Homo sapiens

<400> 1588
aagaagactg gggaatggag agaca                                          25

<210> 1589
<211> 25
<212> DNA
<213> Homo sapiens

<400> 1589
agaagactgg ggaatggaga gacag                                          25

<210> 1590
<211> 25
<212> DNA
<213> Homo sapiens

<400> 1590
gaagactggg gaatggagag acagt                                          25

<210> 1591
<211> 25
<212> DNA
<213> Homo sapiens

<400> 1591
aagactgggg aatggagaga cagtc                                          25

<210> 1592

<211> 25
<212> DNA
<213> Homo sapiens

<400> 1592
agactgggga atggagagac agtca                                          25

<210> 1593
<211> 25
<212> DNA
<213> Homo sapiens

<400> 1593
gactggggaa tggagagaca gtcaa                                          25

<210> 1594
<211> 25
<212> DNA
<213> Homo sapiens

<400> 1594
actggggaat ggagagacag tcaag                                          25

<210> 1595
<211> 25
<212> DNA
<213> Homo sapiens

<400> 1595
ctggggaatg gagagacagt caagg                                          25

<210> 1596
<211> 25
<212> DNA
<213> Homo sapiens

<400> 1596
tggggaatgg agagacagtc aaggg                                          25

<210> 1597
<211> 25
<212> DNA
<213> Homo sapiens

<400> 1597
ggggaatgga gagacagtca agggt                                          25

<210> 1598
<211> 25
<212> DNA
<213> Homo sapiens

<400> 1598
gggaatggag agacagtcaa gggtt                                          25

<210> 1599
<211> 25
<212> DNA
<213> Homo sapiens

<400> 1599

ggaatggaga gacagtcaag ggtta                                          25

<210> 1600
<211> 25
<212> DNA
<213> Homo sapiens

<400> 1600
gaatggagag acagtcaagg gttat                                          25

<210> 1601
<211> 25
<212> DNA
<213> Homo sapiens

<400> 1601
aatggagaga cagtcaaggg ttatg                                          25

<210> 1602
<211> 25
<212> DNA
<213> Homo sapiens

<400> 1602
atggagagac agtcaagggt tatgt                                          25

<210> 1603
<211> 25
<212> DNA
<213> Homo sapiens

<400> 1603
tggagagaca gtcaagggtt atgtc                                          25

<210> 1604
<211> 25
<212> DNA
<213> Homo sapiens

<400> 1604
ggagagacag tcaagggtta tgtca                                          25

<210> 1605
<211> 25
<212> DNA
<213> Homo sapiens

<400> 1605
gagagacagt caagggttat gtcag                                          25

<210> 1606
<211> 25
<212> DNA
<213> Homo sapiens

<400> 1606
agagacagtc aagggttatg tcaga                                          25

<210> 1607
<211> 25
<212> DNA

<213> Homo sapiens

<400> 1607
gagacagtca agggttatgt cagaa                                          25

<210> 1608
<211> 25
<212> DNA
<213> Homo sapiens

<400> 1608
agacagtcaa gggttatgtc agaaa                                          25

<210> 1609
<211> 25
<212> DNA
<213> Homo sapiens

<400> 1609
gacagtcaag ggttatgtca gaaaa                                          25

<210> 1610
<211> 25
<212> DNA
<213> Homo sapiens

<400> 1610
acagtcaagg gttatgtcag aaaag                                          25

<210> 1611
<211> 25
<212> DNA
<213> Homo sapiens

<400> 1611
cagtcaaggg ttatgtcaga aaagg                                          25

<210> 1612
<211> 25
<212> DNA
<213> Homo sapiens

<400> 1612
agtcaagggt tatgtcagaa aagga                                          25

<210> 1613
<211> 25
<212> DNA
<213> Homo sapiens

<400> 1613
gtcaagggtt atgtcagaaa aggat                                          25

<210> 1614
<211> 25
<212> DNA
<213> Homo sapiens

<400> 1614
tcaagggtta tgtcagaaaa ggatg                                          25

<210> 1615
<211> 25
<212> DNA
<213> Homo sapiens

<400> 1615
caagggttat gtcagaaaag gatga                                          25

<210> 1616
<211> 25
<212> DNA
<213> Homo sapiens

<400> 1616
aagggttatg tcagaaaagg atgag                                          25

<210> 1617
<211> 25
<212> DNA
<213> Homo sapiens

<400> 1617
agggttatgt cagaaaagga tgagt                                          25

<210> 1618
<211> 25
<212> DNA
<213> Homo sapiens

<400> 1618
gggttatgtc agaaaaggat gagta                                          25

<210> 1619
<211> 25
<212> DNA
<213> Homo sapiens

<400> 1619
ggttatgtca gaaaaggatg agtat                                          25

<210> 1620
<211> 25
<212> DNA
<213> Homo sapiens

<400> 1620
gttatgtcag aaaaggatga gtatc                                          25

<210> 1621
<211> 25
<212> DNA
<213> Homo sapiens

<400> 1621
ttatgtcaga aaaggatgag tatca                                          25

<210> 1622
<211> 25
<212> DNA
<213> Homo sapiens

<400> 1622
tatgtcagaa aaggatgagt atcag                                          25

<210> 1623
<211> 25
<212> DNA
<213> Homo sapiens

<400> 1623
atgtcagaaa aggatgagta tcagt                                          25

<210> 1624
<211> 25
<212> DNA
<213> Homo sapiens

<400> 1624
tgtcagaaaa ggatgagtat cagtt                                          25

<210> 1625
<211> 25
<212> DNA
<213> Homo sapiens

<400> 1625
gtcagaaaag gatgagtatc agttt                                          25

<210> 1626
<211> 25
<212> DNA
<213> Homo sapiens

<400> 1626
tcagaaaagg atgagtatca gtttc                                          25

<210> 1627
<211> 25
<212> DNA
<213> Homo sapiens

<400> 1627
cagaaaagga tgagtatcag tttca                                          25

<210> 1628
<211> 25
<212> DNA
<213> Homo sapiens

<400> 1628
agaaaaggat gagtatcagt ttcaa                                          25

<210> 1629
<211> 25
<212> DNA
<213> Homo sapiens

<400> 1629
gaaaaggatg agtatcagtt tcaac                                          25

<210> 1630
<211> 25

<212> DNA
<213> Homo sapiens

<400> 1630
aaaaggatga gtatcagttt caaca                                            25

<210> 1631
<211> 25
<212> DNA
<213> Homo sapiens

<400> 1631
aaaggatgag tatcagtttc aacat                                            25

<210> 1632
<211> 25
<212> DNA
<213> Homo sapiens

<400> 1632
aaggatgagt atcagtttca acatc                                            25

<210> 1633
<211> 25
<212> DNA
<213> Homo sapiens

<400> 1633
aggatgagta tcagtttcaa catca                                            25

<210> 1634
<211> 25
<212> DNA
<213> Homo sapiens

<400> 1634
ggatgagtat cagtttcaac atcag                                            25

<210> 1635
<211> 25
<212> DNA
<213> Homo sapiens

<400> 1635
gatgagtatc agtttcaaca tcagg                                            25

<210> 1636
<211> 25
<212> DNA
<213> Homo sapiens

<400> 1636
atgagtatca gtttcaacat caggg                                            25

<210> 1637
<211> 25
<212> DNA
<213> Homo sapiens

<400> 1637
tgagtatcag tttcaacatc aggga                                            25

<210> 1638
<211> 25
<212> DNA
<213> Homo sapiens

<400> 1638
gagtatcagt ttcaacatca gggag                                          25

<210> 1639
<211> 25
<212> DNA
<213> Homo sapiens

<400> 1639
agtatcagtt tcaacatcag ggagc                                          25

<210> 1640
<211> 25
<212> DNA
<213> Homo sapiens

<400> 1640
gtatcagttt caacatcagg gagcg                                          25

<210> 1641
<211> 25
<212> DNA
<213> Homo sapiens

<400> 1641
tatcagtttc aacatcaggg agcgg                                          25

<210> 1642
<211> 25
<212> DNA
<213> Homo sapiens

<400> 1642
atcagtttca acatcaggga gcggt                                          25

<210> 1643
<211> 25
<212> DNA
<213> Homo sapiens

<400> 1643
tcagtttcaa catcagggag cggtg                                          25

<210> 1644
<211> 25
<212> DNA
<213> Homo sapiens

<400> 1644
cagtttcaac atcagggagc ggtgg                                          25

<210> 1645
<211> 25
<212> DNA
<213> Homo sapiens

<400> 1645
agtttcaaca tcagggagcg gtgga                                          25

<210> 1646
<211> 25
<212> DNA
<213> Homo sapiens

<400> 1646
gtttcaacat cagggagcgg tggag                                          25

<210> 1647
<211> 25
<212> DNA
<213> Homo sapiens

<400> 1647
tttcaacatc agggagcggt ggagc                                          25

<210> 1648
<211> 25
<212> DNA
<213> Homo sapiens

<400> 1648
ttcaacatca gggagcggtg gagct                                          25

<210> 1649
<211> 25
<212> DNA
<213> Homo sapiens

<400> 1649
tcaacatcag ggagcggtgg agctg                                          25

<210> 1650
<211> 25
<212> DNA
<213> Homo sapiens

<400> 1650
caacatcagg gagcggtgga gctgc                                          25

<210> 1651
<211> 25
<212> DNA
<213> Homo sapiens

<400> 1651
aacatcaggg agcggtggag ctgct                                          25

<210> 1652
<211> 25
<212> DNA
<213> Homo sapiens

<400> 1652
acatcaggga gcggtggagc tgctt                                          25

<210> 1653

<211> 25
<212> DNA
<213> Homo sapiens

<400> 1653
catcagggag cggtggagct gcttg                                          25

<210> 1654
<211> 25
<212> DNA
<213> Homo sapiens

<400> 1654
atcagggagc ggtggagctg cttgt                                          25

<210> 1655
<211> 25
<212> DNA
<213> Homo sapiens

<400> 1655
tcagggagcg gtggagctgc ttgtc                                          25

<210> 1656
<211> 25
<212> DNA
<213> Homo sapiens

<400> 1656
cagggagcgg tggagctgct tgtct                                          25

<210> 1657
<211> 25
<212> DNA
<213> Homo sapiens

<400> 1657
agggagcggt ggagctgctt gtctt                                          25

<210> 1658
<211> 25
<212> DNA
<213> Homo sapiens

<400> 1658
gggagcggtg gagctgcttg tcttc                                          25

<210> 1659
<211> 25
<212> DNA
<213> Homo sapiens

<400> 1659
ggagcggtgg agctgcttgt cttca                                          25

<210> 1660
<211> 25
<212> DNA
<213> Homo sapiens

<400> 1660

gagcggtgga gctgcttgtc ttcaa 25

<210> 1661
<211> 25
<212> DNA
<213> Homo sapiens

<400> 1661
agcggtggag ctgcttgtct tcaat 25

<210> 1662
<211> 25
<212> DNA
<213> Homo sapiens

<400> 1662
gcggtggagc tgcttgtctt caatt 25

<210> 1663
<211> 25
<212> DNA
<213> Homo sapiens

<400> 1663
cggtggagct gcttgtcttc aattt 25

<210> 1664
<211> 25
<212> DNA
<213> Homo sapiens

<400> 1664
ggtggagctg cttgtcttca atttt 25

<210> 1665
<211> 25
<212> DNA
<213> Homo sapiens

<400> 1665
gtggagctgc ttgtcttcaa ttttt 25

<210> 1666
<211> 25
<212> DNA
<213> Homo sapiens

<400> 1666
tggagctgct tgtcttcaat ttttt 25

<210> 1667
<211> 25
<212> DNA
<213> Homo sapiens

<400> 1667
ggagctgctt gtcttcaatt ttttg 25

<210> 1668
<211> 25
<212> DNA

<213> Homo sapiens

<400> 1668
gagctgcttg tcttcaattt tttgc                                          25

<210> 1669
<211> 25
<212> DNA
<213> Homo sapiens

<400> 1669
agctgcttgt cttcaatttt ttgct                                          25

<210> 1670
<211> 25
<212> DNA
<213> Homo sapiens

<400> 1670
gctgcttgtc ttcaattttt tgctc                                          25

<210> 1671
<211> 25
<212> DNA
<213> Homo sapiens

<400> 1671
ctgcttgtct tcaatttttt gctca                                          25

<210> 1672
<211> 25
<212> DNA
<213> Homo sapiens

<400> 1672
tgcttgtctt caattttttg ctcat                                          25

<210> 1673
<211> 25
<212> DNA
<213> Homo sapiens

<400> 1673
gcttgtcttc aattttttgc tcatc                                          25

<210> 1674
<211> 25
<212> DNA
<213> Homo sapiens

<400> 1674
cttgtcttca attttttgct catcc                                          25

<210> 1675
<211> 25
<212> DNA
<213> Homo sapiens

<400> 1675
ttgtcttcaa ttttttgctc atcct                                          25

<210> 1676
<211> 25
<212> DNA
<213> Homo sapiens

<400> 1676
tgtcttcaat tttttgctca tcctt                                          25

<210> 1677
<211> 25
<212> DNA
<213> Homo sapiens

<400> 1677
gtcttcaatt ttttgctcat cctta                                          25

<210> 1678
<211> 25
<212> DNA
<213> Homo sapiens

<400> 1678
tcttcaattt tttgctcatc cttac                                          25

<210> 1679
<211> 25
<212> DNA
<213> Homo sapiens

<400> 1679
cttcaatttt ttgctcatcc ttacc                                          25

<210> 1680
<211> 25
<212> DNA
<213> Homo sapiens

<400> 1680
ttcaattttt tgctcatcct tacca                                          25

<210> 1681
<211> 25
<212> DNA
<213> Homo sapiens

<400> 1681
tcaatttttt gctcatcctt accat                                          25

<210> 1682
<211> 25
<212> DNA
<213> Homo sapiens

<400> 1682
caattttttg ctcatcctta ccatt                                          25

<210> 1683
<211> 25
<212> DNA
<213> Homo sapiens

<400> 1683
aatttttgc tcatccttac cattt                                        25

<210> 1684
<211> 25
<212> DNA
<213> Homo sapiens

<400> 1684
atttttgct catccttacc atttt                                        25

<210> 1685
<211> 25
<212> DNA
<213> Homo sapiens

<400> 1685
tttttgctc atccttacca ttttg                                        25

<210> 1686
<211> 25
<212> DNA
<213> Homo sapiens

<400> 1686
ttttgctca tccttaccat tttga                                        25

<210> 1687
<211> 25
<212> DNA
<213> Homo sapiens

<400> 1687
tttgctcat ccttaccatt ttgac                                        25

<210> 1688
<211> 25
<212> DNA
<213> Homo sapiens

<400> 1688
tttgctcatc cttaccattt tgaca                                        25

<210> 1689
<211> 25
<212> DNA
<213> Homo sapiens

<400> 1689
ttgctcatcc ttaccatttt gacaa                                        25

<210> 1690
<211> 25
<212> DNA
<213> Homo sapiens

<400> 1690
tgctcatcct taccattttg acaat                                        25

<210> 1691
<211> 25

<212> DNA
<213> Homo sapiens

<400> 1691
gctcatcctt accattttga caatc                                          25

<210> 1692
<211> 25
<212> DNA
<213> Homo sapiens

<400> 1692
ctcatcctta ccattttgac aatct                                          25

<210> 1693
<211> 25
<212> DNA
<213> Homo sapiens

<400> 1693
tcatccttac cattttgaca atctg                                          25

<210> 1694
<211> 25
<212> DNA
<213> Homo sapiens

<400> 1694
catccttacc attttgacaa tctgg                                          25

<210> 1695
<211> 25
<212> DNA
<213> Homo sapiens

<400> 1695
atccttacca ttttgacaat ctggt                                          25

<210> 1696
<211> 25
<212> DNA
<213> Homo sapiens

<400> 1696
tccttaccat tttgacaatc tggtt                                          25

<210> 1697
<211> 25
<212> DNA
<213> Homo sapiens

<400> 1697
ccttaccatt ttgacaatct ggtta                                          25

<210> 1698
<211> 25
<212> DNA
<213> Homo sapiens

<400> 1698
cttaccattt tgacaatctg gttat                                          25

<210> 1699
<211> 25
<212> DNA
<213> Homo sapiens

<400> 1699
ttaccatttt gacaatctgg ttatt                                          25

<210> 1700
<211> 25
<212> DNA
<213> Homo sapiens

<400> 1700
taccattttg acaatctggt tattt                                          25

<210> 1701
<211> 25
<212> DNA
<213> Homo sapiens

<400> 1701
accattttga caatctggtt attta                                          25

<210> 1702
<211> 25
<212> DNA
<213> Homo sapiens

<400> 1702
ccattttgac aatctggtta tttaa                                          25

<210> 1703
<211> 25
<212> DNA
<213> Homo sapiens

<400> 1703
cattttgaca atctggttat ttaaa                                          25

<210> 1704
<211> 25
<212> DNA
<213> Homo sapiens

<400> 1704
attttgacaa tctggttatt taaaa                                          25

<210> 1705
<211> 25
<212> DNA
<213> Homo sapiens

<400> 1705
ttttgacaat ctggttattt aaaaa                                          25

<210> 1706
<211> 25
<212> DNA
<213> Homo sapiens

<400> 1706
tttgacaatc tggttattta aaaat                                          25

<210> 1707
<211> 25
<212> DNA
<213> Homo sapiens

<400> 1707
ttgacaatct ggttatttaa aaatc                                          25

<210> 1708
<211> 25
<212> DNA
<213> Homo sapiens

<400> 1708
tgacaatctg gttatttaaa aatca                                          25

<210> 1709
<211> 25
<212> DNA
<213> Homo sapiens

<400> 1709
gacaatctgg ttatttaaaa atcat                                          25

<210> 1710
<211> 25
<212> DNA
<213> Homo sapiens

<400> 1710
acaatctggt tatttaaaaa tcatc                                          25

<210> 1711
<211> 25
<212> DNA
<213> Homo sapiens

<400> 1711
caatctggtt atttaaaaat catcg                                          25

<210> 1712
<211> 25
<212> DNA
<213> Homo sapiens

<400> 1712
aatctggtta tttaaaaatc atcga                                          25

<210> 1713
<211> 25
<212> DNA
<213> Homo sapiens

<400> 1713
atctggttat ttaaaaatca tcgat                                          25

<210> 1714

<211> 25
<212> DNA
<213> Homo sapiens

<400> 1714
tctggttatt taaaaatcat cgatt                                          25

<210> 1715
<211> 25
<212> DNA
<213> Homo sapiens

<400> 1715
ctggttattt aaaaatcatc gattc                                          25

<210> 1716
<211> 25
<212> DNA
<213> Homo sapiens

<400> 1716
tggttattta aaaatcatcg attcc                                          25

<210> 1717
<211> 25
<212> DNA
<213> Homo sapiens

<400> 1717
ggttatttaa aaatcatcga ttccg                                          25

<210> 1718
<211> 25
<212> DNA
<213> Homo sapiens

<400> 1718
gttatttaaa aatcatcgat tccgc                                          25

<210> 1719
<211> 25
<212> DNA
<213> Homo sapiens

<400> 1719
ttatttaaaa atcatcgatt ccgct                                          25

<210> 1720
<211> 25
<212> DNA
<213> Homo sapiens

<400> 1720
tatttaaaaa tcatcgattc cgctt                                          25

<210> 1721
<211> 25
<212> DNA
<213> Homo sapiens

<400> 1721

atttaaaaat catcgattcc gcttc 25

<210> 1722
<211> 25
<212> DNA
<213> Homo sapiens

<400> 1722
tttaaaaatc atcgattccg cttct 25

<210> 1723
<211> 25
<212> DNA
<213> Homo sapiens

<400> 1723
ttaaaaatca tcgattccgc ttctt 25

<210> 1724
<211> 25
<212> DNA
<213> Homo sapiens

<400> 1724
taaaaatcat cgattccgct tcttg 25

<210> 1725
<211> 25
<212> DNA
<213> Homo sapiens

<400> 1725
aaaaatcatc gattccgctt cttgc 25

<210> 1726
<211> 25
<212> DNA
<213> Homo sapiens

<400> 1726
aaaatcatcg attccgcttc ttgca 25

<210> 1727
<211> 25
<212> DNA
<213> Homo sapiens

<400> 1727
aaatcatcga ttccgcttct tgcat 25

<210> 1728
<211> 25
<212> DNA
<213> Homo sapiens

<400> 1728
aatcatcgat tccgcttctt gcatg 25

<210> 1729
<211> 25
<212> DNA

<213> Homo sapiens

<400> 1729
atcatcgatt ccgcttcttg catga                                          25

<210> 1730
<211> 25
<212> DNA
<213> Homo sapiens

<400> 1730
tcatcgattc cgcttcttgc atgaa                                          25

<210> 1731
<211> 25
<212> DNA
<213> Homo sapiens

<400> 1731
catcgattcc gcttcttgca tgaaa                                          25

<210> 1732
<211> 25
<212> DNA
<213> Homo sapiens

<400> 1732
atcgattccg cttcttgcat gaaac                                          25

<210> 1733
<211> 25
<212> DNA
<213> Homo sapiens

<400> 1733
tcgattccgc ttcttgcatg aaact                                          25

<210> 1734
<211> 25
<212> DNA
<213> Homo sapiens

<400> 1734
cgattccgct tcttgcatga aactg                                          25

<210> 1735
<211> 25
<212> DNA
<213> Homo sapiens

<400> 1735
gattccgctt cttgcatgaa actgg                                          25

<210> 1736
<211> 25
<212> DNA
<213> Homo sapiens

<400> 1736
attccgcttc ttgcatgaaa ctgga                                          25

<210> 1737
<211> 25
<212> DNA
<213> Homo sapiens

<400> 1737
ttccgcttct tgcatgaaac tggag                                              25

<210> 1738
<211> 25
<212> DNA
<213> Homo sapiens

<400> 1738
tccgcttctt gcatgaaact ggagg                                              25

<210> 1739
<211> 25
<212> DNA
<213> Homo sapiens

<400> 1739
ccgcttcttg catgaaactg gagga                                              25

<210> 1740
<211> 25
<212> DNA
<213> Homo sapiens

<400> 1740
cgcttcttgc atgaaactgg aggag                                              25

<210> 1741
<211> 25
<212> DNA
<213> Homo sapiens

<400> 1741
gcttcttgca tgaaactgga ggagc                                              25

<210> 1742
<211> 25
<212> DNA
<213> Homo sapiens

<400> 1742
cttcttgcat gaaactggag gagca                                              25

<210> 1743
<211> 25
<212> DNA
<213> Homo sapiens

<400> 1743
ttcttgcatg aaactggagg agcaa                                              25

<210> 1744
<211> 25
<212> DNA
<213> Homo sapiens

<400> 1744
tcttgcatga aactggagga gcaat                                          25

<210> 1745
<211> 25
<212> DNA
<213> Homo sapiens

<400> 1745
cttgcatgaa actggaggag caatg                                          25

<210> 1746
<211> 25
<212> DNA
<213> Homo sapiens

<400> 1746
ttgcatgaaa ctggaggagc aatgg                                          25

<210> 1747
<211> 25
<212> DNA
<213> Homo sapiens

<400> 1747
tgcatgaaac tggaggagca atggt                                          25

<210> 1748
<211> 25
<212> DNA
<213> Homo sapiens

<400> 1748
gcatgaaact ggaggagcaa tggtg                                          25

<210> 1749
<211> 25
<212> DNA
<213> Homo sapiens

<400> 1749
catgaaactg gaggagcaat ggtgt                                          25

<210> 1750
<211> 25
<212> DNA
<213> Homo sapiens

<400> 1750
atgaaactgg aggagcaatg gtgta                                          25

<210> 1751
<211> 25
<212> DNA
<213> Homo sapiens

<400> 1751
tgaaactgga ggagcaatgg tgtat                                          25

<210> 1752
<211> 25

<212> DNA
<213> Homo sapiens

<400> 1752
gaaactggag gagcaatggt gtatg                                          25

<210> 1753
<211> 25
<212> DNA
<213> Homo sapiens

<400> 1753
aaactggagg agcaatggtg tatgg                                          25

<210> 1754
<211> 25
<212> DNA
<213> Homo sapiens

<400> 1754
aactggagga gcaatggtgt atggc                                          25

<210> 1755
<211> 25
<212> DNA
<213> Homo sapiens

<400> 1755
actggaggag caatggtgta tggcc                                          25

<210> 1756
<211> 25
<212> DNA
<213> Homo sapiens

<400> 1756
ctggaggagc aatggtgtat ggcct                                          25

<210> 1757
<211> 25
<212> DNA
<213> Homo sapiens

<400> 1757
tggaggagca atggtgtatg gcctt                                          25

<210> 1758
<211> 25
<212> DNA
<213> Homo sapiens

<400> 1758
ggaggagcaa tggtgtatgg cctta                                          25

<210> 1759
<211> 25
<212> DNA
<213> Homo sapiens

<400> 1759
gaggagcaat ggtgtatggc cttat                                          25

<210> 1760
<211> 25
<212> DNA
<213> Homo sapiens

<400> 1760
aggagcaatg gtgtatggcc ttata                                          25

<210> 1761
<211> 25
<212> DNA
<213> Homo sapiens

<400> 1761
ggagcaatgg tgtatggcct tataa                                          25

<210> 1762
<211> 25
<212> DNA
<213> Homo sapiens

<400> 1762
gagcaatggt gtatggcctt ataat                                          25

<210> 1763
<211> 25
<212> DNA
<213> Homo sapiens

<400> 1763
agcaatggtg tatggcctta taatg                                          25

<210> 1764
<211> 25
<212> DNA
<213> Homo sapiens

<400> 1764
gcaatggtgt atggccttat aatgg                                          25

<210> 1765
<211> 25
<212> DNA
<213> Homo sapiens

<400> 1765
caatggtgta tggccttata atggg                                          25

<210> 1766
<211> 25
<212> DNA
<213> Homo sapiens

<400> 1766
aatggtgtat ggccttataa tggga                                          25

<210> 1767
<211> 25
<212> DNA
<213> Homo sapiens

<400> 1767
atggtgtatg gccttataat gggac                                          25

<210> 1768
<211> 25
<212> DNA
<213> Homo sapiens

<400> 1768
tggtgtatgg ccttataatg ggact                                          25

<210> 1769
<211> 25
<212> DNA
<213> Homo sapiens

<400> 1769
ggtgtatggc cttataatgg gacta                                          25

<210> 1770
<211> 25
<212> DNA
<213> Homo sapiens

<400> 1770
gtgtatggcc ttataatggg actaa                                          25

<210> 1771
<211> 25
<212> DNA
<213> Homo sapiens

<400> 1771
tgtatggcct tataatggga ctaat                                          25

<210> 1772
<211> 25
<212> DNA
<213> Homo sapiens

<400> 1772
gtatggcctt ataatgggac taatt                                          25

<210> 1773
<211> 25
<212> DNA
<213> Homo sapiens

<400> 1773
tatggcctta taatgggact aattt                                          25

<210> 1774
<211> 25
<212> DNA
<213> Homo sapiens

<400> 1774
atggccttat aatgggacta atttt                                          25

<210> 1775

<211> 25
<212> DNA
<213> Homo sapiens

<400> 1775
tggccttata atgggactaa tttta                                          25

<210> 1776
<211> 25
<212> DNA
<213> Homo sapiens

<400> 1776
ggccttataa tgggactaat tttac                                          25

<210> 1777
<211> 25
<212> DNA
<213> Homo sapiens

<400> 1777
gccttataat gggactaatt ttacg                                          25

<210> 1778
<211> 25
<212> DNA
<213> Homo sapiens

<400> 1778
ccttataatg ggactaattt tacga                                          25

<210> 1779
<211> 25
<212> DNA
<213> Homo sapiens

<400> 1779
cttataatgg gactaatttt acgat                                          25

<210> 1780
<211> 25
<212> DNA
<213> Homo sapiens

<400> 1780
ttataatggg actaatttta cgata                                          25

<210> 1781
<211> 25
<212> DNA
<213> Homo sapiens

<400> 1781
tataatggga ctaattttac gatat                                          25

<210> 1782
<211> 25
<212> DNA
<213> Homo sapiens

<400> 1782

ataatgggac taattttacg atatg 25

<210> 1783
<211> 25
<212> DNA
<213> Homo sapiens

<400> 1783
taatgggact aattttacga tatgc 25

<210> 1784
<211> 25
<212> DNA
<213> Homo sapiens

<400> 1784
aatgggacta attttacgat atgct 25

<210> 1785
<211> 25
<212> DNA
<213> Homo sapiens

<400> 1785
atgggactaa ttttacgata tgcta 25

<210> 1786
<211> 25
<212> DNA
<213> Homo sapiens

<400> 1786
tgggactaat tttacgatat gctac 25

<210> 1787
<211> 25
<212> DNA
<213> Homo sapiens

<400> 1787
gggactaatt ttacgatatg ctaca 25

<210> 1788
<211> 25
<212> DNA
<213> Homo sapiens

<400> 1788
ggactaattt tacgatatgc tacag 25

<210> 1789
<211> 25
<212> DNA
<213> Homo sapiens

<400> 1789
gactaatttt acgatatgct acagc 25

<210> 1790
<211> 25
<212> DNA

<213> Homo sapiens

<400> 1790
actaatttta cgatatgcta cagca                                          25

<210> 1791
<211> 25
<212> DNA
<213> Homo sapiens

<400> 1791
ctaattttac gatatgctac agcac                                          25

<210> 1792
<211> 25
<212> DNA
<213> Homo sapiens

<400> 1792
taattttacg atatgctaca gcacc                                          25

<210> 1793
<211> 25
<212> DNA
<213> Homo sapiens

<400> 1793
aattttacga tatgctacag cacca                                          25

<210> 1794
<211> 25
<212> DNA
<213> Homo sapiens

<400> 1794
attttacgat atgctacagc accaa                                          25

<210> 1795
<211> 25
<212> DNA
<213> Homo sapiens

<400> 1795
ttttacgata tgctacagca ccaac                                          25

<210> 1796
<211> 25
<212> DNA
<213> Homo sapiens

<400> 1796
tttacgatat gctacagcac caact                                          25

<210> 1797
<211> 25
<212> DNA
<213> Homo sapiens

<400> 1797
ttacgatatg ctacagcacc aactg                                          25

<210> 1798
<211> 25
<212> DNA
<213> Homo sapiens

<400> 1798
tacgatatgc tacagcacca actga                                          25

<210> 1799
<211> 25
<212> DNA
<213> Homo sapiens

<400> 1799
acgatatgct acagcaccaa ctgat                                          25

<210> 1800
<211> 25
<212> DNA
<213> Homo sapiens

<400> 1800
cgatatgcta cagcaccaac tgata                                          25

<210> 1801
<211> 25
<212> DNA
<213> Homo sapiens

<400> 1801
gatatgctac agcaccaact gatat                                          25

<210> 1802
<211> 25
<212> DNA
<213> Homo sapiens

<400> 1802
atatgctaca gcaccaactg atatt                                          25

<210> 1803
<211> 25
<212> DNA
<213> Homo sapiens

<400> 1803
tatgctacag caccaactga tattg                                          25

<210> 1804
<211> 25
<212> DNA
<213> Homo sapiens

<400> 1804
atgctacagc accaactgat attga                                          25

<210> 1805
<211> 25
<212> DNA
<213> Homo sapiens

<400> 1805
tgctacagca ccaactgata ttgaa                                          25

<210> 1806
<211> 25
<212> DNA
<213> Homo sapiens

<400> 1806
gctacagcac caactgatat tgaaa                                          25

<210> 1807
<211> 25
<212> DNA
<213> Homo sapiens

<400> 1807
ctacagcacc aactgatatt gaaag                                          25

<210> 1808
<211> 25
<212> DNA
<213> Homo sapiens

<400> 1808
tacagcacca actgatattg aaagt                                          25

<210> 1809
<211> 25
<212> DNA
<213> Homo sapiens

<400> 1809
acagcaccaa ctgatattga aagtg                                          25

<210> 1810
<211> 25
<212> DNA
<213> Homo sapiens

<400> 1810
cagcaccaac tgatattgaa agtgg                                          25

<210> 1811
<211> 25
<212> DNA
<213> Homo sapiens

<400> 1811
agcaccaact gatattgaaa gtgga                                          25

<210> 1812
<211> 25
<212> DNA
<213> Homo sapiens

<400> 1812
gcaccaactg atattgaaag tggaa                                          25

<210> 1813
<211> 25

<212> DNA
<213> Homo sapiens

<400> 1813
caccaactga tattgaaagt ggaac                                          25

<210> 1814
<211> 25
<212> DNA
<213> Homo sapiens

<400> 1814
accaactgat attgaaagtg gaact                                          25

<210> 1815
<211> 25
<212> DNA
<213> Homo sapiens

<400> 1815
ccaactgata ttgaaagtgg aactg                                          25

<210> 1816
<211> 25
<212> DNA
<213> Homo sapiens

<400> 1816
caactgatat tgaaagtgga actgt                                          25

<210> 1817
<211> 25
<212> DNA
<213> Homo sapiens

<400> 1817
aactgatatt gaaagtggaa ctgtc                                          25

<210> 1818
<211> 25
<212> DNA
<213> Homo sapiens

<400> 1818
actgatattg aaagtggaac tgtct                                          25

<210> 1819
<211> 25
<212> DNA
<213> Homo sapiens

<400> 1819
ctgatattga aagtggaact gtcta                                          25

<210> 1820
<211> 25
<212> DNA
<213> Homo sapiens

<400> 1820
tgatattgaa agtggaactg tctat                                          25

<210> 1821
<211> 25
<212> DNA
<213> Homo sapiens

<400> 1821
gatattgaaa gtggaactgt ctatg                                          25

<210> 1822
<211> 25
<212> DNA
<213> Homo sapiens

<400> 1822
atattgaaag tggaactgtc tatga                                          25

<210> 1823
<211> 25
<212> DNA
<213> Homo sapiens

<400> 1823
tattgaaagt ggaactgtct atgac                                          25

<210> 1824
<211> 25
<212> DNA
<213> Homo sapiens

<400> 1824
attgaaagtg gaactgtcta tgact                                          25

<210> 1825
<211> 25
<212> DNA
<213> Homo sapiens

<400> 1825
ttgaaagtgg aactgtctat gactg                                          25

<210> 1826
<211> 25
<212> DNA
<213> Homo sapiens

<400> 1826
tgaaagtgga actgtctatg actgt                                          25

<210> 1827
<211> 25
<212> DNA
<213> Homo sapiens

<400> 1827
gaaagtggaa ctgtctatga ctgtg                                          25

<210> 1828
<211> 25
<212> DNA
<213> Homo sapiens

<400> 1828
aaagtggaac tgtctatgac tgtgt                                          25

<210> 1829
<211> 25
<212> DNA
<213> Homo sapiens

<400> 1829
aagtggaact gtctatgact gtgta                                          25

<210> 1830
<211> 25
<212> DNA
<213> Homo sapiens

<400> 1830
agtggaactg tctatgactg tgtaa                                          25

<210> 1831
<211> 25
<212> DNA
<213> Homo sapiens

<400> 1831
gtggaactgt ctatgactgt gtaaa                                          25

<210> 1832
<211> 25
<212> DNA
<213> Homo sapiens

<400> 1832
tggaactgtc tatgactgtg taaaa                                          25

<210> 1833
<211> 25
<212> DNA
<213> Homo sapiens

<400> 1833
ggaactgtct atgactgtgt aaaac                                          25

<210> 1834
<211> 25
<212> DNA
<213> Homo sapiens

<400> 1834
gaactgtcta tgactgtgta aaact                                          25

<210> 1835
<211> 25
<212> DNA
<213> Homo sapiens

<400> 1835
aactgtctat gactgtgtaa aacta                                          25

<210> 1836

<211> 25
<212> DNA
<213> Homo sapiens

<400> 1836
actgtctatg actgtgtaaa actaa                                          25

<210> 1837
<211> 25
<212> DNA
<213> Homo sapiens

<400> 1837
ctgtctatga ctgtgtaaaa ctaac                                          25

<210> 1838
<211> 25
<212> DNA
<213> Homo sapiens

<400> 1838
tgtctatgac tgtgtaaaac taact                                          25

<210> 1839
<211> 25
<212> DNA
<213> Homo sapiens

<400> 1839
gtctatgact gtgtaaaact aactt                                          25

<210> 1840
<211> 25
<212> DNA
<213> Homo sapiens

<400> 1840
tctatgactg tgtaaaacta acttt                                          25

<210> 1841
<211> 25
<212> DNA
<213> Homo sapiens

<400> 1841
ctatgactgt gtaaaactaa ctttc                                          25

<210> 1842
<211> 25
<212> DNA
<213> Homo sapiens

<400> 1842
tatgactgtg taaaactaac tttca                                          25

<210> 1843
<211> 25
<212> DNA
<213> Homo sapiens

<400> 1843

atgactgtgt aaaactaact ttcag 25

<210> 1844
<211> 25
<212> DNA
<213> Homo sapiens

<400> 1844
tgactgtgta aaactaactt tcagt 25

<210> 1845
<211> 25
<212> DNA
<213> Homo sapiens

<400> 1845
gactgtgtaa aactaacttt cagtc 25

<210> 1846
<211> 25
<212> DNA
<213> Homo sapiens

<400> 1846
actgtgtaaa actaactttc agtcc 25

<210> 1847
<211> 25
<212> DNA
<213> Homo sapiens

<400> 1847
ctgtgtaaaa ctaactttca gtcca 25

<210> 1848
<211> 25
<212> DNA
<213> Homo sapiens

<400> 1848
tgtgtaaaac taactttcag tccat 25

<210> 1849
<211> 25
<212> DNA
<213> Homo sapiens

<400> 1849
gtgtaaaact aactttcagt ccatc 25

<210> 1850
<211> 25
<212> DNA
<213> Homo sapiens

<400> 1850
tgtaaaacta actttcagtc catca 25

<210> 1851
<211> 25
<212> DNA

<213> Homo sapiens

<400> 1851
gtaaaactaa ctttcagtcc atcaa                                          25

<210> 1852
<211> 25
<212> DNA
<213> Homo sapiens

<400> 1852
taaaactaac tttcagtcca tcaac                                          25

<210> 1853
<211> 25
<212> DNA
<213> Homo sapiens

<400> 1853
aaaactaact ttcagtccat caact                                          25

<210> 1854
<211> 25
<212> DNA
<213> Homo sapiens

<400> 1854
aaactaactt tcagtccatc aactc                                          25

<210> 1855
<211> 25
<212> DNA
<213> Homo sapiens

<400> 1855
aactaacttt cagtccatca actct                                          25

<210> 1856
<211> 25
<212> DNA
<213> Homo sapiens

<400> 1856
actaactttc agtccatcaa ctctg                                          25

<210> 1857
<211> 25
<212> DNA
<213> Homo sapiens

<400> 1857
ctaactttca gtccatcaac tctgc                                          25

<210> 1858
<211> 25
<212> DNA
<213> Homo sapiens

<400> 1858
taactttcag tccatcaact ctgct                                          25

<210> 1859
<211> 25
<212> DNA
<213> Homo sapiens

<400> 1859
aactttcagt ccatcaactc tgctg                                          25

<210> 1860
<211> 25
<212> DNA
<213> Homo sapiens

<400> 1860
actttcagtc catcaactct gctgg                                          25

<210> 1861
<211> 25
<212> DNA
<213> Homo sapiens

<400> 1861
ctttcagtcc atcaactctg ctggt                                          25

<210> 1862
<211> 25
<212> DNA
<213> Homo sapiens

<400> 1862
tttcagtcca tcaactctgc tggtt                                          25

<210> 1863
<211> 25
<212> DNA
<213> Homo sapiens

<400> 1863
ttcagtccat caactctgct ggtta                                          25

<210> 1864
<211> 25
<212> DNA
<213> Homo sapiens

<400> 1864
tcagtccatc aactctgctg gttaa                                          25

<210> 1865
<211> 25
<212> DNA
<213> Homo sapiens

<400> 1865
cagtccatca actctgctgg ttaat                                          25

<210> 1866
<211> 25
<212> DNA
<213> Homo sapiens

<400> 1866
agtccatcaa ctctgctggt taata                                          25

<210> 1867
<211> 25
<212> DNA
<213> Homo sapiens

<400> 1867
gtccatcaac tctgctggtt aatat                                          25

<210> 1868
<211> 25
<212> DNA
<213> Homo sapiens

<400> 1868
tccatcaact ctgctggtta atatc                                          25

<210> 1869
<211> 25
<212> DNA
<213> Homo sapiens

<400> 1869
ccatcaactc tgctggttaa tatca                                          25

<210> 1870
<211> 25
<212> DNA
<213> Homo sapiens

<400> 1870
catcaactct gctggttaat atcac                                          25

<210> 1871
<211> 25
<212> DNA
<213> Homo sapiens

<400> 1871
atcaactctg ctggttaata tcact                                          25

<210> 1872
<211> 25
<212> DNA
<213> Homo sapiens

<400> 1872
tcaactctgc tggttaatat cactg                                          25

<210> 1873
<211> 25
<212> DNA
<213> Homo sapiens

<400> 1873
caactctgct ggttaatatc actga                                          25

<210> 1874
<211> 25

<212> DNA
<213> Homo sapiens

<400> 1874
aactctgctg gttaatatca ctgac                                          25

<210> 1875
<211> 25
<212> DNA
<213> Homo sapiens

<400> 1875
actctgctgg ttaatatcac tgacc                                          25

<210> 1876
<211> 25
<212> DNA
<213> Homo sapiens

<400> 1876
ctctgctggt taatatcact gacca                                          25

<210> 1877
<211> 25
<212> DNA
<213> Homo sapiens

<400> 1877
tctgctggtt aatatcactg accaa                                          25

<210> 1878
<211> 25
<212> DNA
<213> Homo sapiens

<400> 1878
ctgctggtta atatcactga ccaag                                          25

<210> 1879
<211> 25
<212> DNA
<213> Homo sapiens

<400> 1879
tgctggttaa tatcactgac caagt                                          25

<210> 1880
<211> 25
<212> DNA
<213> Homo sapiens

<400> 1880
gctggttaat atcactgacc aagtt                                          25

<210> 1881
<211> 25
<212> DNA
<213> Homo sapiens

<400> 1881
ctggttaata tcactgacca agttt                                          25

<210> 1882
<211> 25
<212> DNA
<213> Homo sapiens

<400> 1882
tggttaatat cactgaccaa gttta                                          25

<210> 1883
<211> 25
<212> DNA
<213> Homo sapiens

<400> 1883
ggttaatatc actgaccaag tttat                                          25

<210> 1884
<211> 25
<212> DNA
<213> Homo sapiens

<400> 1884
gttaatatca ctgaccaagt ttatg                                          25

<210> 1885
<211> 25
<212> DNA
<213> Homo sapiens

<400> 1885
ttaatatcac tgaccaagtt tatga                                          25

<210> 1886
<211> 25
<212> DNA
<213> Homo sapiens

<400> 1886
taatatcact gaccaagttt atgaa                                          25

<210> 1887
<211> 25
<212> DNA
<213> Homo sapiens

<400> 1887
aatatcactg accaagttta tgaat                                          25

<210> 1888
<211> 25
<212> DNA
<213> Homo sapiens

<400> 1888
atatcactga ccaagtttat gaata                                          25

<210> 1889
<211> 25
<212> DNA
<213> Homo sapiens

<400> 1889
tatcactgac caagtttatg aatat                                          25

<210> 1890
<211> 25
<212> DNA
<213> Homo sapiens

<400> 1890
atcactgacc aagtttatga atata                                          25

<210> 1891
<211> 25
<212> DNA
<213> Homo sapiens

<400> 1891
tcactgacca agtttatgaa tataa                                          25

<210> 1892
<211> 25
<212> DNA
<213> Homo sapiens

<400> 1892
cactgaccaa gtttatgaat ataaa                                          25

<210> 1893
<211> 25
<212> DNA
<213> Homo sapiens

<400> 1893
actgaccaag tttatgaata taaat                                          25

<210> 1894
<211> 25
<212> DNA
<213> Homo sapiens

<400> 1894
ctgaccaagt ttatgaatat aaata                                          25

<210> 1895
<211> 25
<212> DNA
<213> Homo sapiens

<400> 1895
tgaccaagtt tatgaatata aatac                                          25

<210> 1896
<211> 25
<212> DNA
<213> Homo sapiens

<400> 1896
gaccaagttt atgaatataa ataca                                          25

<210> 1897

<211> 25
<212> DNA
<213> Homo sapiens

<400> 1897
accaagttta tgaatataaa tacaa                                          25

<210> 1898
<211> 25
<212> DNA
<213> Homo sapiens

<400> 1898
ccaagtttat gaatataaat acaaa                                          25

<210> 1899
<211> 25
<212> DNA
<213> Homo sapiens

<400> 1899
caagtttatg aatataaata caaaa                                          25

<210> 1900
<211> 25
<212> DNA
<213> Homo sapiens

<400> 1900
aagtttatga atataaatac aaaag                                          25

<210> 1901
<211> 25
<212> DNA
<213> Homo sapiens

<400> 1901
agtttatgaa tataaataca aaaga                                          25

<210> 1902
<211> 25
<212> DNA
<213> Homo sapiens

<400> 1902
gtttatgaat ataaatacaa aagag                                          25

<210> 1903
<211> 25
<212> DNA
<213> Homo sapiens

<400> 1903
tttatgaata taaatacaaa agaga                                          25

<210> 1904
<211> 25
<212> DNA
<213> Homo sapiens

<400> 1904

ttatgaatat aaatacaaaa gagaa 25

<210> 1905
<211> 25
<212> DNA
<213> Homo sapiens

<400> 1905
tatgaatata aatacaaaag agaaa 25

<210> 1906
<211> 25
<212> DNA
<213> Homo sapiens

<400> 1906
atgaatataa atacaaaaga gaaat 25

<210> 1907
<211> 25
<212> DNA
<213> Homo sapiens

<400> 1907
tgaatataaa tacaaaagag aaata 25

<210> 1908
<211> 25
<212> DNA
<213> Homo sapiens

<400> 1908
gaatataaat acaaaagaga aataa 25

<210> 1909
<211> 25
<212> DNA
<213> Homo sapiens

<400> 1909
aatataaata caaaagagaa ataag 25

<210> 1910
<211> 25
<212> DNA
<213> Homo sapiens

<400> 1910
atataaatac aaaagagaaa taagt 25

<210> 1911
<211> 25
<212> DNA
<213> Homo sapiens

<400> 1911
tataaataca aaagagaaat aagtc 25

<210> 1912
<211> 25
<212> DNA

<213> Homo sapiens

<400> 1912
ataaatacaa aagagaaata agtca                                          25

<210> 1913
<211> 25
<212> DNA
<213> Homo sapiens

<400> 1913
taaatacaaa agagaaataa gtcag                                          25

<210> 1914
<211> 25
<212> DNA
<213> Homo sapiens

<400> 1914
aaatacaaaa gagaaataag tcagc                                          25

<210> 1915
<211> 25
<212> DNA
<213> Homo sapiens

<400> 1915
aatacaaaag agaaataagt cagca                                          25

<210> 1916
<211> 25
<212> DNA
<213> Homo sapiens

<400> 1916
atacaaaaga gaaataagtc agcac                                          25

<210> 1917
<211> 25
<212> DNA
<213> Homo sapiens

<400> 1917
tacaaaagag aaataagtca gcaca                                          25

<210> 1918
<211> 25
<212> DNA
<213> Homo sapiens

<400> 1918
acaaaagaga aataagtcag cacaa                                          25

<210> 1919
<211> 25
<212> DNA
<213> Homo sapiens

<400> 1919
caaaagagaa ataagtcagc acaac                                          25

<210> 1920
<211> 25
<212> DNA
<213> Homo sapiens

<400> 1920
aaaagagaaa taagtcagca caaca                                          25

<210> 1921
<211> 25
<212> DNA
<213> Homo sapiens

<400> 1921
aaagagaaat aagtcagcac aacat                                          25

<210> 1922
<211> 25
<212> DNA
<213> Homo sapiens

<400> 1922
aagagaaata agtcagcaca acatc                                          25

<210> 1923
<211> 25
<212> DNA
<213> Homo sapiens

<400> 1923
agagaaataa gtcagcacaa catca                                          25

<210> 1924
<211> 25
<212> DNA
<213> Homo sapiens

<400> 1924
gagaaataag tcagcacaac atcaa                                          25

<210> 1925
<211> 25
<212> DNA
<213> Homo sapiens

<400> 1925
agaaataagt cagcacaaca tcaat                                          25

<210> 1926
<211> 25
<212> DNA
<213> Homo sapiens

<400> 1926
gaaataagtc agcacaacat caatc                                          25

<210> 1927
<211> 25
<212> DNA
<213> Homo sapiens

<400> 1927
aaataagtca gcacaacatc aatcc                                          25

<210> 1928
<211> 25
<212> DNA
<213> Homo sapiens

<400> 1928
aataagtcag cacaacatca atcct                                          25

<210> 1929
<211> 25
<212> DNA
<213> Homo sapiens

<400> 1929
ataagtcagc acaacatcaa tcctc                                          25

<210> 1930
<211> 25
<212> DNA
<213> Homo sapiens

<400> 1930
taagtcagca caacatcaat cctca                                          25

<210> 1931
<211> 25
<212> DNA
<213> Homo sapiens

<400> 1931
aagtcagcac aacatcaatc ctcat                                          25

<210> 1932
<211> 25
<212> DNA
<213> Homo sapiens

<400> 1932
agtcagcaca acatcaatcc tcatc                                          25

<210> 1933
<211> 25
<212> DNA
<213> Homo sapiens

<400> 1933
gtcagcacaa catcaatcct catca                                          25

<210> 1934
<211> 25
<212> DNA
<213> Homo sapiens

<400> 1934
tcagcacaac atcaatcctc atcaa                                          25

<210> 1935
<211> 25

<212> DNA
<213> Homo sapiens

<400> 1935
cagcacaaca tcaatcctca tcaag                                            25

<210> 1936
<211> 25
<212> DNA
<213> Homo sapiens

<400> 1936
agcacaacat caatcctcat caagg                                            25

<210> 1937
<211> 25
<212> DNA
<213> Homo sapiens

<400> 1937
gcacaacatc aatcctcatc aagga                                            25

<210> 1938
<211> 25
<212> DNA
<213> Homo sapiens

<400> 1938
cacaacatca atcctcatca aggaa                                            25

<210> 1939
<211> 25
<212> DNA
<213> Homo sapiens

<400> 1939
acaacatcaa tcctcatcaa ggaaa                                            25

<210> 1940
<211> 25
<212> DNA
<213> Homo sapiens

<400> 1940
caacatcaat cctcatcaag gaaat                                            25

<210> 1941
<211> 25
<212> DNA
<213> Homo sapiens

<400> 1941
aacatcaatc ctcatcaagg aaatg                                            25

<210> 1942
<211> 25
<212> DNA
<213> Homo sapiens

<400> 1942
acatcaatcc tcatcaagga aatgc                                            25

<210> 1943
<211> 25
<212> DNA
<213> Homo sapiens

<400> 1943
catcaatcct catcaaggaa atgct                                          25

<210> 1944
<211> 25
<212> DNA
<213> Homo sapiens

<400> 1944
atcaatcctc atcaaggaaa tgcta                                          25

<210> 1945
<211> 25
<212> DNA
<213> Homo sapiens

<400> 1945
tcaatcctca tcaaggaaat gctat                                          25

<210> 1946
<211> 25
<212> DNA
<213> Homo sapiens

<400> 1946
caatcctcat caaggaaatg ctata                                          25

<210> 1947
<211> 25
<212> DNA
<213> Homo sapiens

<400> 1947
aatcctcatc aaggaaatgc tatac                                          25

<210> 1948
<211> 25
<212> DNA
<213> Homo sapiens

<400> 1948
atcctcatca aggaaatgct atact                                          25

<210> 1949
<211> 25
<212> DNA
<213> Homo sapiens

<400> 1949
tcctcatcaa ggaaatgcta tactt                                          25

<210> 1950
<211> 25
<212> DNA
<213> Homo sapiens

<400> 1950
cctcatcaag gaaatgctat acttg                                          25

<210> 1951
<211> 25
<212> DNA
<213> Homo sapiens

<400> 1951
ctcatcaagg aaatgctata cttga                                          25

<210> 1952
<211> 25
<212> DNA
<213> Homo sapiens

<400> 1952
tcatcaagga aatgctatac ttgaa                                          25

<210> 1953
<211> 25
<212> DNA
<213> Homo sapiens

<400> 1953
catcaaggaa atgctatact tgaaa                                          25

<210> 1954
<211> 25
<212> DNA
<213> Homo sapiens

<400> 1954
atcaaggaaa tgctatactt gaaaa                                          25

<210> 1955
<211> 25
<212> DNA
<213> Homo sapiens

<400> 1955
tcaaggaaat gctatacttg aaaag                                          25

<210> 1956
<211> 25
<212> DNA
<213> Homo sapiens

<400> 1956
caaggaaatg ctatacttga aaaga                                          25

<210> 1957
<211> 25
<212> DNA
<213> Homo sapiens

<400> 1957
aaggaaatgc tatacttgaa aagat                                          25

<210> 1958

<211> 25
<212> DNA
<213> Homo sapiens

<400> 1958
aggaaatgct atacttgaaa agatg                                          25

<210> 1959
<211> 25
<212> DNA
<213> Homo sapiens

<400> 1959
ggaaatgcta tacttgaaaa gatga                                          25

<210> 1960
<211> 25
<212> DNA
<213> Homo sapiens

<400> 1960
gaaatgctat acttgaaaag atgac                                          25

<210> 1961
<211> 25
<212> DNA
<213> Homo sapiens

<400> 1961
aaatgctata cttgaaaaga tgaca                                          25

<210> 1962
<211> 25
<212> DNA
<213> Homo sapiens

<400> 1962
aatgctatac ttgaaaagat gacat                                          25

<210> 1963
<211> 25
<212> DNA
<213> Homo sapiens

<400> 1963
atgctatact tgaaaagatg acatt                                          25

<210> 1964
<211> 25
<212> DNA
<213> Homo sapiens

<400> 1964
tgctatactt gaaaagatga cattt                                          25

<210> 1965
<211> 25
<212> DNA
<213> Homo sapiens

<400> 1965

gctatacttg aaaagatgac atttg                                          25

<210> 1966
<211> 25
<212> DNA
<213> Homo sapiens

<400> 1966
ctatacttga aaagatgaca tttga                                          25

<210> 1967
<211> 25
<212> DNA
<213> Homo sapiens

<400> 1967
tatacttgaa aagatgacat ttgat                                          25

<210> 1968
<211> 25
<212> DNA
<213> Homo sapiens

<400> 1968
atacttgaaa agatgacatt tgatc                                          25

<210> 1969
<211> 25
<212> DNA
<213> Homo sapiens

<400> 1969
tacttgaaaa gatgacattt gatcc                                          25

<210> 1970
<211> 25
<212> DNA
<213> Homo sapiens

<400> 1970
acttgaaaag atgacatttg atcca                                          25

<210> 1971
<211> 25
<212> DNA
<213> Homo sapiens

<400> 1971
cttgaaaaga tgacatttga tccag                                          25

<210> 1972
<211> 25
<212> DNA
<213> Homo sapiens

<400> 1972
ttgaaaagat gacatttgat ccaga                                          25

<210> 1973
<211> 25
<212> DNA

<213> Homo sapiens

<400> 1973
tgaaaagatg acatttgatc cagaa                                          25

<210> 1974
<211> 25
<212> DNA
<213> Homo sapiens

<400> 1974
gaaaagatga catttgatcc agaaa                                          25

<210> 1975
<211> 25
<212> DNA
<213> Homo sapiens

<400> 1975
aaaagatgac atttgatcca gaaat                                          25

<210> 1976
<211> 25
<212> DNA
<213> Homo sapiens

<400> 1976
aaagatgaca tttgatccag aaatc                                          25

<210> 1977
<211> 25
<212> DNA
<213> Homo sapiens

<400> 1977
aagatgacat ttgatccaga aatct                                          25

<210> 1978
<211> 25
<212> DNA
<213> Homo sapiens

<400> 1978
agatgacatt tgatccagaa atctt                                          25

<210> 1979
<211> 25
<212> DNA
<213> Homo sapiens

<400> 1979
gatgacattt gatccagaaa tcttc                                          25

<210> 1980
<211> 25
<212> DNA
<213> Homo sapiens

<400> 1980
atgacatttg atccagaaat cttct                                          25

<210> 1981
<211> 25
<212> DNA
<213> Homo sapiens

<400> 1981
tgacatttga tccagaaatc ttctt                                             25

<210> 1982
<211> 25
<212> DNA
<213> Homo sapiens

<400> 1982
gacatttgat ccagaaatct tcttc                                             25

<210> 1983
<211> 25
<212> DNA
<213> Homo sapiens

<400> 1983
acatttgatc cagaaatctt cttca                                             25

<210> 1984
<211> 25
<212> DNA
<213> Homo sapiens

<400> 1984
catttgatcc agaaatcttc ttcaa                                             25

<210> 1985
<211> 25
<212> DNA
<213> Homo sapiens

<400> 1985
atttgatcca gaaatcttct tcaat                                             25

<210> 1986
<211> 25
<212> DNA
<213> Homo sapiens

<400> 1986
tttgatccag aaatcttctt caatg                                             25

<210> 1987
<211> 25
<212> DNA
<213> Homo sapiens

<400> 1987
ttgatccaga aatcttcttc aatgt                                             25

<210> 1988
<211> 25
<212> DNA
<213> Homo sapiens

<400> 1988
tgatccagaa atcttcttca atgtt                                          25

<210> 1989
<211> 25
<212> DNA
<213> Homo sapiens

<400> 1989
gatccagaaa tcttcttcaa tgttt                                          25

<210> 1990
<211> 25
<212> DNA
<213> Homo sapiens

<400> 1990
atccagaaat cttcttcaat gtttt                                          25

<210> 1991
<211> 25
<212> DNA
<213> Homo sapiens

<400> 1991
tccagaaatc ttcttcaatg tttta                                          25

<210> 1992
<211> 25
<212> DNA
<213> Homo sapiens

<400> 1992
ccagaaatct tcttcaatgt tttac                                          25

<210> 1993
<211> 25
<212> DNA
<213> Homo sapiens

<400> 1993
cagaaatctt cttcaatgtt ttact                                          25

<210> 1994
<211> 25
<212> DNA
<213> Homo sapiens

<400> 1994
agaaatcttc ttcaatgttt tactg                                          25

<210> 1995
<211> 25
<212> DNA
<213> Homo sapiens

<400> 1995
gaaatcttct tcaatgtttt actgc                                          25

<210> 1996
<211> 25

<212> DNA
<213> Homo sapiens

<400> 1996
aaatcttctt caatgtttta ctgcc                                          25

<210> 1997
<211> 25
<212> DNA
<213> Homo sapiens

<400> 1997
aatcttcttc aatgttttac tgcca                                          25

<210> 1998
<211> 25
<212> DNA
<213> Homo sapiens

<400> 1998
atcttcttca atgttttact gccac                                          25

<210> 1999
<211> 25
<212> DNA
<213> Homo sapiens

<400> 1999
tcttcttcaa tgttttactg ccacc                                          25

<210> 2000
<211> 25
<212> DNA
<213> Homo sapiens

<400> 2000
cttcttcaat gttttactgc cacca                                          25

<210> 2001
<211> 25
<212> DNA
<213> Homo sapiens

<400> 2001
ttcttcaatg ttttactgcc accaa                                          25

<210> 2002
<211> 25
<212> DNA
<213> Homo sapiens

<400> 2002
tcttcaatgt tttactgcca ccaat                                          25

<210> 2003
<211> 25
<212> DNA
<213> Homo sapiens

<400> 2003
cttcaatgtt ttactgccac caatt                                          25

<210> 2004
<211> 25
<212> DNA
<213> Homo sapiens

<400> 2004
ttcaatgttt tactgccacc aatta                                          25

<210> 2005
<211> 25
<212> DNA
<213> Homo sapiens

<400> 2005
tcaatgtttt actgccacca attat                                          25

<210> 2006
<211> 25
<212> DNA
<213> Homo sapiens

<400> 2006
caatgtttta ctgccaccaa ttata                                          25

<210> 2007
<211> 25
<212> DNA
<213> Homo sapiens

<400> 2007
aatgttttac tgccaccaat tatat                                          25

<210> 2008
<211> 25
<212> DNA
<213> Homo sapiens

<400> 2008
atgttttact gccaccaatt atatt                                          25

<210> 2009
<211> 25
<212> DNA
<213> Homo sapiens

<400> 2009
tgttttactg ccaccaatta tattt                                          25

<210> 2010
<211> 25
<212> DNA
<213> Homo sapiens

<400> 2010
gttttactgc caccaattat atttc                                          25

<210> 2011
<211> 25
<212> DNA
<213> Homo sapiens

<400> 2011
ttttactgcc accaattata tttca                                          25

<210> 2012
<211> 25
<212> DNA
<213> Homo sapiens

<400> 2012
tttactgcca ccaattatat ttcat                                          25

<210> 2013
<211> 25
<212> DNA
<213> Homo sapiens

<400> 2013
ttactgccac caattatatt tcatg                                          25

<210> 2014
<211> 25
<212> DNA
<213> Homo sapiens

<400> 2014
tactgccacc aattatattt catgc                                          25

<210> 2015
<211> 25
<212> DNA
<213> Homo sapiens

<400> 2015
actgccacca attatatttc atgca                                          25

<210> 2016
<211> 25
<212> DNA
<213> Homo sapiens

<400> 2016
ctgccaccaa ttatatttca tgcag                                          25

<210> 2017
<211> 25
<212> DNA
<213> Homo sapiens

<400> 2017
tgccaccaat tatatttcat gcagg                                          25

<210> 2018
<211> 25
<212> DNA
<213> Homo sapiens

<400> 2018
gccaccaatt atatttcatg cagga                                          25

<210> 2019

<211> 25
<212> DNA
<213> Homo sapiens

<400> 2019
ccaccaatta tatttcatgc aggat                                          25

<210> 2020
<211> 25
<212> DNA
<213> Homo sapiens

<400> 2020
caccaattat atttcatgca ggata                                          25

<210> 2021
<211> 25
<212> DNA
<213> Homo sapiens

<400> 2021
accaattata tttcatgcag gatat                                          25

<210> 2022
<211> 25
<212> DNA
<213> Homo sapiens

<400> 2022
ccaattatat ttcatgcagg atata                                          25

<210> 2023
<211> 25
<212> DNA
<213> Homo sapiens

<400> 2023
caattatatt tcatgcagga tatag                                          25

<210> 2024
<211> 25
<212> DNA
<213> Homo sapiens

<400> 2024
aattatattt catgcaggat atagt                                          25

<210> 2025
<211> 25
<212> DNA
<213> Homo sapiens

<400> 2025
attatatttc atgcaggata tagtc                                          25

<210> 2026
<211> 25
<212> DNA
<213> Homo sapiens

<400> 2026

ttatatttca tgcaggatat agtct                                          25

<210> 2027
<211> 25
<212> DNA
<213> Homo sapiens

<400> 2027
tatatttcat gcaggatata gtcta                                          25

<210> 2028
<211> 25
<212> DNA
<213> Homo sapiens

<400> 2028
atatttcatg caggatatag tctaa                                          25

<210> 2029
<211> 25
<212> DNA
<213> Homo sapiens

<400> 2029
tatttcatgc aggatatagt ctaaa                                          25

<210> 2030
<211> 25
<212> DNA
<213> Homo sapiens

<400> 2030
atttcatgca ggatatagtc taaag                                          25

<210> 2031
<211> 25
<212> DNA
<213> Homo sapiens

<400> 2031
tttcatgcag gatatagtct aaaga                                          25

<210> 2032
<211> 25
<212> DNA
<213> Homo sapiens

<400> 2032
ttcatgcagg atatagtcta aagaa                                          25

<210> 2033
<211> 25
<212> DNA
<213> Homo sapiens

<400> 2033
tcatgcagga tatagtctaa agaag                                          25

<210> 2034
<211> 25
<212> DNA

<213> Homo sapiens

<400> 2034
catgcaggat atagtctaaa gaaga                                            25

<210> 2035
<211> 25
<212> DNA
<213> Homo sapiens

<400> 2035
atgcaggata tagtctaaag aagag                                            25

<210> 2036
<211> 25
<212> DNA
<213> Homo sapiens

<400> 2036
tgcaggatat agtctaaaga agaga                                            25

<210> 2037
<211> 25
<212> DNA
<213> Homo sapiens

<400> 2037
gcaggatata gtctaaagaa gagac                                            25

<210> 2038
<211> 25
<212> DNA
<213> Homo sapiens

<400> 2038
caggatatag tctaaagaag agaca                                            25

<210> 2039
<211> 25
<212> DNA
<213> Homo sapiens

<400> 2039
aggatatagt ctaaagaaga gacac                                            25

<210> 2040
<211> 25
<212> DNA
<213> Homo sapiens

<400> 2040
ggatatagtc taaagaagag acact                                            25

<210> 2041
<211> 25
<212> DNA
<213> Homo sapiens

<400> 2041
gatatagtct aaagaagaga cactt                                            25

<210> 2042
<211> 25
<212> DNA
<213> Homo sapiens

<400> 2042
atatagtcta aagaagagac acttt                                          25

<210> 2043
<211> 25
<212> DNA
<213> Homo sapiens

<400> 2043
tatagtctaa agaagagaca ctttt                                          25

<210> 2044
<211> 25
<212> DNA
<213> Homo sapiens

<400> 2044
atagtctaaa gaagagacac ttttt                                          25

<210> 2045
<211> 25
<212> DNA
<213> Homo sapiens

<400> 2045
tagtctaaag aagagacact ttttt                                          25

<210> 2046
<211> 25
<212> DNA
<213> Homo sapiens

<400> 2046
agtctaaaga agagacactt ttttc                                          25

<210> 2047
<211> 25
<212> DNA
<213> Homo sapiens

<400> 2047
gtctaaagaa gagacacttt tttca                                          25

<210> 2048
<211> 25
<212> DNA
<213> Homo sapiens

<400> 2048
tctaaagaag agacactttt ttcaa                                          25

<210> 2049
<211> 25
<212> DNA
<213> Homo sapiens

<400> 2049
ctaaagaaga gacacttttt tcaaa                                          25

<210> 2050
<211> 25
<212> DNA
<213> Homo sapiens

<400> 2050
taaagaagag acactttttt caaaa                                          25

<210> 2051
<211> 25
<212> DNA
<213> Homo sapiens

<400> 2051
aaagaagaga cacttttttc aaaac                                          25

<210> 2052
<211> 25
<212> DNA
<213> Homo sapiens

<400> 2052
aagaagagac acttttttca aaact                                          25

<210> 2053
<211> 25
<212> DNA
<213> Homo sapiens

<400> 2053
agaagagaca cttttttcaa aactt                                          25

<210> 2054
<211> 25
<212> DNA
<213> Homo sapiens

<400> 2054
gaagagacac ttttttcaaa actta                                          25

<210> 2055
<211> 25
<212> DNA
<213> Homo sapiens

<400> 2055
aagagacact tttttcaaaa cttag                                          25

<210> 2056
<211> 25
<212> DNA
<213> Homo sapiens

<400> 2056
agagacactt ttttcaaaac ttagg                                          25

<210> 2057
<211> 25

<212> DNA
<213> Homo sapiens

<400> 2057
gagacacttt tttcaaaact tagga                                            25

<210> 2058
<211> 25
<212> DNA
<213> Homo sapiens

<400> 2058
agacactttt ttcaaaactt aggat                                            25

<210> 2059
<211> 25
<212> DNA
<213> Homo sapiens

<400> 2059
gacacttttt tcaaaactta ggatc                                            25

<210> 2060
<211> 25
<212> DNA
<213> Homo sapiens

<400> 2060
acactttttt caaaacttag gatct                                            25

<210> 2061
<211> 25
<212> DNA
<213> Homo sapiens

<400> 2061
   cacttttttc aaaacttagg atcta                                             25

<210> 2062
<211> 25
<212> DNA
<213> Homo sapiens

<400> 2062
   acttttttca aaacttagga tctat                                             25

<210> 2063
<211> 25
<212> DNA
<213> Homo sapiens

<400> 2063
   cttttttcaa aacttaggat ctatt                                             25

<210> 2064
<211> 25
<212> DNA
<213> Homo sapiens

<400> 2064
   ttttttcaaa acttaggatc tattt                                             25

<210> 2065
<211> 25
<212> DNA
<213> Homo sapiens

<400> 2065
tttttcaaaa cttaggatct atttt                                          25

<210> 2066
<211> 25
<212> DNA
<213> Homo sapiens

<400> 2066
ttttcaaaac ttaggatcta tttta                                          25

<210> 2067
<211> 25
<212> DNA
<213> Homo sapiens

<400> 2067
tttcaaaact taggatctat tttaa                                          25

<210> 2068
<211> 25
<212> DNA
<213> Homo sapiens

<400> 2068
ttcaaaactt aggatctatt ttaac                                          25

<210> 2069
<211> 25
<212> DNA
<213> Homo sapiens

<400> 2069
tcaaaactta ggatctattt taacg                                          25

<210> 2070
<211> 25
<212> DNA
<213> Homo sapiens

<400> 2070
caaaacttag gatctatttt aacgt                                          25

<210> 2071
<211> 25
<212> DNA
<213> Homo sapiens

<400> 2071
aaaacttagg atctatttta acgta                                          25

<210> 2072
<211> 25
<212> DNA
<213> Homo sapiens

<400> 2072
aaacttagga tctattttaa cgtat                                          25

<210> 2073
<211> 25
<212> DNA
<213> Homo sapiens

<400> 2073
aacttaggat ctattttaac gtatg                                          25

<210> 2074
<211> 25
<212> DNA
<213> Homo sapiens

<400> 2074
acttaggatc tattttaacg tatgc                                          25

<210> 2075
<211> 25
<212> DNA
<213> Homo sapiens

<400> 2075
cttaggatct attttaacgt atgcc                                          25

<210> 2076
<211> 25
<212> DNA
<213> Homo sapiens

<400> 2076
ttaggatcta ttttaacgta tgcct                                          25

<210> 2077
<211> 25
<212> DNA
<213> Homo sapiens

<400> 2077
taggatctat tttaacgtat gcctt                                          25

<210> 2078
<211> 25
<212> DNA
<213> Homo sapiens

<400> 2078
aggatctatt ttaacgtatg ccttc                                          25

<210> 2079
<211> 25
<212> DNA
<213> Homo sapiens

<400> 2079
ggatctattt taacgtatgc cttct                                          25

<210> 2080

<211> 25
<212> DNA
<213> Homo sapiens

<400> 2080
gatctatttt aacgtatgcc ttctt                                            25

<210> 2081
<211> 25
<212> DNA
<213> Homo sapiens

<400> 2081
atctatttta acgtatgcct tcttg                                            25

<210> 2082
<211> 25
<212> DNA
<213> Homo sapiens

<400> 2082
tctattttaa cgtatgcctt cttgg                                            25

<210> 2083
<211> 25
<212> DNA
<213> Homo sapiens

<400> 2083
ctattttaac gtatgccttc ttggg                                            25

<210> 2084
<211> 25
<212> DNA
<213> Homo sapiens

<400> 2084
tattttaacg tatgccttct tggga                                            25

<210> 2085
<211> 25
<212> DNA
<213> Homo sapiens

<400> 2085
attttaacgt atgccttctt gggaa                                            25

<210> 2086
<211> 25
<212> DNA
<213> Homo sapiens

<400> 2086
ttttaacgta tgccttcttg ggaac                                            25

<210> 2087
<211> 25
<212> DNA
<213> Homo sapiens

<400> 2087

tttaacgtat gccttcttgg gaact                                        25

<210> 2088
<211> 25
<212> DNA
<213> Homo sapiens

<400> 2088
ttaacgtatg ccttcttggg aactg                                        25

<210> 2089
<211> 25
<212> DNA
<213> Homo sapiens

<400> 2089
taacgtatgc cttcttggga actgc                                        25

<210> 2090
<211> 25
<212> DNA
<213> Homo sapiens

<400> 2090
aacgtatgcc ttcttgggaa ctgcc                                        25

<210> 2091
<211> 25
<212> DNA
<213> Homo sapiens

<400> 2091
acgtatgcct tcttgggaac tgcca                                        25

<210> 2092
<211> 25
<212> DNA
<213> Homo sapiens

<400> 2092
cgtatgcctt cttgggaact gccat                                        25

<210> 2093
<211> 25
<212> DNA
<213> Homo sapiens

<400> 2093
gtatgccttc ttgggaactg ccatc                                        25

<210> 2094
<211> 25
<212> DNA
<213> Homo sapiens

<400> 2094
tatgccttct tgggaactgc catct                                        25

<210> 2095
<211> 25
<212> DNA

<213> Homo sapiens

<400> 2095
atgccttctt gggaactgcc atctc                                          25

<210> 2096
<211> 25
<212> DNA
<213> Homo sapiens

<400> 2096
tgccttcttg ggaactgcca tctcc                                          25

<210> 2097
<211> 25
<212> DNA
<213> Homo sapiens

<400> 2097
gccttcttgg gaactgccat ctcct                                          25

<210> 2098
<211> 25
<212> DNA
<213> Homo sapiens

<400> 2098
ccttcttggg aactgccatc tcctg                                          25

<210> 2099
<211> 25
<212> DNA
<213> Homo sapiens

<400> 2099
cttcttggga actgccatct cctgc                                          25

<210> 2100
<211> 25
<212> DNA
<213> Homo sapiens

<400> 2100
ttcttgggaa ctgccatctc ctgca                                          25

<210> 2101
<211> 25
<212> DNA
<213> Homo sapiens

<400> 2101
tcttgggaac tgccatctcc tgcat                                          25

<210> 2102
<211> 25
<212> DNA
<213> Homo sapiens

<400> 2102
cttgggaact gccatctcct gcatc                                          25

<210> 2103
<211> 25
<212> DNA
<213> Homo sapiens

<400> 2103
ttgggaactg ccatctcctg catcg                                          25

<210> 2104
<211> 25
<212> DNA
<213> Homo sapiens

<400> 2104
tgggaactgc catctcctgc atcgt                                          25

<210> 2105
<211> 25
<212> DNA
<213> Homo sapiens

<400> 2105
gggaactgcc atctcctgca tcgtc                                          25

<210> 2106
<211> 25
<212> DNA
<213> Homo sapiens

<400> 2106
ggaactgcca tctcctgcat cgtca                                          25

<210> 2107
<211> 25
<212> DNA
<213> Homo sapiens

<400> 2107
gaactgccat ctcctgcatc gtcat                                          25

<210> 2108
<211> 25
<212> DNA
<213> Homo sapiens

<400> 2108
aactgccatc tcctgcatcg tcata                                          25

<210> 2109
<211> 25
<212> DNA
<213> Homo sapiens

<400> 2109
actgccatct cctgcatcgt catag                                          25

<210> 2110
<211> 25
<212> DNA
<213> Homo sapiens

<400> 2110
ctgccatctc ctgcatcgtc atagg                                          25

<210> 2111
<211> 25
<212> DNA
<213> Homo sapiens

<400> 2111
tgccatctcc tgcatcgtca taggg                                          25

<210> 2112
<211> 25
<212> DNA
<213> Homo sapiens

<400> 2112
gccatctcct gcatcgtcat agggt                                          25

<210> 2113
<211> 25
<212> DNA
<213> Homo sapiens

<400> 2113
ccatctcctg catcgtcata gggtt                                          25

<210> 2114
<211> 25
<212> DNA
<213> Homo sapiens

<400> 2114
catctcctgc atcgtcatag ggtta                                          25

<210> 2115
<211> 25
<212> DNA
<213> Homo sapiens

<400> 2115
atctcctgca tcgtcatagg gttaa                                          25

<210> 2116
<211> 25
<212> DNA
<213> Homo sapiens

<400> 2116
tctcctgcat cgtcataggg ttaat                                          25

<210> 2117
<211> 25
<212> DNA
<213> Homo sapiens

<400> 2117
ctcctgcatc gtcatagggt taatt                                          25

<210> 2118
<211> 25

<212> DNA
<213> Homo sapiens

<400> 2118
tcctgcatcg tcatagggtt aatta                                            25

<210> 2119
<211> 25
<212> DNA
<213> Homo sapiens

<400> 2119
cctgcatcgt catagggtta attat                                            25

<210> 2120
<211> 25
<212> DNA
<213> Homo sapiens

<400> 2120
ctgcatcgtc atagggttaa ttatg                                            25

<210> 2121
<211> 25
<212> DNA
<213> Homo sapiens

<400> 2121
tgcatcgtca tagggttaat tatgt                                            25

<210> 2122
<211> 25
<212> DNA
<213> Homo sapiens

<400> 2122
gcatcgtcat agggttaatt atgta                                            25

<210> 2123
<211> 25
<212> DNA
<213> Homo sapiens

<400> 2123
catcgtcata gggttaatta tgtat                                            25

<210> 2124
<211> 25
<212> DNA
<213> Homo sapiens

<400> 2124
atcgtcatag ggttaattat gtatg                                            25

<210> 2125
<211> 25
<212> DNA
<213> Homo sapiens

<400> 2125
tcgtcatagg gttaattatg tatgg                                            25

<210> 2126
<211> 25
<212> DNA
<213> Homo sapiens

<400> 2126
cgtcataggg ttaattatgt atggt                                          25

<210> 2127
<211> 25
<212> DNA
<213> Homo sapiens

<400> 2127
gtcatagggt taattatgta tggtt                                          25

<210> 2128
<211> 25
<212> DNA
<213> Homo sapiens

<400> 2128
tcatagggtt aattatgtat ggttt                                          25

<210> 2129
<211> 25
<212> DNA
<213> Homo sapiens

<400> 2129
catagggtta attatgtatg gtttt                                          25

<210> 2130
<211> 25
<212> DNA
<213> Homo sapiens

<400> 2130
atagggttaa ttatgtatgg ttttg                                          25

<210> 2131
<211> 25
<212> DNA
<213> Homo sapiens

<400> 2131
tagggttaat tatgtatggt tttgt                                          25

<210> 2132
<211> 25
<212> DNA
<213> Homo sapiens

<400> 2132
agggttaatt atgtatggtt ttgtg                                          25

<210> 2133
<211> 25
<212> DNA
<213> Homo sapiens

<400> 2133
gggttaatta tgtatggttt tgtga                                          25

<210> 2134
<211> 25
<212> DNA
<213> Homo sapiens

<400> 2134
ggttaattat gtatggtttt gtgaa                                          25

<210> 2135
<211> 25
<212> DNA
<213> Homo sapiens

<400> 2135
gttaattatg tatggttttg tgaag                                          25

<210> 2136
<211> 25
<212> DNA
<213> Homo sapiens

<400> 2136
ttaattatgt atggttttgt gaagg                                          25

<210> 2137
<211> 25
<212> DNA
<213> Homo sapiens

<400> 2137
taattatgta tggttttgtg aaggc                                          25

<210> 2138
<211> 25
<212> DNA
<213> Homo sapiens

<400> 2138
aattatgtat ggttttgtga aggct                                          25

<210> 2139
<211> 25
<212> DNA
<213> Homo sapiens

<400> 2139
attatgtatg gttttgtgaa ggcta                                          25

<210> 2140
<211> 25
<212> DNA
<213> Homo sapiens

<400> 2140
ttatgtatgg ttttgtgaag gctat                                          25

<210> 2141

<211> 25
<212> DNA
<213> Homo sapiens

<400> 2141
tatgtatggt tttgtgaagg ctatg                                             25

<210> 2142
<211> 25
<212> DNA
<213> Homo sapiens

<400> 2142
atgtatggtt ttgtgaaggc tatga                                             25

<210> 2143
<211> 25
<212> DNA
<213> Homo sapiens

<400> 2143
tgtatggttt tgtgaaggct atgat                                             25

<210> 2144
<211> 25
<212> DNA
<213> Homo sapiens

<400> 2144
gtatggtttt gtgaaggcta tgata                                             25

<210> 2145
<211> 25
<212> DNA
<213> Homo sapiens

<400> 2145
tatggttttg tgaaggctat gatac                                             25

<210> 2146
<211> 25
<212> DNA
<213> Homo sapiens

<400> 2146
atggttttgt gaaggctatg ataca                                             25

<210> 2147
<211> 25
<212> DNA
<213> Homo sapiens

<400> 2147
tggttttgtg aaggctatga tacat                                             25

<210> 2148
<211> 25
<212> DNA
<213> Homo sapiens

<400> 2148

ggttttgtga aggctatgat acatg                                          25

<210> 2149
<211> 25
<212> DNA
<213> Homo sapiens

<400> 2149
gttttgtgaa ggctatgata catgc                                          25

<210> 2150
<211> 25
<212> DNA
<213> Homo sapiens

<400> 2150
ttttgtgaag gctatgatac atgct                                          25

<210> 2151
<211> 25
<212> DNA
<213> Homo sapiens

<400> 2151
tttgtgaagg ctatgataca tgctg                                          25

<210> 2152
<211> 25
<212> DNA
<213> Homo sapiens

<400> 2152
ttgtgaaggc tatgatacat gctgg                                          25

<210> 2153
<211> 25
<212> DNA
<213> Homo sapiens

<400> 2153
tgtgaaggct atgatacatg ctggc                                          25

<210> 2154
<211> 25
<212> DNA
<213> Homo sapiens

<400> 2154
gtgaaggcta tgatacatgc tggcc                                          25

<210> 2155
<211> 25
<212> DNA
<213> Homo sapiens

<400> 2155
tgaaggctat gatacatgct ggcca                                          25

<210> 2156
<211> 25
<212> DNA

<213> Homo sapiens

<400> 2156
gaaggctatg atacatgctg gccag                                          25

<210> 2157
<211> 25
<212> DNA
<213> Homo sapiens

<400> 2157
aaggctatga tacatgctgg ccagc                                          25

<210> 2158
<211> 25
<212> DNA
<213> Homo sapiens

<400> 2158
aggctatgat acatgctggc cagct                                          25

<210> 2159
<211> 25
<212> DNA
<213> Homo sapiens

<400> 2159
ggctatgata catgctggcc agctg                                          25

<210> 2160
<211> 25
<212> DNA
<213> Homo sapiens

<400> 2160
gctatgatac atgctggcca gctga                                          25

<210> 2161
<211> 25
<212> DNA
<213> Homo sapiens

<400> 2161
ctatgataca tgctggccag ctgaa                                          25

<210> 2162
<211> 25
<212> DNA
<213> Homo sapiens

<400> 2162
tatgatacat gctggccagc tgaaa                                          25

<210> 2163
<211> 25
<212> DNA
<213> Homo sapiens

<400> 2163
atgatacatg ctggccagct gaaaa                                          25

<210> 2164
<211> 25
<212> DNA
<213> Homo sapiens

<400> 2164
tgatacatgc tggccagctg aaaaa                                          25

<210> 2165
<211> 25
<212> DNA
<213> Homo sapiens

<400> 2165
gatacatgct ggccagctga aaaat                                          25

<210> 2166
<211> 25
<212> DNA
<213> Homo sapiens

<400> 2166
atacatgctg gccagctgaa aaatg                                          25

<210> 2167
<211> 25
<212> DNA
<213> Homo sapiens

<400> 2167
tacatgctgg ccagctgaaa aatgg                                          25

<210> 2168
<211> 25
<212> DNA
<213> Homo sapiens

<400> 2168
acatgctggc cagctgaaaa atgga                                          25

<210> 2169
<211> 25
<212> DNA
<213> Homo sapiens

<400> 2169
catgctggcc agctgaaaaa tggag                                          25

<210> 2170
<211> 25
<212> DNA
<213> Homo sapiens

<400> 2170
atgctggcca gctgaaaaat ggaga                                          25

<210> 2171
<211> 25
<212> DNA
<213> Homo sapiens

<400> 2171
tgctggccag ctgaaaaatg gagac                                          25

<210> 2172
<211> 25
<212> DNA
<213> Homo sapiens

<400> 2172
gctggccagc tgaaaaatgg agact                                          25

<210> 2173
<211> 25
<212> DNA
<213> Homo sapiens

<400> 2173
ctggccagct gaaaaatgga gactt                                          25

<210> 2174
<211> 25
<212> DNA
<213> Homo sapiens

<400> 2174
tggccagctg aaaaatggag acttt                                          25

<210> 2175
<211> 25
<212> DNA
<213> Homo sapiens

<400> 2175
ggccagctga aaaatggaga ctttc                                          25

<210> 2176
<211> 25
<212> DNA
<213> Homo sapiens

<400> 2176
gccagctgaa aaatggagac tttca                                          25

<210> 2177
<211> 25
<212> DNA
<213> Homo sapiens

<400> 2177
ccagctgaaa aatggagact ttcat                                          25

<210> 2178
<211> 25
<212> DNA
<213> Homo sapiens

<400> 2178
cagctgaaaa atggagactt tcatt                                          25

<210> 2179
<211> 25

<212> DNA
<213> Homo sapiens

<400> 2179
agctgaaaaa tggagacttt cattt                                            25

<210> 2180
<211> 25
<212> DNA
<213> Homo sapiens

<400> 2180
gctgaaaaat ggagactttc atttc                                            25

<210> 2181
<211> 25
<212> DNA
<213> Homo sapiens

<400> 2181
ctgaaaaatg gagactttca tttca                                            25

<210> 2182
<211> 25
<212> DNA
<213> Homo sapiens

<400> 2182
tgaaaaatgg agactttcat ttcac                                            25

<210> 2183
<211> 25
<212> DNA
<213> Homo sapiens

<400> 2183
gaaaaatgga gactttcatt tcact                                            25

<210> 2184
<211> 25
<212> DNA
<213> Homo sapiens

<400> 2184
aaaaatggag actttcattt cactg                                            25

<210> 2185
<211> 25
<212> DNA
<213> Homo sapiens

<400> 2185
aaaatggaga ctttcatttc actga                                            25

<210> 2186
<211> 25
<212> DNA
<213> Homo sapiens

<400> 2186
aaatggagac tttcatttca ctgac                                            25

<210> 2187
<211> 25
<212> DNA
<213> Homo sapiens

<400> 2187
aatggagact ttcatttcac tgact                                          25

<210> 2188
<211> 25
<212> DNA
<213> Homo sapiens

<400> 2188
atggagactt tcatttcact gactg                                          25

<210> 2189
<211> 25
<212> DNA
<213> Homo sapiens

<400> 2189
tggagacttt catttcactg actgt                                          25

<210> 2190
<211> 25
<212> DNA
<213> Homo sapiens

<400> 2190
ggagactttc atttcactga ctgtt                                          25

<210> 2191
<211> 25
<212> DNA
<213> Homo sapiens

<400> 2191
gagactttca tttcactgac tgttt                                          25

<210> 2192
<211> 25
<212> DNA
<213> Homo sapiens

<400> 2192
agactttcat ttcactgact gttta                                          25

<210> 2193
<211> 25
<212> DNA
<213> Homo sapiens

<400> 2193
gactttcatt tcactgactg tttat                                          25

<210> 2194
<211> 25
<212> DNA
<213> Homo sapiens

<400> 2194
actttcattt cactgactgt ttatt                                          25

<210> 2195
<211> 25
<212> DNA
<213> Homo sapiens

<400> 2195
ctttcatttc actgactgtt tattt                                          25

<210> 2196
<211> 25
<212> DNA
<213> Homo sapiens

<400> 2196
tttcatttca ctgactgttt atttt                                          25

<210> 2197
<211> 25
<212> DNA
<213> Homo sapiens

<400> 2197
ttcatttcac tgactgttta ttttt                                          25

<210> 2198
<211> 25
<212> DNA
<213> Homo sapiens

<400> 2198
tcatttcact gactgtttat ttttt                                          25

<210> 2199
<211> 25
<212> DNA
<213> Homo sapiens

<400> 2199
catttcactg actgtttatt ttttg                                          25

<210> 2200
<211> 25
<212> DNA
<213> Homo sapiens

<400> 2200
atttcactga ctgtttattt tttgg                                          25

<210> 2201
<211> 25
<212> DNA
<213> Homo sapiens

<400> 2201
tttcactgac tgtttatttt ttggt                                          25

<210> 2202

<211> 25
<212> DNA
<213> Homo sapiens

<400> 2202
ttcactgact gtttattttt tggtt                                            25

<210> 2203
<211> 25
<212> DNA
<213> Homo sapiens

<400> 2203
tcactgactg tttatttttt ggttc                                            25

<210> 2204
<211> 25
<212> DNA
<213> Homo sapiens

<400> 2204
cactgactgt ttattttttg gttca                                            25

<210> 2205
<211> 25
<212> DNA
<213> Homo sapiens

<400> 2205
actgactgtt tattttttgg ttcac                                            25

<210> 2206
<211> 25
<212> DNA
<213> Homo sapiens

<400> 2206
ctgactgttt attttttggt tcact                                            25

<210> 2207
<211> 25
<212> DNA
<213> Homo sapiens

<400> 2207
tgactgttta ttttttggtt cactg                                            25

<210> 2208
<211> 25
<212> DNA
<213> Homo sapiens

<400> 2208
gactgtttat tttttggttc actga                                            25

<210> 2209
<211> 25
<212> DNA
<213> Homo sapiens

<400> 2209

actgtttatt ttttggttca ctgat                                          25

<210> 2210
<211> 25
<212> DNA
<213> Homo sapiens

<400> 2210
ctgtttattt tttggttcac tgatg                                          25

<210> 2211
<211> 25
<212> DNA
<213> Homo sapiens

<400> 2211
tgtttatttt ttggttcact gatgt                                          25

<210> 2212
<211> 25
<212> DNA
<213> Homo sapiens

<400> 2212
gtttattttt tggttcactg atgtc                                          25

<210> 2213
<211> 25
<212> DNA
<213> Homo sapiens

<400> 2213
tttatttttt ggttcactga tgtct                                          25

<210> 2214
<211> 25
<212> DNA
<213> Homo sapiens

<400> 2214
ttattttttg gttcactgat gtctg                                          25

<210> 2215
<211> 25
<212> DNA
<213> Homo sapiens

<400> 2215
tattttttgg ttcactgatg tctgc                                          25

<210> 2216
<211> 25
<212> DNA
<213> Homo sapiens

<400> 2216
attttttggt tcactgatgt ctgct                                          25

<210> 2217
<211> 25
<212> DNA

<213> Homo sapiens

<400> 2217
   ttttttggtt cactgatgtc tgcta                                          25

<210> 2218
<211> 25
<212> DNA
<213> Homo sapiens

<400> 2218
tttttggttc actgatgtct gctac                                           25

<210> 2219
<211> 25
<212> DNA
<213> Homo sapiens

<400> 2219
ttttggttca ctgatgtctg ctaca                                           25

<210> 2220
<211> 25
<212> DNA
<213> Homo sapiens

<400> 2220
tttggttcac tgatgtctgc tacag                                           25

<210> 2221
<211> 25
<212> DNA
<213> Homo sapiens

<400> 2221
ttggttcact gatgtctgct acaga                                           25

<210> 2222
<211> 25
<212> DNA
<213> Homo sapiens

<400> 2222
tggttcactg atgtctgcta cagat                                           25

<210> 2223
<211> 25
<212> DNA
<213> Homo sapiens

<400> 2223
ggttcactga tgtctgctac agatc                                           25

<210> 2224
<211> 25
<212> DNA
<213> Homo sapiens

<400> 2224
gttcactgat gtctgctaca gatcc                                           25

<210> 2225
<211> 25
<212> DNA
<213> Homo sapiens

<400> 2225
ttcactgatg tctgctacag atcca                                          25

<210> 2226
<211> 25
<212> DNA
<213> Homo sapiens

<400> 2226
tcactgatgt ctgctacaga tccag                                          25

<210> 2227
<211> 25
<212> DNA
<213> Homo sapiens

<400> 2227
cactgatgtc tgctacagat ccagt                                          25

<210> 2228
<211> 25
<212> DNA
<213> Homo sapiens

<400> 2228
actgatgtct gctacagatc cagtg                                          25

<210> 2229
<211> 25
<212> DNA
<213> Homo sapiens

<400> 2229
ctgatgtctg ctacagatcc agtga                                          25

<210> 2230
<211> 25
<212> DNA
<213> Homo sapiens

<400> 2230
tgatgtctgc tacagatcca gtgac                                          25

<210> 2231
<211> 25
<212> DNA
<213> Homo sapiens

<400> 2231
gatgtctgct acagatccag tgaca                                          25

<210> 2232
<211> 25
<212> DNA
<213> Homo sapiens

<400> 2232
atgtctgcta cagatccagt gacag                                          25

<210> 2233
<211> 25
<212> DNA
<213> Homo sapiens

<400> 2233
tgtctgctac agatccagtg acagt                                          25

<210> 2234
<211> 25
<212> DNA
<213> Homo sapiens

<400> 2234
gtctgctaca gatccagtga cagtg                                          25

<210> 2235
<211> 25
<212> DNA
<213> Homo sapiens

<400> 2235
tctgctacag atccagtgac agtgc                                          25

<210> 2236
<211> 25
<212> DNA
<213> Homo sapiens

<400> 2236
ctgctacaga tccagtgaca gtgct                                          25

<210> 2237
<211> 25
<212> DNA
<213> Homo sapiens

<400> 2237
tgctacagat ccagtgacag tgctg                                          25

<210> 2238
<211> 25
<212> DNA
<213> Homo sapiens

<400> 2238
gctacagatc cagtgacagt gctgg                                          25

<210> 2239
<211> 25
<212> DNA
<213> Homo sapiens

<400> 2239
ctacagatcc agtgacagtg ctggc                                          25

<210> 2240
<211> 25

<212> DNA
<213> Homo sapiens

<400> 2240
tacagatcca gtgacagtgc tggcc                                          25

<210> 2241
<211> 25
<212> DNA
<213> Homo sapiens

<400> 2241
acagatccag tgacagtgct ggcca                                          25

<210> 2242
<211> 25
<212> DNA
<213> Homo sapiens

<400> 2242
cagatccagt gacagtgctg gccat                                          25

<210> 2243
<211> 25
<212> DNA
<213> Homo sapiens

<400> 2243
agatccagtg acagtgctgg ccatt                                          25

<210> 2244
<211> 25
<212> DNA
<213> Homo sapiens

<400> 2244
gatccagtga cagtgctggc cattt                                          25

<210> 2245
<211> 25
<212> DNA
<213> Homo sapiens

<400> 2245
atccagtgac agtgctggcc atttt                                          25

<210> 2246
<211> 25
<212> DNA
<213> Homo sapiens

<400> 2246
tccagtgaca gtgctggcca ttttc                                          25

<210> 2247
<211> 25
<212> DNA
<213> Homo sapiens

<400> 2247
ccagtgacag tgctggccat tttcc                                          25

<210> 2248
<211> 25
<212> DNA
<213> Homo sapiens

<400> 2248
cagtgacagt gctggccatt ttcca                                          25

<210> 2249
<211> 25
<212> DNA
<213> Homo sapiens

<400> 2249
agtgacagtg ctggccattt tccat                                          25

<210> 2250
<211> 25
<212> DNA
<213> Homo sapiens

<400> 2250
gtgacagtgc tggccatttt ccatg                                          25

<210> 2251
<211> 25
<212> DNA
<213> Homo sapiens

<400> 2251
tgacagtgct ggccattttc catga                                          25

<210> 2252
<211> 25
<212> DNA
<213> Homo sapiens

<400> 2252
gacagtgctg gccattttcc atgaa                                          25

<210> 2253
<211> 25
<212> DNA
<213> Homo sapiens

<400> 2253
acagtgctgg ccattttcca tgaac                                          25

<210> 2254
<211> 25
<212> DNA
<213> Homo sapiens

<400> 2254
cagtgctggc cattttccat gaact                                          25

<210> 2255
<211> 25
<212> DNA
<213> Homo sapiens

<400> 2255
agtgctggcc attttccatg aactg                                          25

<210> 2256
<211> 25
<212> DNA
<213> Homo sapiens

<400> 2256
gtgctggcca ttttccatga actgc                                          25

<210> 2257
<211> 25
<212> DNA
<213> Homo sapiens

<400> 2257
tgctggccat tttccatgaa ctgca                                          25

<210> 2258
<211> 25
<212> DNA
<213> Homo sapiens

<400> 2258
gctggccatt ttccatgaac tgcac                                          25

<210> 2259
<211> 25
<212> DNA
<213> Homo sapiens

<400> 2259
ctggccattt tccatgaact gcacg                                          25

<210> 2260
<211> 25
<212> DNA
<213> Homo sapiens

<400> 2260
tggccatttt ccatgaactg cacgt                                          25

<210> 2261
<211> 25
<212> DNA
<213> Homo sapiens

<400> 2261
ggccattttc catgaactgc acgtc                                          25

<210> 2262
<211> 25
<212> DNA
<213> Homo sapiens

<400> 2262
gccattttcc atgaactgca cgtcg                                          25

<210> 2263

<211> 25
<212> DNA
<213> Homo sapiens

<400> 2263
ccattttcca tgaactgcac gtcga                                          25

<210> 2264
<211> 25
<212> DNA
<213> Homo sapiens

<400> 2264
cattttccat gaactgcacg tcgac                                          25

<210> 2265
<211> 25
<212> DNA
<213> Homo sapiens

<400> 2265
attttccatg aactgcacgt cgacc                                          25

<210> 2266
<211> 25
<212> DNA
<213> Homo sapiens

<400> 2266
ttttccatga actgcacgtc gaccc                                          25

<210> 2267
<211> 25
<212> DNA
<213> Homo sapiens

<400> 2267
tttccatgaa ctgcacgtcg accct                                          25

<210> 2268
<211> 25
<212> DNA
<213> Homo sapiens

<400> 2268
ttccatgaac tgcacgtcga ccctg                                          25

<210> 2269
<211> 25
<212> DNA
<213> Homo sapiens

<400> 2269
tccatgaact gcacgtcgac cctga                                          25

<210> 2270
<211> 25
<212> DNA
<213> Homo sapiens

<400> 2270

ccatgaactg cacgtcgacc ctgac 25

<210> 2271
<211> 25
<212> DNA
<213> Homo sapiens

<400> 2271
catgaactgc acgtcgaccc tgacc 25

<210> 2272
<211> 25
<212> DNA
<213> Homo sapiens

<400> 2272
atgaactgca cgtcgaccct gacct 25

<210> 2273
<211> 25
<212> DNA
<213> Homo sapiens

<400> 2273
tgaactgcac gtcgaccctg acctg 25

<210> 2274
<211> 25
<212> DNA
<213> Homo sapiens

<400> 2274
gaactgcacg tcgaccctga cctgt 25

<210> 2275
<211> 25
<212> DNA
<213> Homo sapiens

<400> 2275
aactgcacgt cgaccctgac ctgta 25

<210> 2276
<211> 25
<212> DNA
<213> Homo sapiens

<400> 2276
actgcacgtc gaccctgacc tgtac 25

<210> 2277
<211> 25
<212> DNA
<213> Homo sapiens

<400> 2277
ctgcacgtcg accctgacct gtaca 25

<210> 2278
<211> 25
<212> DNA

<213> Homo sapiens

<400> 2278
tgcacgtcga ccctgacctg tacac                                          25

<210> 2279
<211> 25
<212> DNA
<213> Homo sapiens

<400> 2279
gcacgtcgac cctgacctgt acaca                                          25

<210> 2280
<211> 25
<212> DNA
<213> Homo sapiens

<400> 2280
cacgtcgacc ctgacctgta cacac                                          25

<210> 2281
<211> 25
<212> DNA
<213> Homo sapiens

<400> 2281
acgtcgaccc tgacctgtac acact                                          25

<210> 2282
<211> 25
<212> DNA
<213> Homo sapiens

<400> 2282
cgtcgaccct gacctgtaca cactc                                          25

<210> 2283
<211> 25
<212> DNA
<213> Homo sapiens

<400> 2283
gtcgaccctg acctgtacac actct                                          25

<210> 2284
<211> 25
<212> DNA
<213> Homo sapiens

<400> 2284
tcgaccctga cctgtacaca ctctt                                          25

<210> 2285
<211> 25
<212> DNA
<213> Homo sapiens

<400> 2285
cgaccctgac ctgtacacac tcttg                                          25

<210> 2286
<211> 25
<212> DNA
<213> Homo sapiens

<400> 2286
gaccctgacc tgtacacact cttgt                                          25

<210> 2287
<211> 25
<212> DNA
<213> Homo sapiens

<400> 2287
accctgacct gtacacactc ttgtt                                          25

<210> 2288
<211> 25
<212> DNA
<213> Homo sapiens

<400> 2288
ccctgacctg tacacactct tgttt                                          25

<210> 2289
<211> 25
<212> DNA
<213> Homo sapiens

<400> 2289
cctgacctgt acacactctt gtttg                                          25

<210> 2290
<211> 25
<212> DNA
<213> Homo sapiens

<400> 2290
ctgacctgta cacactcttg tttgg                                          25

<210> 2291
<211> 25
<212> DNA
<213> Homo sapiens

<400> 2291
tgacctgtac acactcttgt ttgga                                          25

<210> 2292
<211> 25
<212> DNA
<213> Homo sapiens

<400> 2292
gacctgtaca cactcttgtt tggag                                          25

<210> 2293
<211> 25
<212> DNA
<213> Homo sapiens

<400> 2293
acctgtacac actcttgttt ggaga                                          25

<210> 2294
<211> 25
<212> DNA
<213> Homo sapiens

<400> 2294
cctgtacaca ctcttgtttg gagag                                          25

<210> 2295
<211> 25
<212> DNA
<213> Homo sapiens

<400> 2295
ctgtacacac tcttgtttgg agaga                                          25

<210> 2296
<211> 25
<212> DNA
<213> Homo sapiens

<400> 2296
tgtacacact cttgtttgga gagag                                          25

<210> 2297
<211> 25
<212> DNA
<213> Homo sapiens

<400> 2297
gtacacactc ttgtttggag agagt                                          25

<210> 2298
<211> 25
<212> DNA
<213> Homo sapiens

<400> 2298
tacacactct tgtttggaga gagtg                                          25

<210> 2299
<211> 25
<212> DNA
<213> Homo sapiens

<400> 2299
acacactctt gtttggagag agtgt                                          25

<210> 2300
<211> 25
<212> DNA
<213> Homo sapiens

<400> 2300
cacactcttg tttggagaga gtgtg                                          25

<210> 2301
<211> 25

<212> DNA
<213> Homo sapiens

<400> 2301
acactcttgt ttggagagag tgtgt                                            25

<210> 2302
<211> 25
<212> DNA
<213> Homo sapiens

<400> 2302
cactcttgtt tggagagagt gtgtt                                            25

<210> 2303
<211> 25
<212> DNA
<213> Homo sapiens

<400> 2303
actcttgttt ggagagagtg tgttg                                            25

<210> 2304
<211> 25
<212> DNA
<213> Homo sapiens

<400> 2304
ctcttgtttg gagagagtgt gttga                                            25

<210> 2305
<211> 25
<212> DNA
<213> Homo sapiens

<400> 2305
tcttgtttgg agagagtgtg ttgaa                                            25

<210> 2306
<211> 25
<212> DNA
<213> Homo sapiens

<400> 2306
cttgtttgga gagagtgtgt tgaat                                            25

<210> 2307
<211> 25
<212> DNA
<213> Homo sapiens

<400> 2307
ttgtttggag agagtgtgtt gaatg                                            25

<210> 2308
<211> 25
<212> DNA
<213> Homo sapiens

<400> 2308
tgtttggaga gagtgtgttg aatga                                            25

<210> 2309
<211> 25
<212> DNA
<213> Homo sapiens

<400> 2309
gtttggagag agtgtgttga atgat                                          25

<210> 2310
<211> 25
<212> DNA
<213> Homo sapiens

<400> 2310
tttggagaga gtgtgttgaa tgatg                                          25

<210> 2311
<211> 25
<212> DNA
<213> Homo sapiens

<400> 2311
ttggagagag tgtgttgaat gatgc                                          25

<210> 2312
<211> 25
<212> DNA
<213> Homo sapiens

<400> 2312
tggagagagt gtgttgaatg atgca                                          25

<210> 2313
<211> 25
<212> DNA
<213> Homo sapiens

<400> 2313
ggagagagtg tgttgaatga tgcag                                          25

<210> 2314
<211> 25
<212> DNA
<213> Homo sapiens

<400> 2314
gagagagtgt gttgaatgat gcagt                                          25

<210> 2315
<211> 25
<212> DNA
<213> Homo sapiens

<400> 2315
agagagtgtg ttgaatgatg cagtg                                          25

<210> 2316
<211> 25
<212> DNA
<213> Homo sapiens

<400> 2316
gagagtgtgt tgaatgatgc agtgg                                          25

<210> 2317
<211> 25
<212> DNA
<213> Homo sapiens

<400> 2317
agagtgtgtt gaatgatgca gtggc                                          25

<210> 2318
<211> 25
<212> DNA
<213> Homo sapiens

<400> 2318
gagtgtgttg aatgatgcag tggcc                                          25

<210> 2319
<211> 25
<212> DNA
<213> Homo sapiens

<400> 2319
agtgtgttga atgatgcagt ggcca                                          25

<210> 2320
<211> 25
<212> DNA
<213> Homo sapiens

<400> 2320
gtgtgttgaa tgatgcagtg gccat                                          25

<210> 2321
<211> 25
<212> DNA
<213> Homo sapiens

<400> 2321
tgtgttgaat gatgcagtgg ccata                                          25

<210> 2322
<211> 25
<212> DNA
<213> Homo sapiens

<400> 2322
gtgttgaatg atgcagtggc catag                                          25

<210> 2323
<211> 25
<212> DNA
<213> Homo sapiens

<400> 2323
tgttgaatga tgcagtggcc atagt                                          25

<210> 2324

<211> 25
<212> DNA
<213> Homo sapiens

<400> 2324
gttgaatgat gcagtggcca tagtc                                          25

<210> 2325
<211> 25
<212> DNA
<213> Homo sapiens

<400> 2325
ttgaatgatg cagtggccat agtcc                                          25

<210> 2326
<211> 25
<212> DNA
<213> Homo sapiens

<400> 2326
tgaatgatgc agtggccata gtcct                                          25

<210> 2327
<211> 25
<212> DNA
<213> Homo sapiens

<400> 2327
gaatgatgca gtggccatag tcctt                                          25

<210> 2328
<211> 25
<212> DNA
<213> Homo sapiens

<400> 2328
aatgatgcag tggccatagt cctta                                          25

<210> 2329
<211> 25
<212> DNA
<213> Homo sapiens

<400> 2329
atgatgcagt ggccatagtc cttac                                          25

<210> 2330
<211> 25
<212> DNA
<213> Homo sapiens

<400> 2330
tgatgcagtg gccatagtcc ttaca                                          25

<210> 2331
<211> 25
<212> DNA
<213> Homo sapiens

<400> 2331

gatgcagtgg ccatagtcct tacat                                          25

<210> 2332
<211> 25
<212> DNA
<213> Homo sapiens

<400> 2332
atgcagtggc catagtcctt acata                                          25

<210> 2333
<211> 25
<212> DNA
<213> Homo sapiens

<400> 2333
tgcagtggcc atagtcctta catat                                          25

<210> 2334
<211> 25
<212> DNA
<213> Homo sapiens

<400> 2334
gcagtggcca tagtccttac atatt                                          25

<210> 2335
<211> 25
<212> DNA
<213> Homo sapiens

<400> 2335
cagtggccat agtccttaca tattc                                          25

<210> 2336
<211> 25
<212> DNA
<213> Homo sapiens

<400> 2336
agtggccata gtccttacat attct                                          25

<210> 2337
<211> 25
<212> DNA
<213> Homo sapiens

<400> 2337
gtggccatag tccttacata ttcta                                          25

<210> 2338
<211> 25
<212> DNA
<213> Homo sapiens

<400> 2338
tggccatagt ccttacatat tctat                                          25

<210> 2339
<211> 25
<212> DNA

<213> Homo sapiens

<400> 2339
ggccatagtc cttacatatt ctata                                            25

<210> 2340
<211> 25
<212> DNA
<213> Homo sapiens

<400> 2340
gccatagtcc ttacatattc tatat                                            25

<210> 2341
<211> 25
<212> DNA
<213> Homo sapiens

<400> 2341
ccatagtcct tacatattct atatc                                            25

<210> 2342
<211> 25
<212> DNA
<213> Homo sapiens

<400> 2342
catagtcctt acatattcta tatcc                                            25

<210> 2343
<211> 25
<212> DNA
<213> Homo sapiens

<400> 2343
atagtcctta catattctat atcca                                            25

<210> 2344
<211> 25
<212> DNA
<213> Homo sapiens

<400> 2344
tagtccttac atattctata tccat                                            25

<210> 2345
<211> 25
<212> DNA
<213> Homo sapiens

<400> 2345
agtccttaca tattctatat ccatt                                            25

<210> 2346
<211> 25
<212> DNA
<213> Homo sapiens

<400> 2346
gtccttacat attctatatc cattt                                            25

<210> 2347
<211> 25
<212> DNA
<213> Homo sapiens

<400> 2347
tccttacata ttctatatcc attta                                          25

<210> 2348
<211> 25
<212> DNA
<213> Homo sapiens

<400> 2348
ccttacatat tctatatcca tttac                                          25

<210> 2349
<211> 25
<212> DNA
<213> Homo sapiens

<400> 2349
cttacatatt ctatatccat ttaca                                          25

<210> 2350
<211> 25
<212> DNA
<213> Homo sapiens

<400> 2350
ttacatattc tatatccatt tacag                                          25

<210> 2351
<211> 25
<212> DNA
<213> Homo sapiens

<400> 2351
tacatattct atatccattt acagt                                          25

<210> 2352
<211> 25
<212> DNA
<213> Homo sapiens

<400> 2352
acatattcta tatccattta cagtc                                          25

<210> 2353
<211> 25
<212> DNA
<213> Homo sapiens

<400> 2353
catattctat atccatttac agtcc                                          25

<210> 2354
<211> 25
<212> DNA
<213> Homo sapiens

<400> 2354
atattctata tccatttaca gtccc                                          25

<210> 2355
<211> 25
<212> DNA
<213> Homo sapiens

<400> 2355
tattctatat ccatttacag tccca                                          25

<210> 2356
<211> 25
<212> DNA
<213> Homo sapiens

<400> 2356
attctatatc catttacagt cccaa                                          25

<210> 2357
<211> 25
<212> DNA
<213> Homo sapiens

<400> 2357
ttctatatcc atttacagtc ccaag                                          25

<210> 2358
<211> 25
<212> DNA
<213> Homo sapiens

<400> 2358
tctatatcca tttacagtcc caagg                                          25

<210> 2359
<211> 25
<212> DNA
<213> Homo sapiens

<400> 2359
ctatatccat ttacagtccc aagga                                          25

<210> 2360
<211> 25
<212> DNA
<213> Homo sapiens

<400> 2360
tatatccatt tacagtccca aggag                                          25

<210> 2361
<211> 25
<212> DNA
<213> Homo sapiens

<400> 2361
atatccattt acagtcccaa ggaga                                          25

<210> 2362
<211> 25

<212> DNA
<213> Homo sapiens

<400> 2362
tatccattta cagtcccaag gagaa                                          25

<210> 2363
<211> 25
<212> DNA
<213> Homo sapiens

<400> 2363
atccatttac agtcccaagg agaat                                          25

<210> 2364
<211> 25
<212> DNA
<213> Homo sapiens

<400> 2364
tccatttaca gtcccaagga gaatc                                          25

<210> 2365
<211> 25
<212> DNA
<213> Homo sapiens

<400> 2365
ccatttacag tcccaaggag aatcc                                          25

<210> 2366
<211> 25
<212> DNA
<213> Homo sapiens

<400> 2366
catttacagt cccaaggaga atcca                                          25

<210> 2367
<211> 25
<212> DNA
<213> Homo sapiens

<400> 2367
atttacagtc ccaaggagaa tccaa                                          25

<210> 2368
<211> 25
<212> DNA
<213> Homo sapiens

<400> 2368
tttacagtcc caaggagaat ccaaa                                          25

<210> 2369
<211> 25
<212> DNA
<213> Homo sapiens

<400> 2369
ttacagtccc aaggagaatc caaat                                          25

<210> 2370
<211> 25
<212> DNA
<213> Homo sapiens

<400> 2370
tacagtccca aggagaatcc aaatg                                          25

<210> 2371
<211> 25
<212> DNA
<213> Homo sapiens

<400> 2371
acagtcccaa ggagaatcca aatgc                                          25

<210> 2372
<211> 25
<212> DNA
<213> Homo sapiens

<400> 2372
cagtcccaag gagaatccaa atgca                                          25

<210> 2373
<211> 25
<212> DNA
<213> Homo sapiens

<400> 2373
agtcccaagg agaatccaaa tgcat                                          25

<210> 2374
<211> 25
<212> DNA
<213> Homo sapiens

<400> 2374
gtcccaagga gaatccaaat gcatt                                          25

<210> 2375
<211> 25
<212> DNA
<213> Homo sapiens

<400> 2375
tcccaaggag aatccaaatg cattt                                          25

<210> 2376
<211> 25
<212> DNA
<213> Homo sapiens

<400> 2376
cccaaggaga atccaaatgc atttg                                          25

<210> 2377
<211> 25
<212> DNA
<213> Homo sapiens

<400> 2377
ccaaggagaa tccaaatgca tttga                                          25

<210> 2378
<211> 25
<212> DNA
<213> Homo sapiens

<400> 2378
caaggagaat ccaaatgcat ttgat                                          25

<210> 2379
<211> 25
<212> DNA
<213> Homo sapiens

<400> 2379
aaggagaatc caaatgcatt tgatg                                          25

<210> 2380
<211> 25
<212> DNA
<213> Homo sapiens

<400> 2380
aggagaatcc aaatgcattt gatgc                                          25

<210> 2381
<211> 25
<212> DNA
<213> Homo sapiens

<400> 2381
ggagaatcca aatgcatttg atgcc                                          25

<210> 2382
<211> 25
<212> DNA
<213> Homo sapiens

<400> 2382
gagaatccaa atgcatttga tgccg                                          25

<210> 2383
<211> 25
<212> DNA
<213> Homo sapiens

<400> 2383
agaatccaaa tgcatttgat gccgc                                          25

<210> 2384
<211> 25
<212> DNA
<213> Homo sapiens

<400> 2384
gaatccaaat gcatttgatg ccgca                                          25

<210> 2385

<211> 25
<212> DNA
<213> Homo sapiens

<400> 2385
aatccaaatg catttgatgc cgcag                                          25

<210> 2386
<211> 25
<212> DNA
<213> Homo sapiens

<400> 2386
atccaaatgc atttgatgcc gcagc                                          25

<210> 2387
<211> 25
<212> DNA
<213> Homo sapiens

<400> 2387
tccaaatgca tttgatgccg cagca                                          25

<210> 2388
<211> 25
<212> DNA
<213> Homo sapiens

<400> 2388
ccaaatgcat ttgatgccgc agcat                                          25

<210> 2389
<211> 25
<212> DNA
<213> Homo sapiens

<400> 2389
caaatgcatt tgatgccgca gcatt                                          25

<210> 2390
<211> 25
<212> DNA
<213> Homo sapiens

<400> 2390
aaatgcattt gatgccgcag cattc                                          25

<210> 2391
<211> 25
<212> DNA
<213> Homo sapiens

<400> 2391
aatgcatttg atgccgcagc attct                                          25

<210> 2392
<211> 25
<212> DNA
<213> Homo sapiens

<400> 2392

atgcatttga tgccgcagca ttctt                                          25

<210> 2393
<211> 25
<212> DNA
<213> Homo sapiens

<400> 2393
tgcatttgat gccgcagcat tcttc                                          25

<210> 2394
<211> 25
<212> DNA
<213> Homo sapiens

<400> 2394
gcatttgatg ccgcagcatt cttcc                                          25

<210> 2395
<211> 25
<212> DNA
<213> Homo sapiens

<400> 2395
catttgatgc cgcagcattc ttcca                                          25

<210> 2396
<211> 25
<212> DNA
<213> Homo sapiens

<400> 2396
atttgatgcc gcagcattct tccag                                          25

<210> 2397
<211> 25
<212> DNA
<213> Homo sapiens

<400> 2397
tttgatgccg cagcattctt ccagt                                          25

<210> 2398
<211> 25
<212> DNA
<213> Homo sapiens

<400> 2398
ttgatgccgc agcattcttc cagtc                                          25

<210> 2399
<211> 25
<212> DNA
<213> Homo sapiens

<400> 2399
tgatgccgca gcattcttcc agtct                                          25

<210> 2400
<211> 25
<212> DNA

<213> Homo sapiens

<400> 2400
gatgccgcag cattcttcca gtctg                                          25

<210> 2401
<211> 25
<212> DNA
<213> Homo sapiens

<400> 2401
atgccgcagc attcttccag tctgt                                          25

<210> 2402
<211> 25
<212> DNA
<213> Homo sapiens

<400> 2402
tgccgcagca ttcttccagt ctgtg                                          25

<210> 2403
<211> 25
<212> DNA
<213> Homo sapiens

<400> 2403
gccgcagcat tcttccagtc tgtgg                                          25

<210> 2404
<211> 25
<212> DNA
<213> Homo sapiens

<400> 2404
ccgcagcatt cttccagtct gtggg                                          25

<210> 2405
<211> 25
<212> DNA
<213> Homo sapiens

<400> 2405
cgcagcattc ttccagtctg tgggg                                          25

<210> 2406
<211> 25
<212> DNA
<213> Homo sapiens

<400> 2406
gcagcattct tccagtctgt gggga                                          25

<210> 2407
<211> 25
<212> DNA
<213> Homo sapiens

<400> 2407
cagcattctt ccagtctgtg gggaa                                          25

<210> 2408
<211> 25
<212> DNA
<213> Homo sapiens

<400> 2408
agcattcttc cagtctgtgg ggaat                                          25

<210> 2409
<211> 25
<212> DNA
<213> Homo sapiens

<400> 2409
gcattcttcc agtctgtggg gaatt                                          25

<210> 2410
<211> 25
<212> DNA
<213> Homo sapiens

<400> 2410
cattcttcca gtctgtgggg aattt                                          25

<210> 2411
<211> 25
<212> DNA
<213> Homo sapiens

<400> 2411
attcttccag tctgtgggga atttc                                          25

<210> 2412
<211> 25
<212> DNA
<213> Homo sapiens

<400> 2412
ttcttccagt ctgtggggaa tttcc                                          25

<210> 2413
<211> 25
<212> DNA
<213> Homo sapiens

<400> 2413
tcttccagtc tgtggggaat ttcct                                          25

<210> 2414
<211> 25
<212> DNA
<213> Homo sapiens

<400> 2414
cttccagtct gtggggaatt tcctg                                          25

<210> 2415
<211> 25
<212> DNA
<213> Homo sapiens

<400> 2415
ttccagtctg tggggaattt cctgg                                          25

<210> 2416
<211> 25
<212> DNA
<213> Homo sapiens

<400> 2416
tccagtctgt ggggaatttc ctggg                                          25

<210> 2417
<211> 25
<212> DNA
<213> Homo sapiens

<400> 2417
ccagtctgtg gggaatttcc tggga                                          25

<210> 2418
<211> 25
<212> DNA
<213> Homo sapiens

<400> 2418
cagtctgtgg ggaatttcct gggaa                                          25

<210> 2419
<211> 25
<212> DNA
<213> Homo sapiens

<400> 2419
agtctgtggg gaatttcctg ggaat                                          25

<210> 2420
<211> 25
<212> DNA
<213> Homo sapiens

<400> 2420
gtctgtgggg aatttcctgg gaatc                                          25

<210> 2421
<211> 25
<212> DNA
<213> Homo sapiens

<400> 2421
tctgtgggga atttcctggg aatct                                          25

<210> 2422
<211> 25
<212> DNA
<213> Homo sapiens

<400> 2422
ctgtggggaa tttcctggga atctt                                          25

<210> 2423
<211> 25

<212> DNA
<213> Homo sapiens

<400> 2423
tgtggggaat ttcctgggaa tcttc                                            25

<210> 2424
<211> 25
<212> DNA
<213> Homo sapiens

<400> 2424
gtggggaatt tcctgggaat cttcg                                            25

<210> 2425
<211> 25
<212> DNA
<213> Homo sapiens

<400> 2425
tggggaattt cctgggaatc ttcgc                                            25

<210> 2426
<211> 25
<212> DNA
<213> Homo sapiens

<400> 2426
ggggaatttc ctgggaatct tcgct                                            25

<210> 2427
<211> 25
<212> DNA
<213> Homo sapiens

<400> 2427
gggaatttcc tgggaatctt cgctg                                            25

<210> 2428
<211> 25
<212> DNA
<213> Homo sapiens

<400> 2428
ggaatttcct gggaatcttc gctgg                                            25

<210> 2429
<211> 25
<212> DNA
<213> Homo sapiens

<400> 2429
gaatttcctg ggaatcttcg ctggc                                            25

<210> 2430
<211> 25
<212> DNA
<213> Homo sapiens

<400> 2430
aatttcctgg gaatcttcgc tggct                                            25

<210> 2431
<211> 25
<212> DNA
<213> Homo sapiens

<400> 2431
atttcctggg aatcttcgct ggctc                                            25

<210> 2432
<211> 25
<212> DNA
<213> Homo sapiens

<400> 2432
tttcctggga atcttcgctg gctca                                            25

<210> 2433
<211> 25
<212> DNA
<213> Homo sapiens

<400> 2433
ttcctgggaa tcttcgctgg ctcat                                            25

<210> 2434
<211> 25
<212> DNA
<213> Homo sapiens

<400> 2434
tcctgggaat cttcgctggc tcatt                                            25

<210> 2435
<211> 25
<212> DNA
<213> Homo sapiens

<400> 2435
cctgggaatc ttcgctggct cattt                                            25

<210> 2436
<211> 25
<212> DNA
<213> Homo sapiens

<400> 2436
ctgggaatct tcgctggctc atttg                                            25

<210> 2437
<211> 25
<212> DNA
<213> Homo sapiens

<400> 2437
tgggaatctt cgctggctca tttgc                                            25

<210> 2438
<211> 25
<212> DNA
<213> Homo sapiens

<400> 2438
gggaatcttc gctggctcat ttgca                                          25

<210> 2439
<211> 25
<212> DNA
<213> Homo sapiens

<400> 2439
ggaatcttcg ctggctcatt tgcaa                                          25

<210> 2440
<211> 25
<212> DNA
<213> Homo sapiens

<400> 2440
gaatcttcgc tggctcattt gcaat                                          25

<210> 2441
<211> 25
<212> DNA
<213> Homo sapiens

<400> 2441
aatcttcgct ggctcatttg caatg                                          25

<210> 2442
<211> 25
<212> DNA
<213> Homo sapiens

<400> 2442
atcttcgctg gctcatttgc aatgg                                          25

<210> 2443
<211> 25
<212> DNA
<213> Homo sapiens

<400> 2443
tcttcgctgg ctcatttgca atggg                                          25

<210> 2444
<211> 25
<212> DNA
<213> Homo sapiens

<400> 2444
cttcgctggc tcatttgcaa tgggg                                          25

<210> 2445
<211> 25
<212> DNA
<213> Homo sapiens

<400> 2445
ttcgctggct catttgcaat ggggt                                          25

<210> 2446

<211> 25
<212> DNA
<213> Homo sapiens

<400> 2446
tcgctggctc atttgcaatg gggtc                                          25

<210> 2447
<211> 25
<212> DNA
<213> Homo sapiens

<400> 2447
cgctggctca tttgcaatgg ggtct                                          25

<210> 2448
<211> 25
<212> DNA
<213> Homo sapiens

<400> 2448
gctggctcat ttgcaatggg gtctg                                          25

<210> 2449
<211> 25
<212> DNA
<213> Homo sapiens

<400> 2449
ctggctcatt tgcaatgggg tctgc                                          25

<210> 2450
<211> 25
<212> DNA
<213> Homo sapiens

<400> 2450
tggctcattt gcaatggggt ctgcg                                          25

<210> 2451
<211> 25
<212> DNA
<213> Homo sapiens

<400> 2451
ggctcatttg caatggggtc tgcgt                                          25

<210> 2452
<211> 25
<212> DNA
<213> Homo sapiens

<400> 2452
gctcatttgc aatggggtct gcgta                                          25

<210> 2453
<211> 25
<212> DNA
<213> Homo sapiens

<400> 2453

ctcatttgca atggggtctg cgtat 25

<210> 2454
<211> 25
<212> DNA
<213> Homo sapiens

<400> 2454
tcatttgcaa tggggtctgc gtatg 25

<210> 2455
<211> 25
<212> DNA
<213> Homo sapiens

<400> 2455
catttgcaat ggggtctgcg tatgc 25

<210> 2456
<211> 25
<212> DNA
<213> Homo sapiens

<400> 2456
atttgcaatg gggtctgcgt atgcc 25

<210> 2457
<211> 25
<212> DNA
<213> Homo sapiens

<400> 2457
tttgcaatgg ggtctgcgta tgcca 25

<210> 2458
<211> 25
<212> DNA
<213> Homo sapiens

<400> 2458
ttgcaatggg gtctgcgtat gccat 25

<210> 2459
<211> 25
<212> DNA
<213> Homo sapiens

<400> 2459
tgcaatgggg tctgcgtatg ccatc 25

<210> 2460
<211> 25
<212> DNA
<213> Homo sapiens

<400> 2460
gcaatggggt ctgcgtatgc catca 25

<210> 2461
<211> 25
<212> DNA

<213> Homo sapiens

<400> 2461
caatggggtc tgcgtatgcc atcat                                          25

<210> 2462
<211> 25
<212> DNA
<213> Homo sapiens

<400> 2462
aatggggtct gcgtatgcca tcatc                                          25

<210> 2463
<211> 25
<212> DNA
<213> Homo sapiens

<400> 2463
atggggtctg cgtatgccat catca                                          25

<210> 2464
<211> 25
<212> DNA
<213> Homo sapiens

<400> 2464
tggggtctgc gtatgccatc atcac                                          25

<210> 2465
<211> 25
<212> DNA
<213> Homo sapiens

<400> 2465
ggggtctgcg tatgccatca tcaca                                          25

<210> 2466
<211> 25
<212> DNA
<213> Homo sapiens

<400> 2466
gggtctgcgt atgccatcat cacag                                          25

<210> 2467
<211> 25
<212> DNA
<213> Homo sapiens

<400> 2467
ggtctgcgta tgccatcatc acagc                                          25

<210> 2468
<211> 25
<212> DNA
<213> Homo sapiens

<400> 2468
gtctgcgtat gccatcatca cagca                                          25

<210> 2469
<211> 25
<212> DNA
<213> Homo sapiens

<400> 2469
tctgcgtatg ccatcatcac agcac                                            25

<210> 2470
<211> 25
<212> DNA
<213> Homo sapiens

<400> 2470
ctgcgtatgc catcatcaca gcact                                            25

<210> 2471
<211> 25
<212> DNA
<213> Homo sapiens

<400> 2471
tgcgtatgcc atcatcacag cactg                                            25

<210> 2472
<211> 25
<212> DNA
<213> Homo sapiens

<400> 2472
gcgtatgcca tcatcacagc actgt                                            25

<210> 2473
<211> 25
<212> DNA
<213> Homo sapiens

<400> 2473
cgtatgccat catcacagca ctgtt                                            25

<210> 2474
<211> 25
<212> DNA
<213> Homo sapiens

<400> 2474
gtatgccatc atcacagcac tgttg                                            25

<210> 2475
<211> 25
<212> DNA
<213> Homo sapiens

<400> 2475
tatgccatca tcacagcact gttga                                            25

<210> 2476
<211> 25
<212> DNA
<213> Homo sapiens

<400> 2476
atgccatcat cacagcactg ttgac                                          25

<210> 2477
<211> 25
<212> DNA
<213> Homo sapiens

<400> 2477
tgccatcatc acagcactgt tgacc                                          25

<210> 2478
<211> 25
<212> DNA
<213> Homo sapiens

<400> 2478
gccatcatca cagcactgtt gacca                                          25

<210> 2479
<211> 25
<212> DNA
<213> Homo sapiens

<400> 2479
ccatcatcac agcactgttg accaa                                          25

<210> 2480
<211> 25
<212> DNA
<213> Homo sapiens

<400> 2480
catcatcaca gcactgttga ccaaa                                          25

<210> 2481
<211> 25
<212> DNA
<213> Homo sapiens

<400> 2481
atcatcacag cactgttgac caaat                                          25

<210> 2482
<211> 25
<212> DNA
<213> Homo sapiens

<400> 2482
tcatcacagc actgttgacc aaatt                                          25

<210> 2483
<211> 25
<212> DNA
<213> Homo sapiens

<400> 2483
catcacagca ctgttgacca aattt                                          25

<210> 2484
<211> 25

<212> DNA
<213> Homo sapiens

<400> 2484
atcacagcac tgttgaccaa attta                                            25

<210> 2485
<211> 25
<212> DNA
<213> Homo sapiens

<400> 2485
tcacagcact gttgaccaaa tttac                                            25

<210> 2486
<211> 25
<212> DNA
<213> Homo sapiens

<400> 2486
cacagcactg ttgaccaaat ttacc                                            25

<210> 2487
<211> 25
<212> DNA
<213> Homo sapiens

<400> 2487
acagcactgt tgaccaaatt tacca                                            25

<210> 2488
<211> 25
<212> DNA
<213> Homo sapiens

<400> 2488
cagcactgtt gaccaaattt accaa                                            25

<210> 2489
<211> 25
<212> DNA
<213> Homo sapiens

<400> 2489
agcactgttg accaaattta ccaag                                            25

<210> 2490
<211> 25
<212> DNA
<213> Homo sapiens

<400> 2490
gcactgttga ccaaatttac caagc                                            25

<210> 2491
<211> 25
<212> DNA
<213> Homo sapiens

<400> 2491
cactgttgac caaatttacc aagct                                            25

<210> 2492
<211> 25
<212> DNA
<213> Homo sapiens

<400> 2492
actgttgacc aaatttacca agctg                                          25

<210> 2493
<211> 25
<212> DNA
<213> Homo sapiens

<400> 2493
ctgttgacca aatttaccaa gctgt                                          25

<210> 2494
<211> 25
<212> DNA
<213> Homo sapiens

<400> 2494
tgttgaccaa atttaccaag ctgtg                                          25

<210> 2495
<211> 25
<212> DNA
<213> Homo sapiens

<400> 2495
gttgaccaaa tttaccaagc tgtgt                                          25

<210> 2496
<211> 25
<212> DNA
<213> Homo sapiens

<400> 2496
ttgaccaaat ttaccaagct gtgtg                                          25

<210> 2497
<211> 25
<212> DNA
<213> Homo sapiens

<400> 2497
tgaccaaatt taccaagctg tgtga                                          25

<210> 2498
<211> 25
<212> DNA
<213> Homo sapiens

<400> 2498
gaccaaattt accaagctgt gtgag                                          25

<210> 2499
<211> 25
<212> DNA
<213> Homo sapiens

<400> 2499
accaaattta ccaagctgtg tgagt                                          25

<210> 2500
<211> 25
<212> DNA
<213> Homo sapiens

<400> 2500
ccaaatttac caagctgtgt gagtt                                          25

<210> 2501
<211> 25
<212> DNA
<213> Homo sapiens

<400> 2501
caaatttacc aagctgtgtg agttc                                          25

<210> 2502
<211> 25
<212> DNA
<213> Homo sapiens

<400> 2502
aaatttacca agctgtgtga gttcc                                          25

<210> 2503
<211> 25
<212> DNA
<213> Homo sapiens

<400> 2503
aatttaccaa gctgtgtgag ttccc                                          25

<210> 2504
<211> 25
<212> DNA
<213> Homo sapiens

<400> 2504
atttaccaag ctgtgtgagt tcccg                                          25

<210> 2505
<211> 25
<212> DNA
<213> Homo sapiens

<400> 2505
tttaccaagc tgtgtgagtt cccga                                          25

<210> 2506
<211> 25
<212> DNA
<213> Homo sapiens

<400> 2506
ttaccaagct gtgtgagttc ccgat                                          25

<210> 2507

<211> 25
<212> DNA
<213> Homo sapiens

<400> 2507
taccaagctg tgtgagttcc cgatg                                          25

<210> 2508
<211> 25
<212> DNA
<213> Homo sapiens

<400> 2508
accaagctgt gtgagttccc gatgc                                          25

<210> 2509
<211> 25
<212> DNA
<213> Homo sapiens

<400> 2509
ccaagctgtg tgagttcccg atgct                                          25

<210> 2510
<211> 25
<212> DNA
<213> Homo sapiens

<400> 2510
caagctgtgt gagttcccga tgctg                                          25

<210> 2511
<211> 25
<212> DNA
<213> Homo sapiens

<400> 2511
aagctgtgtg agttcccgat gctgg                                          25

<210> 2512
<211> 25
<212> DNA
<213> Homo sapiens

<400> 2512
agctgtgtga gttcccgatg ctgga                                          25

<210> 2513
<211> 25
<212> DNA
<213> Homo sapiens

<400> 2513
gctgtgtgag ttcccgatgc tggaa                                          25

<210> 2514
<211> 25
<212> DNA
<213> Homo sapiens

<400> 2514

ctgtgtgagt tcccgatgct ggaaa 25

<210> 2515
<211> 25
<212> DNA
<213> Homo sapiens

<400> 2515
tgtgtgagtt cccgatgctg gaaac 25

<210> 2516
<211> 25
<212> DNA
<213> Homo sapiens

<400> 2516
gtgtgagttc ccgatgctgg aaacc 25

<210> 2517
<211> 25
<212> DNA
<213> Homo sapiens

<400> 2517
tgtgagttcc cgatgctgga aaccg 25

<210> 2518
<211> 25
<212> DNA
<213> Homo sapiens

<400> 2518
gtgagttccc gatgctggaa accgg 25

<210> 2519
<211> 25
<212> DNA
<213> Homo sapiens

<400> 2519
tgagttcccg atgctggaaa ccggc 25

<210> 2520
<211> 25
<212> DNA
<213> Homo sapiens

<400> 2520
gagttcccga tgctggaaac cggcc 25

<210> 2521
<211> 25
<212> DNA
<213> Homo sapiens

<400> 2521
agttcccgat gctggaaacc ggcct 25

<210> 2522
<211> 25
<212> DNA

<213> Homo sapiens

<400> 2522
gttcccgatg ctggaaaccg gcctg                                          25

<210> 2523
<211> 25
<212> DNA
<213> Homo sapiens

<400> 2523
ttcccgatgc tggaaaccgg cctgt                                          25

<210> 2524
<211> 25
<212> DNA
<213> Homo sapiens

<400> 2524
tcccgatgct ggaaaccggc ctgtt                                          25

<210> 2525
<211> 25
<212> DNA
<213> Homo sapiens

<400> 2525
cccgatgctg gaaaccggcc tgttt                                          25

<210> 2526
<211> 25
<212> DNA
<213> Homo sapiens

<400> 2526
ccgatgctgg aaaccggcct gtttt                                          25

<210> 2527
<211> 25
<212> DNA
<213> Homo sapiens

<400> 2527
cgatgctgga aaccggcctg ttttt                                          25

<210> 2528
<211> 25
<212> DNA
<213> Homo sapiens

<400> 2528
gatgctggaa accggcctgt ttttc                                          25

<210> 2529
<211> 25
<212> DNA
<213> Homo sapiens

<400> 2529
atgctggaaa ccggcctgtt tttcc                                          25

<210> 2530
<211> 25
<212> DNA
<213> Homo sapiens

<400> 2530
tgctggaaac cggcctgttt ttcct                                          25

<210> 2531
<211> 25
<212> DNA
<213> Homo sapiens

<400> 2531
gctggaaacc ggcctgtttt tcctg                                          25

<210> 2532
<211> 25
<212> DNA
<213> Homo sapiens

<400> 2532
ctggaaaccg gcctgttttt cctgc                                          25

<210> 2533
<211> 25
<212> DNA
<213> Homo sapiens

<400> 2533
tggaaaccgg cctgtttttc ctgct                                          25

<210> 2534
<211> 25
<212> DNA
<213> Homo sapiens

<400> 2534
ggaaaccggc ctgtttttcc tgctt                                          25

<210> 2535
<211> 25
<212> DNA
<213> Homo sapiens

<400> 2535
gaaaccggcc tgtttttcct gcttt                                          25

<210> 2536
<211> 25
<212> DNA
<213> Homo sapiens

<400> 2536
aaaccggcct gtttttcctg ctttc                                          25

<210> 2537
<211> 25
<212> DNA
<213> Homo sapiens

<400> 2537
aaccggcctg tttttcctgc tttct                                          25

<210> 2538
<211> 25
<212> DNA
<213> Homo sapiens

<400> 2538
accggcctgt ttttcctgct ttctt                                          25

<210> 2539
<211> 25
<212> DNA
<213> Homo sapiens

<400> 2539
ccggcctgtt tttcctgctt tcttg                                          25

<210> 2540
<211> 25
<212> DNA
<213> Homo sapiens

<400> 2540
cggcctgttt ttcctgcttt cttgg                                          25

<210> 2541
<211> 25
<212> DNA
<213> Homo sapiens

<400> 2541
ggcctgtttt tcctgctttc ttgga                                          25

<210> 2542
<211> 25
<212> DNA
<213> Homo sapiens

<400> 2542
gcctgttttt cctgctttct tggag                                          25

<210> 2543
<211> 25
<212> DNA
<213> Homo sapiens

<400> 2543
cctgtttttc ctgctttctt ggagt                                          25

<210> 2544
<211> 25
<212> DNA
<213> Homo sapiens

<400> 2544
ctgtttttcc tgctttcttg gagtg                                          25

<210> 2545
<211> 25

<212> DNA
<213> Homo sapiens

<400> 2545
tgtttttcct gctttcttgg agtgc                                          25

<210> 2546
<211> 25
<212> DNA
<213> Homo sapiens

<400> 2546
gtttttcctg ctttcttgga gtgcc                                          25

<210> 2547
<211> 25
<212> DNA
<213> Homo sapiens

<400> 2547
tttttcctgc tttcttggag tgcct                                          25

<210> 2548
<211> 25
<212> DNA
<213> Homo sapiens

<400> 2548
ttttcctgct ttcttggagt gcctt                                          25

<210> 2549
<211> 25
<212> DNA
<213> Homo sapiens

<400> 2549
tttcctgctt tcttggagtg ccttc                                          25

<210> 2550
<211> 25
<212> DNA
<213> Homo sapiens

<400> 2550
ttcctgcttt cttggagtgc cttcc                                          25

<210> 2551
<211> 25
<212> DNA
<213> Homo sapiens

<400> 2551
tcctgctttc ttggagtgcc ttcct                                          25

<210> 2552
<211> 25
<212> DNA
<213> Homo sapiens

<400> 2552
cctgctttct tggagtgcct tcctg                                          25

<210> 2553
<211> 25
<212> DNA
<213> Homo sapiens

<400> 2553
ctgctttctt ggagtgcctt cctgt                                          25

<210> 2554
<211> 25
<212> DNA
<213> Homo sapiens

<400> 2554
tgctttcttg gagtgccttc ctgtc                                          25

<210> 2555
<211> 25
<212> DNA
<213> Homo sapiens

<400> 2555
gctttcttgg agtgccttcc tgtct                                          25

<210> 2556
<211> 25
<212> DNA
<213> Homo sapiens

<400> 2556
ctttcttgga gtgccttcct gtctg                                          25

<210> 2557
<211> 25
<212> DNA
<213> Homo sapiens

<400> 2557
tttcttggag tgccttcctg tctgc                                          25

<210> 2558
<211> 25
<212> DNA
<213> Homo sapiens

<400> 2558
ttcttggagt gccttcctgt ctgcc                                          25

<210> 2559
<211> 25
<212> DNA
<213> Homo sapiens

<400> 2559
tcttggagtg ccttcctgtc tgccg                                          25

<210> 2560
<211> 25
<212> DNA
<213> Homo sapiens

<400> 2560
cttggagtgc cttcctgtct gccga                                          25

<210> 2561
<211> 25
<212> DNA
<213> Homo sapiens

<400> 2561
ttggagtgcc ttcctgtctg ccgag                                          25

<210> 2562
<211> 25
<212> DNA
<213> Homo sapiens

<400> 2562
tggagtgcct tcctgtctgc cgagg                                          25

<210> 2563
<211> 25
<212> DNA
<213> Homo sapiens

<400> 2563
ggagtgcctt cctgtctgcc gaggc                                          25

<210> 2564
<211> 25
<212> DNA
<213> Homo sapiens

<400> 2564
gagtgccttc ctgtctgccg aggct                                          25

<210> 2565
<211> 25
<212> DNA
<213> Homo sapiens

<400> 2565
agtgccttcc tgtctgccga ggctg                                          25

<210> 2566
<211> 25
<212> DNA
<213> Homo sapiens

<400> 2566
gtgccttcct gtctgccgag gctgc                                          25

<210> 2567
<211> 25
<212> DNA
<213> Homo sapiens

<400> 2567
tgccttcctg tctgccgagg ctgcc                                          25

<210> 2568

<211> 25
<212> DNA
<213> Homo sapiens

<400> 2568
gccttcctgt ctgccgaggc tgccg                                          25

<210> 2569
<211> 25
<212> DNA
<213> Homo sapiens

<400> 2569
ccttcctgtc tgccgaggct gccgg                                          25

<210> 2570
<211> 25
<212> DNA
<213> Homo sapiens

<400> 2570
cttcctgtct gccgaggctg ccggc                                          25

<210> 2571
<211> 25
<212> DNA
<213> Homo sapiens

<400> 2571
ttcctgtctg ccgaggctgc cggcc                                          25

<210> 2572
<211> 25
<212> DNA
<213> Homo sapiens

<400> 2572
tcctgtctgc cgaggctgcc ggcct                                          25

<210> 2573
<211> 25
<212> DNA
<213> Homo sapiens

<400> 2573
cctgtctgcc gaggctgccg gccta                                          25

<210> 2574
<211> 25
<212> DNA
<213> Homo sapiens

<400> 2574
ctgtctgccg aggctgccgg cctaa                                          25

<210> 2575
<211> 25
<212> DNA
<213> Homo sapiens

<400> 2575

tgtctgccga ggctgccggc ctaac                                          25

<210> 2576
<211> 25
<212> DNA
<213> Homo sapiens

<400> 2576
gtctgccgag gctgccggcc taaca                                          25

<210> 2577
<211> 25
<212> DNA
<213> Homo sapiens

<400> 2577
tctgccgagg ctgccggcct aacag                                          25

<210> 2578
<211> 25
<212> DNA
<213> Homo sapiens

<400> 2578
ctgccgaggc tgccggccta acagg                                          25

<210> 2579
<211> 25
<212> DNA
<213> Homo sapiens

<400> 2579
tgccgaggct gccggcctaa caggg                                          25

<210> 2580
<211> 25
<212> DNA
<213> Homo sapiens

<400> 2580
gccgaggctg ccggcctaac aggga                                          25

<210> 2581
<211> 25
<212> DNA
<213> Homo sapiens

<400> 2581
ccgaggctgc cggcctaaca gggat                                          25

<210> 2582
<211> 25
<212> DNA
<213> Homo sapiens

<400> 2582
cgaggctgcc ggcctaacag ggata                                          25

<210> 2583
<211> 25
<212> DNA

<213> Homo sapiens

<400> 2583
gaggctgccg gcctaacagg gatag                                          25

<210> 2584
<211> 25
<212> DNA
<213> Homo sapiens

<400> 2584
aggctgccgg cctaacaggg atagt                                          25

<210> 2585
<211> 25
<212> DNA
<213> Homo sapiens

<400> 2585
ggctgccggc ctaacaggga tagtt                                          25

<210> 2586
<211> 25
<212> DNA
<213> Homo sapiens

<400> 2586
gctgccggcc taacagggat agttg                                          25

<210> 2587
<211> 25
<212> DNA
<213> Homo sapiens

<400> 2587
ctgccggcct aacagggata gttgc                                          25

<210> 2588
<211> 25
<212> DNA
<213> Homo sapiens

<400> 2588
tgccggccta acagggatag ttgct                                          25

<210> 2589
<211> 25
<212> DNA
<213> Homo sapiens

<400> 2589
gccggcctaa cagggatagt tgctg                                          25

<210> 2590
<211> 25
<212> DNA
<213> Homo sapiens

<400> 2590
ccggcctaac agggatagtt gctgt                                          25

<210> 2591
<211> 25
<212> DNA
<213> Homo sapiens

<400> 2591
cggcctaaca gggatagttg ctgtt                                          25

<210> 2592
<211> 25
<212> DNA
<213> Homo sapiens

<400> 2592
ggcctaacag ggatagttgc tgttc                                          25

<210> 2593
<211> 25
<212> DNA
<213> Homo sapiens

<400> 2593
gcctaacagg gatagttgct gttct                                          25

<210> 2594
<211> 25
<212> DNA
<213> Homo sapiens

<400> 2594
cctaacaggg atagttgctg ttctc                                          25

<210> 2595
<211> 25
<212> DNA
<213> Homo sapiens

<400> 2595
ctaacaggga tagttgctgt tctct                                          25

<210> 2596
<211> 25
<212> DNA
<213> Homo sapiens

<400> 2596
taacagggat agttgctgtt ctctt                                          25

<210> 2597
<211> 25
<212> DNA
<213> Homo sapiens

<400> 2597
aacagggata gttgctgttc tcttc                                          25

<210> 2598
<211> 25
<212> DNA
<213> Homo sapiens

<400> 2598
acagggatag ttgctgttct cttct                                          25

<210> 2599
<211> 25
<212> DNA
<213> Homo sapiens

<400> 2599
cagggatagt tgctgttctc ttctg                                          25

<210> 2600
<211> 25
<212> DNA
<213> Homo sapiens

<400> 2600
agggatagtt gctgttctct tctgt                                          25

<210> 2601
<211> 25
<212> DNA
<213> Homo sapiens

<400> 2601
gggatagttg ctgttctctt ctgtg                                          25

<210> 2602
<211> 25
<212> DNA
<213> Homo sapiens

<400> 2602
ggatagttgc tgttctcttc tgtgg                                          25

<210> 2603
<211> 25
<212> DNA
<213> Homo sapiens

<400> 2603
gatagttgct gttctcttct gtgga                                          25

<210> 2604
<211> 25
<212> DNA
<213> Homo sapiens

<400> 2604
atagttgctg ttctcttctg tggag                                          25

<210> 2605
<211> 25
<212> DNA
<213> Homo sapiens

<400> 2605
tagttgctgt tctcttctgt ggagt                                          25

<210> 2606
<211> 25

<212> DNA
<213> Homo sapiens

<400> 2606
agttgctgtt ctcttctgtg gagtc                                             25

<210> 2607
<211> 25
<212> DNA
<213> Homo sapiens

<400> 2607
gttgctgttc tcttctgtgg agtca                                             25

<210> 2608
<211> 25
<212> DNA
<213> Homo sapiens

<400> 2608
ttgctgttct cttctgtgga gtcac                                             25

<210> 2609
<211> 25
<212> DNA
<213> Homo sapiens

<400> 2609
tgctgttctc ttctgtggag tcaca                                             25

<210> 2610
<211> 25
<212> DNA
<213> Homo sapiens

<400> 2610
gctgttctct tctgtggagt cacac                                             25

<210> 2611
<211> 25
<212> DNA
<213> Homo sapiens

<400> 2611
ctgttctctt ctgtggagtc acaca                                             25

<210> 2612
<211> 25
<212> DNA
<213> Homo sapiens

<400> 2612
tgttctcttc tgtggagtca cacaa                                             25

<210> 2613
<211> 25
<212> DNA
<213> Homo sapiens

<400> 2613
gttctcttct gtggagtcac acaag                                             25

<210> 2614
<211> 25
<212> DNA
<213> Homo sapiens

<400> 2614
ttctcttctg tggagtcaca caagc                                          25

<210> 2615
<211> 25
<212> DNA
<213> Homo sapiens

<400> 2615
tctcttctgt ggagtcacac aagca                                          25

<210> 2616
<211> 25
<212> DNA
<213> Homo sapiens

<400> 2616
ctcttctgtg gagtcacaca agcac                                          25

<210> 2617
<211> 25
<212> DNA
<213> Homo sapiens

<400> 2617
tcttctgtgg agtcacacaa gcaca                                          25

<210> 2618
<211> 25
<212> DNA
<213> Homo sapiens

<400> 2618
cttctgtgga gtcacacaag cacat                                          25

<210> 2619
<211> 25
<212> DNA
<213> Homo sapiens

<400> 2619
ttctgtggag tcacacaagc acatt                                          25

<210> 2620
<211> 25
<212> DNA
<213> Homo sapiens

<400> 2620
tctgtggagt cacacaagca catta                                          25

<210> 2621
<211> 25
<212> DNA
<213> Homo sapiens

<400> 2621
ctgtggagtc acacaagcac attat                                          25

<210> 2622
<211> 25
<212> DNA
<213> Homo sapiens

<400> 2622
tgtggagtca cacaagcaca ttata                                          25

<210> 2623
<211> 25
<212> DNA
<213> Homo sapiens

<400> 2623
gtggagtcac acaagcacat tatac                                          25

<210> 2624
<211> 25
<212> DNA
<213> Homo sapiens

<400> 2624
tggagtcaca caagcacatt atacc                                          25

<210> 2625
<211> 25
<212> DNA
<213> Homo sapiens

<400> 2625
ggagtcacac aagcacatta tacct                                          25

<210> 2626
<211> 25
<212> DNA
<213> Homo sapiens

<400> 2626
gagtcacaca agcacattat accta                                          25

<210> 2627
<211> 25
<212> DNA
<213> Homo sapiens

<400> 2627
agtcacacaa gcacattata cctac                                          25

<210> 2628
<211> 25
<212> DNA
<213> Homo sapiens

<400> 2628
gtcacacaag cacattatac ctaca                                          25

<210> 2629

<211> 25
<212> DNA
<213> Homo sapiens

<400> 2629
tcacacaagc acattatacc tacaa                                          25

<210> 2630
<211> 25
<212> DNA
<213> Homo sapiens

<400> 2630
cacacaagca cattatacct acaac                                          25

<210> 2631
<211> 25
<212> DNA
<213> Homo sapiens

<400> 2631
acacaagcac attataccta caaca                                          25

<210> 2632
<211> 25
<212> DNA
<213> Homo sapiens

<400> 2632
cacaagcaca ttatacctac aacaa                                          25

<210> 2633
<211> 25
<212> DNA
<213> Homo sapiens

<400> 2633
acaagcacat tatacctaca acaat                                          25

<210> 2634
<211> 25
<212> DNA
<213> Homo sapiens

<400> 2634
caagcacatt atacctacaa caatc                                          25

<210> 2635
<211> 25
<212> DNA
<213> Homo sapiens

<400> 2635
aagcacatta tacctacaac aatct                                          25

<210> 2636
<211> 25
<212> DNA
<213> Homo sapiens

<400> 2636

agcacattat acctacaaca atctg                                          25

<210> 2637
<211> 25
<212> DNA
<213> Homo sapiens

<400> 2637
gcacattata cctacaacaa tctgt                                          25

<210> 2638
<211> 25
<212> DNA
<213> Homo sapiens

<400> 2638
cacattatac ctacaacaat ctgtc                                          25

<210> 2639
<211> 25
<212> DNA
<213> Homo sapiens

<400> 2639
acattatacc tacaacaatc tgtct                                          25

<210> 2640
<211> 25
<212> DNA
<213> Homo sapiens

<400> 2640
cattatacct acaacaatct gtctt                                          25

<210> 2641
<211> 25
<212> DNA
<213> Homo sapiens

<400> 2641
attataccta caacaatctg tcttc                                          25

<210> 2642
<211> 25
<212> DNA
<213> Homo sapiens

<400> 2642
ttatacctac aacaatctgt cttcg                                          25

<210> 2643
<211> 25
<212> DNA
<213> Homo sapiens

<400> 2643
tatacctaca acaatctgtc ttcgg                                          25

<210> 2644
<211> 25
<212> DNA

<213> Homo sapiens

<400> 2644
atacctacaa caatctgtct tcgga                                            25

<210> 2645
<211> 25
<212> DNA
<213> Homo sapiens

<400> 2645
tacctacaac aatctgtctt cggat                                            25

<210> 2646
<211> 25
<212> DNA
<213> Homo sapiens

<400> 2646
acctacaaca atctgtcttc ggatt                                            25

<210> 2647
<211> 25
<212> DNA
<213> Homo sapiens

<400> 2647
cctacaacaa tctgtcttcg gattc                                            25

<210> 2648
<211> 25
<212> DNA
<213> Homo sapiens

<400> 2648
ctacaacaat ctgtcttcgg attcc                                            25

<210> 2649
<211> 25
<212> DNA
<213> Homo sapiens

<400> 2649
tacaacaatc tgtcttcgga ttcca                                            25

<210> 2650
<211> 25
<212> DNA
<213> Homo sapiens

<400> 2650
acaacaatct gtcttcggat tccaa                                            25

<210> 2651
<211> 25
<212> DNA
<213> Homo sapiens

<400> 2651
caacaatctg tcttcggatt ccaaa                                            25

<210> 2652
<211> 25
<212> DNA
<213> Homo sapiens

<400> 2652
aacaatctgt cttcggattc caaaa                                             25

<210> 2653
<211> 25
<212> DNA
<213> Homo sapiens

<400> 2653
acaatctgtc ttcggattcc aaaat                                             25

<210> 2654
<211> 25
<212> DNA
<213> Homo sapiens

<400> 2654
caatctgtct tcggattcca aaata                                             25

<210> 2655
<211> 25
<212> DNA
<213> Homo sapiens

<400> 2655
aatctgtctt cggattccaa aataa                                             25

<210> 2656
<211> 25
<212> DNA
<213> Homo sapiens

<400> 2656
atctgtcttc ggattccaaa ataag                                             25

<210> 2657
<211> 25
<212> DNA
<213> Homo sapiens

<400> 2657
tctgtcttcg gattccaaaa taaga                                             25

<210> 2658
<211> 25
<212> DNA
<213> Homo sapiens

<400> 2658
ctgtcttcgg attccaaaat aagaa                                             25

<210> 2659
<211> 25
<212> DNA
<213> Homo sapiens

<400> 2659
tgtcttcgga ttccaaaata agaac                                          25

<210> 2660
<211> 25
<212> DNA
<213> Homo sapiens

<400> 2660
gtcttcggat tccaaaataa gaact                                          25

<210> 2661
<211> 25
<212> DNA
<213> Homo sapiens

<400> 2661
tcttcggatt ccaaaataag aacta                                          25

<210> 2662
<211> 25
<212> DNA
<213> Homo sapiens

<400> 2662
cttcggattc caaaataaga actaa                                          25

<210> 2663
<211> 25
<212> DNA
<213> Homo sapiens

<400> 2663
ttcggattcc aaaataagaa ctaaa                                          25

<210> 2664
<211> 25
<212> DNA
<213> Homo sapiens

<400> 2664
tcggattcca aaataagaac taaac                                          25

<210> 2665
<211> 25
<212> DNA
<213> Homo sapiens

<400> 2665
cggattccaa aataagaact aaaca                                          25

<210> 2666
<211> 25
<212> DNA
<213> Homo sapiens

<400> 2666
ggattccaaa ataagaacta aacag                                          25

<210> 2667
<211> 25

<212> DNA
<213> Homo sapiens

<400> 2667
gattccaaaa taagaactaa acagt                                          25

<210> 2668
<211> 25
<212> DNA
<213> Homo sapiens

<400> 2668
attccaaaat aagaactaaa cagtt                                          25

<210> 2669
<211> 25
<212> DNA
<213> Homo sapiens

<400> 2669
ttccaaaata agaactaaac agttg                                          25

<210> 2670
<211> 25
<212> DNA
<213> Homo sapiens

<400> 2670
tccaaaataa gaactaaaca gttgt                                          25

<210> 2671
<211> 25
<212> DNA
<213> Homo sapiens

<400> 2671
ccaaaataag aactaaacag ttgtt                                          25

<210> 2672
<211> 25
<212> DNA
<213> Homo sapiens

<400> 2672
caaaataaga actaaacagt tgttt                                          25

<210> 2673
<211> 25
<212> DNA
<213> Homo sapiens

<400> 2673
aaaataagaa ctaaacagtt gtttg                                          25

<210> 2674
<211> 25
<212> DNA
<213> Homo sapiens

<400> 2674
aaataagaac taaacagttg tttga                                          25

<210> 2675
<211> 25
<212> DNA
<213> Homo sapiens

<400> 2675
aataagaact aaacagttgt ttgaa                                            25

<210> 2676
<211> 25
<212> DNA
<213> Homo sapiens

<400> 2676
ataagaacta aacagttgtt tgaat                                            25

<210> 2677
<211> 25
<212> DNA
<213> Homo sapiens

<400> 2677
taagaactaa acagttgttt gaatt                                            25

<210> 2678
<211> 25
<212> DNA
<213> Homo sapiens

<400> 2678
aagaactaaa cagttgtttg aattt                                            25

<210> 2679
<211> 25
<212> DNA
<213> Homo sapiens

<400> 2679
agaactaaac agttgtttga attta                                            25

<210> 2680
<211> 25
<212> DNA
<213> Homo sapiens

<400> 2680
gaactaaaca gttgtttgaa tttat                                            25

<210> 2681
<211> 25
<212> DNA
<213> Homo sapiens

<400> 2681
aactaaacag ttgtttgaat ttatg                                            25

<210> 2682
<211> 25
<212> DNA
<213> Homo sapiens

<400> 2682
actaaacagt tgtttgaatt tatga                                          25

<210> 2683
<211> 25
<212> DNA
<213> Homo sapiens

<400> 2683
ctaaacagtt gtttgaattt atgaa                                          25

<210> 2684
<211> 25
<212> DNA
<213> Homo sapiens

<400> 2684
taaacagttg tttgaattta tgaac                                          25

<210> 2685
<211> 25
<212> DNA
<213> Homo sapiens

<400> 2685
aaacagttgt ttgaatttat gaact                                          25

<210> 2686
<211> 25
<212> DNA
<213> Homo sapiens

<400> 2686
aacagttgtt tgaatttatg aactt                                          25

<210> 2687
<211> 25
<212> DNA
<213> Homo sapiens

<400> 2687
acagttgttt gaatttatga acttt                                          25

<210> 2688
<211> 25
<212> DNA
<213> Homo sapiens

<400> 2688
cagttgtttg aatttatgaa ctttt                                          25

<210> 2689
<211> 25
<212> DNA
<213> Homo sapiens

<400> 2689
agttgtttga atttatgaac ttttt                                          25

<210> 2690

<211> 25
<212> DNA
<213> Homo sapiens

<400> 2690
gttgtttgaa tttatgaact ttttg                                            25

<210> 2691
<211> 25
<212> DNA
<213> Homo sapiens

<400> 2691
ttgtttgaat ttatgaactt tttgg                                            25

<210> 2692
<211> 25
<212> DNA
<213> Homo sapiens

<400> 2692
tgtttgaatt tatgaacttt ttggc                                            25

<210> 2693
<211> 25
<212> DNA
<213> Homo sapiens

<400> 2693
gtttgaattt atgaactttt tggcg                                            25

<210> 2694
<211> 25
<212> DNA
<213> Homo sapiens

<400> 2694
tttgaattta tgaacttttt ggcgg                                            25

<210> 2695
<211> 25
<212> DNA
<213> Homo sapiens

<400> 2695
ttgaatttat gaactttttg gcgga                                            25

<210> 2696
<211> 25
<212> DNA
<213> Homo sapiens

<400> 2696
tgaatttatg aactttttgg cggag                                            25

<210> 2697
<211> 25
<212> DNA
<213> Homo sapiens

<400> 2697

gaatttatga actttttggc ggaga                                          25

<210> 2698
<211> 25
<212> DNA
<213> Homo sapiens

<400> 2698
aatttatgaa ctttttggcg gagaa                                          25

<210> 2699
<211> 25
<212> DNA
<213> Homo sapiens

<400> 2699
atttatgaac tttttggcgg agaac                                          25

<210> 2700
<211> 25
<212> DNA
<213> Homo sapiens

<400> 2700
tttatgaact ttttggcgga gaacg                                          25

<210> 2701
<211> 25
<212> DNA
<213> Homo sapiens

<400> 2701
ttatgaactt tttggcggag aacgt                                          25

<210> 2702
<211> 25
<212> DNA
<213> Homo sapiens

<400> 2702
tatgaacttt ttggcggaga acgtc                                          25

<210> 2703
<211> 25
<212> DNA
<213> Homo sapiens

<400> 2703
atgaactttt tggcggagaa cgtca                                          25

<210> 2704
<211> 25
<212> DNA
<213> Homo sapiens

<400> 2704
tgaacttttt ggcggagaac gtcat                                          25

<210> 2705
<211> 25
<212> DNA

<213> Homo sapiens

<400> 2705
   gaactttttg gcggagaacg tcatc                                          25

<210> 2706
<211> 25
<212> DNA
<213> Homo sapiens

<400> 2706
aactttttgg cggagaacgt catct                                          25

<210> 2707
<211> 25
<212> DNA
<213> Homo sapiens

<400> 2707
actttttggc ggagaacgtc atctt                                          25

<210> 2708
<211> 25
<212> DNA
<213> Homo sapiens

<400> 2708
ctttttggcg gagaacgtca tcttc                                          25

<210> 2709
<211> 25
<212> DNA
<213> Homo sapiens

<400> 2709
tttttggcgg agaacgtcat cttct                                          25

<210> 2710
<211> 25
<212> DNA
<213> Homo sapiens

<400> 2710
ttttggcgga gaacgtcatc ttctg                                          25

<210> 2711
<211> 25
<212> DNA
<213> Homo sapiens

<400> 2711
tttggcggag aacgtcatct tctgt                                          25

<210> 2712
<211> 25
<212> DNA
<213> Homo sapiens

<400> 2712
ttggcggaga acgtcatctt ctgtt                                          25

<210> 2713
<211> 25
<212> DNA
<213> Homo sapiens

<400> 2713
tggcggagaa cgtcatcttc tgtta                                          25

<210> 2714
<211> 25
<212> DNA
<213> Homo sapiens

<400> 2714
ggcggagaac gtcatcttct gttac                                          25

<210> 2715
<211> 25
<212> DNA
<213> Homo sapiens

<400> 2715
gcggagaacg tcatcttctg ttaca                                          25

<210> 2716
<211> 25
<212> DNA
<213> Homo sapiens

<400> 2716
cggagaacgt catcttctgt tacat                                          25

<210> 2717
<211> 25
<212> DNA
<213> Homo sapiens

<400> 2717
ggagaacgtc atcttctgtt acatg                                          25

<210> 2718
<211> 25
<212> DNA
<213> Homo sapiens

<400> 2718
gagaacgtca tcttctgtta catgg                                          25

<210> 2719
<211> 25
<212> DNA
<213> Homo sapiens

<400> 2719
agaacgtcat cttctgttac atggg                                          25

<210> 2720
<211> 25
<212> DNA
<213> Homo sapiens

<400> 2720
gaacgtcatc ttctgttaca tgggc                                          25

<210> 2721
<211> 25
<212> DNA
<213> Homo sapiens

<400> 2721
aacgtcatct tctgttacat gggcc                                          25

<210> 2722
<211> 25
<212> DNA
<213> Homo sapiens

<400> 2722
acgtcatctt ctgttacatg ggcct                                          25

<210> 2723
<211> 25
<212> DNA
<213> Homo sapiens

<400> 2723
cgtcatcttc tgttacatgg gcctg                                          25

<210> 2724
<211> 25
<212> DNA
<213> Homo sapiens

<400> 2724
gtcatcttct gttacatggg cctgg                                          25

<210> 2725
<211> 25
<212> DNA
<213> Homo sapiens

<400> 2725
tcatcttctg ttacatgggc ctggc                                          25

<210> 2726
<211> 25
<212> DNA
<213> Homo sapiens

<400> 2726
catcttctgt tacatgggcc tggca                                          25

<210> 2727
<211> 25
<212> DNA
<213> Homo sapiens

<400> 2727
atcttctgtt acatgggcct ggcac                                          25

<210> 2728
<211> 25

<212> DNA
<213> Homo sapiens

<400> 2728
tcttctgtta catgggcctg gcact                                            25

<210> 2729
<211> 25
<212> DNA
<213> Homo sapiens

<400> 2729
cttctgttac atgggcctgg cactg                                            25

<210> 2730
<211> 25
<212> DNA
<213> Homo sapiens

<400> 2730
ttctgttaca tgggcctggc actgt                                            25

<210> 2731
<211> 25
<212> DNA
<213> Homo sapiens

<400> 2731
tctgttacat gggcctggca ctgtt                                            25

<210> 2732
<211> 25
<212> DNA
<213> Homo sapiens

<400> 2732
ctgttacatg ggcctggcac tgttc                                            25

<210> 2733
<211> 25
<212> DNA
<213> Homo sapiens

<400> 2733
tgttacatgg gcctggcact gttca                                            25

<210> 2734
<211> 25
<212> DNA
<213> Homo sapiens

<400> 2734
gttacatggg cctggcactg ttcac                                            25

<210> 2735
<211> 25
<212> DNA
<213> Homo sapiens

<400> 2735
ttacatgggc ctggcactgt tcacg                                            25

<210> 2736
<211> 25
<212> DNA
<213> Homo sapiens

<400> 2736
tacatgggcc tggcactgtt cacgt                                            25

<210> 2737
<211> 25
<212> DNA
<213> Homo sapiens

<400> 2737
acatgggcct ggcactgttc acgtt                                            25

<210> 2738
<211> 25
<212> DNA
<213> Homo sapiens

<400> 2738
catgggcctg gcactgttca cgttc                                            25

<210> 2739
<211> 25
<212> DNA
<213> Homo sapiens

<400> 2739
atgggcctgg cactgttcac gttcc                                            25

<210> 2740
<211> 25
<212> DNA
<213> Homo sapiens

<400> 2740
tgggcctggc actgttcacg ttcca                                            25

<210> 2741
<211> 25
<212> DNA
<213> Homo sapiens

<400> 2741
gggcctggca ctgttcacgt tccag                                            25

<210> 2742
<211> 25
<212> DNA
<213> Homo sapiens

<400> 2742
ggcctggcac tgttcacgtt ccaga                                            25

<210> 2743
<211> 25
<212> DNA
<213> Homo sapiens

<400> 2743
gcctggcact gttcacgttc cagaa                                          25

<210> 2744
<211> 25
<212> DNA
<213> Homo sapiens

<400> 2744
cctggcactg ttcacgttcc agaat                                          25

<210> 2745
<211> 25
<212> DNA
<213> Homo sapiens

<400> 2745
ctggcactgt tcacgttcca gaatc                                          25

<210> 2746
<211> 25
<212> DNA
<213> Homo sapiens

<400> 2746
tggcactgtt cacgttccag aatca                                          25

<210> 2747
<211> 25
<212> DNA
<213> Homo sapiens

<400> 2747
ggcactgttc acgttccaga atcat                                          25

<210> 2748
<211> 25
<212> DNA
<213> Homo sapiens

<400> 2748
gcactgttca cgttccagaa tcata                                          25

<210> 2749
<211> 25
<212> DNA
<213> Homo sapiens

<400> 2749
cactgttcac gttccagaat catat                                          25

<210> 2750
<211> 25
<212> DNA
<213> Homo sapiens

<400> 2750
actgttcacg ttccagaatc atatc                                          25

<210> 2751

<211> 25
<212> DNA
<213> Homo sapiens

<400> 2751
ctgttcacgt tccagaatca tatct                                          25

<210> 2752
<211> 25
<212> DNA
<213> Homo sapiens

<400> 2752
tgttcacgtt ccagaatcat atctt                                          25

<210> 2753
<211> 25
<212> DNA
<213> Homo sapiens

<400> 2753
gttcacgttc cagaatcata tcttt                                          25

<210> 2754
<211> 25
<212> DNA
<213> Homo sapiens

<400> 2754
ttcacgttcc agaatcatat cttta                                          25

<210> 2755
<211> 25
<212> DNA
<213> Homo sapiens

<400> 2755
tcacgttcca gaatcatatc tttaa                                          25

<210> 2756
<211> 25
<212> DNA
<213> Homo sapiens

<400> 2756
cacgttccag aatcatatct ttaat                                          25

<210> 2757
<211> 25
<212> DNA
<213> Homo sapiens

<400> 2757
acgttccaga atcatatctt taatg                                          25

<210> 2758
<211> 25
<212> DNA
<213> Homo sapiens

<400> 2758

cgttccagaa tcatatcttt aatgc 25

<210> 2759
<211> 25
<212> DNA
<213> Homo sapiens

<400> 2759
gttccagaat catatcttta atgct 25

<210> 2760
<211> 25
<212> DNA
<213> Homo sapiens

<400> 2760
ttccagaatc atatctttaa tgctc 25

<210> 2761
<211> 25
<212> DNA
<213> Homo sapiens

<400> 2761
tccagaatca tatctttaat gctct 25

<210> 2762
<211> 25
<212> DNA
<213> Homo sapiens

<400> 2762
ccagaatcat atctttaatg ctctt 25

<210> 2763
<211> 25
<212> DNA
<213> Homo sapiens

<400> 2763
cagaatcata tctttaatgc tcttt 25

<210> 2764
<211> 25
<212> DNA
<213> Homo sapiens

<400> 2764
agaatcatat ctttaatgct ctttt 25

<210> 2765
<211> 25
<212> DNA
<213> Homo sapiens

<400> 2765
gaatcatatc tttaatgctc ttttt 25

<210> 2766
<211> 25
<212> DNA

<213> Homo sapiens

<400> 2766
aatcatatct ttaatgctct tttta                                          25

<210> 2767
<211> 25
<212> DNA
<213> Homo sapiens

<400> 2767
atcatatctt taatgctctt tttat                                          25

<210> 2768
<211> 25
<212> DNA
<213> Homo sapiens

<400> 2768
tcatatcttt aatgctcttt ttata                                          25

<210> 2769
<211> 25
<212> DNA
<213> Homo sapiens

<400> 2769
catatcttta atgctctttt tatac                                          25

<210> 2770
<211> 25
<212> DNA
<213> Homo sapiens

<400> 2770
atatctttaa tgctcttttt atact                                          25

<210> 2771
<211> 25
<212> DNA
<213> Homo sapiens

<400> 2771
tatctttaat gctcttttta tactt                                          25

<210> 2772
<211> 25
<212> DNA
<213> Homo sapiens

<400> 2772
atctttaatg ctctttttat acttg                                          25

<210> 2773
<211> 25
<212> DNA
<213> Homo sapiens

<400> 2773
tctttaatgc tctttttata cttgg                                          25

<210> 2774
<211> 25
<212> DNA
<213> Homo sapiens

<400> 2774
ctttaatgct ctttttatac ttgga                                          25

<210> 2775
<211> 25
<212> DNA
<213> Homo sapiens

<400> 2775
tttaatgctc tttttatact tggag                                          25

<210> 2776
<211> 25
<212> DNA
<213> Homo sapiens

<400> 2776
ttaatgctct ttttatactt ggagc                                          25

<210> 2777
<211> 25
<212> DNA
<213> Homo sapiens

<400> 2777
taatgctctt tttatacttg gagcc                                          25

<210> 2778
<211> 25
<212> DNA
<213> Homo sapiens

<400> 2778
aatgctcttt ttatacttgg agcct                                          25

<210> 2779
<211> 25
<212> DNA
<213> Homo sapiens

<400> 2779
atgctctttt tatacttgga gcctt                                          25

<210> 2780
<211> 25
<212> DNA
<213> Homo sapiens

<400> 2780
tgctcttttt atacttggag ccttt                                          25

<210> 2781
<211> 25
<212> DNA
<213> Homo sapiens

<400> 2781
gctcttttta tacttggagc ctttc                                          25

<210> 2782
<211> 25
<212> DNA
<213> Homo sapiens

<400> 2782
ctctttttat acttggagcc tttct                                          25

<210> 2783
<211> 25
<212> DNA
<213> Homo sapiens

<400> 2783
tctttttata cttggagcct ttcta                                          25

<210> 2784
<211> 25
<212> DNA
<213> Homo sapiens

<400> 2784
ctttttatac ttggagcctt tctag                                          25

<210> 2785
<211> 25
<212> DNA
<213> Homo sapiens

<400> 2785
tttttatact tggagccttt ctagc                                          25

<210> 2786
<211> 25
<212> DNA
<213> Homo sapiens

<400> 2786
ttttatactt ggagcctttc tagca                                          25

<210> 2787
<211> 25
<212> DNA
<213> Homo sapiens

<400> 2787
tttatacttg gagcctttct agcaa                                          25

<210> 2788
<211> 25
<212> DNA
<213> Homo sapiens

<400> 2788
ttatacttgg agcctttcta gcaat                                          25

<210> 2789
<211> 25

<212> DNA
<213> Homo sapiens

<400> 2789
tatacttgga gcctttctag caatt                                          25

<210> 2790
<211> 25
<212> DNA
<213> Homo sapiens

<400> 2790
atacttggag cctttctagc aattt                                          25

<210> 2791
<211> 25
<212> DNA
<213> Homo sapiens

<400> 2791
tacttggagc ctttctagca atttt                                          25

<210> 2792
<211> 25
<212> DNA
<213> Homo sapiens

<400> 2792
acttggagcc tttctagcaa ttttt                                          25

<210> 2793
<211> 25
<212> DNA
<213> Homo sapiens

<400> 2793
cttggagcct ttctagcaat ttttg                                          25

<210> 2794
<211> 25
<212> DNA
<213> Homo sapiens

<400> 2794
ttggagcctt tctagcaatt tttgt                                          25

<210> 2795
<211> 25
<212> DNA
<213> Homo sapiens

<400> 2795
tggagccttt ctagcaattt ttgtt                                          25

<210> 2796
<211> 25
<212> DNA
<213> Homo sapiens

<400> 2796
ggagcctttc tagcaatttt tgttg                                          25

<210> 2797
<211> 25
<212> DNA
<213> Homo sapiens

<400> 2797
gagcctttct agcaattttt gttgc                                          25

<210> 2798
<211> 25
<212> DNA
<213> Homo sapiens

<400> 2798
agcctttcta gcaatttttg ttgcc                                          25

<210> 2799
<211> 25
<212> DNA
<213> Homo sapiens

<400> 2799
gcctttctag caatttttgt tgcca                                          25

<210> 2800
<211> 25
<212> DNA
<213> Homo sapiens

<400> 2800
cctttctagc aatttttgtt gccag                                          25

<210> 2801
<211> 25
<212> DNA
<213> Homo sapiens

<400> 2801
ctttctagca atttttgttg ccaga                                          25

<210> 2802
<211> 25
<212> DNA
<213> Homo sapiens

<400> 2802
tttctagcaa tttttgttgc cagag                                          25

<210> 2803
<211> 25
<212> DNA
<213> Homo sapiens

<400> 2803
ttctagcaat ttttgttgcc agagc                                          25

<210> 2804
<211> 25
<212> DNA
<213> Homo sapiens

<400> 2804
tctagcaatt tttgttgcca gagcc                                          25

<210> 2805
<211> 25
<212> DNA
<213> Homo sapiens

<400> 2805
ctagcaattt ttgttgccag agcct                                          25

<210> 2806
<211> 25
<212> DNA
<213> Homo sapiens

<400> 2806
tagcaatttt tgttgccaga gcctg                                          25

<210> 2807
<211> 25
<212> DNA
<213> Homo sapiens

<400> 2807
agcaattttt gttgccagag cctgc                                          25

<210> 2808
<211> 25
<212> DNA
<213> Homo sapiens

<400> 2808
gcaatttttg ttgccagagc ctgca                                          25

<210> 2809
<211> 25
<212> DNA
<213> Homo sapiens

<400> 2809
caatttttgt tgccagagcc tgcaa                                          25

<210> 2810
<211> 25
<212> DNA
<213> Homo sapiens

<400> 2810
aatttttgtt gccagagcct gcaac                                          25

<210> 2811
<211> 25
<212> DNA
<213> Homo sapiens

<400> 2811
atttttgttg ccagagcctg caaca                                          25

<210> 2812

<211> 25
<212> DNA
<213> Homo sapiens

<400> 2812
tttttgttgc cagagcctgc aacat                                            25

<210> 2813
<211> 25
<212> DNA
<213> Homo sapiens

<400> 2813
ttttgttgcc agagcctgca acata                                            25

<210> 2814
<211> 25
<212> DNA
<213> Homo sapiens

<400> 2814
tttgttgcca gagcctgcaa catat                                            25

<210> 2815
<211> 25
<212> DNA
<213> Homo sapiens

<400> 2815
ttgttgccag agcctgcaac atata                                            25

<210> 2816
<211> 25
<212> DNA
<213> Homo sapiens

<400> 2816
tgttgccaga gcctgcaaca tatat                                            25

<210> 2817
<211> 25
<212> DNA
<213> Homo sapiens

<400> 2817
gttgccagag cctgcaacat atatc                                            25

<210> 2818
<211> 25
<212> DNA
<213> Homo sapiens

<400> 2818
ttgccagagc ctgcaacata tatcc                                            25

<210> 2819
<211> 25
<212> DNA
<213> Homo sapiens

<400> 2819

tgccagagcc tgcaacatat atccc                                          25

<210> 2820
<211> 25
<212> DNA
<213> Homo sapiens

<400> 2820
gccagagcct gcaacatata tcccc                                          25

<210> 2821
<211> 25
<212> DNA
<213> Homo sapiens

<400> 2821
ccagagcctg caacatatat cccct                                          25

<210> 2822
<211> 25
<212> DNA
<213> Homo sapiens

<400> 2822
cagagcctgc aacatatatc ccctc                                          25

<210> 2823
<211> 25
<212> DNA
<213> Homo sapiens

<400> 2823
agagcctgca acatatatcc cctct                                          25

<210> 2824
<211> 25
<212> DNA
<213> Homo sapiens

<400> 2824
gagcctgcaa catatatccc ctctc                                          25

<210> 2825
<211> 25
<212> DNA
<213> Homo sapiens

<400> 2825
agcctgcaac atatatcccc tctcc                                          25

<210> 2826
<211> 25
<212> DNA
<213> Homo sapiens

<400> 2826
gcctgcaaca tatatcccct ctcct                                          25

<210> 2827
<211> 25
<212> DNA

<213> Homo sapiens

<400> 2827
cctgcaacat atatcccctc tcctt                                          25

<210> 2828
<211> 25
<212> DNA
<213> Homo sapiens

<400> 2828
ctgcaacata tatcccctct ccttc                                          25

<210> 2829
<211> 25
<212> DNA
<213> Homo sapiens

<400> 2829
tgcaacatat atcccctctc cttcc                                          25

<210> 2830
<211> 25
<212> DNA
<213> Homo sapiens

<400> 2830
gcaacatata tcccctctcc ttcct                                          25

<210> 2831
<211> 25
<212> DNA
<213> Homo sapiens

<400> 2831
caacatatat cccctctcct tcctc                                          25

<210> 2832
<211> 25
<212> DNA
<213> Homo sapiens

<400> 2832
aacatatatc ccctctcctt cctcc                                          25

<210> 2833
<211> 25
<212> DNA
<213> Homo sapiens

<400> 2833
acatatatcc cctctccttc ctcct                                          25

<210> 2834
<211> 25
<212> DNA
<213> Homo sapiens

<400> 2834
catatatccc ctctccttcc tcctg                                          25

<210> 2835
<211> 25
<212> DNA
<213> Homo sapiens

<400> 2835
atatatcccc tctccttcct cctga                                            25

<210> 2836
<211> 25
<212> DNA
<213> Homo sapiens

<400> 2836
tatatcccct ctccttcctc ctgaa                                            25

<210> 2837
<211> 25
<212> DNA
<213> Homo sapiens

<400> 2837
atatcccctc tccttcctcc tgaat                                            25

<210> 2838
<211> 25
<212> DNA
<213> Homo sapiens

<400> 2838
tatcccctct ccttcctcct gaatc                                            25

<210> 2839
<211> 25
<212> DNA
<213> Homo sapiens

<400> 2839
atcccctctc cttcctcctg aatct                                            25

<210> 2840
<211> 25
<212> DNA
<213> Homo sapiens

<400> 2840
tcccctctcc ttcctcctga atcta                                            25

<210> 2841
<211> 25
<212> DNA
<213> Homo sapiens

<400> 2841
cccctctcct tcctcctgaa tctag                                            25

<210> 2842
<211> 25
<212> DNA
<213> Homo sapiens

<400> 2842
ccctctcctt cctcctgaat ctagg                                          25

<210> 2843
<211> 25
<212> DNA
<213> Homo sapiens

<400> 2843
cctctccttc ctcctgaatc taggc                                          25

<210> 2844
<211> 25
<212> DNA
<213> Homo sapiens

<400> 2844
ctctccttcc tcctgaatct aggcc                                          25

<210> 2845
<211> 25
<212> DNA
<213> Homo sapiens

<400> 2845
tctccttcct cctgaatcta ggccg                                          25

<210> 2846
<211> 25
<212> DNA
<213> Homo sapiens

<400> 2846
ctccttcctc ctgaatctag gccga                                          25

<210> 2847
<211> 25
<212> DNA
<213> Homo sapiens

<400> 2847
tccttcctcc tgaatctagg ccgaa                                          25

<210> 2848
<211> 25
<212> DNA
<213> Homo sapiens

<400> 2848
ccttcctcct gaatctaggc cgaaa                                          25

<210> 2849
<211> 25
<212> DNA
<213> Homo sapiens

<400> 2849
cttcctcctg aatctaggcc gaaaa                                          25

<210> 2850
<211> 25

<212> DNA
<213> Homo sapiens

<400> 2850
ttcctcctga atctaggccg aaaac                                          25

<210> 2851
<211> 25
<212> DNA
<213> Homo sapiens

<400> 2851
tcctcctgaa tctaggccga aaaca                                          25

<210> 2852
<211> 25
<212> DNA
<213> Homo sapiens

<400> 2852
cctcctgaat ctaggccgaa aacag                                          25

<210> 2853
<211> 25
<212> DNA
<213> Homo sapiens

<400> 2853
ctcctgaatc taggccgaaa acaga                                          25

<210> 2854
<211> 25
<212> DNA
<213> Homo sapiens

<400> 2854
tcctgaatct aggccgaaaa cagaa                                          25

<210> 2855
<211> 25
<212> DNA
<213> Homo sapiens

<400> 2855
cctgaatcta ggccgaaaac agaag                                          25

<210> 2856
<211> 25
<212> DNA
<213> Homo sapiens

<400> 2856
ctgaatctag gccgaaaaca gaaga                                          25

<210> 2857
<211> 25
<212> DNA
<213> Homo sapiens

<400> 2857
tgaatctagg ccgaaaacag aagat                                          25

<210> 2858
<211> 25
<212> DNA
<213> Homo sapiens

<400> 2858
gaatctaggc cgaaaacaga agatc                                          25

<210> 2859
<211> 25
<212> DNA
<213> Homo sapiens

<400> 2859
aatctaggcc gaaaacagaa gatcc                                          25

<210> 2860
<211> 25
<212> DNA
<213> Homo sapiens

<400> 2860
atctaggccg aaaacagaag atccc                                          25

<210> 2861
<211> 25
<212> DNA
<213> Homo sapiens

<400> 2861
tctaggccga aaacagaaga tcccc                                          25

<210> 2862
<211> 25
<212> DNA
<213> Homo sapiens

<400> 2862
ctaggccgaa aacagaagat cccct                                          25

<210> 2863
<211> 25
<212> DNA
<213> Homo sapiens

<400> 2863
taggccgaaa acagaagatc ccctg                                          25

<210> 2864
<211> 25
<212> DNA
<213> Homo sapiens

<400> 2864
aggccgaaaa cagaagatcc cctgg                                          25

<210> 2865
<211> 25
<212> DNA
<213> Homo sapiens

<400> 2865
ggccgaaaac agaagatccc ctgga                                          25

<210> 2866
<211> 25
<212> DNA
<213> Homo sapiens

<400> 2866
gccgaaaaca gaagatcccc tggaa                                          25

<210> 2867
<211> 25
<212> DNA
<213> Homo sapiens

<400> 2867
ccgaaaacag aagatcccct ggaac                                          25

<210> 2868
<211> 25
<212> DNA
<213> Homo sapiens

<400> 2868
cgaaaacaga agatcccctg gaact                                          25

<210> 2869
<211> 25
<212> DNA
<213> Homo sapiens

<400> 2869
gaaaacagaa gatcccctgg aactt                                          25

<210> 2870
<211> 25
<212> DNA
<213> Homo sapiens

<400> 2870
aaaacagaag atcccctgga acttt                                          25

<210> 2871
<211> 25
<212> DNA
<213> Homo sapiens

<400> 2871
aaacagaaga tcccctggaa ctttc                                          25

<210> 2872
<211> 25
<212> DNA
<213> Homo sapiens

<400> 2872
aacagaagat cccctggaac tttca                                          25

<210> 2873

<211> 25
<212> DNA
<213> Homo sapiens

<400> 2873
acagaagatc ccctggaact ttcag                                          25

<210> 2874
<211> 25
<212> DNA
<213> Homo sapiens

<400> 2874
cagaagatcc cctggaactt tcagc                                          25

<210> 2875
<211> 25
<212> DNA
<213> Homo sapiens

<400> 2875
agaagatccc ctggaacttt cagca                                          25

<210> 2876
<211> 25
<212> DNA
<213> Homo sapiens

<400> 2876
gaagatcccc tggaactttc agcac                                          25

<210> 2877
<211> 25
<212> DNA
<213> Homo sapiens

<400> 2877
aagatcccct ggaactttca gcaca                                          25

<210> 2878
<211> 25
<212> DNA
<213> Homo sapiens

<400> 2878
agatcccctg gaactttcag cacat                                          25

<210> 2879
<211> 25
<212> DNA
<213> Homo sapiens

<400> 2879
gatcccctgg aactttcagc acatg                                          25

<210> 2880
<211> 25
<212> DNA
<213> Homo sapiens

<400> 2880

atccccctgga actttcagca catga                                        25

<210> 2881
<211> 25
<212> DNA
<213> Homo sapiens

<400> 2881
tcccctggaa ctttcagcac atgat                                        25

<210> 2882
<211> 25
<212> DNA
<213> Homo sapiens

<400> 2882
cccctggaac tttcagcaca tgatg                                        25

<210> 2883
<211> 25
<212> DNA
<213> Homo sapiens

<400> 2883
ccctggaact ttcagcacat gatga                                        25

<210> 2884
<211> 25
<212> DNA
<213> Homo sapiens

<400> 2884
cctggaactt tcagcacatg atgat                                        25

<210> 2885
<211> 25
<212> DNA
<213> Homo sapiens

<400> 2885
ctggaacttt cagcacatga tgatg                                        25

<210> 2886
<211> 25
<212> DNA
<213> Homo sapiens

<400> 2886
tggaactttc agcacatgat gatgt                                        25

<210> 2887
<211> 25
<212> DNA
<213> Homo sapiens

<400> 2887
ggaactttca gcacatgatg atgtt                                        25

<210> 2888
<211> 25
<212> DNA

<213> Homo sapiens

<400> 2888
gaactttcag cacatgatga tgttt                                          25

<210> 2889
<211> 25
<212> DNA
<213> Homo sapiens

<400> 2889
aactttcagc acatgatgat gtttt                                          25

<210> 2890
<211> 25
<212> DNA
<213> Homo sapiens

<400> 2890
actttcagca catgatgatg ttttc                                          25

<210> 2891
<211> 25
<212> DNA
<213> Homo sapiens

<400> 2891
ctttcagcac atgatgatgt tttca                                          25

<210> 2892
<211> 25
<212> DNA
<213> Homo sapiens

<400> 2892
tttcagcaca tgatgatgtt ttcag                                          25

<210> 2893
<211> 25
<212> DNA
<213> Homo sapiens

<400> 2893
ttcagcacat gatgatgttt tcagg                                          25

<210> 2894
<211> 25
<212> DNA
<213> Homo sapiens

<400> 2894
tcagcacatg atgatgtttt caggt                                          25

<210> 2895
<211> 25
<212> DNA
<213> Homo sapiens

<400> 2895
cagcacatga tgatgttttc aggtt                                          25

<210> 2896
<211> 25
<212> DNA
<213> Homo sapiens

<400> 2896
agcacatgat gatgttttca ggttt                                          25

<210> 2897
<211> 25
<212> DNA
<213> Homo sapiens

<400> 2897
gcacatgatg atgttttcag gtttg                                          25

<210> 2898
<211> 25
<212> DNA
<213> Homo sapiens

<400> 2898
cacatgatga tgttttcagg tttgc                                          25

<210> 2899
<211> 25
<212> DNA
<213> Homo sapiens

<400> 2899
acatgatgat gttttcaggt ttgcg                                          25

<210> 2900
<211> 25
<212> DNA
<213> Homo sapiens

<400> 2900
catgatgatg ttttcaggtt tgcga                                          25

<210> 2901
<211> 25
<212> DNA
<213> Homo sapiens

<400> 2901
atgatgatgt tttcaggttt gcgag                                          25

<210> 2902
<211> 25
<212> DNA
<213> Homo sapiens

<400> 2902
tgatgatgtt ttcaggtttg cgagg                                          25

<210> 2903
<211> 25
<212> DNA
<213> Homo sapiens

<400> 2903
gatgatgttt tcaggtttgc gagga                                          25

<210> 2904
<211> 25
<212> DNA
<213> Homo sapiens

<400> 2904
atgatgtttt caggtttgcg aggag                                          25

<210> 2905
<211> 25
<212> DNA
<213> Homo sapiens

<400> 2905
tgatgttttc aggtttgcga ggagc                                          25

<210> 2906
<211> 25
<212> DNA
<213> Homo sapiens

<400> 2906
gatgttttca ggtttgcgag gagcg                                          25

<210> 2907
<211> 25
<212> DNA
<213> Homo sapiens

<400> 2907
atgttttcag gtttgcgagg agcga                                          25

<210> 2908
<211> 25
<212> DNA
<213> Homo sapiens

<400> 2908
tgttttcagg tttgcgagga gcgat                                          25

<210> 2909
<211> 25
<212> DNA
<213> Homo sapiens

<400> 2909
gttttcaggt ttgcgaggag cgatc                                          25

<210> 2910
<211> 25
<212> DNA
<213> Homo sapiens

<400> 2910
ttttcaggtt tgcgaggagc gatcg                                          25

<210> 2911
<211> 25

<212> DNA
<213> Homo sapiens

<400> 2911
tttcaggttt gcgaggagcg atcgc                                          25

<210> 2912
<211> 25
<212> DNA
<213> Homo sapiens

<400> 2912
ttcaggtttg cgaggagcga tcgca                                          25

<210> 2913
<211> 25
<212> DNA
<213> Homo sapiens

<400> 2913
tcaggtttgc gaggagcgat cgcat                                          25

<210> 2914
<211> 25
<212> DNA
<213> Homo sapiens

<400> 2914
caggtttgcg aggagcgatc gcatt                                          25

<210> 2915
<211> 25
<212> DNA
<213> Homo sapiens

<400> 2915
aggtttgcga ggagcgatcg cattt                                          25

<210> 2916
<211> 25
<212> DNA
<213> Homo sapiens

<400> 2916
ggtttgcgag gagcgatcgc atttg                                          25

<210> 2917
<211> 25
<212> DNA
<213> Homo sapiens

<400> 2917
gtttgcgagg agcgatcgca tttgc                                          25

<210> 2918
<211> 25
<212> DNA
<213> Homo sapiens

<400> 2918
tttgcgagga gcgatcgcat ttgcc                                          25

<210> 2919
<211> 25
<212> DNA
<213> Homo sapiens

<400> 2919
ttgcgaggag cgatcgcatt tgcct                                          25

<210> 2920
<211> 25
<212> DNA
<213> Homo sapiens

<400> 2920
tgcgaggagc gatcgcattt gcctt                                          25

<210> 2921
<211> 25
<212> DNA
<213> Homo sapiens

<400> 2921
gcgaggagcg atcgcatttg cctta                                          25

<210> 2922
<211> 25
<212> DNA
<213> Homo sapiens

<400> 2922
cgaggagcga tcgcatttgc cttag                                          25

<210> 2923
<211> 25
<212> DNA
<213> Homo sapiens

<400> 2923
gaggagcgat cgcatttgcc ttagc                                          25

<210> 2924
<211> 25
<212> DNA
<213> Homo sapiens

<400> 2924
aggagcgatc gcatttgcct tagct                                          25

<210> 2925
<211> 25
<212> DNA
<213> Homo sapiens

<400> 2925
ggagcgatcg catttgcctt agcta                                          25

<210> 2926
<211> 25
<212> DNA
<213> Homo sapiens

<400> 2926
gagcgatcgc atttgcctta gctat                                          25

<210> 2927
<211> 25
<212> DNA
<213> Homo sapiens

<400> 2927
agcgatcgca tttgccttag ctatt                                          25

<210> 2928
<211> 25
<212> DNA
<213> Homo sapiens

<400> 2928
gcgatcgcat ttgccttagc tattc                                          25

<210> 2929
<211> 25
<212> DNA
<213> Homo sapiens

<400> 2929
cgatcgcatt tgccttagct attcg                                          25

<210> 2930
<211> 25
<212> DNA
<213> Homo sapiens

<400> 2930
gatcgcattt gccttagcta ttcgg                                          25

<210> 2931
<211> 25
<212> DNA
<213> Homo sapiens

<400> 2931
atcgcatttg ccttagctat tcgga                                          25

<210> 2932
<211> 25
<212> DNA
<213> Homo sapiens

<400> 2932
tcgcatttgc cttagctatt cggaa                                          25

<210> 2933
<211> 25
<212> DNA
<213> Homo sapiens

<400> 2933
cgcatttgcc ttagctattc ggaac                                          25

<210> 2934

<211> 25
<212> DNA
<213> Homo sapiens

<400> 2934
gcatttgcct tagctattcg gaaca                                          25

<210> 2935
<211> 25
<212> DNA
<213> Homo sapiens

<400> 2935
catttgcctt agctattcgg aacac                                          25

<210> 2936
<211> 25
<212> DNA
<213> Homo sapiens

<400> 2936
atttgcctta gctattcgga acaca                                          25

<210> 2937
<211> 25
<212> DNA
<213> Homo sapiens

<400> 2937
tttgccttag ctattcggaa cacag                                          25

<210> 2938
<211> 25
<212> DNA
<213> Homo sapiens

<400> 2938
ttgccttagc tattcggaac acaga                                          25

<210> 2939
<211> 25
<212> DNA
<213> Homo sapiens

<400> 2939
tgccttagct attcggaaca cagaa                                          25

<210> 2940
<211> 25
<212> DNA
<213> Homo sapiens

<400> 2940
gccttagcta ttcggaacac agaat                                          25

<210> 2941
<211> 25
<212> DNA
<213> Homo sapiens

<400> 2941
   ccttagctat tcggaacaca gaatc                                          25

<210> 2942
<211> 25
<212> DNA
<213> Homo sapiens

<400> 2942
cttagctatt cggaacacag aatct                                          25

<210> 2943
<211> 25
<212> DNA
<213> Homo sapiens

<400> 2943
ttagctattc ggaacacaga atctc                                          25

<210> 2944
<211> 25
<212> DNA
<213> Homo sapiens

<400> 2944
tagctattcg gaacacagaa tctca                                          25

<210> 2945
<211> 25
<212> DNA
<213> Homo sapiens

<400> 2945
agctattcgg aacacagaat ctcag                                          25

<210> 2946
<211> 25
<212> DNA
<213> Homo sapiens

<400> 2946
gctattcgga acacagaatc tcagc                                          25

<210> 2947
<211> 25
<212> DNA
<213> Homo sapiens

<400> 2947
ctattcggaa cacagaatct cagcc                                          25

<210> 2948
<211> 25
<212> DNA
<213> Homo sapiens

<400> 2948
tattcggaac acagaatctc agccc                                          25

<210> 2949
<211> 25
<212> DNA

<213> Homo sapiens

<400> 2949
attcggaaca cagaatctca gccca                                          25

<210> 2950
<211> 25
<212> DNA
<213> Homo sapiens

<400> 2950
ttcggaacac agaatctcag cccaa                                          25

<210> 2951
<211> 25
<212> DNA
<213> Homo sapiens

<400> 2951
tcggaacaca gaatctcagc ccaaa                                          25

<210> 2952
<211> 25
<212> DNA
<213> Homo sapiens

<400> 2952
cggaacacag aatctcagcc caaac                                          25

<210> 2953
<211> 25
<212> DNA
<213> Homo sapiens

<400> 2953
ggaacacaga atctcagccc aaaca                                          25

<210> 2954
<211> 25
<212> DNA
<213> Homo sapiens

<400> 2954
gaacacagaa tctcagccca aacaa                                          25

<210> 2955
<211> 25
<212> DNA
<213> Homo sapiens

<400> 2955
aacacagaat ctcagcccaa acaaa                                          25

<210> 2956
<211> 25
<212> DNA
<213> Homo sapiens

<400> 2956
acacagaatc tcagcccaaa caaat                                          25

<210> 2957
<211> 25
<212> DNA
<213> Homo sapiens

<400> 2957
cacagaatct cagcccaaac aaatg                                            25

<210> 2958
<211> 25
<212> DNA
<213> Homo sapiens

<400> 2958
acagaatctc agcccaaaca aatga                                            25

<210> 2959
<211> 25
<212> DNA
<213> Homo sapiens

<400> 2959
cagaatctca gcccaaacaa atgat                                            25

<210> 2960
<211> 25
<212> DNA
<213> Homo sapiens

<400> 2960
agaatctcag cccaaacaaa tgatg                                            25

<210> 2961
<211> 25
<212> DNA
<213> Homo sapiens

<400> 2961
gaatctcagc ccaaacaaat gatgt                                            25

<210> 2962
<211> 25
<212> DNA
<213> Homo sapiens

<400> 2962
aatctcagcc caaacaaatg atgtt                                            25

<210> 2963
<211> 25
<212> DNA
<213> Homo sapiens

<400> 2963
atctcagccc aaacaaatga tgttt                                            25

<210> 2964
<211> 25
<212> DNA
<213> Homo sapiens

<400> 2964
tctcagccca aacaaatgat gttta                                          25

<210> 2965
<211> 25
<212> DNA
<213> Homo sapiens

<400> 2965
ctcagcccaa acaaatgatg tttac                                          25

<210> 2966
<211> 25
<212> DNA
<213> Homo sapiens

<400> 2966
tcagcccaaa caaatgatgt ttacc                                          25

<210> 2967
<211> 25
<212> DNA
<213> Homo sapiens

<400> 2967
cagcccaaac aaatgatgtt tacca                                          25

<210> 2968
<211> 25
<212> DNA
<213> Homo sapiens

<400> 2968
agcccaaaca aatgatgttt accac                                          25

<210> 2969
<211> 25
<212> DNA
<213> Homo sapiens

<400> 2969
gcccaaacaa atgatgttta ccact                                          25

<210> 2970
<211> 25
<212> DNA
<213> Homo sapiens

<400> 2970
cccaaacaaa tgatgtttac cacta                                          25

<210> 2971
<211> 25
<212> DNA
<213> Homo sapiens

<400> 2971
ccaaacaaat gatgtttacc actac                                          25

<210> 2972
<211> 25

<212> DNA
<213> Homo sapiens

<400> 2972
caaacaaatg atgtttacca ctacg                                          25

<210> 2973
<211> 25
<212> DNA
<213> Homo sapiens

<400> 2973
aaacaaatga tgtttaccac tacgc                                          25

<210> 2974
<211> 25
<212> DNA
<213> Homo sapiens

<400> 2974
aacaaatgat gtttaccact acgct                                          25

<210> 2975
<211> 25
<212> DNA
<213> Homo sapiens

<400> 2975
acaaatgatg tttaccacta cgctg                                          25

<210> 2976
<211> 25
<212> DNA
<213> Homo sapiens

<400> 2976
caaatgatgt ttaccactac gctgc                                          25

<210> 2977
<211> 25
<212> DNA
<213> Homo sapiens

<400> 2977
aaatgatgtt taccactacg ctgct                                          25

<210> 2978
<211> 25
<212> DNA
<213> Homo sapiens

<400> 2978
aatgatgttt accactacgc tgctc                                          25

<210> 2979
<211> 25
<212> DNA
<213> Homo sapiens

<400> 2979
atgatgttta ccactacgct gctcc                                          25

<210> 2980
<211> 25
<212> DNA
<213> Homo sapiens

<400> 2980
tgatgtttac cactacgctg ctcct                                          25

<210> 2981
<211> 25
<212> DNA
<213> Homo sapiens

<400> 2981
gatgtttacc actacgctgc tcctc                                          25

<210> 2982
<211> 25
<212> DNA
<213> Homo sapiens

<400> 2982
atgtttacca ctacgctgct cctcg                                          25

<210> 2983
<211> 25
<212> DNA
<213> Homo sapiens

<400> 2983
tgtttaccac tacgctgctc ctcgt                                          25

<210> 2984
<211> 25
<212> DNA
<213> Homo sapiens

<400> 2984
gtttaccact acgctgctcc tcgtg                                          25

<210> 2985
<211> 25
<212> DNA
<213> Homo sapiens

<400> 2985
tttaccacta cgctgctcct cgtgt                                          25

<210> 2986
<211> 25
<212> DNA
<213> Homo sapiens

<400> 2986
ttaccactac gctgctcctc gtgtt                                          25

<210> 2987
<211> 25
<212> DNA
<213> Homo sapiens

<400> 2987
taccactacg ctgctcctcg tgttc                                          25

<210> 2988
<211> 25
<212> DNA
<213> Homo sapiens

<400> 2988
accactacgc tgctcctcgt gttct                                          25

<210> 2989
<211> 25
<212> DNA
<213> Homo sapiens

<400> 2989
ccactacgct gctcctcgtg ttctt                                          25

<210> 2990
<211> 25
<212> DNA
<213> Homo sapiens

<400> 2990
cactacgctg ctcctcgtgt tcttc                                          25

<210> 2991
<211> 25
<212> DNA
<213> Homo sapiens

<400> 2991
actacgctgc tcctcgtgtt cttca                                          25

<210> 2992
<211> 25
<212> DNA
<213> Homo sapiens

<400> 2992
ctacgctgct cctcgtgttc ttcac                                          25

<210> 2993
<211> 25
<212> DNA
<213> Homo sapiens

<400> 2993
tacgctgctc ctcgtgttct tcact                                          25

<210> 2994
<211> 25
<212> DNA
<213> Homo sapiens

<400> 2994
acgctgctcc tcgtgttctt cactg                                          25

<210> 2995

<211> 25
<212> DNA
<213> Homo sapiens

<400> 2995
cgctgctcct cgtgttcttc actgt                                          25

<210> 2996
<211> 25
<212> DNA
<213> Homo sapiens

<400> 2996
gctgctcctc gtgttcttca ctgtc                                          25

<210> 2997
<211> 25
<212> DNA
<213> Homo sapiens

<400> 2997
ctgctcctcg tgttcttcac tgtct                                          25

<210> 2998
<211> 25
<212> DNA
<213> Homo sapiens

<400> 2998
tgctcctcgt gttcttcact gtctg                                          25

<210> 2999
<211> 25
<212> DNA
<213> Homo sapiens

<400> 2999
gctcctcgtg ttcttcactg tctgg                                          25

<210> 3000
<211> 25
<212> DNA
<213> Homo sapiens

<400> 3000
ctcctcgtgt tcttcactgt ctggg                                          25

<210> 3001
<211> 25
<212> DNA
<213> Homo sapiens

<400> 3001
tcctcgtgtt cttcactgtc tgggt                                          25

<210> 3002
<211> 25
<212> DNA
<213> Homo sapiens

<400> 3002

cctcgtgttc ttcactgtct gggta                                          25

<210> 3003
<211> 25
<212> DNA
<213> Homo sapiens

<400> 3003
ctcgtgttct tcactgtctg ggtat                                          25

<210> 3004
<211> 25
<212> DNA
<213> Homo sapiens

<400> 3004
tcgtgttctt cactgtctgg gtatt                                          25

<210> 3005
<211> 25
<212> DNA
<213> Homo sapiens

<400> 3005
cgtgttcttc actgtctggg tattt                                          25

<210> 3006
<211> 25
<212> DNA
<213> Homo sapiens

<400> 3006
gtgttcttca ctgtctgggt atttg                                          25

<210> 3007
<211> 25
<212> DNA
<213> Homo sapiens

<400> 3007
tgttcttcac tgtctgggta tttgg                                          25

<210> 3008
<211> 25
<212> DNA
<213> Homo sapiens

<400> 3008
gttcttcact gtctgggtat ttgga                                          25

<210> 3009
<211> 25
<212> DNA
<213> Homo sapiens

<400> 3009
ttcttcactg tctgggtatt tggag                                          25

<210> 3010
<211> 25
<212> DNA

<213> Homo sapiens

<400> 3010
tcttcactgt ctgggtattt ggagg                                          25

<210> 3011
<211> 25
<212> DNA
<213> Homo sapiens

<400> 3011
cttcactgtc tgggtatttg gagga                                          25

<210> 3012
<211> 25
<212> DNA
<213> Homo sapiens

<400> 3012
ttcactgtct gggtatttgg aggag                                          25

<210> 3013
<211> 25
<212> DNA
<213> Homo sapiens

<400> 3013
tcactgtctg ggtatttgga ggagg                                          25

<210> 3014
<211> 25
<212> DNA
<213> Homo sapiens

<400> 3014
cactgtctgg gtatttggag gagga                                          25

<210> 3015
<211> 25
<212> DNA
<213> Homo sapiens

<400> 3015
actgtctggg tatttggagg aggaa                                          25

<210> 3016
<211> 25
<212> DNA
<213> Homo sapiens

<400> 3016
ctgtctgggt atttggagga ggaac                                          25

<210> 3017
<211> 25
<212> DNA
<213> Homo sapiens

<400> 3017
tgtctgggta tttggaggag gaaca                                          25

<210> 3018
<211> 25
<212> DNA
<213> Homo sapiens

<400> 3018
gtctgggtat ttggaggagg aacaa                                            25

<210> 3019
<211> 25
<212> DNA
<213> Homo sapiens

<400> 3019
tctgggtatt tggaggagga acaac                                            25

<210> 3020
<211> 25
<212> DNA
<213> Homo sapiens

<400> 3020
ctgggtattt ggaggaggaa caacc                                            25

<210> 3021
<211> 25
<212> DNA
<213> Homo sapiens

<400> 3021
tgggtatttg gaggaggaac aaccc                                            25

<210> 3022
<211> 25
<212> DNA
<213> Homo sapiens

<400> 3022
gggtatttgg aggaggaaca acccc                                            25

<210> 3023
<211> 25
<212> DNA
<213> Homo sapiens

<400> 3023
ggtatttgga ggaggaacaa ccccc                                            25

<210> 3024
<211> 25
<212> DNA
<213> Homo sapiens

<400> 3024
gtatttggag gaggaacaac cccca                                            25

<210> 3025
<211> 25
<212> DNA
<213> Homo sapiens

<400> 3025
tatttggagg aggaacaacc cccat                                          25

<210> 3026
<211> 25
<212> DNA
<213> Homo sapiens

<400> 3026
atttggagga ggaacaaccc ccatg                                          25

<210> 3027
<211> 25
<212> DNA
<213> Homo sapiens

<400> 3027
tttggaggag gaacaacccc catgt                                          25

<210> 3028
<211> 25
<212> DNA
<213> Homo sapiens

<400> 3028
ttggaggagg aacaaccccc atgtt                                          25

<210> 3029
<211> 25
<212> DNA
<213> Homo sapiens

<400> 3029
tggaggagga acaaccccca tgttg                                          25

<210> 3030
<211> 25
<212> DNA
<213> Homo sapiens

<400> 3030
ggaggaggaa caacccccat gttga                                          25

<210> 3031
<211> 25
<212> DNA
<213> Homo sapiens

<400> 3031
gaggaggaac aacccccatg ttgac                                          25

<210> 3032
<211> 25
<212> DNA
<213> Homo sapiens

<400> 3032
aggaggaaca acccccatgt tgact                                          25

<210> 3033
<211> 25

<212> DNA
<213> Homo sapiens

<400> 3033
ggaggaacaa cccccatgtt gactt                                            25

<210> 3034
<211> 25
<212> DNA
<213> Homo sapiens

<400> 3034
gaggaacaac ccccatgttg acttg                                            25

<210> 3035
<211> 25
<212> DNA
<213> Homo sapiens

<400> 3035
aggaacaacc cccatgttga cttgg                                            25

<210> 3036
<211> 25
<212> DNA
<213> Homo sapiens

<400> 3036
ggaacaaccc ccatgttgac ttggc                                            25

<210> 3037
<211> 25
<212> DNA
<213> Homo sapiens

<400> 3037
gaacaacccc catgttgact tggct                                            25

<210> 3038
<211> 25
<212> DNA
<213> Homo sapiens

<400> 3038
aacaaccccc atgttgactt ggctt                                            25

<210> 3039
<211> 25
<212> DNA
<213> Homo sapiens

<400> 3039
acaaccccca tgttgacttg gcttc                                            25

<210> 3040
<211> 25
<212> DNA
<213> Homo sapiens

<400> 3040
caacccccat gttgacttgg cttca                                            25

<210> 3041
<211> 25
<212> DNA
<213> Homo sapiens

<400> 3041
aacccccatg ttgacttggc ttcag                                          25

<210> 3042
<211> 25
<212> DNA
<213> Homo sapiens

<400> 3042
acccccatgt tgacttggct tcaga                                          25

<210> 3043
<211> 25
<212> DNA
<213> Homo sapiens

<400> 3043
cccccatgtt gacttggctt cagat                                          25

<210> 3044
<211> 25
<212> DNA
<213> Homo sapiens

<400> 3044
ccccatgttg acttggcttc agatc                                          25

<210> 3045
<211> 25
<212> DNA
<213> Homo sapiens

<400> 3045
cccatgttga cttggcttca gatca                                          25

<210> 3046
<211> 25
<212> DNA
<213> Homo sapiens

<400> 3046
ccatgttgac ttggcttcag atcag                                          25

<210> 3047
<211> 25
<212> DNA
<213> Homo sapiens

<400> 3047
catgttgact tggcttcaga tcaga                                          25

<210> 3048
<211> 25
<212> DNA
<213> Homo sapiens

<400> 3048
atgttgactt ggcttcagat cagag                                          25

<210> 3049
<211> 25
<212> DNA
<213> Homo sapiens

<400> 3049
tgttgacttg gcttcagatc agagt                                          25

<210> 3050
<211> 25
<212> DNA
<213> Homo sapiens

<400> 3050
gttgacttgg cttcagatca gagtt                                          25

<210> 3051
<211> 25
<212> DNA
<213> Homo sapiens

<400> 3051
ttgacttggc ttcagatcag agttg                                          25

<210> 3052
<211> 25
<212> DNA
<213> Homo sapiens

<400> 3052
tgacttggct tcagatcaga gttgg                                          25

<210> 3053
<211> 25
<212> DNA
<213> Homo sapiens

<400> 3053
gacttggctt cagatcagag ttggc                                          25

<210> 3054
<211> 25
<212> DNA
<213> Homo sapiens

<400> 3054
acttggcttc agatcagagt tggcg                                          25

<210> 3055
<211> 25
<212> DNA
<213> Homo sapiens

<400> 3055
cttggcttca gatcagagtt ggcgt                                          25

<210> 3056

<211> 25
<212> DNA
<213> Homo sapiens

<400> 3056
ttggcttcag atcagagttg gcgtg                                          25